# FASCICULE DE BREVET EUROPEEN

(11) **EP 0 879 223 B1**
(45) Date de publication et mention de la délivrance du brevet: **07.11.2001**
(21) Numéro de dépôt: 97900659.0
(22) Date de dépôt: 16.01.1997
(51) Int. Cl.: C07C 63/66, A61K 31/19, C07C 63/74, C07C 229/00, C07D 333/00, C07D 257/04, C07F 9/02

(54) **DERIVES POLYCYCLIQUES AROMATIQUES DE TYPE RETINOIDE, LEUR PROCEDE DE PREPARATION ET LEUR UTILISATION POUR LA FABRICATION DE COMPOSITIONS PHARMACEUTIQUES ET COSMETIQUES**
AROMATISCHE POLYCYCLISCHE DERIVATE DES RETINOIDTYPS, VERFAHREN ZU IHRER HERSTELLUNG UND IHRE VERWENDUNG ZUR HERSTELLUNG DER PHARMAZEUTISCHEN UND KOSMETISCHEN ZUSAMMENSETZUNGEN
AROMATIC POLYCYCLIC RETINOID-TYPE DERIVATIVES, METHOD FOR PREPARING SAME, AND USE THEREOF FOR MAKING PHARMACEUTICAL AND COSMETIC COMPOSITIONS

(30) Priorité: 17.01.1996 FR 9600497
(43) Date de publication de la demande: 25.11.1998
(73) Titulaire: Centre Europeen de Bioprospective -CEB, 76131 Mont-Saint-Aignan Cédex (FR)
(72) Inventeur: LEBLOND, Bertrand, F-76000 Rouen (FR); DARRO, Francis, B-1070 Bruxelles (BE); DEYINE, Abdallah, F-76000 Rouen (FR); SALES-SALLANS, Véronique, F-76240 Le-Mesnil-Esnard (FR); DUHAMEL, Pierre, F-76130 Mont-Saint-Aignan (FR); KISS, Robert, B-1440 Wauthier-Braine (BE); SCHOOFS, Alain-René, F-92400 Courbevoie (FR); GERMAIN, Pierre, F-87500 Saint-Yrieix-la-Perche (FR); POURRIAS, Bernard, F-91570 Bievres (FR)
(74) Mandataire: Breese, Pierre
(86) Numéro de dépôt international: FR9700079
(87) Numéro de publication internationale: WO9726237

(56) Documents cités:
- EP-A- 0 002 742
- DE-A- 3 715 955

## Description

La présente invention concerne des dérivés polycycliques aromatiques de type rétinoïdes de formule générale : 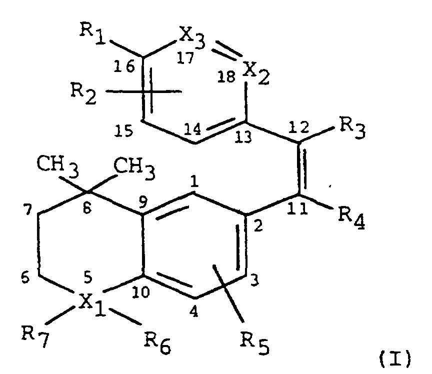 dans laquelle les groupements R₃ et R₄ portés par la double liaison entre les carbones 11 et 12 sont en cis et R₁, R₂, R₃, R₄, R₅, R₆, R₇, X₁, X₂ et X₃ ont les significations suivantes :
- R₁ représente un groupe tétrazoyle ou un groupe - COOH ou
- R₂ représente un atome d'hydrogène, un atome d'halogène et plus particulièrement un atome de fluor, un radical alkyle en C₁ à C₆ un groupe de formule -COOH, -OR₁₁, -SR₁₁, -(CF₂)ₙCF₃ où n est un nombre entier compris entre 0 et 10, ou un groupe -OCOR₁₁, et quand cela est possible leurs sels avec des. acides tolérés physiologiquement, ou un groupe aminé de formule : 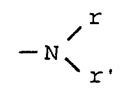 dans laquelle r et r' identiques ou différents, représeptent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle. et R₁₁ représente un atome d'hydrogène, un radical alkyle en C₁ à C₆, un radical fluoroalkyle ayant de 1 à 6 atomes de carbone et de 3 à 7 atomes de fluor, un radical aryle ou un radical aralkyle.
- R₃ représente un atome d'hydrogène, un radical trifluorométhyle, un radical aryle, un radical aralkyle ou un radical alkyle en C₁ à C₆ éventuellement substitué par un hydroxyle ou par un ou plusieurs atomes de fluor, par un alcoxy en C₁ à C₆ ou par un groupe de formule - (C=O)R₁₂, dans laquelle R₁₂ représente un atome d'hydrogène, un radical alkyle inférieur, un radical hydroxyle, un radical alcoxy en C₁ à C₆ ou un groupe aminé de formule : 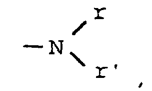 dans laquelle r et r' ont la même signification que précédemment.
- R₄ représente un atome d'hydrogène ou un radical aryle.
- R₅ représente un atome d'hydrogène, un radical alkyle en C₁ à C₆, un atome d'halogène, un radical fluoroalkyle ayant de 1 à 6 atomes de carbone et de 3 à 7 atomes de fluor, ou un groupe de formule -OR₁₃ où R₁₃ représente un atome d'hydrogène, un radical alkyle en C₁ à C₆, un radical aryle ou un radical aralkyle ou un groupement trifluorométhyle.
- X₁ représente un atome de carbone, ou un atome de soufre, et alors,
- R₆ et R₇ sont :
   - des radicaux méthyles, dans le cas où X₁ est un atome de carbone,
   - identiques ou différents et représentent soit rien (cas d'un sulfure -S-), soit un oxygène (cas d'un sulfoxyde -SO- ou d'une sulfone -SO₂-) dans le cas où X₁ est un atome de soufre,
- X₂ et X₃, identiques ou différents, représentent un atome de carbone, un atome d'oxygène ou un atome d'azote, ou encore X₂-X₃ sont un seul atome de soufre, d'oxygène, ou d'azote, ainsi le noyau porteur de X₂ et X₃ peut être benzénique, pyridinique, thiophénique, furanique, pyrrolique, ou, dans le cas où X₂ est un atome d'oxygène et X₃ un atome de carbone, C₁₃ et C₁₄ représente un seul et même atome de carbone, ainsi le noyau porteur de X₂ et X₃ peut être isoxazolique.

A titre de sels pharmaceutiquement acceptables des dérivés précédents, on peut citer de manière non limitative : les sels d'acides acétique, chlorhydrique, cinnamique, citrique, formique, hydrobromique, hydroiodique, hydrofluorique, malonique, méthanesulfonique, oxalique, picrique, maléique, lactique, nicotinique, phénylacétique, phosphorique, succinique, sulfurique, tartrique, les sels d'ammonium, de pipérazine, de diéthylamine, de nicotinamide, d'urée, de sodium, de potassium, de calcium, de magnésium, de zinc, de lithium, de méthylamino, de diméthylamino, de triméthylamino, de tris(hydroxyméthyl)aminométhane.

Le terme alkyle ou alcoxy inférieur désigne des groupes de 1 à 6 atomes de carbone linéaires ou ramifiés tels que les radicaux méthyle, éthyle, propyle, isopropyle, butyle, isobutyle, butyle secondaire, méthoxy, éthoxy, propoxy, isopropoxy, butyloxy, isobutyloxy, butyloxy secondaire.

L'acide tout-trans rétinoïque, métabolite de la vitamine A présente un grand nombre de propriétés biologiques. Plusieurs molécules construites à partir de modifications chimiques de cet acide ont été synthétisées et se sont révélées biologiquement actives. Ces analogues synthétiques et leurs dérivés sont appelés rétinoïdes, selon la définition de Sporn, M. B. et Roberts, A. B., Ciba. Found. Symp., 113, 1-5, 1985. Parmi ces composés, on peut citer ceux décrits dans les demandes de brevet européen publiées sous les numéros 350 846, 303 186, 253 302, dans la demande de brevet internationale PCT publiée sous le numéro WO 93/11755, ou encore dans les brevets américains 5 300 522, 5 420 273, 4 578 498 et les brevets allemands 3602473 et 3715955, ainsi que dans les articles de Marcia I. Dawson et al. (J. Med. Chem., 1989, 32, 1504-1517 ; J. Med. Chem. , 1993, 36, 2605-2613).

Les composés qui présentent une activité de type rétinoïde sont utiles pour le traitement des mammifères et plus particulièrement de l'homme par chimioprévention et par chimiothérapie, notamment dans le traitement des symptômes de nombreuses maladies telles que les dermatoses, l'acné, la maladie de Darier, le psoriasis, l'icthyose, l'eczéma. Ces composés sont aussi utilisés pour le traitement et la prévention de maladies cancéreuses et de nombreuses maladies hyperproliferatives malignes telles que les cancers du sein, de la prostate, du poumon, de la tête et du cou, ainsi que de certains types de cancers d'origine épithéliale et des leucémies myélocytaires. Les composés présentant une activité de type rétinoïde sont aussi utiles pour le traitement et la prévention de l'athérosclérose, de la resténose issue d'hyperprolifération néointimale, des pathologies hyperprolifératives bénignes telles que l'hyperplasie endométriale, l'hypertrophie bégnine de la prostate, la rétinopathie proliférative, pour le traitement des maladies auto-immunes et des désordres immunologiques tels que le lupus érythémateux, pour le traitement et la prévention de maladies associées au métabolisme des lipides et pour le traitement des effets du soleil sur la peau.

Toutefois, ces composés de type rétinoïde présentent des effets secondaires importants, notamment une forte irritation au niveau de la peau et des muqueuses, une toxicité lipidique, et sont même tératogènes, ce qui rend leur usage délicat en clinique (Kistler, A. et al. Arch Toxicol, 64 : 616-622 ; "Retinoids in Oncology", Edited by Waun Ki Hong & Reuben Lotan, The University of Texas M. D. Anderson Cancer Center, Houston, Texas, USA, Marcel Dekker Inc., pages 127-146 ; "Retinoids in Clinical Practice", Edited by Gideon Koren The Motherisk Program, The Hospital for Sick Children and The University of Toronto, Toronto, Ontario, Canada, Marcel Dekker Inc.).

Les effets néfastes rapportés ci-dessus ont conduit la Demanderesse à rechercher des dérivés polycycliques aromatiques de type rétinoïde actifs dans le traitement et la prévention des maladies précédentes mais ne présentant pas d'effets secondaires.

De façon surprenante, la Demanderesse a mis en évidence que les composés de formule I dans laquelle les groupements R₃ et R₄ de la double liaison entre les carbones 11 et 12 sont de configuration cis présentent une activité supérieure à celle des isomères trans correspondants ou du mélange d'isoméres cis/trans, et pourraient ne pas présenter les effets secondaires notamment tératogènes généralement associés à l'usage des composés de type rétinoïde. L'art antérieur et notamment les brevets et demandes de brevet sus-mentionnés indiquent logiquement l'existence d'isomères cis et trans du fait de la double liaison entre les carbones 11 et 12, mais s'incéressent spécifiquement aux composés de configuration trans car cette configuration est celle rencontrée dans l'arotinoïde de référence : l'acide (E) 4-[2-(5,6,7,8-tétrahydro-5,5,8,8-tétraméthyl-naphtalényl)-1-propényl] benzoïque (TTNPB).

Toujours conformément à la structure du TTNPB, ces composés de l'art antérieur sont caractérisés par la présence d'un substituant, plus particulièrement d'un méthyle, sur le carbone 11 (R₄ = -CH₃ dans la formule I), alors que le carbone 13 n'est pas substitué (R₃ = H dans la formule I), alors que les composés de l'invention possèdent, au contraire, un substituant, notamment un alkyle, au niveau du carbone 12 et pas au niveau du carbone 11.

Les travaux de recherche réalisés par la Demanderesse sur les dérivés de formule générale (I) dont les groupements R3 et R₄ sont en cis, l'ont conduit à démontrer que ces composés présentent une activité intrinsèque leur permettant de moduler la prolifération et la différenciation cellulaire, et autorisant leur application dans le traitement et la chimioprévention de maladies telles que le cancer du sein, le cancer de la prostate, le cancer des poumons, les cancers cutanés et les leucémies promyélocytaires dans des compositions non tératogéniques.

La présente invention a donc pour objet les dérivés rétinoïdes polycycliques aromatiques de formule générale I définie précédemment, leur procédé de préparation ainsi que leur utilisation en médecine humaine et vétérinaire et en cosmétique.

Parmi les dérivés de formule (I), une série préférée est celle dans laquelle R₃ représente un alkyle en C₁ à C₆ ou un radical trifluorométhyle ou un groupe -(CH₂)nCF₃ où n est un nombre entier compris entre 0 et 10, et R4 représente un atome d'hydrogène.

Une autre série préférée de dérivés de formule (I) selon l'invention sont ceux dans lesquels R₂ représente un atome d'hydrogène et R₁ représente un groupe tétrazoyle ou un groupe COOH. Parmi ces derniers, la série de dérivés pour lesquels R₁ et R₂ sont différents d'un groupe -COOH présentent des propriétés plus particulièrement intéressantes; de tels dérivés sont par exemple les composés désignés CB92834, CB77402, CB61692, CB63237 et CB39122 ci-dessous. Ces dérivés se distinguent donc nettement des composés de type rétinoïde issus du TTNPB de l'art antérieur qui portent sur le carbone 16 un groupe -COOH.

L'invention envisage à titre spécifique les dérivés de formule (I) suivants :

L'acide (Z) 2-[1-(5,6,7,8-tétrahydro-5,5,8,8 -tétraméthyl-2-naphthalényl)-2-propényl]pyridinyl-5-carboxylique, désigné CB38416 et répondant à la formule suivante :

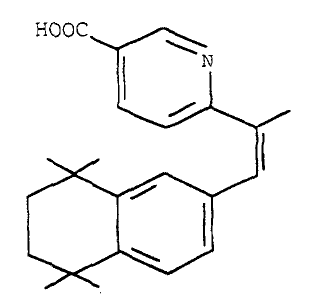

L'acide (E) 4-[1-trifluorométhyl-2-(5,6,7,8-tétrahydro-5,5,8,8-tétraméthyl-2-naphtalényl)-1-éthényl] benzoïque, désigné CB36493 et répondant à la formule suivante :

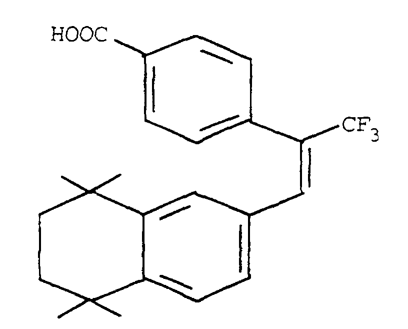

L'acide (Z) 4-[1-(5,6,7,8-tétrahydro-3,5,5,8,8-pentaméthyl-2-naphtalényl)-2-propényl]benzoïque, désigné CB32706 et répondant à la formule suivante :

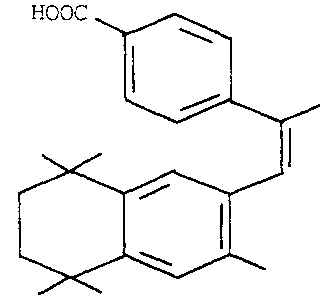

L'acide (E) 4-[1-trifluorométhyl-2-(5,6,7,8-tétrahydro-3,5,5,8,8-pentaméthyl-2-naphtalényl)-1-éthényl] benzoïque, désigné CB62899 et répondant à la formule suivante :

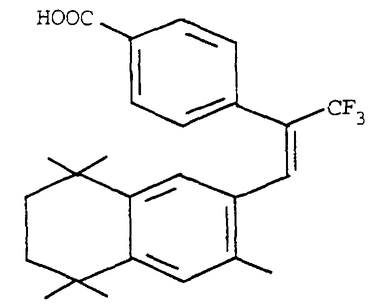

L'acide (Z) 4-[1-(3',4'-dihydro-4',4' diméthyl-1',1'-dioxyde-2'H-1'-benzothiopyran-6'-yl)-2-propényl]benzoïque, désigné CB72484 et répondant à la formule suivante :

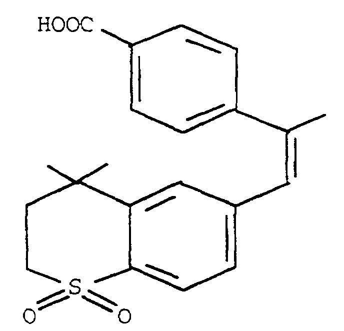

Le (Z) 5-[4-[1-(5,6,7,8-tétrahydro-5,5,8,8-tétraméthyl-2-naphtalényl)-2-propényl]phényl]-lH-tétrazole, désigné CB92834 et répondant à la formule suivante :

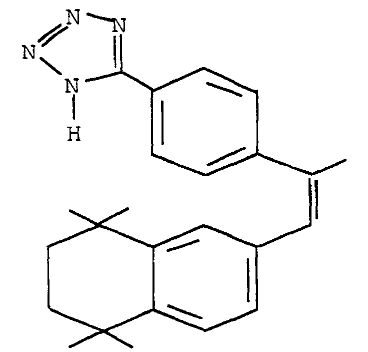

L'acide (E) 5-[4-[1-trifluorométhyl-2-(5,6,7,8-tétrahydro-5,5,8,8-tétraméthyl-2-naphtalényl)-1-éthényl]phényl]-1H-tétrazole, désigné CB77402 et répondant à la formule suivante :

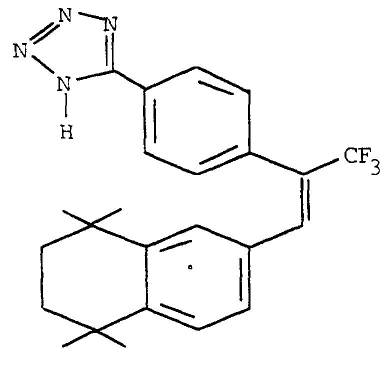

Le (Z) 5-[4-[1-(5,6,7,8-tétrahydro-3,5,5,8,8-pentaméthyl-2-naphtalényl)-2-propényl]phényl]-1H-tétrazole, désigné CB61692 et répondant à la formule suivante :

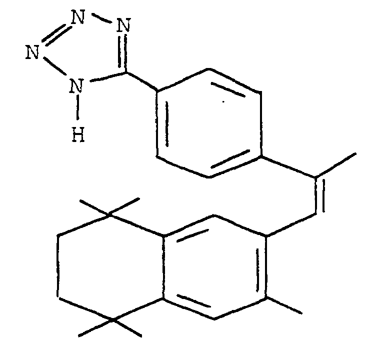

L'acide (E) 5-[4-[1-trifluorométhyl-2-(5,6,7,8-tétrahydro-3,5,5,8,8-pentaméthyl-2-naphtalényl)-1-éthényl]phényl]-1*H*-tétrazole, désigné CB63237 et répondant à la formule suivante :

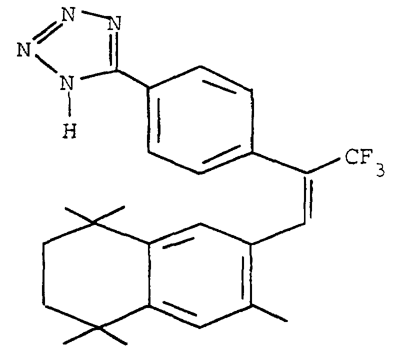

Le (Z) 5-[4-[1-(3',4'-dihydro-4',4'-diméthyl-1',1'-dioxyde-2'*H*-1'-benzothiopyran-6'-yl)-2-propényl] phényl]-1*H*-tétrazole, désigné CB39122 et répondant à la formule suivante :

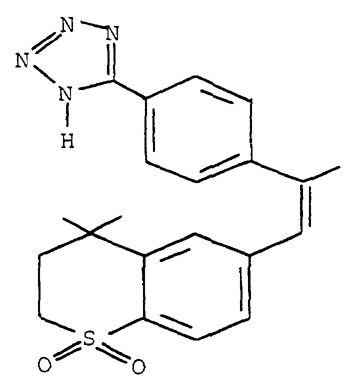

L'acide (Z) 2-[1-(5,6,7,8-tétrahydro-5,5,8,8-tétraméthyl-2-naphtalényl)-2-propényl]thiényl-5-carboxylique, désigné CB30382 et répondant à la formule suivante :

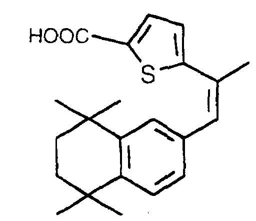

Le (Z) 5-[2-[1-(5,6,7,8-tétrahydro-5,5,8,8-tétraméthyl-2-naphtalényl)-2-propényl)]5-thiényl]-1*H* tétrazole, désigné CB92855 et répondant à la formule suivante :

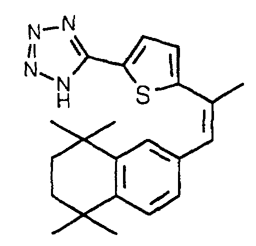

L'acide (Z) 5-[1-(5,6,7,8-tétrahydro-5,5,8,8-tétraméthyl-2-naphtalényl)-2-propényl]pyridinyl-2-carboxylique, désigné CB16279 et répondant à la formule suivante :

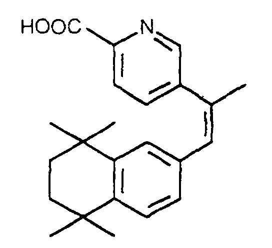

L'acide (Z) 2-[1-(5,6,7,8-tétrahydro-3,5,5,8,8-pentaméthyl-2-naphtalényl)-2-propényl]pyridinyl-5-carboxylique, désigné CB90525 et répondant à la formule suivante :

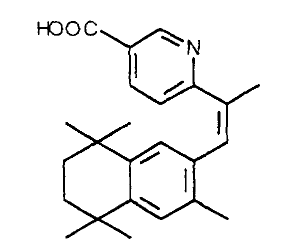

L'acide (Z) 5-[1-(5,6,7,8-tétrahydro-3,5,5,8,8-pentaméthyl-2-naphtalényl)-2-propényl]pyridinyl-2-carboxylique, désigné CB56004 et répondant à la formule suivante :

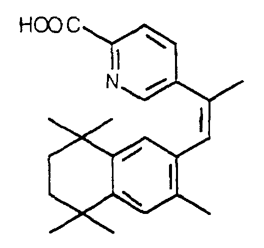

L'acide (Z) 5-[1-(5,6,7,8-tétrahydro-5,5,8,8-tétraméthyl-2-naphtalényl)-2-propényl)]isoxazole-3-carboxylique, désigné CB73069 et répondant à la formule suivante :

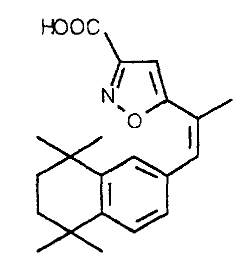

L'acide (Z) 5-[1-(5,6,7,8-tétrahydro-3,5,5,8,8-pentaméthyl-2-naphtalényl)-2-propényl)]isoxazole-3-carboxylique, désigné CB54647 et répondant à la formule suivante :

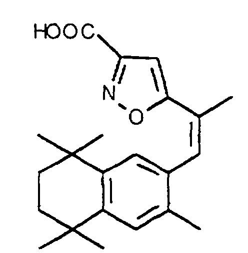

Le (Z) 1-[4-[1-(5,6,7,8-tétrahydro-5,5,8,8-tétraméthyl-2-naphtalényl)-2-propényl]phényl]-5-trifluorométhyl-1*H*-tétrazole, désigné CB29830 et répondant à la formule suivante :

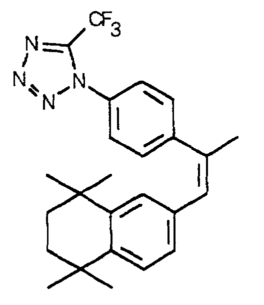

L'invention concerne aussi la préparation des dérivés de formule (I) et l'utilisation de ces derniers pour le traitement et la prévention des cancers du type tumeurs solides, tels que les cancers du sein, des poumons, de la prostate ou du foie, ainsi que pour le traitement et la prévention de maladies de la peau, tels que le psoriasis et l'acné. L'invention concerne également les compositions pharmaceutiques ou cosmétiques contenant comme principe actif au moins un dérivé de formule (I).

Les dérivés de formule (I) et leurs isomères de configuration trans peuvent être obtenus selon la méthode connue de Wittig en condensant un composé de formule : 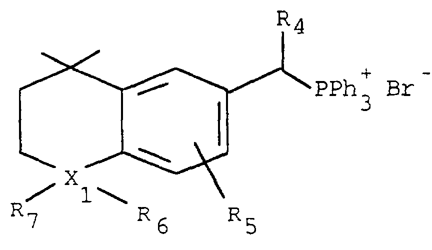 avec un composé de formule : 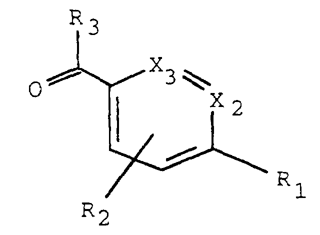 dans lesquelles R₁, R₂, R₃, R₄, R₅, R₆, R₇, X₁, X₂ et X₃ ont la même signification que dans la formule (I).

Les dérivés de formule (I) peuvent être préparés par tout autre méthode connue de l'homme du métier, telle qu'une déshydratation d'un alcool répondant à l'une ou l'autre des formules suivantes : 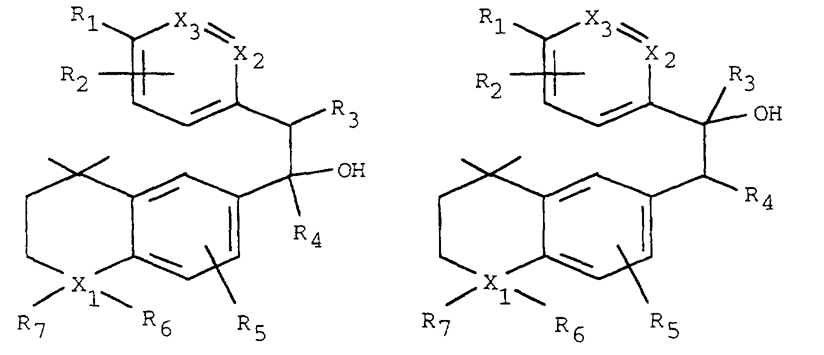 dans lesquelles R₁, R₂, R₃, R₄, R₅, R₆, R₇, X₁, X₂ et X₃ ont la même signification que dans la formule (I).

Il peut aussi s'agir d'une méthode du type Horner-Emmons consistant à condenser un composé carbonylé (aldéhyde ou cétone) de formule : 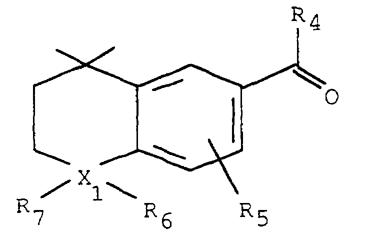 avec un phosphonate de formule : 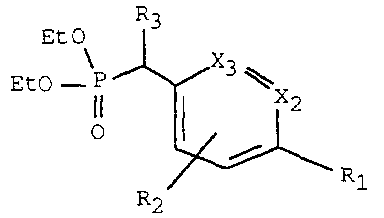 dans lesquelles R₁ , R₂, R₃, R₄, R₅, R₆, R₇, X₁, X₂ et X₃ ont la même signification que dans la formule (I).

Les formes isomériques cis et trans des dérivés obtenus par ces différentes méthodes peuvent être séparées et purifiées soit au cours du processus de synthèse par changement de solvant ou addition de sel (March, J., Modern Organic Synthesis, 3rd Edition, Wiley Interscience, p. 845-854), soit au stade final, selon des techniques connues, telles que, par exemple, la recristallisation, l'HPLC préparative ou la chromatographie.

En outre, il est possible à partir des dérivés de formule (I) et de leurs isomères trans obtenus selon les méthodes précédentes, de préparer d'autres dérivés par des réactions classiques au niveau d'un ou plusieurs des radicaux R₁, R₂, R₃, R₄, R₅, R₆, R₇, X₁, X₂ et X₃. On peut citer par exemple les réactions suivantes :
- Un ester d'acide carboxylique de formule (I) dans laquelle R₁ est un groupe -COOR₁₀, peut être saponifié par des méthodes connues, par exemple par traitement avec des solutions alcalines, plus spécialement par traitement avec une solution hydro-alcoolique d'hydroxyde de sodium ou de potassium à des températures comprises entre l'ambiante au point d'ébullition du mélange réactionnel. L'acide carboxylique ainsi obtenu peut être transformé en amide via passage par un halogénure d'acide ou transformé en amide directement en utilisant une réaction de couplage peptidique, par exemple en utilisant comme agent de couplage du carbonyldiimidazole (CDI) et comme amine le 5-aminotétrazole en solution dans du THF à température ambiante (Paul R., Anderson G. W., J. Am. Chem. Soc., 82, 4596, 1960).
- Un acide carboxylique de formule (I) dans laquelle R₁ est un groupe -COOH, peut être converti par une méthode connue, par exemple par traitement avec du chlorure de thionyle, dans du toluène ou de la pyridine, ou par du trichlorure ou pentachlorure de phosphore dans du toluène, en chlorure d'acide. Ce chlorure d'acide peut être converti, en ester par réaction avec un alcool, ou en amide correspondant par réaction avec une amine.
- Un acide carboxylique ou un ester d'acide carboxylique de formule (I) dans laquelle R₁ est un groupe
- COOH ou un groupe -COOR₁₀, peut être réduit par des méthodes connues de manière à donner l'alcool correspondant où R₁ est un groupe -CH₂OH.
- Un sulfure de formule (I), ou son isomère trans, dans laquelle X₁ est un atome de soufre (-S-), peut être oxydé en sulfoxyde (-SO-) ou en sulfone (-SO₂-), R₆ et R₇ sont alors identiques ou différents et représentent soit rien (cas d'un sulfure : -S-), soit un oxygène (cas d'un sulfoxyde : -SO-) ou deux oxygènes (cas d'une sulfone : -SO₂-). L'oxydation d'un groupement sulfure peut être réalisée en utilisant des agents d'oxydation tels que des périodates (par exemple le périodate de sodium) ou en utilisant des peracides organiques tel que l'acide *m* chloroperbenzoïque (mCPBA). Quand l'oxydation est réalisée en utilisant un peracide organique, environ 1 équivalent permet d'obtenir un sulfoxyde alors que l'utilisation de 2 équivalents de peracide conduisent à la sulfone.
- Un amide de formule (I) dans laquelle R₁ est un groupe -CONrr', peut être réduit par des méthodes connues de manière à donner un aldéhyde (R₁ = -CHO), par exemple par l'hydrure de diisobutylaluminium en solution dans le toluène, préférablement en utilisant du THF comme solvant réactionnel à des températures comprises entre - 78°C et l'ambiante.
- Un aldéhyde de formule (I) dans laquelle R₁ est un groupe -CHO, peut être oxydé par des méthodes connues de manière à donner un acide carboxylique (R₁ = -COOH) ou un ester d'acide carboxylique (R₁ = -COOR₁₀), par exemple par la méthode de Corey (Corey E. J. et al., J. Am. Chem. Soc., 90, 5616, 1968) mettant en jeu le dioxyde de manganèse, du cyanure de sodium, de l'acide acétique et du méthanol à température ambiante.
- Un dérivé nitrile de formule (I) dans laquelle R₁ est un groupe -CN, peut être hydrolysé en acide carboxylique (R₁ = -COOH) correspondant par des méthodes connues, par exemple par traitement avec des bases alcalines, plus spécialement par traitement avec une solution hydro-alcoolique d'hydroxyde de sodium ou de potassium à des températures comprises entre l'ambiante et le point d'ébullition du mélange réactionnel.
- Un dérivé nitrile de formule (I) dans laquelle R₁ est un groupe -CN, peut être transformé en 1*H*-tétrazole par des méthodes connues, par exemple par traitement avec de l'azoture de triméthylsilane N₃SiMe₃ en présence ou non de catalyseur tel que de l'oxyde de dibutylétain (Bu)₂SnO dans des solvants aromatiques préférablement du toluène ou du benzène à des températures comprises entre l'ambiante et le point d'ébullition du mélange réactionnel.
- Un dérivé aromatique bromé, iodé ou chloré, de formule (I) dans laquelle R₁ est un atome de brome, d'iode ou de chlore, peut être transformé par des méthodes connues en dérivé nitrile (R₁ = -CN), par exemple par la réaction de Rosenmund-von Braun utilisant du cyanure cuivreux dans un solvant, préférablement du diméthylformamide ou de la quinoléine à des températures comprises entre l'ambiante et le point d'ébullition du mélange réactionnel.
- Un dérivé aromatique bromé, iodé ou chloré, de formule (I) dans laquelle R₁ est un atome de brome, d'iode ou de chlore, peut être transformé par des méthodes connues en acide carboxylique (R₁ = -COOH), par exemple par échange halogène-métal en utilisant du butyllithium (primaire, secondaire ou tertiaire) en solution dans du THF à froid (-78°C à 0°C) et condensation de dioxyde de carbone puis remontée à l'ambiante.
- Un dérivé aromatique bromé, iodé ou chloré, de formule (I) dans laquelle R₁ est un atome de brome, d'iode ou de chlore, peut être transformé par des méthodes connues en ester d'acide carboxylique (R₁ = -COOR), par exemple par échange halogène-métal en utilisant du butyllithium (primaire, secondaire ou tertiaire) en solution dans du THF à froid (-78°C à 0°C) et condensation sur un chloroformiate d'alkyle.
- Les dérivés de formule (I) dans laquelle R₁ est un groupe -COOH ou -tétrazoyl, peuvent être transformés par des méthodes connues en sels par des bases physiologiquement acceptables, non toxiques, inorganiques ou organiques, par exemple en sels de métaux alcalins ou en sels de métaux alcalino-terreux, par exemple du sodium, du potassium, du magnésium ou des sels de calcium, aussi bien que des sels avec de l'ammoniaque ou des amines non toxiques.
- Un acide carboxylique de formule (I) dans laquelle R₁ est un groupe -COOH, peut être converti par une méthode connue, par exemple par un réarrangement de type Curtius, avec de l'azoture de diphénylphosphorane en présence de triéthylamine dans du toluène à 80°C, suivi d'une addition d'un alcool de type ROH, préférentiellement l'alcool méthylique (R = Me) ou benzylique (R = benzyl), en carbamate -NHCOOR, qui par traitement par de la soude aqueuse à 10% (R = Me) ou par hydrogénation (R = benzyl) conduit à une aniline de formule (I) où R₁ est un groupe -NH₂.
- Une aniline de formule (I) dans laquelle R₁ est un groupe -NH₂, peut être convertie par des méthodes connues de trifluoroacétylation, par exemple en utilisant la 2-(trifluoroacétyloxy)pyridine (TFAP) dans l'éther à des températures comprises entre 0°C au point d'ébullition du mélange réactionnel (T. Keumi et al., Bull. Chem. Soc. Jpn., 63, 2252, 1990), en l'amide trifluorométhylé de formule (I) où R₁ est un groupe -NH(C=O)CF₃. L'amide trifluorométhylé de formule (I) peut être converti par des méthodes connues en imine chlorée, préférentiellement en utilisant de la triphénylphosphine et du tétrachlorure de carbone (K. Tamura et al. J. Org. Chem., 58, 32, 1993), pour conduire au composé de formule (I) où R₁ est un groupe -N=C(CF₃)(Cl).
- Un chlorure de trifluoroacétimidoyle de formule (I) où R₁ est un groupe -N=C(CF₃)(Cl) peut être cyclisé par des méthodes connues, préférentiellement par de l'azoture de sodium dans l'acide acétique à 70°C (D. Armour et al., Bioorg. & Med. Chem. Lett., 6, 1015, 1996), pour conduire à un tétrazole de formule (I) où R₁ est un groupe de formule suivante : 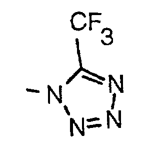
- Un composé halogéné de formule (I) où R₁ est un brome, un chlore ou un iode peut être converti par réaction d'Arbuzov, préférentiellement par traitement du diéthylphosphite dans du toluène au point d'ébullition du mélange réactionnel, en phosphonate de diéthyle de formule (I) où R₁ est un groupe -P(O)(OEt)₂.
- Un phosphonate de formule (I) où R₁ est un groupe -P(O)(OEt)₂ peut être hydrolysé par des méthodes connues, par exemple en présence d'iodure de triméthylsilyle, pour conduire à un acide phosphonique de formule (I) où R₁ est un groupe -PO₃H₂.

Les dérivés de formule (I) présentent des propriétés très intéressantes sur la différenciation et la prolifération cellulaire, permettant d'envisager leur utilisation à des fins thérapeutiques, dermatologiques et cosmétiques. Parmi les utilisations thérapeutiques, on peut citer le traitement et la prévention des cancers du type tumeurs solides, tels que les cancers du sein, des poumons, de la prostate ou du foie, ainsi que le traitement et la prévention de maladies de la peau, tels que le psoriasis et l'acné.

En outre, les travaux de biologie moléculaire, rapportés ci-après, ont permis de définir les profils d'activité des composés de l'invention sur les récepteurs rétinoiques et certains facteurs transcriptionnels.

Or, il a été récemment proposé d'utiliser des composés RXR agonistes dans le traitement du diabète non-insulino-dépendant, des maladies inflammatoires et immunitaires. En conséquence, les composés de l'invention sont utiles pour le traitement du diabéte non insulinodépendant, des maladies inflammatoires et immunitaires.

L'invention concerne donc aussi l'utilisation des dérivés de formule (I) pour la fabrication de compositions pharmaceutiques utiles dans le traitement ou la prévention des cancers iansi que pour le traitement du diabéte non insulino-dépendant, des maladies anti-inflammatoires et immunitaires. Ils peuvent aussi être utilisés pour la fabrication de compositions cosmétiques utiles dans le traitement ou la prévention de maladies de la peau.

L'invention concerne aussi l'utilisation d'un dérivé de formule suivante 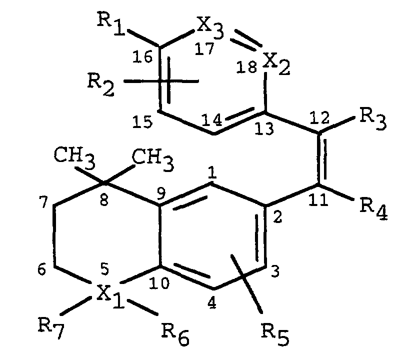
dans laquelle R₁ représente un atome d'hydrogène, un radical alkyle en C₁ à C₆, ou un groupe de formule -CH₂OH, -OH, -CHO, -COOH, -COR₈, -CH₂OCOR₉, -SH, -S-alkyl, phydroxyphénylaminocarbonyl, tétrazol-5-ylaminocarbonyl, tétrazol-5-yl, et quand cela est possible leurs sels avec des acides tolérés physiologiquement, où R₈ et R₉ sont :
   . un atome d'hydrogène, un groupe -OH, un radical alkyle en C₁ à C₆ ou un groupe de formule -OR₁₀, où R₁₀ représente un radical alkyle ramifié ou non ayant de 1 à 20 atomes de carbone, un radical alkényle ramifié ou non ayant de 2 à 20 atomes de carbone, un radical aryle ou aralkyle, ou
   . un groupe aminé de formule : 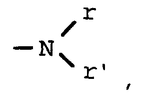 dans laquelle r et r', identiques ou différents, représentent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle.
et R₂, R₃, R₄, R₅, R₆, R₇, X₁, X₂ et X₃ ont les mêmes significations que précédemment, pour la fabrication de compositions thérapeutiques utiles dans le traitement ou la prévention des cancers ainsi que pour le traitement du diabète non insulino-dépendant, des maladies inflammatoires et immunitaires. Ces dérivés peuvent être aussi utilisés pour la fabrication de compositions cosmétiques utiles dans la traitement ou la prévention des maladies de la peau fabrication de compositions cosmétiques utiles dans le traitement ou la prévention de maladies de la peau.

En tant que médicament, les dérivés de l'invention sont administrés sous forme d'une composition pharmaceutique comprenant au moins desdits dérivés, sous forme libre ou sous forme d'un sel pharmaceutiquement acceptable, en association avec un véhicule ou diluant traditionnel. De telles compositions, qui font également partie de l'invention, peuvent se présenter pour l'administration par voie entérale, par exemple sous forme de comprimés, ou pour l'administration par voie parentérale, par exemple sous forme de solutions ou de suspensions injectables par voie intraveineuse ou musculaire, ou encore pour l'administration sous forme de pulvérisation nasale.

En tant que cosmétique, les dérivés de l'invention sont administrés sous forme d'une composition cosmétique comprenant au moins desdits dérivés, sous forme libre ou sous forme d'un sel pharmaceutiquement acceptable, en association avec un véhicule ou diluant traditionnel. De telles compositions, qui font également partie de l'invention, peuvent se présenter pour l'administration par voie entérale, par exemple sous forme de comprimés, ou pour l'administration par voie parentérale, par exemple sous forme de solutions ou de suspensions injectables par voie intraveineuse ou musculaire, ou encore pour l'administration sous forme de pulvérisation nasale, ou avantageusement pour une application topique, sous forme de crèmes, pommades, de laits, de poudres ou de gels.

Les véhicules et diluants susceptibles d'être utilisés en association avec les dérivés de l'invention sont ceux généralement utilisés dans ce type d'indications.

Pour les indications précédentes, la dose dépend du mode d'administration et du traitement désiré. On obtient des résultats satisfaisants lorsque le dérivé est administré à une dose quotidienne comprise entre 0,1 mg/kg et environ 100 mg/kg. Chez l'homme, l'administration est effectuée par exemple par voie intraveineuse, en une dose unique par jour ou en doses fractionnées jusqu'à plusieurs fois par jour, sous forme de doses unitaires contenant une concentration de 0,001 % à environ 0,01 % de substance active.

### I - Activité de dérivés de formule (I) sur la prolifération cellulaire.

### A) Tests MTT et SAMBA.

L'activité de ces dérivés sur la prolifération cellulaire a été évaluée à l'aide du test MTT mis au point par T. Mosman (J. Immunol. Method., 65, 55-63, 1983). Ce test est actuellement utilisé au NCI (National Cancer Institute) dans le cadre du programme de criblage des molécules anticancéreuses (Alley M.C. et al. Cancer Res., 48, 489-601, 1988). Par ailleurs, de nombreux laboratoires spécialisés en recherche sur la chimiothérapie et la radiothérapie font appel également à ce modèle (Arnould R. et al. Anticancer Res, 10, 145-154, 1990 ; Campling B.G. Leuk. Res., 12, 823-831, 1988 ; Kasugai S. et al. Japan Pharmacol., 52, 95-100, 1990 ; Price P. et al. Cancer Res., 50, 1392-1396, 1990).

Les molécules qui se sont révélées les plus intéressantes sur le test MTT ont été évaluées sur un test d'analyse d'images dénommé SAMBA. En effet les récentes avancées dans le domaine de l'analyse d'images ont permis de nouvelles approches d'évaluation de différents paramètres de cinétique cellulaire et de mesure de la répartition de la chromatine. Les données ainsi obtenues sont traitées par différents tests statistiques.

L'utilisation du Système d'Analyses Microscopiques à Balayage Automatique 2005 (SAMBA 2005, Alcatel TITN, France) permet de mesurer l'influence des composés étudiés à trois niveaux:
- la prolifération cellulaire,
- la cinétique cellulaire,
- la répartition et la texture de la chromatine.

Ces modifications sont représentatives de l'influence du composé testé sur la différenciation cellulaire.

### 1 - Réactifs, milieux de culture et souches cellulaires.

Les molécules originales et les produits dits de référence testés ont été synthétisés suivant les procédés décrits précédemment ou selon des procédés connus de l'homme du métier.

L'acide rétinoïque tout-trans désigné CB16178 et le 13-cis-acide rétinoïque désigné CB81808 ont été acquis auprès la société Sigma (Saint Quentin Fallavier, France).

Plusieurs produits de référence ont été sélectionnés :

### a) Les ligands naturels des deux classes de récepteurs nucléaires RAR et RXR connus de l'homme de l'art:

. le tout-trans acide rétinoïque désigné CB16178.
. le 9-cis acide rétinoïque préparé selon la méthode de Corey (Corey E. J., Gilman N. W. et Ganem B. E., *J*. *Am. Chem*. *Soc.,* 90, 5616, 1968), désigné CB13407 et un de leurs isomères,
. le 13-cis acide rétinoïque désigné CB81808.

### b) Trois rétinoïdes de référence connus de l'homme du métier :

. le TTNPB (Loeliger P. et al. Eur. J. Med. Chem., 15, 9-15, 1980) désigné CB01570,
. le LGD1069 aussi désigné CB32934 et le LGD-CB14499 (Boehm M.F. et al., J. Med.Chem., 37, 2930-2941, 1994).

Les cellules mises en culture proviennent de l'ATCC (American Type Culture Collection). Parmi les lignées cellulaires tumorales testées, seuls les résultats obtenus avec quatre d'entres-elles sont présentés ci-dessous :
- A549 (code ATCC : CCL 185) et A-427 (code ATCC : HTB 53) sont des cellules cancéreuses pulmonaires humaines.
- ZR-75-1 (code ATCC : CRL 1500) et T47D (code ATCC : HTB 133) sont des cellules cancéreuses mammaires humaines.

Les lignées cellulaires sont maintenues en culture en monocouche à 37°C dans des boîtes de culture fermées (Nunc, Gibco BRL, Life Technologies, Belgique) contenant du milieu minimum essentiel (MEM, Gibco) supplémenté avec 5 % de sérum de veau foetal (FCS, Gibco), 0,6 mg/ml de glutamine (Gibco) et un mélange de 200 UI/ml de pénicilline (Gibco), 200µg/ml de streptomycine (Gibco) et 0,1 mg/ml de gentamycine (Gibco). Le sérum de veau foetal est décomplémenté pendant 1 heure à 56°C.

### 2 - Protocoles expérimentaux.

### a) Test MTT.

Le test MTT est réalisé selon la méthode de Carmichael J. et al., Cancer Res., 47, 936-942, 1987 comportant certaines modifications (Etievant C., Anticancer Res., 11, 305-312, 1991).

Ce test est basé sur la réduction mitochondriale par les cellules vivantes métaboliquement actives du MTT (bromure de 3-(4,5-diméthylthiazol-2-yl)-2,5-diphényl tétrazolium) de couleur jaunâtre en un produit de couleur bleu, le formazan. La quantité de formazan obtenue est directement proportionnelle au nombre de cellules vivantes. Après incubation des cellules pendant 24 heures dans des microplaques de 96 puits à fond plat, le milieu de culture est remplacé par 100 µl de milieu de culture frais contenant la drogue à tester. Après 72 heures d'incubation en présence ou non de la molécule à étudier, le milieu de culture est alors remplacé par 100 µl de MTT (Sigma, Belgique) dissous à raison de 1 mg/ml dans du RPMI 1640 (Roswell Park Memorial Institute, Gibco).

Les microplaques sont alors incubées pendant 3 h à 37°C et centrifugées pendant 10 min à 400 g (GPR centrifuge, Beckman). La solution de MTT est remplacée par 100 µl de diméthylsulfoxide (Merck). Les microplaques sont agitées (Vari-Shaker, Dynatech) pendant 10 minutes. La mesure de la densité optique est alors réalisée (Lecteur DIAS, Dynatech) à la longueur d'ondes de 570 nm et à la longueur d'ondes de référence (absorbance maximale du bruit de fond) de 630 nm (Alley M.C. et al., Cancer Res., 48, 489-601, 1988).

Dans ces microplaques, les cellules contrôles comme les cellules traitées ont été incubées dans un milieu sans les composés à tester pendant 24 heures. Les cellules contrôles sont incubées dans un milieu sans drogue pendant toute l'expérimentation, tandis que les cellules traitées sont incubées dans différents milieux. Ces milieux contiennent la molécule à tester aux différentes concentrations: 10 ⁻⁸ M, 10 ⁻⁶ M et 10⁻⁴ M. Tous les essais sont effectués en 6 duplicats.

### Analyses statistigues:

Les résultats sont présentés sous forme de moyenne ± l'erreur standard sur la moyenne (ESM). Les comparaisons statistiques sont effectuées d'après le test de Fisher: NS * = P < 0,05 ; ** = P < 0,01 ; *** P < 0,001.

### b) Test SAMBA.

Les cellules en croissance exponentielle sont mises en culture sur des lamelles de verre (Kiss R. et al. Eur. J. Cancer. 27, 1268-1274, 1991) à raison de 20 x 10³ M à 60 x 10³ M cellules/ml (selon la lignée cellulaire considérée) dans 3 ml de milieu minimum essentiel (MEM, Gibco). Les lamelles de 18 x 18 mm sont placées dans des boîtes de Pétri de dimension 35 x 10 mm (Becton-Dickison). Les cellules sont ainsi cultivées pendant 96 h. Le traitement des cellules, s'il a lieu, est réalisé 24 h après le repiquage. Le milieu de culture est alors remplacé par des milieux comprenant différentes concentrations de substances à étudier.

Les cellules contrôles sont cultivées en l'absence de drogues.

Pour chaque condition expérimentale, 3 lamelles sont fixées après 96 h de culture dans l'EFA (75 volumes d'éthanol 96°, 20 volumes de formol neutre 40% et 5 volumes d'acide acétique pur) pendant 20 min. Les cellules contrôles sont fixées en même temps. Après fixation, les lamelles de verre sont montées sur des lames histologiques à l'aide de baume du Canada (DPX, BDH Chemicals) et conservées à 4°C dans l'obscurité.

Les lames supportant les lamelles sont ensuite colorées par la réaction de Feulgen R. (Z. Physiol. Chem., 135, 203-248, 1924) selon le protocole décrit par Kiss R. et al. (Modern. Pathol. 5, 655-660, 1992). Toutes les lames d'une condition expérimentale donnée sont colorées en même temps pendant 1 h dans le réactif de Feulgen (pararosalinine Chloride-C.I., Aldrich, France ; Noritt PN5, BDH) après hydrolyse dans du HCl pendant 1 h à 22-24°C. Les cellules sont maintenues à température ambiante avant analyse.

L'intérêt de la coloration de Feulgen réside dans la possibilité de colorer l'ADN de façon spécifique et stoechiométrique (Giroud F., Cell, 44, 177-188, 1982 ; Kiss R. et al., J. Histochem. Cytochem., 41, 6, 935-945, 1993). Pour chaque condition expérimentale, 900 noyaux sont analysés par l'analyseur d'images. Chaque noyau est caractérisé par 15 paramètres morphonucléaires dont 1 est de type morphométrique, 5 de type densitométrique et 9 de type textural (Brugal G. et al., J. Histochem. Cytochem., 27, 144-52, 1979 ; Pauwels O. et Kiss R., Meth. Find. Exp. Clin. Pharmacol., 15, 113-124, 1993).

Ces 15 paramètres sont calculés par des logiciels spécifiques, à partir :
- Des histogrammes des valeurs de densité optique.
- Des matrices des longueurs de section (Galloway M.M., Comput. Graph. Image Proc., 4, 172-179, 1975).
- Des matrices de co-occurence (Haralick R.M et al., IEE Trans. Syst. Man Cybern. SMC -3, 610-620, 1973).

### Statistiques et analyse mathématique :

Les mesures de la croissance cellulaire et les caractéristiques morphonucléaires sont présentées sous forme de moyenne (± erreur standard sur la moyenne : ESM). La comparaison des moyennes est réalisée à l'aide du test F de Fisher pour les probabilités de p < 0,5 ; p < 0,01 et p < 0,001.

Une analyse en composante principale suivie d'une transformation canonique est utilisée pour distinguer les noyaux " typiques " des noyaux traités par les drogues à tester. Ces analyses font appel à l'analyse multivariée décrite par Bartels P.H. (Anal. Quant. Cytol., 11, 433-439, 1980).

### 3 - Résultats.

### a) Test MTT.

Les résultats sont consignés dans les tableaux 1 à 6 présentés ci-dessous. La densité optique moyenne mesurée pour chaque condition expérimentale est exprimée en pourcentage par rapport à la condition contrôle posée égale à 100%.

Le tableau 1 ci-dessous rapporte les résultats de l'étude structure/activité de composés répondant à la formule générale suivante, sur les lignées ZR-75-1 et T-47D:

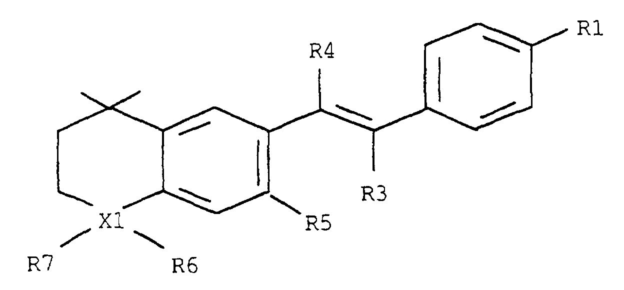

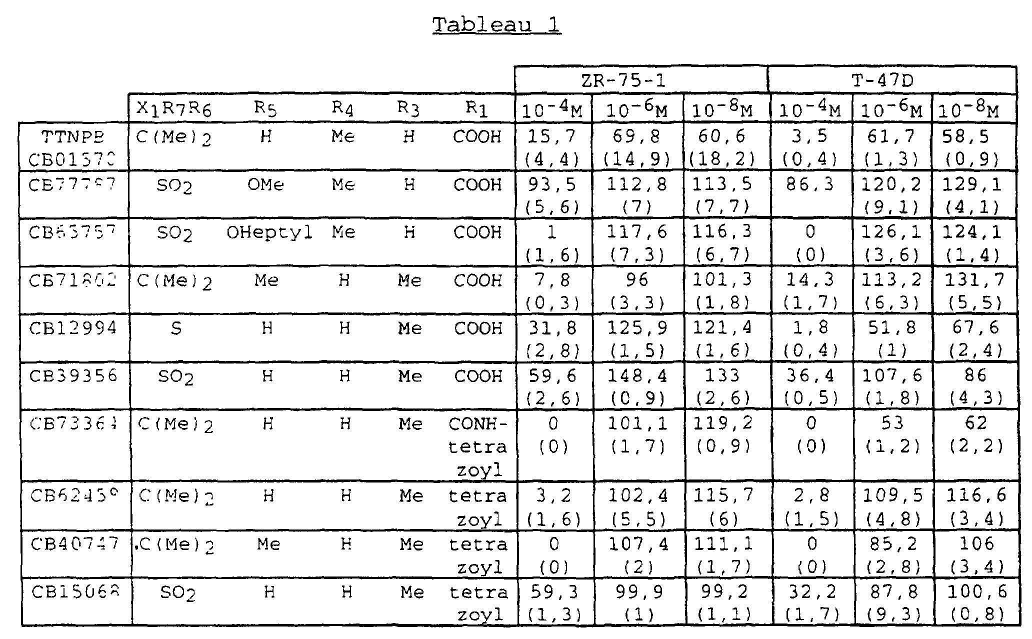

Le niveau de prolifération obtenu pour chaque lignée et pour chaque produit est exprimé en pourcentage par rapport à la condition contrôle (100% ± au maximum, 6% d'erreur standard sur la moyenne).

Le tableau 2 ci-dessous rapporte les résultats de l'étude structure/activité de composés répondant à la formule générale suivante, sur les lignées ZR-75-1 et T-47D:

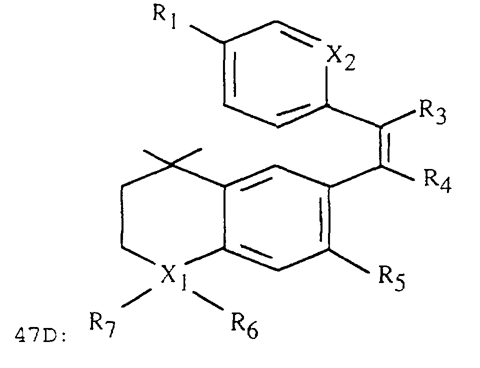

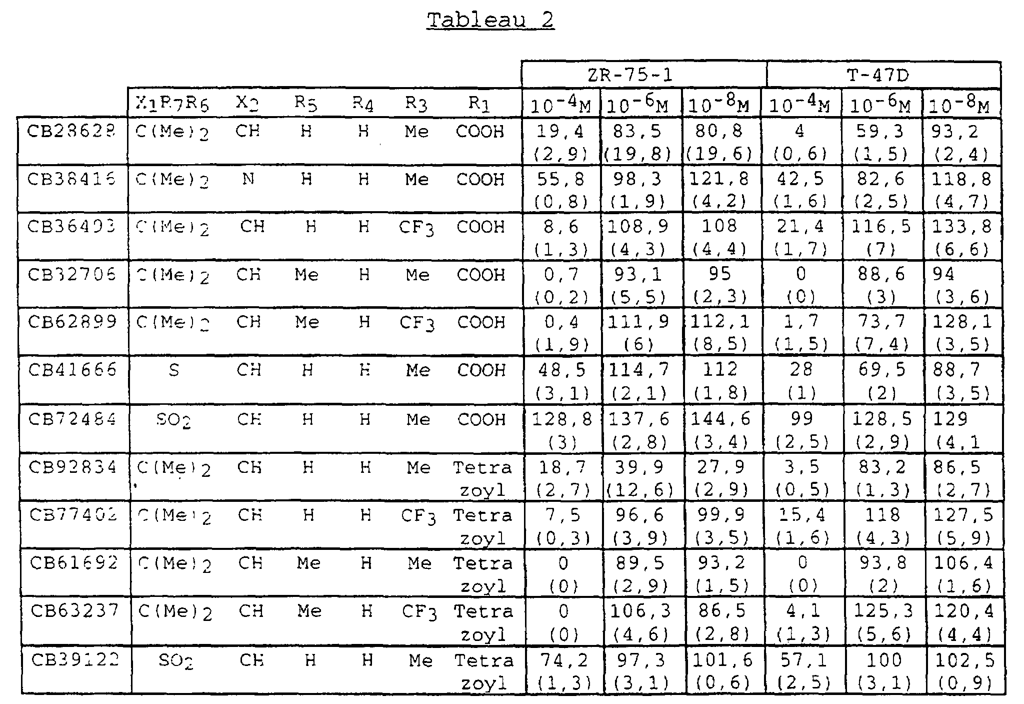

Le niveau de prolifération obtenu pour chaque lignée et pour chaque produit est exprimé en pourcentage par rapport à la condition contrôle (100% ± au maximum, 6% d'erreur standard sur la moyenne).

Le tableau 3 ci-dessous rapporte les résultats de l'étude structure/activité de composés répondant à la formule générale suivante, sur les lignées ZR-75-1 et T-47D:

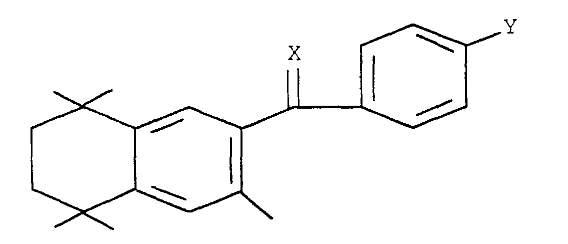

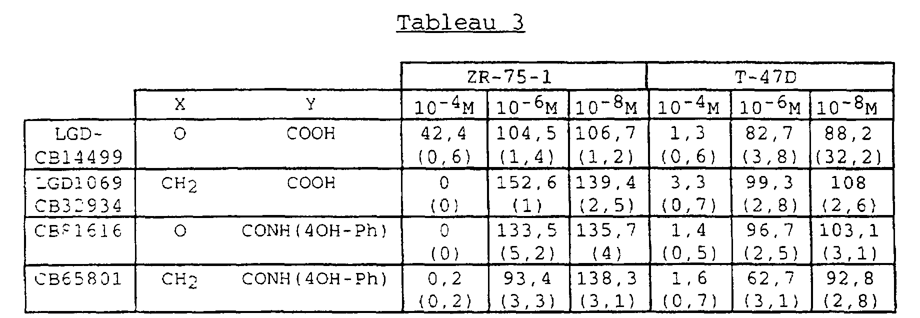

Le niveau de prolifération obtenu pour chaque lignée et pour chaque produit est exprimé en pourcentage par rapport à la condition contrôle (100% ± au maximum, 6% d'erreur standard sur la moyenne).

Le tableau 4 ci-dessous rapporte les résultats de l'étude structure/activité de composés répondant à la formule générale suivante, sur les lignées ZR-75-1 et T-47D:

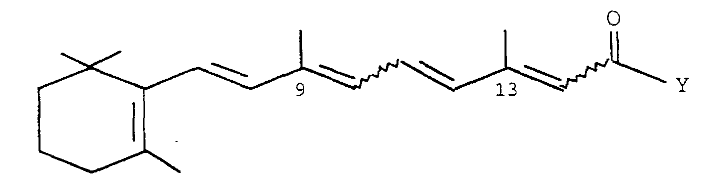

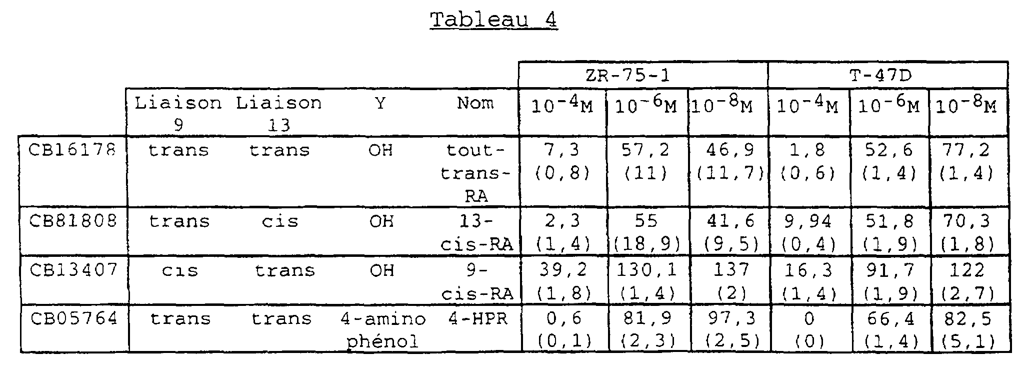

Le niveau de prolifération obtenu pour chaque lignée et pour chaque produit est exprimé en pourcentage par rapport à la condition contrôle (100% ± au maximum, 6% d'erreur scandard sur la moyenne).

Le tableau 5 ci-dessous rapporte les résultats de l'étude structure/activité de composés répondant à la formule générale suivante, sur les lignées A549 et A-427 :

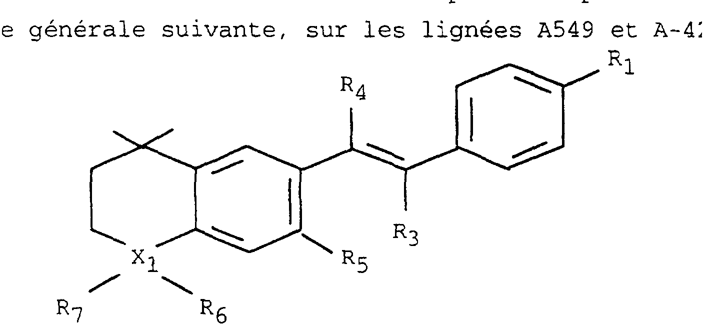

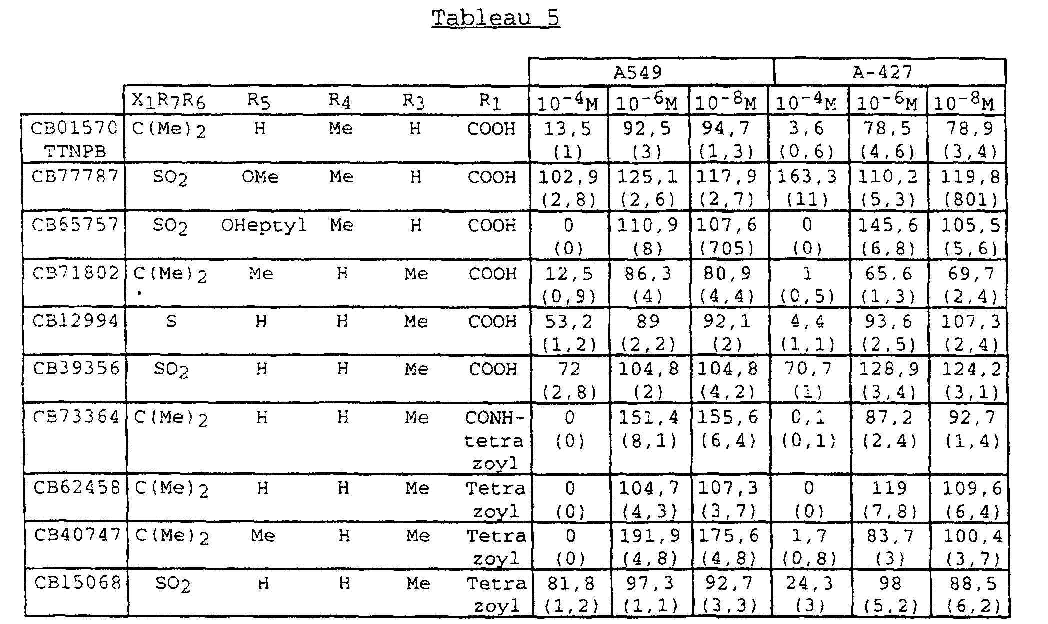

Le niveau de prolifération obtenu pour chaque lignée et pour chaque produit est exprimé en pourcentage par rapport à la condition contrôle (100% ± au maximum, 6% d'erreur standard sur la moyenne).

Le tableau 6 ci-dessous rapporte les résultats de l'étude structure/activité de composés répondant à la formule générale suivante, sur les lignées A549 et A-427 :

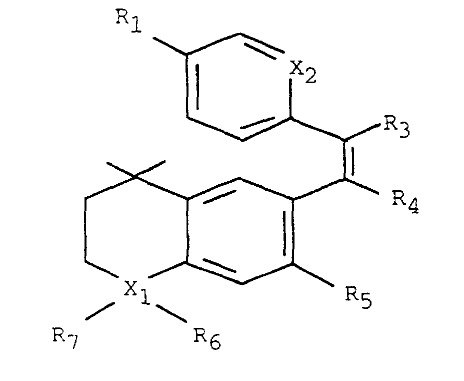

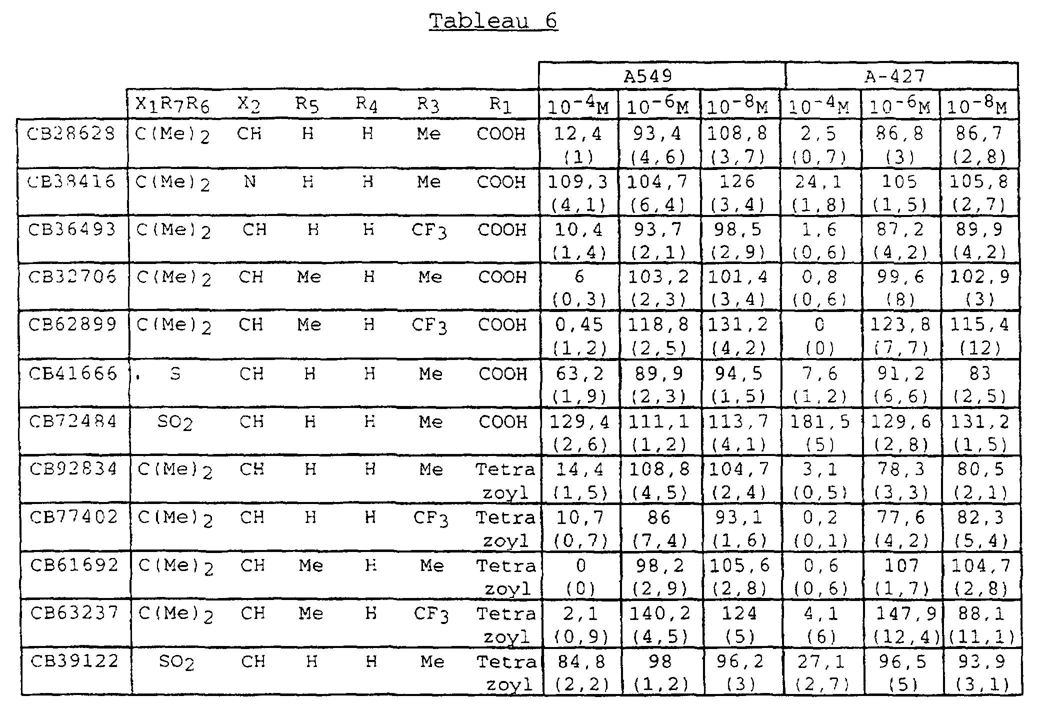

Le niveau de prolifération obtenu pour chaque lignée et pour chaque produit est exprimé en pourcentage par rapport à la condition contrôle (100% ± au maximum, 6% d'erreur standard sur la moyenne).

Le tableau 7 ci-dessous rapporte les résultats de l'étude structure/activité de composés de référence CB16178, CB81808, CB13407, CB01570 et CB32934 sur les lignées A549 et A-427 :

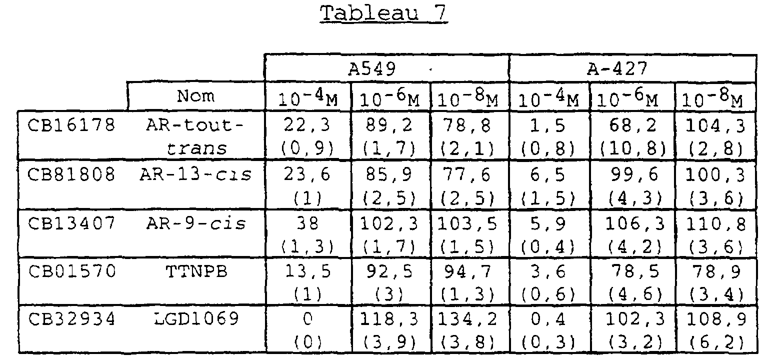

Le niveau de prolifération obtenu pour chaque lignée et pour chaque produit est exprimé en pourcentage par rapport à la condition contrôle (100% ± au maximum, 6% d'erreur standard sur la moyenne).

### Résultats concernant les lignées pulmonaires A549 et A-427.

Les produits de référence sont globalement inactifs sur ces lignées aux concentrations de 10 ⁻⁶ M et 10⁻⁸ M. Seuls le tout-trans acide rétinoïque (CB16178) et le 13-*cis*-acide rétinoïque (CB81808) inhibent la prolifération de la lignée A549 à 10⁻⁶ M et 10⁻⁸ M. Le TTNPB (CB01570) inhibe la lignée A-427 à 10⁻⁶ M et à 10⁻⁸ M. Le LGD1069 (CB32934) induit une stimulation de la prolifération des cellules A549 à 10⁻⁶ M et 10⁻⁸ M. Parmi les dérivés dont les groupements R₃ et R₄ portés par la double liaison entre les carbones 11 et 12 sont de configuration trans, le composé CB71802 inhibe le plus fortement la croissance de ces deux lignées pulmonaires. Par contre, une stimulation de la croissance cellulaire est observée avec CB39356 (A-427), CB73364 (A549) et CB40747 (A-427). Les autres molécules ne présentent pas ou peu d'activité sur ces lignées. Parmi les dérivés de formule
(I) de conformes à la présente invention, les composé CB92834 et CB77402 présentent une activité sur la croissance de ces deux lignées. Les autres molécules ne présentent pas d'activité significative sur ces deux lignées.

### Résultats concernant les lignées mammaires ZR-75-1 et T47D.

Parmi les produits de référence, l'acide rétinoïque tout-trans (CB16178) et le 13-cis acide rétinoïque (CB81808) comme le TTNPB (CB01570) inhibent la croissance cellulaire des deux lignées mammaires aux concentrations testées. Le 9-cis acide rétinoïque (CB13407) stimule la croissance cellulaire à 10⁻⁶ M et 10⁻⁸ M.

Les différents dérivés dont les groupements R₃ et R₄ portés par la double liaison entre les carbones 11 et 12 sont de configuration trans, comme le composé CB62458 et les bioisostères CB39356 et CB15068 n'influencent pas la croissance cellulaire des lignées considérées. Seul le composé CB73364 inhibe la croissance des cellules T-47D.

Parmi les dérivés de formule (I) conformes à la présente invention, le composé CB92834 montre une forte activité inhibitrice de la croissance cellulaire des deux lignées. Les autres molécules s'avèrent moins puissantes voire inactives.

### Conclusions.

### a) Test MTT :

Parmi les rétinoïdes de référence testés : l'acide rétinoïque tout-trans (CB16178), le 13-cis acide rétinoique (CB81808), le 9-*cis*-acide rétinoïque (CB13407), le TTNPB (CB01570) et le LGD1069 (CB32934), le TTNPB induit la plus forte inhibition des lignées cellulaires. Le produit CB92834 apparaît aussi actif sur le test MTT que les produits de référence testés. Le composé CB92834 est plus actif sur les lignées oestrogéno-sensibles telles les lignées mammaires ZR-75-1 et T-47D que sur les lignées pulmonaires. L'activité antiproliférative du CB92834 sur les lignées tumorales mammaires, déterminée in vitro à l'aide du test MTT, est retrouvée et corrélée aux résultats obtenus sur le système d'analyse d'images SAMBA. Ces deux techniques différentes mettent ainsi en évidence l'activité anti-néoplasique de ce rétinoïde.

L'analyse quantitative de l'image des noyaux colorés de façon spécifique et stochiométrique repose sur le calcul de 15 paramètres morphonucléaires déterminés soit à partir :
- de l'histogramme des valeurs de densité optique,
- des matrices de longueur de section,
- des matrices de co-occurence.

Le traitement statistique des données obtenues permet alors de déterminer et de caractériser l'activité antitumorale des rétinoïdes au niveau :
- de l'indice de prolifération,
- de la cinétique cellulaire,
- de l'organisation de la répartition et de la texture de la chromatine.

### b) Test SAMBA :

L'analyse d'images des noyaux colorés permet d'obtenir un histogramme de l'ADN et de calculer un index de prolifération. En plus de l'étude de la cinétique du cycle cellulaire, l'analyse d'images de ces noyaux au travers de la mesure de 15 paramètres et de la quantification de la chromatine permet de déterminer l'influence de la drogue sur un état cytologique de la différenciation.

Le TTNPB (CB01570) et le composé CB92834 influencent différemment la différenciation morphologique de la lignée T-47D alors que leurs capacités d'inhibition de la prolifération cellulaire globale (test MTT) des cellules néoplasiques s'avèrent comparables. L'effet dose-dépendant du TTNPB sur la valeur des différents paramètres morphonucléaires traités en analyse multivariée suggère fortement une corrélation directe entre l'effet cytotoxique de la molécule et son activité antitumorale. A l'inverse l'effet antitumoral du CB92834 n'est pas relié à un effet cytotoxique mais à une modulation du rapport gain cellulaire sur perte cellulaire et à une induction de la différenciation cellulaire.

Le TTNPB (CB01570) exerce son activité antitumorale par un effet cytotoxique (cytocidal) direct tandis que le pouvoir antitumoral du composé CB92834 serait médié par une activation de la mort cellulaire et/ou à une inhibition de la prolifération due à l'induction d'une différenciation cellulaire.

Les dérivés de formule (I), et plus particulièrement les composés CB92834 et CB77402, se signalent donc par d'intéressantes propriétés pharmacologiques, et peuvent par conséquent être utilisés en thérapeutique humaine ou animale comme médicament ainsi qu'en cosmétologie.

### B) Essais clonogéniques.

Par définition une cellule est considérée clonogénique si elle possède la capacité de proliférer et de donner naissance à une colonie cellulaire contenant au minimum 50 cellules. Les "human tumor stem cells" ou "cellules souches tumorales humaines" sont les cellules clonogéniques qui sont à l'origine des cellules néoplasiques qui constituent une tumeur donnée. Ces cellules souches tumorales sont responsables des processus de récidives observables après résection chirurgicale des tumeurs primaires et sont également responsables de la formation des métastases. Elles sont ainsi devenues la première cible de certaines chimiothérapies anticancéreuses (Fialkow P.J. et al., Am. J. Med., 1977, 63, 125-130 ; Hamburger A.W. et Salmon S.E., Science, 1977, 197, 461-463 ; Selby P. et al., N. England J. Med., 1983, 308, 129-134; Steel G.G., Growth kinetics of tumor, Clarendon Press, 1977). Au niveau d'une tumeur ou d'une lignée cellulaire tumorale ces cellules souches clonogéniques se différencient, des autres cellules clonogéniques de la tumeur ou de la lignée cellulaire néoplasique considérée, par le fait qu'elles conservent leur capacité à proliférer en l'absence de tout support solide.

Une méthode simple appelée "test clonogénique" (colony-forming assays ou stem cell assay) a été développée en 1977 par Hamburger A. H. et Salmon S. E. Cette technique est appropriée pour la mise en culture in vitro de la plupart des tumeurs et des lignées cellulaires tumorales de différents types histologiques. Dans le cadre de ce test, les cellules néoplasiques sont mises en culture sur un milieu de culture semi-solide "soft agar" constitué d'un mélange d'agar et d'agarose. Sur ce support sélectif, ne subsistent et ne prolifèrent que les cellules souches tumorales. En effet, sur un tel milieu de culture les cellules normales, tels par exemple les fibroblastes, ne survivent pas. Dans ces conditions, en fonction de la tumeur ou de la lignée cellulaire tumorale considérée, le pourcentage de cellules tumorales capables de proliférer et de donner naissance à une colonie cellulaire est compris entre 0,1 et 0,001% seulement. Les caractéristiques morphologiques et la croissance des colonies cellulaires sont également fonction du type histologique des tumeurs considérées.

Cette technique demeure la plus communément utilisée et la mieux adaptée à l'étude des effets des drogues anticancéreuses sur les cellules souches des tumeurs humaines. En effet les premières études in vitro faisant appel à ce type de test indiquent qu'une prédiction correcte de la réponse au traitement par chimiothérapie est possible dans environ 60% des cas et qu'une prédiction correcte de la résistance d'une tumeur à un traitement par chimiothérapie peut être mis en évidence dans plus de 90 % des cas (Van Hoff D.D., Semin. Oncol. 1985, 12, 327-331; Bertelsen C.A., Cancer, 1984, 53, 1240-1245). Ce test permet d'étudier et de décrire les propriétés biologiques, la sensibilité aux différents agents anticancéreux utilisés en chimiothérapie. Pour un même patient, ce test permet d'étudier l'hétérogénéité de la réponse à une drogue donnée au sein d'une même tumeur, entre la tumeur primaire et ses métastases et enfin entre les différentes métastases (Tanigawa N. et al. Cancer Res., 1984, 44, 2309-2312). L'étude de la croissance clonogénique des cellules tumorales d'une tumeur donnée sur un milieu de culture semi-solide semble être reliée au degré de malignité de la tumeur considérée (Dittrich C. et al., J. Clin. Oncol. 1991, 9, 381-388; Von Hoff D.D. et al., Cancer Res. 1983, 43, 1926-1931 ; Alberts D.S. et al., Lancet, 1980, 2, 340-343 ; Meyskens F.L. et al. Br. J. Cancer, 1981, 44, 787-797 ; Salmon S.E. et al. Cancer Research, 1980, 74, 300-305 ; Salmon S.E. et al., Cloning of human tumor stem cells, 1980, 223-245 ; Von Hoff D.D. et al., Am. J. Med. 1981, 70,1027-1032).

Dans le cadre de l'invention, l'influence de divers rétinoïdes sur la croissance des colonies cellulaires obtenues en cultivant la lignée tumorale mammaire T-47D sur ce milieu de culture semi-solide "soft-agar" a été mesurée. Ces conditions de culture sont également appelées "anchorage-indépendant".

### 1) Matériels et méthodes.

### a) Réactifs, milieu de culture et lignée cellulaire tumorale.

### - Réactifs.

Les dérivés de l'invention et les produits de référence testés ont été synthétisés suivant les procédés qui seront décrits plus loin ou selon des procédés connus de l'homme de métier.

Le tamoxifène commercial, désigné CB58707, et le 4-hydroxytamoxifène, désigné CB05764, ont été acquis auprès de la société Sigma (Saint Quentin Fallavier, France).

Plusieurs rétinoïdes de référence, ligands des deux classes de récepteurs nucléaires RAR et RXR connus de l'homme de l'art, ont été sélectionnés :
- Le 9-cis acide rétinoïque, ligand naturel, désigné CB13407.
- Le TTNPB désigné CB01570.
- Un analogue du TTNPB SRI désigné CB28628.
- Le LGD1069 désigné CB32934.
- Milieu de culture.

Le milieu de base "MEM 25 MM HEPES" (Minimum Essential Medium, Life technologies, Belgique) contient des sels de Eagle mais pas de L-glutamine. Ce milieu est bien adapté à la croissance d'une gamme de cellules variées diploïdes ou primaires de mammifères. Ce milieu est ensuite additionné :
- d'une quantité de 5% de SVF (Sérum de Veau Foetal, Life Technologies, Belgique). décomplémenté à 56°C pendant 1 heure,
- de 0,6 mg/ml de L-glutamine (Life Technologies, Belgique),
- de 200 IU/ml de pénicilline (Life Technologies, Belgique),
- de 200 µg/ml de streptomycine (Life Technologies, Belgique),
- de 0,1 mg/ml de gentamicine (Life Technologies, Belgique).
- Lignée cellulaire tumorale.

La lignée cellulaire tumorale humaine d'origine mammaire T-47D utilisée dans le cadre de ce travail provient de l'ATCC (American Type Culture Collection, code ATCC : HTB 133).

### b) Protocole expérimental.

Les cellules tumorales sont maintenues en culture dans des boîtes falcon de 25 cm² (Life Technologies, Belgique). Elles sont ensuite trypsinisées et individualisées. Leur taux de viabilité est déterminé par coloration au bleu trypan (Sigma, Belgique). Il doit être supérieur à 90%. Dans le cas où cette condition est respectée une suspension cellulaire à la concentration souhaitée est préparée dans une solution d'agar à 0,3%. Les cellules sont ensuite ensemencées entre deux couches d'agar (Sigma, Belgique) et dans des boîtes de Pétri de 35 mm (Life Technologies, Belgique). La couche de base est constituée d'une solution d'agar à 0,5% et la couche supérieure est constituée d'une solution d'agar à 0,3%. Les cupules sont ensuite placées et maintenues dans un incubateur à 37°C et 5% de CO₂. Vingt quatre heures après l'ensemencement, les cellules sont traitées en déposant sur la couche supérieure les différents produits à tester à des concentrations 100 fois supérieures à la concentration finale souhaitée car le volume de la solution traitante ajoutée est 100 fois inférieur au volume total d'agar déposé dans les boîtes de Pétri. Après le traitement les cupules sont maintenues dans l'incubateur pendant 21 jours. Il est conseillé d'examiner, au cours de l'expérimentation, l'apparition et la croissance des colonies cellulaires. Si après 10 jours aucune colonie n'apparaît ou se développe l'expérimentation doit être renouvelée. Dans le cas contraire au 21^{ème} jour 100 µl d'une solution de MTT (3-(4,5-diméthylthiazol-2-yl)-2,5 diphényl tétrazolium bromide; Sigma, Belgique) solubilisée dans du milieu de culture RPMI (Roswell Park Memorial Institute, Life technologies, Belgique) à la concentration de 1mg/ml sont déposés sur la couche supérieure de l'ensemble des cupules. Les cellules sont maintenues au contact de cette solution au minimum 3 heures à 37°C. Les cellules métaboliquement actives transforment alors par réduction mitochondriale cette solution jaunâtre en cristaux bleus de formazan permettant ainsi de mettre en évidence les colonies cellulaires présentes (Mosmann, 1983 ; Carmichael et coll., 1987).

A l'aide d'un microscope inversé et à faible grossissement le nombre de clones dont la surface est supérieure à 100 µm² est déterminé dans l'ensemble des cupules représentants l'ensemble des différentes conditions expérimentales. Ensuite pour chacune de ces conditions expérimentales le nombre moyen de colonies cellulaires ± l'erreur standard sur la moyenne (ESM) est calculé.

### c) Analyse statistique.

Les résultats obtenus dans les conditions traitées sont comparés à ceux obtenus dans la condition contrôle par l'intermédiaire d'une analyse statistique de type non paramétrique et selon le test de Mann-Withney (NS : P > 0,05 ; * : P < 0,05 ; ** : P < 0,01 ; *** : P < 0,001).

### 2) Résultats.

Les résultats sont consignés dans les tableaux 8 à 10. Le nombre moyen de colonies cellulaires déterminé pour chaque condition expérimentale est exprimé en pourcentage par rapport à la condition contrôle posée égale à 100%.

### a) Résultats concernant les produits de référence.

Le tableau 8 ci-après regroupe les résultats obtenus avec les produits de référence choisis.

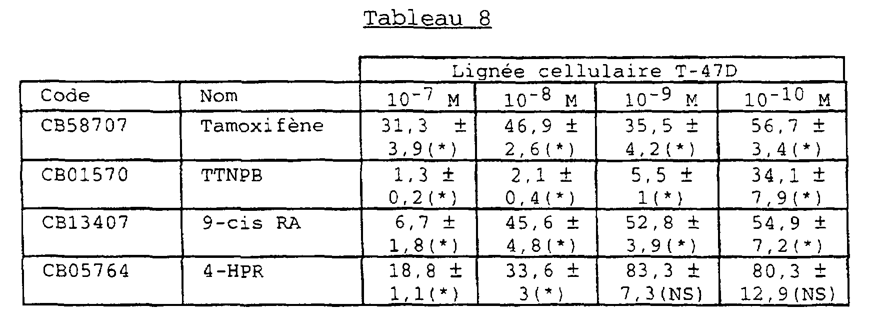

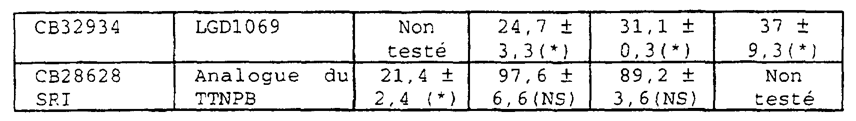

Les résultats obtenus montrent que les produits de référence choisis inhibent de manière différente la croissance des colonies cellulaires de la lignée tumorale mammaire humaine T-47D.

Le tamoxifène (CB58707), molécule à activité anti-oestrogénique, induit aux concentrations testées ici (10⁻⁷ M à 10⁻¹⁰ M) une inhibition significative de la croissance de ces colonies mais son activité n'est pas dose dépendante, ce qui n'est pas le cas de son homologue le 4-HPR. Ce dernier, très fortement actif à la concentration de 10 ⁻⁷ M, perd son activité inhibitrice significative dès la concentration de 10⁻⁹ M. Le rétinoïde naturel 9-cis acide rétinoique (CB13407) présente une activité inhibitrice significative et dose dépendante. Enfin le rétinoïde synthétique TTNPB (CB01570) inhibe très fortement, de manière significative et dose dépendante la croissance des colonies cellulaire de la lignée T-47D. Il est à noter que son activité inhibitrice est encore de 66% à la concentration de 10⁻¹⁰ M. Le LGD1069, ligand spécifique des récepteurs nucléaires RXR, inhibe fortement et significativement la croissance des colonies cellulaires de la lignée T-47D. Si son activité inhibitrice est faiblement dose dépendante, elle demeure néanmoins très importante à la concentration de 10⁻⁸ M (63%). Le rétinoïde SRI-CB28628 inhibe fortement et significativement la croissance des colonies cellulaires à la concentration de 10⁻⁷ M mais perd très rapidement son activité, dès la concentration de 10⁻⁸ M.

### b) Résultats concernant les produits de la série (E).

Le tableau 9 ci-après résume l'ensemble des résultats relatifs aux rétinoïdes de la série trans (E).

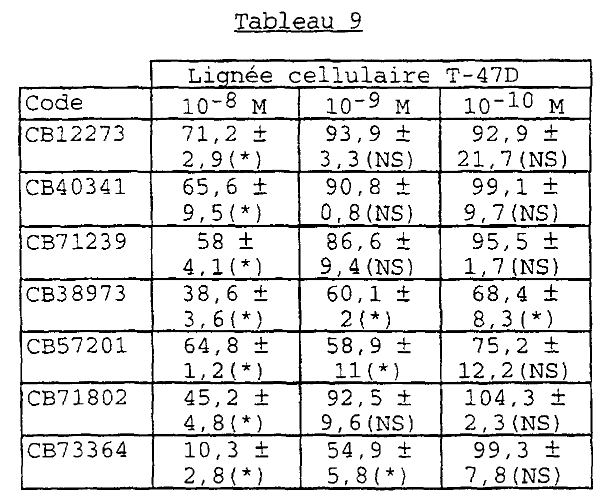

Les arotinoïdes de cette série trans (E) induisent une inhibition de la croissance des colonies cellulaires de la lignée T-47D de manière significative à la concentration de 10 ⁻⁸ M. A cette concentration le pourcentage d'inhibition des différents produits s'échelonne de 69,7%, pour le produit CB73364, à 28,8%, pour le produit CB12273. A la concentration de 10⁻⁹ M seuls trois (CB38973, CB57201 et CB73364) sur les sept produits testés inhibent la croissance des colonies cellulaires. A la concentration de 10⁻¹⁰ M, seulement deux produits (CB38973 et CB57201) sur les sept produits inhibent significativement la croissance des colonies. A ces deux concentrations de 10⁻⁹ M et de 10⁻¹⁰ M, aucune des inhibitions induites n'est supérieure à 50%.

### c) Résultats concernant les produits de la série cis (Z).

Le tableau 10 ci-après résume l'ensemble des résultats relatifs aux rétinoïdes de la série cis (Z).

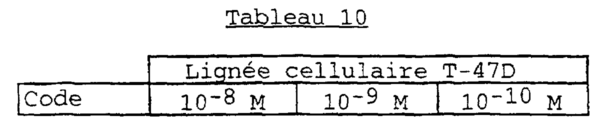

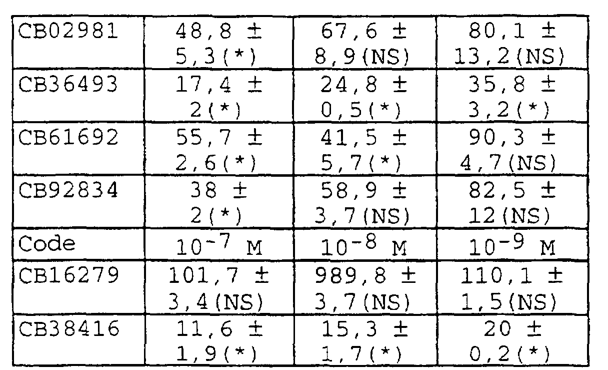

Parmi les six produits testés dans cette série le produit CB16279 n'induit aucune modification significative de la croissance des colonies cellulaires et ce même à la concentration de 10⁻⁷ M. En revanche les cinq autres produits induisent à la concentration de 10⁻⁸ M une inhibition significative de la croissance de ces colonies. Aux concentrations de 10⁻⁹ M et 10⁻¹⁰ M trois produits (CB 36493, CB61692 et CB38416) sur six et un produit (CB36493) sur quatre inhibent significativement la croissance des colonies cellulaires de la lignée tumorale T-47D. A ces deux concentrations les inhibitions induites par les produits CB 36493, CB 61692 et CB 38416 sont toutes supérieures à 50%.

### 3) Discussion et conclusion.

Les résultats ci-dessus montrent que le test clonogénique est un test adapté à la sélection et au screnning de rétinoïdes divers.

Dans les mêmes conditions expérimentales, les inhibitions induites par les produits de la série cis (Z) sur la croissance des colonies cellulaires de la lignée tumorale mammaire humaine T-47D cultivée sur soft agar sont pour la plupart significativement plus importantes que celles induites par les produits de la série trans (E). On remarque que le produit CB92834 présente une activité inhibitrice globale et dose dépendante aussi importante que celle obtenue avec les meilleurs produits de la série trans (E). Les produits de la série cis (Z) se présentent comme de plus puissants inhibiteurs de la croissance des cellules souches tumorales de la lignée T-47D que les produits de la série trans (E). De plus les produits CB36493 et CB38416 de la série cis (Z) apparaissent aussi actifs que les produits de référence CB13407 (9-cis RA) et LGD1069. Enfin seul le produit de référence TTNPB (CB01570) est plus actif que le produit CB36493 de la série cis (Z).

### II - Activités des dérivés de formule (I) sur les récepteurs rétinoïques et certains facteurs transcriptionnels.

### I) Modèles et molécules de références.

L'acide rétinoïque tout trans (ttRA) et ses stéréoisomères 9-cis, 11-cis et 13-cis se lient et activent, plus ou moins sélectivement, des récepteurs intranucléaires dits récepteurs rétinoïques. Une avancée importante dans le mécanisme d'action moléculaire du signal de transduction de l'acide rétinoïque a été établie notamment grâce aux travaux pionniers de R. M. Evans et al. (Sciences, 1988, 240, 889-895).

Les composés de l'invention du type rétinoïde ou arotinoïde possèdent des profils de sélectivité différents vis à vis des sous-types de récepteurs de l'acide rétinoïque (RARs) et des récepteurs X rétinoïdes (RXRs).

Un grand nombre de résultats cliniques récents ont montré que l'acide rétinoïque, certains de ses isomères et des dérivés formant la classe des rétinoïdes, sont utilisés pour le traitement de maladies telles que l'acné, le psoriasis et certains cancers (U. Reichert et al, Pharmacology of Retinoids in the Skin, Karger AG Eds, Basel, 1989 ; M.S. Tallman et al, Retinoids in Cancer Treatment, J. Clin. Pharmacol., 1992, 32, 868-888 ; Warrell et al, N. Engl. J. Med., 1991, 324, 1385-1393).

Ces rétinoïdes sont aussi évalués dans d'autres domaines thérapeutiques tels que, à titre indicatif : l'arthrite (Vinienti M.P. et al, Using Inhibitors of Metalloproteinases to treat Arthritis, Arthritis Rheumatoidism, 1994, 37, 1115-1126) ; la dyslipidémie (Rottman et al, A RARE Element in the Apolipoprotein AI Gene Distinguishes Between Two Different Retinoic Acid Response Pathways, Mol. Cell. Biol., 1991, 3814-3820) ; la prévention de la lymphopénie VIH induite (Yang Y. et al, 9-cis RA Inhibits Activation-Driven T-Cell Apoptosis: Implications for Retinoid X Receptor Involvement in Thymocyte Development, Proc.Natl. Acad. Sci. USA, 1993, 90, 6170-6174). Ces effets thérapeutiques résultent de la capacité de l'acide rétinoïque et de certains rétinoïdes à contrôler des situations cellulaires anormales par la modulation de la croissance cellulaire, de la différenciation cellulaire et/ou de l'apoptose ou mort cellulaire programmée (The Retinoids, Biology, Chemistry and Medecine, M.B. Sporn, A.B. Roberts et D.S Goodman, Raven Press Eds, 2nd ed, New York 1994). Ces régulations ont été attribuées pour large partie à la formation de complexes ligand(s)-récepteurs. Ces protéines appartiennent à la superfamille des récepteurs nucléaires, et fonctionnent comme facteurs de transcriptions ligands dépendants. Ce sont ces interactions qui sont responsables de l'activation transcriptionnelle et des effets physiologiques associés.

A l'aide de ligands endogènes et synthétiques, cette famille a été classée en deux séries dénommées RAR et RXR, composées chacune en trois sous-types de récepteurs appelés α, β et γ. De plus, ces rétinoïdes se sont montrés capables de réguler l'expression d'autres gènes par un effet inhibiteur de facteurs transcriptionnels comme le complexe AP-1 composé des protéines oncogènes c-Fos et c-Jun. Toutes ces protéines récepteurs modulent l'expression de certains gènes par liaison sélective, sous forme de dimère, à des régions spécifiques de l'ADN appelées RARE's (pour Retinoic Acid Response Element's, M.B. Sporn et al, p.319-349, D.J. Mangelsdorf et al., Proc. Natl. Acad. Sci. USA., 1991, 88, 3559-3563).

Les récepteurs RXR fonctionnent comme homodimères ou peuvent s'hétérodimériser avec les récepteurs RAR ainsi qu'avec les autres membres de la superfamille des récepteurs intracellulaires.

L'acide rétinoïque tout trans (ttRA) est le ligand naturel des récepteurs RARs, alors que son isomère 9-cis (9-cis RA) est à la fois ligand des récepteurs RXRs et RARs sous forme d'homodimères et d'hétérodimères (M. B. Sporn, page 5-178, X-K. Zhang et al, Homodimer formation of Retinoid X receptor induced by 9-cis RA, Nature, 1992, 358, 587-591, Heyman R.A. et al , 9-cis RA is a high affinity ligand for the Retinoic Receptor X, Cell, 1992, 68, 397-406, Levin A. A. et al, 9-cis RA stereoisomer binds and activates the nuclear receptor RXRα, Nature, 1992, 355, 359-361).

Il a été montré que ces récepteurs sont significativement différents : les structures primaires des domaines de liaisons (composition en acides aminés) sont différentes à plus de 80%. De même, une distribution différente de ces sous-types de récepteurs est fonction de ia nature des tissus. Par exemple, les RARs ne sont pas exprimés dans les viscères, par contre les RXRα mRNA sont les plus abondants dans le foie, les reins, les poumons, l'intestin et les muscles.

Les voies hormono-dépendantes des RARs peuvent être activées par des ligands RAR spécifiques qui se lient à la partie RAR des RAR-RXR hétérodimères, tandis que des ligands specifiques RXR se révèlent incapables d'activer ces mêmes voies en se fixant sur la partie RXR. Des ligands RXR présentent une synergie d'activation des gènes répondant au ttRA quand ils sont utilisés en association avec des ligands spécifiques RARs (Roy B. et al, Mol. Cell Biol., 1995, 15, 6481-6487). Les RXRs forment des homodimères, en présence de ligands RXR et régulent la transcription de gènes qui sont distincts de ceux contrôlés par les hétérodimères RAR-RXR (Zhang X.-K. et al. cité ci-dessus).

Ainsi des rétinoides qui sont sélectifs pour des sous-types de récepteurs seront utiles à un contrôle sélectif voir indépendant des voies physiologiques médiées par ces mêmes sous-types. Par comparaison un panagoniste sera utile pour contrôler les voies physiologiques médiées par plusieurs de ces sous-types. Il apparaît que des rétinoïdes agissant sélectivement sur ces sous types pourront augmenter l'efficacité thérapeutique et réduire le profil des effets secondaires. Un panagoniste est défini comme un agent qui se lie et active au moins un des récepteurs de la sous-famille RAR et de la sous famille RXR. Un véritable panagoniste active tous les membres des sous familles RAR et RXR.

L'acide rétinoique tout trans (ttRA), comme son isomère 13-cis, a lors de tout traitement chronique un puissant effet d'hypervitaminose, de toxicité mucocutanée et de tératogénécité. De plus, ttRA est inducteur de son propre métabolisme ce qui a pour effet direct de diminuer rapidement son efficacité thérapeutique.

C'est pourquoi, la présente invention vise à fournir de nouveaux composés ayant une plus grande stabilité chimique et métabolique, et des profils d'activité différents vis à vis de ces sous-types de récepteurs reliés à des activités anti-tumorales et anti-prolifératives sélectives bien établies. Une telle stratégie a conduit à la formation de molécules :
- panagonistes RAR-RXR,
- sélectives RAR ou RXR,
- dissociantes anti-AP-1.

Par leur propriété propre de co-activation des protéines RAR, des rétinoides RXR-sélectifs constituent un nouveau plus thérapeutique. A des doses où ils sont inactifs par eux-mêmes, ils peuvent augmenter l'activité de rétinoïdes RAR-sélectifs, et notamment RARα, alors utiles dans le traitement (régression ou rémission) de cancers de type leucémies, de tumeurs solides, plus particulièrement du sein, de la tête et du cou, mais aussi de manière plus classique dans les épisodes d'acné, d'acné sévère et de peau endommagée par le soleil. L'administration de rétinoïdes utilisés en combinaison, peut être concommitante ou simultanée. Dans ce cas, l'écart d'administration entre les rétinoïdes ne doit pas dépasser quelques heures, de telle sorte que les rétinoides RXR et RAR seraient dans des concentrations sanguines telles que la potentialisation soit effective.

### 1) Expression des récepteurs RAR, RXR et RE en fonction des lignées cellulaires.

Les cellules MCF-7 et HeLa sont cultivées dans du DMEM avec rouge de phénol additionné de sérum de veau foetal 5%. Les cellules T47-D sont cultivées dans du RPMI additionné de sérum de veau foetal 10%. Les essais expérimentaux sont réalisés dans du DMEM sans rouge de phénol additionné de sérum de veau foetal traîté au charbon dextran à 3 %. Les lignées cellulaires transfectées de façon stable, issues des lignées MCF-7 et HeLa, sont établies selon le protocole décrit par D. Gagne et al. (J. Biolumin. Chemilumin., 1994, 9, 201-209). Les expériences utilisant les différents rétinoïdes sont réalisées à l'abri de la lumière afin d'éviter toute isomérisation.

Le tableau 11 ci-dessous rapporte la différence d'expression des récepteurs de l'acide rétinoïque et du récepteur aux estrogènes RE par les cellules HeLa et MCF-7 (Titcomb M. W. et al., Mol. Endocrinol., 1994, 8, 870-877). Les résultats du tableau 11 sont exprimés en fentomoles de récepteur par mg de protéines.

**Tableau 11**

| Type de récepteur | HeLa | MCF-7 |
|---|---|---|
| RE | non détecté | exprimé |
| RARα | 28 | 80 |
| RARβ | 9 | non détecté |
| RARγ | 16 | 34 |
| RXRα | 50 | 12 |
| RXRβ | 28 | non détecté |
| RXRγ | 9 | non détecté |

### 2) Spécificité des molécules de référence dans des modèles de transfections transitoires.

### a) Récepteurs chimérigues Ga14-RAR.

Les études de spécificité transactivatrice des rétinoïdes ont été réalisées par transfection transitoire de ellules HeLa. Deux types de récepteurs chimériques peuvent alors être exprimés par les cellules. Les plasmides Gal-RARα, Gal-RARβ et Gal-RARγ (J.Y. Chen et al., EMBO J., 1995, 14, 1187-1197) codent pour des récepteurs chimériques Gal4-RAR dans lesquels le domaine de liaison à l'ADN de la protéine Ga14 de levure est fusionné avec les régions E et F régions contenant le domaine de liaison au ligand et la ronction d'activation AF-2) des récepteurs de l'acide rétinoique. La région C (domaine de liaison à l'ADN) et les régions A et B (domaine d'activation AF-1) sont supprimées.

Ces récepteurs chimériques activés par un agoniste stimulent spécifiquement la transcription du gène de la luciférase présent dans un plasmide cotransfecté (17M)₅-βG-Luc) où 17M est l'élément de réponse de Gal4. La coopérativité transcriptionnelle entre AF-1 et AF-2 n'existe pas avec ce type de récepteur.

L'utilisation des molécules de référence a permis de vérifier la validité du modèle GAL-RAR pour déterminer la spécificité de molécules agonistes : ce modèle GAL-RAR traduit l'affinité d'un composé pour le domaine de liaison à l'hormone de RAR. L'arotinoïde TTNPB, à la concentration 10⁻⁸ M, est utilisé comme référence de transactivation maximale (100%) obtenue avec un agoniste synthétique. Ainsi TTNPB et ttRA sont de bons agonistes RAR tandis que Am580 se comporte bien comme un RARα spécifique à la concentration de 10 nM. Les composés décrits comme RXR spécifiques (LGD1069 et LGD-CB14499) ne permettent pas d'observer une bonne transactivation médiée par RAR. Le tableau 12 ci-dessous rapporte ces résulats, où l'activité des composés est exprimée en pourcentage de l'activité mesurée pour le TTNPB 10⁻⁸ M.

Le composé désigné Am580 est l'acide 4-[(5,6,7,8-tétrahydro-5,5,8,8-tetraméthyl-2-naphtalényl) carboxamido]benzoïque (Shudo K. et al., J. Med. Chem., 1988, 31, 2182-2192).

**Tableau 12**

| GAL4-RAR | | | | |
|---|---|---|---|---|
| Produit | Concentration (Log M) | RARα (%) | RARβ (%) | RARγ (%) |
| | -8 | 82 | 104 | 106 |
| ttRA | -7 | 102 | 93 | 104 |
| | -6 | 108 | 92 | 111 |
| | -8 | 110 | 22 | 12 |
| Am580 | -7 | 102 | 77 | 64 |
| | -6 | 113 | 111 | 96 |
| | -8 | 100 | 100 | 100 |
| TTNPB | -7 | 101 | 116 | 94 |
| | -6 | 105 | 109 | 112 |
| | -8 | 0 | 1 | 1 |
| LGD1069 | -7 | 7 | 5 | 8 |
| | -6 | 21 | 22 | 29 |
| LGD- | -8 | 1 | 0 | 0 |
| CB14499 | -7 | 4 | 1 | 0 |
| | -6 | 17 | 9 | 13 |

b) Récepteurs chimériques ERcassettes. Les récepteurs chimériques RAR-ERcassettes ont été décrits par Petkovitch et al. (Nature, 1987, 330, 444-450). Les cellules HeLa sont cotransfectées par un plasmide qui code pour un récepteur rétinoïque dans lequel le domaine C de liaison à l'ADN est substitué par celui du récepteur aux estrogènes RE, et un plasmide qui place l'expression de la luciférase sous le contrôle d'un ERE (Elément de Réponse aux estrogènes) . Les récepteurs chimériques exprimés sont désignés comme RARα-ERcassette, RARβ-ERcassette, RARγ-ERcassette et RXRα-ERcassette. Les régions A, B, D, E et F du récepteur naturel sont conservées ainsi que la coopérativité transcriptionnelle entre les domaines d'activation AF-1 et AF-2. Ces expériences de transfections transitoires suivent la méthode de coprécipitation au phosphate de calcium. Les cellules HeLa sont cotransfectées par 0,25 mg de plasmide codant pour le récepteur chimérique, 1 mg de plasmide rapporteur et 0,5 mg de vecteur d'expression CMV-β-galactosidase utilisé comme contrôle interne de transfection. 24 heures après la transfection, les cellules sont incubées pendant 16 heures avec les différents effecteurs.

Dans ce type de récepteur chimérique, le domaine C de liaison à l'ADN de RAR est substitué par celui du récepteur aux estrogènes RE. La protéine obtenue est proche du récepteur RAR naturel et conserve les propriétés transcriptionnelles des domaines AF-1 et AF-2. Elle module la transcription par l'intermédiaire d'un ERE. Ce modèle RAR-ERcassette permet d'observer une réponse transcriptionnelle à un ligand plus physiologique.

L'effet des hormones naturelles (ttRA et 9-cis RA) est remarquable. Ces ligands induisent une surexpression de luciférase en comparaison à l'induction provoquée par un composé synthétique (TTNPB). Ce phénomène est également observé sur d'autres réponses (cf notamment résultats de transactivation sur les modèles HRLN). Cela montre que les constructions RAR-ERcassettes reflètent un contexte physiologique. La spécificité RARα de Am580 est à nouveau constatée pour les concentrations inférieures à 10 nM. Le profil défini en RAR-ERcassette pour les agonistes RXR (LGD1069 et LGD-CB14499) diffère de leur profil GAL-RAR. En comparaison au TTNPB, leur pouvoir transcriptionnel apparaît plus élevé notamment par l'intermédiaire de RARβ-ERcassette. Ce résultat peut s'expliquer par une hétérodimérisation de RAR-ERcassette avec les récepteurs RXR endogènes exprimés par les cellules HeLa transfectées. LGD1069 1 µM permet une coactivation de RAR et de RXR, entraînant une suractivation de l'hétérodimère par rapport à l'activation RAR spécifique du TTNPB. Le modèle RAR-ERcassette traduit donc une activité RAR d'un composé et aussi une activité RXR. Ainsi, toute molécule panagoniste entraîne une transcription maximale supérieure à celle du TTNPB. Cette observation est confirmée par le fait que si une molécule RAR spécifique est associée à une molécule RXR spécifique, une surexpression de luciférase intervient. L'utilisation de la construction RAR-ERcassette permet ainsi de visualiser et mettre en évidence une activité RXR comme rapporté dans le tableau 13 ci-dessous.

**Tableau 13**

| RAR-ERcassettes | | | | |
|---|---|---|---|---|
| Produit | Concentration (LogM) | RARα (%) | RARβ (%) | RARγ (%) |
| ttRA | -9 | 65 | 93 | 86 |
| | -8 | 127 | 128 | 114 |
| | -7 | 249 | 270 | 122 |
| | -6 | 443 | 475 | 189 |
| 9-cis RA | -9 | 7 | 16 | 11 |
| | -8 | 60 | 94 | 56 |
| | -7 | 187 | 212 | 108 |
| | -6 | 348 | 369 | 157 |
| Am580 | -9 | 88 | 6 | 9 |
| | -8 | 113 | 70 | 45 |
| | -7 | 115 | 99 | 93 |
| | -6 | 106 | 89 | 100 |
| TTNPB | -9 | 52 | 93 | 66 |
| | -8 | 100 | 100 | 100 |
| | -7 | 119 | 104 | 97 |
| | -6 | 123 | 116 | 112 |
| LGD1069 | -8 | 14 | 78 | 19 |
| | -7 | 36 | 194 | 41 |
| | -6 | 70 | 237 | 62 |
| LGD- CB14499 | -8 | 4 | 10 | 13 |
| | -7 | 12 | 58 | 31 |
| | -6 | 42 | 130 | 66 |

### 3) Effet des rétinoïdes de référence sur la prolifération estrogéno-induite - Lignées cellulaires MCF-7 et T-47D.

Afin de tester l'effet antiprolifératif des rétinoïdes, des expériences de croissances en conditions estrogéniques sur des cellules MCF-7 et T-47D ont été réalisées. Ce sont des cellules cancéreuses mammaires humaines estrogéno-dépendantes qui expriment le récepteur aux estrogènes RE. L'effet des molécules est évalué après 7 jours de culture en conditions estrogéniques (estradiol 10⁻ ⁹ M) par dosage de l'ADN cellulaire. Les cellules sont distribuées dans des plaques 24 puits à une densité de 2.10⁴ cellules par puits. Les essais avec les différents rétinoïdes sont réalisés en triple et le milieu de culture est changé après 4 jours de croissance. L'ADN cellulaire est mesuré par la méthode du 4,6-diamidino-2-phenylindole (C.F. Brunck et al., Anal Biochem., 1979, 92, 497-500). L'activité des composés est exprimée en pourcentage, 100% représentant la quantité d'ADN mesurée avec l'estradiol 10⁻⁹ M.

Le tableau 14 ci-dessous présente les concentrations de rétinoide nécessaires pour inhiber de 50% la croissance des cellules MCF-7 et T-47D, ou le pourcentage d'inhibition de croissance à la concentration de 1 µM. Les molécules RAR spécifiques (TTNPB et Am580) exercent un effet inhibiteur plus fort que les ligands naturels (ttRA et 9-cis RA) et que LGD1069 (RXR agoniste). Ces résultats confirment ceux rapportés par Dawson et al., Cancer Res., 1995, 55, 446-451, qui ont montré que des RARα agonistes sont des inhibiteurs efficaces de la croissance des cellules MCF-7, et que l'affinité des rétinoïdes pour RARα est étroitement corrélée à leur activité anti-proliférative. A la concentration de 10⁻⁸ M, LGD1069 est RXRα spécifique et n'exerce aucun effet sur la croissance en condition estrogénique des cellules MCF-7 et T-47D.

**Tableau 14**

| EFFET ANTIPROLIFERATIF | | |
|---|---|---|
| Produit | T-47D IC₅₀ (nM) | MCF-7 IC₅₀ (nM) |
| ttRA | 39,1 | 14,1 +/- 10,3 |
| 9-cis RA | 25,1 +/ -1,4 | Non déterminé |
| Am580 | 74% | 67% |
| TTNPB | 3,3 +/- 1,1 | 0,35 +/- 0,07 |
| LGD1069 | 20% | 33% |

Certaines propriétés des rétinoïdes ont été déterminées gràce à des modèles cellulaires plus élaborés. Ces modèles consistent en des lignées cellulaires transfectées de façon stable par des plasmides recombinants qui placent l'expression du gène de la luciférase sous le contrôle de différents éléments de réponse nucléaire. Les effets observés correspondent alors à des régulations physiologiques et à une activité des récepteurs endogènes. Les essais réalisés en double sont décrits ci-dessous pour chacun de ces modèles.

### 4) Activité transactivatrice des rétinoïdes de référence médiée par les récepteurs de l'acide rétinoïque-Lignées cellulaires HRLN et HRL⁺N transfectées de facon stable.

Les lignées cellulaires HRLN et HRL⁺N permettent d'étudier l'activation d'un RARE par des récepteurs endogènes en utilisant des ligands à concentrations physiologiques. Ces lignées dérivent de cellules HeLa transfectées de façon stable par un gène rapporteur qui place l'expression du gène de la luciférase sous le contrôle d'un élément de réponse nucléaire RARE (RARE₃-tk-Luc). Les cellules HeLa expriment tous les récepteurs de l'acide rétinoïque connus (RARα,β,γ et RXRα,β,γ) avec une prédominance de RARα et RXRα. L'élément de réponse RARE utilisé pour les cellules HRLN correspond à la séquence du gène naturel du récepteur RARβ (GGTTCAnnnnnAGTTCA). Les cellules HRL⁺N comportent la séquence GAGTGAnnnnnCGGTGA.

### a) Lignée HRLN.

Cette lignée comporte l'élément de réponse RARE du gène naturel du récepteur RARβ qui contrôle l'expression du gène de la luciférase. ttRA et 9-cis RA induisent une activation dose-dépendante. Une suractivation par rapport au TTNPB est observée à forte concentration (1 µM), comparable à celle observée avec les constructions ERcassettes. Une coactivation de RAR et de RXR au niveau de l'hétérodimère est certainement impliquée.

Les résultats concernant TTNPB et Am580 indiquent l'activation induite spécifiquement par les récepteurs RAR. Les EC₅₀ de ces deux molécules sont similaires. Am580 induit une transactivation médiée par RARα et TTNPB par RARα,γ comme le montre l'utilisation de l'antagoniste RARα Ro 41-5253 (Apfel, C. et al., PNAS, USA, 1992, 89, 7129-7133) qui abolit totalement la réponse de Am580 et partiellement celle du TTNPB. Cependant, RARα apparaît comme le récepteur prédominant pour la transactivation dans les cellules HeLa. Le ligand RXRα spécifique LGD1069 transactive avec une EC₅₀ de ~10 nM, ce qui correspond à son activité RXR. La lignée HRLN permet de mettre clairement en évidence une activité RAR physiologique des composés, mais une faible activité RXR est observée. Le tableau 15 ci-dessous rapporte ces résultats où le 100% d'expression correspond à l'induction provoquée par TTNPB 10⁻⁸ M. Les EC₅₀ sont déterminées à partir des résultats obtenus avec une gamme de concentrations allant de 1 nM à 1 µM.

**Tableau 15**

| TRANSACTIVATION HRLN | | |
|---|---|---|
| Produit | E max % | EC₅₀ (nM) |
| ttRA | 208 | 2,5 +/- 4 |
| 9-cis RA | 196 | non déterminé |
| Am580 | 104 | 0,10 +/- 0,06 |
| TTNPB | 100 | 0,55 +/- 0,72 |
| LGD1069 | 73 | 9,4 +/- 6,3 |
| LGD-CB14499 | 30 | non déterminé |

### b) Lignée HRL+N.

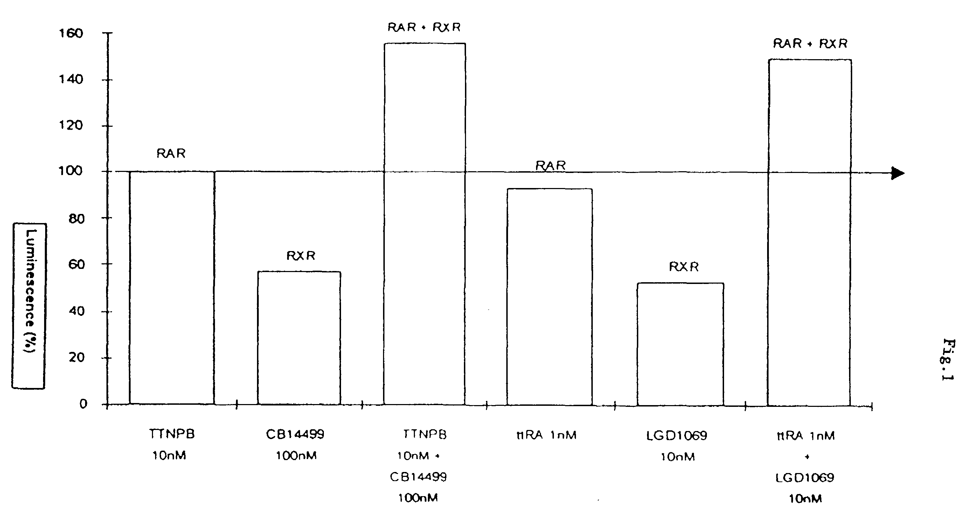

Les résultats de transactivation obtenus avec la lignée HRL⁺N sont comparables à ceux obtenus avec la lignée HRLN pour les molécules RAR agonistes (TTNPB et Am580) et les ligands naturels (ttRA et 9-cis RA). Les agonistes RXR (LGD1069 et LGD-CB14499) induisent une transactivation plus forte avec les cellules HRL⁺N et LGD1069 1 µM est plus efficace que les molécules RAR spécifiques. De plus, l'association d'un agoniste RAR et d'un agoniste RXR (par exemple TTNPB + LGD-CB14499, figure 1 en annexe) permet une meilleure transactivation que celle provoquée par chaque molécule utilisée séparément. La lignée HRL⁺N permet de visualiser une coactivation des récepteurs RAR et RXR au niveau de l'hétérodimére RAR-RXR. Ainsi, LGD1069 1 µM ayant une activité RAR à cette concentration se comporte comme un panagoniste. Ce résultat est à corréler à la suractivation induite par les molécules RXR agonistes avec les modèles RAR-ERcassettes. La lignée HRL⁺N permet clairement de mettre en évidence une activité RAR et RXR des molécules. Ces résultats sont rapportés dans le tableau 16 ci-dessous où l'activité transcriptionnelle des produits est exprimée en pourcentage, 100% correspondant au niveau d'activité mesuré en présence de TTNPB 10⁻⁸ M.

**Tableau 16**

| TRANSACTIVATION HRL+N | |
|---|---|
| Produit (10⁻⁶ M) | Emax (%) |
| ttRA | 225 |
| Am580 | 99 |
| TTNPB (10⁻⁸ M) | 100 |
| LGD1069 | 131 |
| LGD-CB14499 | 94 |

La figure 1 en annexe montre la suractivation induite par une molécule RXR sélective en présence d'un agoniste RAR spécifique sur le modèle HRL+N.

### 5) Effet anti-AP-1 des rétinoïdes de référence sur des cellules estrogéno-dépendantes activées par le TPA-Lignée cellulaire MTLN.

L'effet transrepresseur anti-facteur AP-1 des rétinoïdes est déterminé à l'aide de la lignée MTLN, issue de cellules MCF-7 transfectées de façon stable par un vecteur p(TRE)3-tk-Luc qui place l'expression du gène de la luciférase sous le contrôle du TPA (12-O-tétradécanoyl-phorbol-13-acétate). Le TPA active le complexe AP-1 formé des protéines de la famille des protooncogènes nucléaires (c-Jun et c-Fos). Cette lignée MTLN permet d'étudier la relation existant entre les voies estrogéniques et AP-1 (M.E. Astruc et al., Endocrinology, 1995, 136, 824-832), et de montrer la dissociation entre l'activité transactivatrice et l'effet anti-AP-1 de rétinoïdes de synthèses (J.Y. Chen et al., EMBO J., 1995, 14, 1187-1197). Les expériences se font avec des cellules MTLN activées par du TPA 10⁻⁷ M.

Les résultats rapportés dans le tableau 17 ci-dessous montrent que les 3 types de récepteurs de l'acide rétinoïque exprimés par les cellules MCF-7 (RARα,γ et RXRα) médient un effet inhibiteur de la voie AP-1. L'utilisation de l'antagoniste RARα sélectif Ro 41-5253 ne permet pas de lever totalement l'inhibition induite par le TTNPB, ce qui indique que l'activation de RARγ médie un effet anti-AP-1. L'activation par un agoniste RARα (Am580 10 nM) ou RXRα (LGD1069 et LGD-CB14499) entraîne également une inhibition de la voie AP-1. L'association d'une molécule RAR spécifique et d'une molécule RXR spécifique provoque un fort effet inhibiteur anti-AP-1, il y a effet additif entre les deux voies d'inhibition. De part son profil panagoniste à 1 µM, LGD1069 apparaît comme le composé le plus efficace testé dans 'ce modèle.

**Tableau 17**

| EFFET ANTI-AP-1 | | |
|---|---|---|
| Produit | Concentration (Log M) | Inhibition (%) |
| ttRA | -8 | 22 - 33 |
| | -7 | 28 - 44 |
| 9cisRA | -8 | 30 - 37 |
| Am580 | -8 | 40 - 46 |
| | -7 | 41 - 48 |
| TTNPB | -8 | 26 - 53 |
| | -7 | 29 - 54 |
| LGD1069 | -8 | 18 - 29 |
| | -7 | 48 - 62 |
| LGD-CB14499 | -7 | 17 - 30 |

L'effet des composés sur des cellules MTLN activées par le TPA 10⁻⁷ M est exprimé en pourcentage. 100% représente le niveau d'activité maximale mesuré avec le TPA qui est 5 à 6 fois supérieur à l'activité basale des cellules. Les propriétés inhibitrices des produits sont calculées après déduction de l'activité basale des cellules. Les pourcentages rapportés correspondent à une fourchette d'inhibitions déterminées à partir de plusieurs expérimentations.

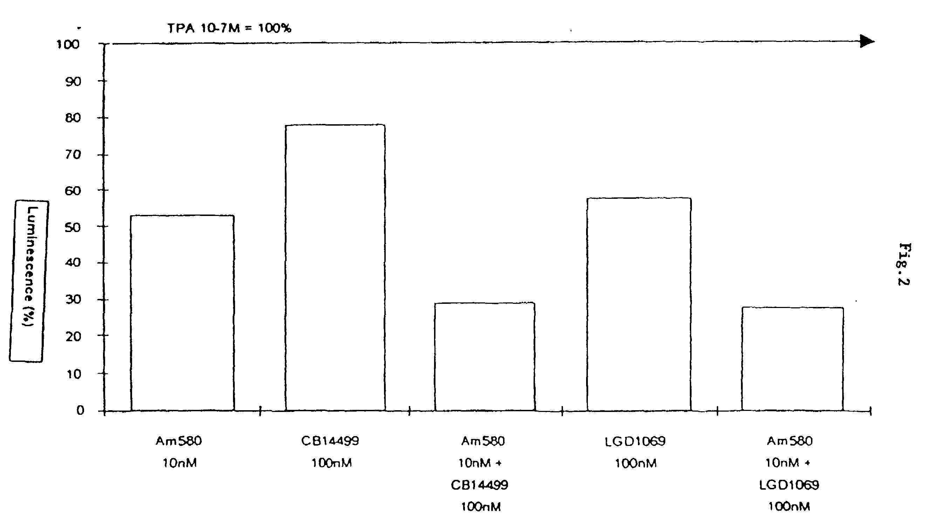

La figure 2 en annexe montre l'additivité de l'effet inhibiteur anti-AP-1 sur la lignée MTLN d'un agoniste RXR et d'un agoniste RAR sélectif.

### 6) Effet anti-estrogénique des rétinoïdes de référence sur des cellules estrogéno-dépendantes activées par l'estradiol - Lignée MELN.

Les cellules MCF-7 expriment RARα,γ et RXRα. La transfection de cellules MCF-7 par un gène estrogéno-dépendant (ERE-βGlob-Luc) a permis d'établir la lignée cellulaire MELN, utilisée pour déterminer l'activité anti-estrogénique des rétinoïdes. Ces cellules contiennent le gène de la luciférase sous le contrôle transcriptionnel du promoteur de la βGlobine et de l'élément de réponse ERE isolé du gène de la vitélogénine A2 de poulet. Les expériences sont réalisées avec des cellules MELN activées par de l'estradiol 10⁻⁹ M. Le niveau d'activité basale des cellules MELN est obtenu avec l'antiestrogène 4-OH-tamoxifène (hydroxytamoxifène) 1 µM. Ce niveau est toujours de 8 à 10 fois plus bas que l'activité maximale de référence mesurée en présence d'estradiol 10⁻⁹ M qui représente 100%. ttRA réduit la croissance cellulaire ainsi que l'expression de gènes estrogéno-dépendants (E. Demirpence et al., Cancer Res., 1994, 54, 1458-1464), ce que cette lignée MELN permet de bien vérifier.

Les molécules RAR sélectives (Am580 et TTNPB) induisent une inhibition de l'ordre de 40%. Am580 à une concentration RARα spécifique (10 nM) permet une inhibition maximale, ce qui indique que RARα médie l'effet antiestrogénique dans les cellules MCF-7. L'utilisation de l'antagoniste RARα Ro 41-5253 lève l'effet inhibiteur de Am580 mais aussi du TTNPB qui, dans ces conditions, conserve une activité RARγ et perd son activité RARα. Les composés RXR sélectifs (LGD1069 et LGD-CB14499) sont inactifs. L'activation de RXR n'est donc pas impliquée dans l'inhibition de la voie estrogénique par les rétinoïdes dans les cellules MCF-7.

Ces résultats sont rapportés dans le tableau 18 ci-dessous, ils sont parfaitement corrélés aux expériences de prolifération cellulaire estrogéno-induites. L'association d'un agoniste RAR spécifique et d'un agoniste RXR spécifique ne permet pas d'observer un effet inhibiteur supérieur à celui exercé par l'agoniste RAR seul.

**Tableau 18**

| EFFET ANTI- ESTROGENIQUE | | |
|---|---|---|
| Produit | Concentration (Log M) | Inhibition (%) |
| ttRA | -7 | 35 |
| 9-cis RA | -7 | 35 |
| Am580 | -8 | 36-51 |
| TTNPB | -8 | 30-41 |
| | -8 | 0 |
| LGD1069 | -7 | 0 |
| LGD- | -7 | 0 |
| CB14499 | -6 | 0 |

L'effet des produits testés sur des cellules activées par l'estradiol 10⁻⁹ M est exprimé en pourcentage, le niveau d'activité basale étant retranché.

### 6) Conclusion.

Les molécules de référence ont permis de montrer l'efficacité et la complémentarité des différents modèles utilisés et conduisent aux conclusions suivantes :
- les constructions chimériques GAL-RAR et RAR-ERcassette permettent de déterminer le profil RAR agoniste de molécules, mais aussi une activité RXR agoniste avec RAR-ERcassette,
- la lignée HRLN traduit principalement l'activité d'un composé médiée par RARα endogène,
- la lignée HRL+N met également en évidence une activité RXR agoniste des rétinoïdes,
- l'effet anti-estrogénique des rétinoïdes (prolifération cellulaire estrogéno-induite et lignée MELN) est médiée par RARα,
- l'effet anti-AP-1 est médié dans les cellules MCF-7 (lignée MTLN) par RARα, RARγ et RXRα, et un effet additif existe entre les voies RAR et RXR.

### B) Activités des dérivés du type rétinoide de série trans (E).

Les résultats obtenus avec les molécules de la série (E) sont à comparer à ceux présentés pour les molécules de référence et pour la molécule SRI3946 (CB24159). Ce composé est RAR sélectif.

### 1) Spécificité des dérivés testés.

L'activité RAR agoniste de composés de la série (E) a été évaluée avec le modèle RAR-ERcassette. A une concentration de 1 µM, les molécules de l'invention de cette série permettent une transactivation médiée par les trois types de récepteurs RAR. Leur activité est rapportée dans le tableau 19 ci-dessous et, à cette concentration, apparait comparable à celle du TTNPB.

**Tableau 19**

| RAR-ERcassettes | | | | |
|---|---|---|---|---|
| Produit | Concentration (Log M) | RARα (%) | RARβ (%) | RARγ (%) |
| TTNPB | -8 | 100 | 100 | 100 |
| CB24159 | -6 | 113 | 117 | 89 |
| CB73364 | -6 | 74 | 118 | 78 |
| CB12273 | -6 | 90 | 123 | 106 |
| CB57201 | -6 | 73 | 132 | 84 |

### 2) Effet des dérivés de la série trans (E) sur la prolifération estrogéno-induite - Lignées cellulaires MCF-7 et T-47D.

Le tableau 20 ci-dessous rapporte l'effet des rétinoïdes sur la croissance des cellules MCF-7 et T-47D qui est évaluée après 7 jours de culture en présence d'estradiol 10⁻⁹ M par dosage de l'ADN cellulaire. La molécule CB24159 inhibe la croissance des cellules T-47D de manière dose dépendante avec une efficacité supérieure à celle du TTNPB. CB73364 exerce un effet comparable au TTNPB. Les autres molécules induisent une inhibition de l'ordre de 50% à la concentration de 0,3 µM qui correspond à leur activité RARα observée en spécificité à cette concentration (cf. modèle RAR-ERcassette).

**Tableau 20**

| EFFET ANTIPROLIFERATIF | | |
|---|---|---|
| Produit | Concentration (Log M) | T-47D |
| CB24159 | | 1,4 +/- 0,2 |
| CB73364 | | 4,1 +/- 0,7 |
| CB71802 | -6 | 72% |
| CB12273 | | 252,8 +/- 109,7 |
| CB57201 | | 233,0 +/- 90, 6 |
| CB38973 | -6 | 55% |

### 3) Activité transactivatrice des dérivés trans (E) médiée par les récepteurs de l'acide rétinoïque-Lignées cellulaires HRLN.

Comme indiqué dans le tableau 21 ci-dessous, les modifications structurales effectuées sur les molécules de la série trans (E) entraînent une diminution d'efficacité importante de ces molécules en transactivation. Cependant, à 1 µM, elles permettent une induction comparable à celle du TTNPB et de CB24159. Cette activité correspond au profil RAR de ces molécules déterminé avec le modèle RAR-ERcassette.

**Tableau 21**

| TRANSACTIVATION HRLN | | |
|---|---|---|
| Produit | E max % | EC₅₀ (nM) |
| TTNPB | 100 | 0,55 +/- ,072 |
| CB24159 | 100 | 0,08 +/- 0,10 |
| CB73364 | 89 | 33 |
| CB71802 | 76 | 31,4 +/- 16,9 |
| CB12273 | 106 | 21,4 +/- 5,6 |
| CB57201 | 70 | 55,8 +/- 70,1 |
| CB80660 | 106 | 38,3 +/- 9,5 |
| CB38973 | 89 | 14,9 +/- 12,3 |

### 4) Effet anti-estrogénique des dérivés trans (E) sur des cellules estrogéno-dépendantes activées par l'estradiol - Lignée MELN.

Comme indiqué dans le tableau 22 ci-dessous, le dérivé CB24159 est le composé le plus inhibiteur de la transcription estrogénique sur le modèle MELN. Son activité est parfaitement corrélée à ses propriétés transcriptionnelles médiées par RAR dans la lignée HRLN. Comme pour CB24159, le maximum d'inhibition provoqué par les molécules trans (E) est de l'ordre de 30 à 40%. L'effet anti-estrogénique des rétinoides étant médié par RARα dans les cellules MCF-7, ces composés exercent leur inhibition par l'intermédiaire de ce récepteur, ce qui correspond à leur pouvoir transcriptionnel RARα médié (cf. modèles HRLN et RARα-ERcassette).

**Tableau 22**

| EFFET ANTI-ESTROGENIQUE | | |
|---|---|---|
| Produit | Concentration (Log M) | Inhibition (%) |
| | -8 | 31-43 |
| CB24159 | -7 | 26-43 |
| | -6 | 22-37 |
| | -8 | 13 |
| CB73364 | -7 | 24 |
| | -6 | 20-41 |
| | -8 | 5 |
| CB71802 | -7 | 37 |
| | -6 | 32-48 |
| | -8 | 0 |
| CB12273 | -7 | 7-16 |
| | -6 | 21-41 |
| | -8 | 0 |
| CB57201 | -7 | 1-9 |
| | -6 | 23-41 |
| CB38973 | -6 | 30 |
| | -8 | 26 |
| CB80660 | -7 | 20-42 |
| | -6 | 18-37 |

### C) Activités des dérivés du type rétinoïde de l'invention de série cis (Z).

Ces dérivés se divisent en deux familles :
- les molécules carboxylées (substituant R₁),
- les molécules dont le carboxyle est remplacé par un tétrazole.

### 1) Spécificité des dérivés cis (Z).

Les deux types de constructions (Gal-RAR et RAR-ERcassette) ont été utilisé pour mettre en évidence l'intérêt de cette série.

### a) Récepteurs chimériques Gal4-RAR

Les constructions Gal-RAR ont permis d'évaluer l'activité RAR agoniste des dérivés de la série cis (Z). Les résultats rapportés dans le tableau 23 ci-dessous montrent que la molécule de référence SRI (CB28628) est agoniste complet pour les trois types de récepteurs RAR et permet la même transactivation maximale que TTNPB (RAR agoniste sélectif). Les molécules dérivées de CB28628 apparaissent moins efficaces. Les molécules carboxylées (CB36493 et CB16279) sont des agonistes partiels à 1 µM pour les trois récepteurs RAR tandis que CB38416 est agoniste partiel pour RARα et RARβ, mais complet pour RARγ. Ces molécules ont la propriété d'induire une transactivation RAR médiée. Les composés comportant un tétrazole (CB92834 et CB77402) sont incapables de transactiver par l'intermédiaire des récepteurs RAR. Ils ne sont pas RAR agonistes.

**Tableau 23**

| GAL-RAR | | | | |
|---|---|---|---|---|
| Produit | Concentration (Log M) | RARα (%) | RARβ (%) | RARγ (%) |
| TTNPB | -8 | 100 | 100 | 100 |
| SRI (CB28628) | -7 | 103 | 102 | 106 |
| CB38416 | -6 | 58 | 51 | 99 |
| CB36493 | -6 | 38 | 44 | 50 |
| CB16279 | -6 | 33 | 47 | 59 |
| CB92834 | -6 | 8 | 0 | 2 |
| CB77402 | -6 | 6 | 0 | 0 |

### b) Récepteurs chimériques ERcassettes

Comme rapporté dans le tableau 24 ci-dessous, le profil défini avec le modèle RAR-ERcassette pour les dérivés cis (Z) diffère du profil Gal-RAR. TTNPB 10 nM présente la transactivation maximale induite par un agoniste synthétique RAR sélectif. SRI (CB28628) est agoniste complet pour RARγ-ERcassette et induit une suractivation de RARα-ERcassette et RARβ-ERcassette à 1 µM. Cette suractivation traduit des propriétés panagonistes (RXR et RAR agoniste). Les composés carboxylés (CB30382, CB38416, CB36493 et CB16279) induisent une transactivation médiée par les trois récepteurs. Une suractivation de RARβ-ERcassette intervient avec ces molécules, ce qui traduit une activité RXR. Les dérivés cis (Z) carboxylés sont aussi des panagonistes. Les molécules comportant un tétrazole (CB92834 et CB77402) ne permettent pas de transactivation médiée par RARα-ERcassette et RARγ-ERcassette. Cependant, une transactivation partielle de RARβ-ERcassette est constatée. Dans la mesure où ces composés sont inactifs sur Gal-RARβ, cet effet agoniste partiel peut être attribué à une activité RXR. De plus, l'association de CB92834 ou de CB77402 avec un agoniste RAR spécifique permet un suractivation de l'effet de la molécule RAR seule pour les trois récepteurs RAR-ERcassettes, ce qui tend à confirmer l'activité RXR agoniste de CB92834 et de CB77402.

**Tableau 24**

| RAR-ERcassettes | | | | |
|---|---|---|---|---|
| Produit | Concentration (Log M) | RARα (%) | RARβ (%) | RARγ (%) |
| TTNPB | -8 | 100 | 100 | 100 |
| | -8 | 35 | 88 | 55 |
| SRI (CB28628) | -7 | 95 | 115 | 87 |
| | -6 | 179 | 183 | 92 |
| CB30382 | -7 | 12 | 39 | 29 |
| | -6 | 93 | 130 | 83 |
| | -8 | 0 | 10 | 4 |
| CB38416 | -7 | 7 | 57 | 14 |
| | -6 | 65 | 198 | 62 |
| | -8 | 1 | 0 | 0 |
| CB36493 | -7 | 15 | 34 | 5 |
| | -6 | 91 | 205 | 26 |
| CB16279 | -7 | 6 | 60 | 15 |
| | -6 | 60 | 118 | 71 |
| | -8 | 0 | 0 | 2 |
| CB92834 | -7 | 9 | 36 | 7 |
| | -6 | 11 | 43 | 0 |
| | -8 | 0 | 0 | 0 |
| CB77402 | -7 | 1 | 31 | 0 |
| | -6 | 8 | 37 | 8 |

### 2) Effet des molécules de la série cis (Z) sur la prolifération estrogéno-induite - Lignées cellulaires MCF-7 et T- 47D.

Le tableau 25 ci-dessous rapporte l'effet des dérivés cis (Z) sur la croissance des cellules T-47D qui est évaluée après 7 jours de culture en présence d'estradiol 10⁻⁹ M par dosage de l'ADN cellulaire. Les molécules carboxylées induisent une inhibition de l'ordre de 20 à 50% qui correspond à leur profil FARα agoniste partiel déterminé en transfections transitoires (Gal-RAR et RAR-ERcassette). SRI (CB28628) agoniste complet, permet une inhibition de l'ordre de 70%, comparable à celle exercée par Am580. CB92834 est inactif sur une croissance estrogéno-induite.

**Tableau 25**

| EFFET ANTIPROLIFERATIF T-47D | | |
|---|---|---|
| PRODUIT | Concentration (Log M) | Croissance 100% = E2 10⁻⁹ M |
| SRI (CB28628) | -6 | 33 |
| CB36493 | -6 | 68-78 |
| CB30382 | -6 | 73-94 |
| CB38416 | -6 | 50-60 |
| CB16279 | -6 | 84 |
| CB92834 | -6 | 94 |

### 3) Activité transactivatrice des dérivés cis (Z) médiée par les récepteurs de l'acide rétinoïque - Lignées cellulaires HRLN et HRL+N.

### a) Lignée HRLN.

L'activité RAR agoniste des dérivés cis (Z) carboxylés (CB38416, CB30382, CB36493 et CB16279) constatée avec les modèles chimériques est confirmée avec le modèle HRLN, comme rapporté dans le tableau 26 ci-dessous. A 1 µM, ces composés induisent une transactivation proche de celle du TTNPB. Cependant, les modifications structurales effectuées sur ces dérivés entraînent, par rapport à la référence SRI (CB28628) une diminution d'affinité, et donc d'efficacité RAR médiée. Les composés comportant un tétrazole (CB92834 et CB77402) sont trés faiblement actifs, ce qui correspond à leur activité en RAR-ERcassette.

**Tableau 26**

| TRANSACTIVATION HRLN | |
|---|---|
| Produit | E max % |
| TTNPB | 100 |
| SRI (CB28628) | 98 |
| CB30382 | 102 |
| CB38416 | 81 |
| CB36493 | 97 |
| CB92834 | 21 |
| CB77402 | 18 |

### b) Lignée HRL+N.

La lignée HRL+N permet de mettre en évidence une activité RXR agoniste. Les résultats rapportés dans le tableau 27 ci-dessous sont corrélés à ceux obtenus avec le modèle RAR-ERcassette. SRI (CB28628) est une molécule panagoniste. Elle induit un maximum de transactivation supérieur à celui du TTNPB. Les composés de la série cis (Z) carboxylés (CB38416, CB16279) apparaissent comme des panagonistes, en accord avec les résultats constatés en RAR-ERcassette, et provoquent une transactivation supérieure aux agonistes RAR sélectifs (TTNPB). Les molécules CB (Z) non cycliques comportant un tétrazole (CB92834, CB77402) permettent d'observer une faible transactivation, inférieure à celle du TTNPB. L'association de CB92834 ou de CB77402 avec un agoniste RAR spécifique induit une transactivation plus forte que celle observée pour chaque composé testé séparément, ce qui correspond au profil RXR agoniste de CB92834 et CB77402.

**Tableau 27**

| TRANSACTIVATION HRL+N | |
|---|---|
| Produit | E max % |
| TTNPB | 100 |
| SRI-CB28628 | 158 |
| CB38416 | 131 |
| CB92834 | 42 |
| CB77402 | 33 |

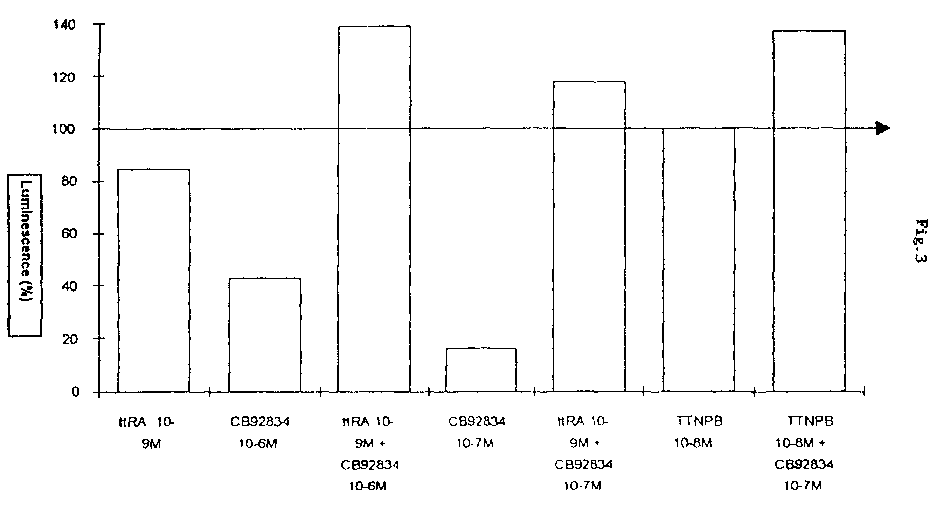

La figure 3 en annexe représente la suractivation par CB92834 de la transactivation induite par un agoniste RAR sélectif sur la lignée HRL+N.

### 4) Effet anti-AP-1 des dérivés cis (Z) sur des cellules estrogéno-dépendantes activées par le TPA - Lignée cellulaire MTLN.

La lignée MTLN activée par le TPA permet d'étudier l'effet des dérivés cis (Z) sur la voie AP-1. L'interprétation des résultats rapportés dans le tableau 28 ci-dessous est basée sur les conclusions faites à partir des effets observés avec les molécules de références. Les activations de RARα, RARγ et RXRα médient un effet anti-AP-1 dans les cellules MCF-7 et il existe une additivité des effets de RAR et RXR. CB28628 présente un fort effet anti-AP-1 sur les cellules MCF-7. Comme LGD1069, cette molécule exerce une forte inhibition de part ses propriétés panagonistes. Toutes les molécules de la série (Z) présentent un profil inhibiteur de la voie AP-1. Les composés carboxylés (CB36493 et CB30382) à la concentration de 1 µM exercent un effet inhibiteur comparable à celui de l'effet de CB28628. Ces dérivés panagonistes (cf. résultats RAR-ERcassettes et HRL+N) induisent un effet anti-AP-1 par activation des récepteurs RAR et RXR. CB92834 et CB77402 présentent également un profil inhibiteur. L'inhibition mesurée est de l'ordre de 20 à 30%, comparable à celle constatée pour LGD1069 10⁻⁸M (RXR sélectif à cette concentration). CB92834 et CB77402 sont des molécules anti-AP-1 par l'intermédiaire de RXR. En association avec un agoniste RAR sélectif, on observe un effet anti-AP-1 additif. Ces deux composés présentent donc l'avantage d'exercer une transrépression de la voie AP-1 sans induire une activation de la transcription d'un gène contrôlé par un RARE.

**Tableau 28**

| EFFET ANTI-AP-1 MTLN | | |
|---|---|---|
| Produit | Concentration (Log M) | Inhibition (%) |
| TTNPB | -8 | 26-53 |
| CB28628 | -7 | 40 |
| | -6 | 58 |
| CB30382 | -7 | 21 |
| | -6 | 42-47 |
| CB38416 | -7 | 7-14 |
| | -6 | 21-25 |
| CB36493 | -7 | 26 |
| | -6 | 38-46 |
| CB16279 | -6 | 23-26 |
| CB92834 | -7 | 4-9 |
| | -6 | 20-23 |
| CB77402 | -7 | 21-23 |
| | -6 | 30-33 |

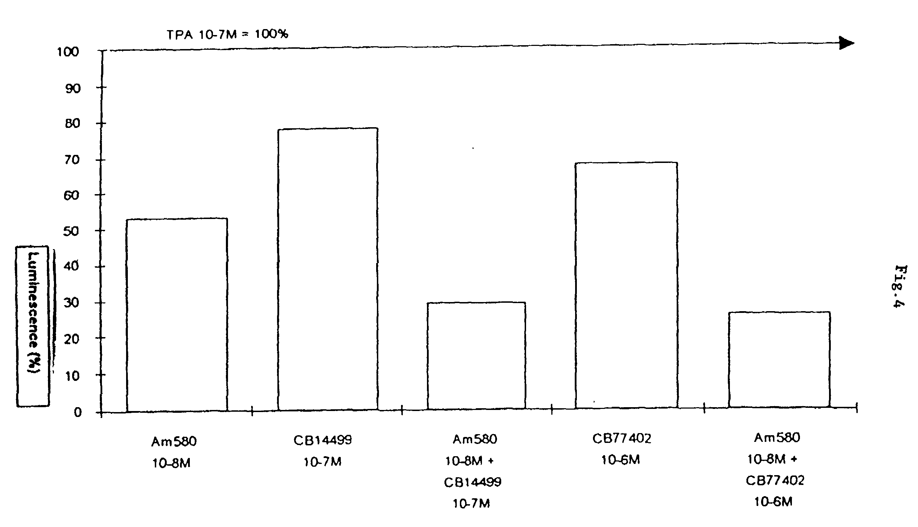

La figure 4 représente l'additivité de l'effet inhibiteur anti-AP-1 sur la lignée MTLN de CB77402 et de Am580.

### 5) Effet anti-estrogénique des dérivés cis (Z) sur des cellules estrogéno-dépendantes activées par l'estradiol - Lignée MELN.

L'utilisation des molécules de référence a permis de montrer que l'effet anti-estrogénique des rétinoides est médié par RARα. L'action anti-estrogénique des dérivés cis (Z) évaluée avec la lignée MELN, activée par l'estradiol 10⁻⁹ M, suit leur profil RARα. Comme rapporté dans le tableau 29 ci-dessous, les dérivés carboxylés (CB36493, CB38416, CB30382 et CB16279), partiellement agonistes, permettent une transrépression d'un gène contrôlé par un ERE. CB92834 et CB77402 sont inactifs, ce qui est en parfait accord avec leur incapacité à activer RARα.

**Tableau 29**

| EFFET ANTI-ESTROGENIQUE MELN | | |
|---|---|---|
| Produit | Concentration (Log M) | Inhibition (%) |
| TTNPB | -8 | 30-41 |
| SRI (CB28628) | -7 | 31 |
| | -6 | 29-41 |
| CB30382 | -7 | 0 |
| | -6 | 2-20 |
| CB38416 | -7 | 0 |
| | -6 | 19 |
| CB36493 | -7 | 0 |
| | -6 | 17-32 |
| CB73069 | -6 | 17-21 |
| CB16279 | -6 | 32 |
| CB92834 | -6 | 0 |
| CB77402 | -6 | 0 |

### 6) Conclusion.

Les dérivés de l'invention de série cis (Z) présentent des propriétés très intéressantes. Les composés CB92834 et CB77402 présentent une activité RXR spécifique, en comparaison aux molécules cis (Z) carboxylées qui sont panagonistes(CB36493, CB30382, CB38416, CB16279). Ainsi, la substitution du carboxyle par un radical tétrazole entraîne une perte d'activité RAR et permet d'orienter ces molécules vers une spécificité RXR. Ces composés exercent un effet inhibiteur sur la voie AP-1 et sont inactifs sur la transcription d'un gène contrôlé par un RARE. Leur association avec une molécule RAR spécifique permet d'optimiser l'effet anti-AP-1 de ces molécules.

D'autres caractéristiques et avantages de l'invention apparaîtront à la lecture des exemples qui suivent concernant la préparation et l'analyse de composés de référence et de dérivés de l'invention, étant entendu que ces exemples ne sauraient être interprétés comme tendant à réduire la portée des revendications.

### Exemple 1 : Préparation de l'acide (E) 4-[1-(5,6,7,8-tétrahydro-3,5,5,8,8-pentaméthyl-2-naphtalényl)-2-propényl]benzoïque (CB71802) de formule :

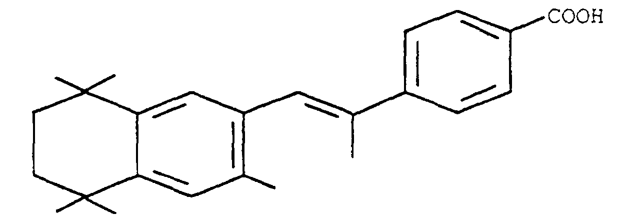

### a) Préparation du 2,5-dichloro-2,5-diméthylhexane:

A une solution de 2,5-diméthyl-2,5-hexanediol (20,0 g, 136,8 mmol) dans 250 ml de dichlorométhane est additionné à l'ambiante du chlorure de thionyle (25 ml, 341,8 mmol). Le milieu réactionnel jaunit et on poursuit l'agitation pendant 4 h. L'avancement de la réaction est contrôlé par chromatographie sur couche mince (CCM) (éluant éther:éther de pétrole = 50:50). En fin de réaction, on additionne 250 ml d'eau distillée, décante et la phase organique est neutralisée par 2 x 250 ml d'une solution de NaHCO₃ 10%. On sèche alors par MgSO₄, filtre et évapore à sec. On obtient 18,37 g (Rdt brut = 73%) d'un solide le 2,5-dichloro-2,5-diméthylhexane.

RMN¹H 200 MHz (CDCl₃) : 1,57 (s, 12H, Me); 1,92 (s, 4H, -CH₂-).

### b) Préparation du 1,1,4,4,6,7-hexaméthyl-1,2,3,4-tétrahydronaphtalène :

Dans un tricol de 250 ml muni d'un réfrigérant, d'une agitation magnétique et d'un thermomètre, on pèse du trichlorure d'aluminiun (3,0 g, 22,5 mmol). On additionne 70 ml de o-xylène (Aldrich) puis avec une ampoule à brome on additionne une solution de 2,5-dichloro-2,5-diméthylhexane préparé à l'étape précédente (15,00 g, 81,9 mmol) dans 30 ml de o-xylène. On chauffe à 100°C le milieu réactionnel pendant 3 h, il y a un important dégagement de HCl et le milieu réactionnel devient rouge sombre. Après refroidissement du milieu réactionnel, celui-ci est versé sur un mélange eau-glace (200 ml), on extrait par de l'éther (3 x 80 ml), lave par une solution saturée de NaHCO₃ (2 x 150 ml), sèche la phase éthérée par MgSO₄, filtre et évapore. Le produit brut est purifié par distillation sous vide, on obtient 15,20 g d'un solide le 1,1,4,4,6,7-hexaméthyl-1,2,3,4-tétrahydronaphtalène (Rdt = 86%).

RMN¹H 200MHz (CDCl₃) : 1,31 (s, 12H, 1-Me et 4-Me); 1,71 (s , 4H, -CH₂-); 2,26 (s, 6H, 6 et 7 Me); 7,11 (s, 2H, ArH).

### c) Préparation du 6-bromométhyl-1,1,4,4,7-pentaméthyl-1,2,3,4-tétrahydronaphtalène de formule :

Une solution de 1,1,4,4,6,7-hexaméthyl-1,2,3,4-tétrahydronaphtalène (6,91 g, 31,9 mmol), de NBS (5,97 g, 33,5 mmol), de peroxyde de benzoyle (0,22 g, 0,9 mmol) dans 100 ml de CCl₄ est portée à reflux pendant 5 h. La solution est alors refroidie, concentrée à l'évaporateur rotatif, reprise à l'éther (100 ml), filtrée pour éliminer le succinimide formé (le succinimide étant lavé à l'éther) et concentrée. On obtient 8,80 g d'un produit brut composé (RMN¹H 200MHz) d'environ 30 % de 1,1,4,4,6,7-hexaméthyl-1,2,3,4-tétrahydronaphtalène, d'environ 65 % de 6-bromométhyl-1,1,4,4,7-pentaméthyl-1,2,3,4-tétrahydronaphtalène désiré et d'environ 5 % de composé dibromé correspondant. Ce produit brut sera utilisé tel quel pour la formation du sel de phosphonium correspondant.

RMN¹H 200MHz (CDCl₃) : 1,27 (s, 12H, 5,8-CH₃); 1,67 (s , 4H, 6,7-CH₂-); 2,36 (s, 3H, ArMe); 4,50 (s, 2H, BnzH); 7,05 (s, 1H, ArH ortho Me); 7,22 (s, 1H, ArH méta Me).

### d) Préparation du bromure de (5, 6,7,8-tétrahydro-3,5,5,8,8-pentaméthyl-2-naphtalényl)-méthyl triphénylphosphonium de formule :

Le 6-bromométhyl-1,1,4,4,7-pentaméthyl-1,2,3,4-tétrahydronaphtalène brut (8,80 g, environ 32 mmol) préparé précédemment est mélangé avec de la triphénylphosphine (10,1 g, 38,4 mmol) en solution dans 50 ml de dichlorométhane pendant 24 h, puis dilué à l'éther éthylique (200 ml). Le bromure de [(5,6,7,8-tétrahydro-3,5,5,8,8-pentaméthyl-2-naphtalényl)méthyl] triphénylphosphonium précipite, est filtré et lavé abondamment à l'éther. On obtient 10,72 g de cristaux blancs, le bromure de (5,6,7,8-tétrahydro-3,5,5,8,8-pentaméthyl-2-naphtalényl)-méthyl-triphénylphosphonium (Rdt = 60%) après séchage. RMN¹H 200MHz (CDCl₃) : 0,74 (s, 6H, 5-CH₃) ; 1,15 (s, 6H, 8-CH₃); 1,52 (s large, 2H, 6-CH₂); 1,56 (s large, 2H, 7-CH₂); 1,56 (s, 3H, ArMe) ; 4,98 (d, 2H, -CH₂-P *J* 16,8Hz); 6,77 (s, 1H, ArH *J* 2,8Hz); 6,84 (s, 1H, ArH) ; 7,78-7,46 (m, 15H, ArH).

### e) Préparation des (E) et (Z) 4-[1-(5,6,7,8-tétrahydro-3,5,5,8,8-pentaméthyl-2-naphtalényl)-2-propényl]benzonitriles de formules

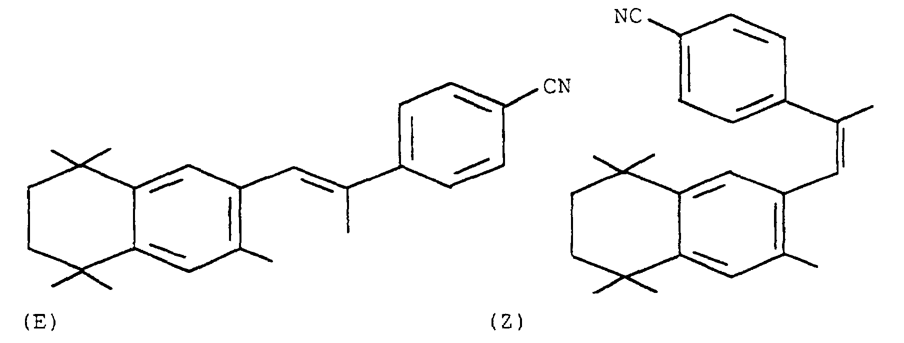

Une solution de bromure de (5,6,7,8-tétrahydro-3,5,5,8,8-pentaméthyl-2-naphtalényl)-méthyl triphénylphosphonium (3, 65 g, 6,5 mmol) préparé précédemment de NaH (Aldrich, 6,0 mmol) provenant de 0,24 g d'une dispersion de 60% de NaH dans l'huile dans 5 ml de DMSO anhydre est agitée sous argon pendant 30 min, puis est ajoutée une solution de 4-cyanoacétophénone (1,45 g, 10,0 mmol) dans 5,5 ml de DMSO. La solution est agitée pendant 6 h à l'ambiante puis versée sur de la glace (100 g). On extrait alors à l'éther (5 x 100 ml). Le produit brut est purifié par chromatographie éclair sur silice (éluant éther de pétrole pur) puis de nouveau purifié par chromatographie éclair sur silice (éluant éther : éther de pétrole = 1 : 99) afin de séparer les isomères. On obtient 0,25 g d'un solide blanc (Z) 4-[-1-(5,6,7,8-tétrahydro-3,5,5,8,8-pentaméthyl-2-naphtalényl)-2-propény]benzonitrile (Rdt = 14%) puis 0,20 g d'un autre solide blanc le (E) 4-[-1-(5,6,7,8-tétrahydro-3,5,5,8,8-pentaméthyl-2-naphtalényl)-2-propényl]benzonitrile (Rdt = 11%) et 0,18 g d'un mélange des deux isomères (Rdt = 10%).

### Isomère (E) :

RMN¹H 200MHz (CDCl₃) : 1,28 (s, 12H, 5,8-CH₃); 1,68 (s, 4H, 6,7-CH₂-); 2,16 (d, 3H, Me vinylique J 0,8Hz); 2,24 (s, 3H, ArMe); 6,91 (s large, 1H, H vinylique); 7,13 (s, 1H, ArH ); 7,16 (s, 1H, ArH) ; 7,62 (s, 2H, ArH méta au CN); 7,63 (s, 2H, ArH ortho au CN).

### Isomère (Z) :

RMN¹H 200MHz (CDCl₃) : 0,78 (s, 6H, 5-Me); 1,20 (s, 6H, 5,8-Me); 1,40-1,60 (m, 4H, 6,7-CH₂); 2,21 (d, 3H, Me vinylique *J* 1,5Hz); 2,25 (s, 3H, ArMe); 6,51 (s, 1H, ArH ); 6,59 (s large, 1H, H vinylique); 7,00 (s, 1H, ArH); 7,19 (d, 2H, ArH méta au CN *J* 8,4Hz); 7,46 (d, 2H, ArH ortho au CN *J* 8,4Hz).

### f) Préparation de l'acide (E) 4-[1-(5,6,7,8-tétrahydro-3,5,5,8,8-pentaméthyl-2-naphtalényl)-2-propényl]benzoïque (composé CB71802) de formule :

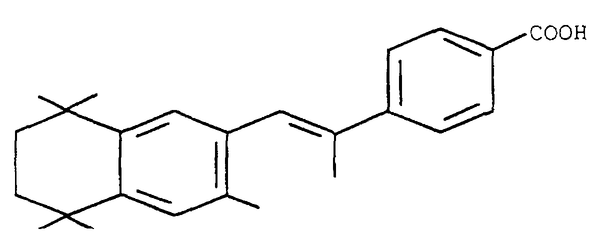

Une suspension du dérivé (E) 4-[1-(5,6,7,8-tétrahydro-3,5,5,8,8-pentaméthyl-2-naphtalényl)-2-propényl]benzonitrile (0,22 g, 0,64 mmol) préparé précédemment en solution dans de la potasse (0,94 g, 16,8 mmol) hydroéthanolique (H₂O 0,55 ml et EtOH 3,3 ml) est chauffée à 70°C sous agitation magnétique pendant 6 h. L'avancement de la réaction est suivi par HPLC. Après refroidissement du milieu réactionnel, on reprend à l'eau (50 ml), acidifie par HCl 1N, extrait à l'éther (4 x 20 ml), sèche la phase étherée par MgSO₄, filtre et évapore pour obtenir un produit brut que l'on purifie par lavage à l'hexane. Après séchage à 50°C (3 h) au dessiccateur à vide, on obtient 0,18 g (Rdt = 75 %) d'un solide blanchâtre, l'acide (E) 4-[-1-(5,6,7,8-tétrahydro-3,5,5,8,8-pentaméthyl-2-naphtalényl)-2-propényl]benzoïque pur.
F(°C) = 209-210 (déc.).
RMN¹H 200MHz (CDCl₃) : 1,29 (s, 12H, 5,8-CH₃); 1,69 (s, 4H, 6,7-CH₂); 2,19 (d, 3H, Me vinylique *J* 1,1Hz); 2,26 (s, 3H, ArMe); 6,95 (s large, 1H, H vinylique); 7,13 (s, 1H, ArH ); 7,19 (s, 1H, ArH); 7,63 (d, 2H, ArH méta au COOH *J* 8,4Hz); 8,11 (d, 2H, ArH ortho au COOH *J* 8,4Hz).
MS EI 70 eV (m/z, % intensité) : 362 (M⁺, 79%); 347 (M⁺-CH₃, 100).
HRMS EI 70 eV : Mₜᵣ = 362,2220 pour C₂₅H₃₀O₂ Mₜₕ = 362,2246
HPLC : Colonne ODS, Ultrasphère, 5 µ, 250 x 4,6 mm, détecteur Shimadzu UV, 260 nm, débit 1 ml/min, éluant MeOH : H₂O = 90 : 10 + 0,1% TFA, acide (composé CB71802) tr = 11,4 min 99,0% ; impuretés tr = 8,0 min 0,5% et tr = 9,0 min 0,3%.
IR (pur, cm⁻¹) : 3400-2500; 2958; 1688, 1604, 1420, 1288, 1186, 1124, 1066, 1016, 908, 850.

### Exemple 2 : Préparation de l'acide (Z) 4-[1-(5,6,7,8-tétrahydro-3,5,5,8,8-pentaméthyl-2-naphtalényl)-2-propényl]benzoïque (composé CB32706) de formule :

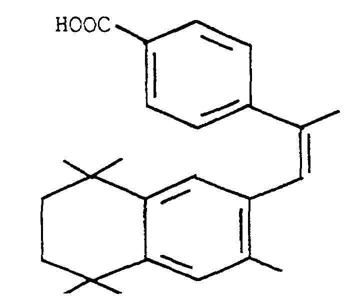

Une suspension du dérivé (Z) 4-[1-(5,6,7,8-tétrahydro-3,5,5,8,8-pentaméthyl-2-naphtalényl)-2-propényl]benzonitrile (30 mg, 0,08 mmol) préparé précédemment, dans de la potasse (0,21 g, 3,84 mmol) hydroéthanolique (H₂O 0,11 ml et EtOH 0,7 ml) est chauffée à reflux sous agitation magnétique pendant 6 h. On évapore l'éthanol à l'évaporateur rotatif, reprend à l'eau (20 ml), acidifie par HCl 1N, extrait (5 x 40 ml) à l'éther, sèche la phase éthérée sur MgSO₄, filtre et évapore. Le produit est recristallisé dans l'hexane, filtré et séché. On obtient 110 mg d'un solide blanc, l'acide (Z) 4-[1-(5,6,7,8-tétrahydro-3,5,5,8,8-pentaméthyl-2-naphtalényl)-2-propényl]benzoïque (composé CB32706) (Rdt = 95%).
F (°C) = 185.
RMN¹H 200MHz (CDCl₃) : 0,76 (s, 6H, 2 Me); 1,20 (s, 6H, 2 Me); 1,40-1,60 (m, 4H, 2 -CH₂-); 2,23 (d, 3H, Me vinylique *J* 1,3 Hz); 2,25 (s, 3H, Me); 6,57 (s, 2H, ArH); 6,99 (s, 1H, H vinylique); 7,20-7,30 (m, 2H, ArH); 7,90 (d, 2H, ArH, *J* 8Hz).
MS EI 70ev (m/z, % intensité) : 362 (M⁺, 69,5 %); 347 (100); 149 (13).
HPLC : Colonne ODS, Ultrasphère, 5 µ, 250 x 4,6 mm, détecteur Shimadzu UV, 260 nm, débit 1 ml/min, éluant MeOH:H₂O = 90:10 + 0,1% TFA, débit 1 ml/min, acide (CB32706) tr = 12,1 min 99,2%.
IR (cm⁻¹) : 2926; 1688; 1606; 1418; 1280.

### Exemple 3 : Préparation du (E) 5-[4-[1-(5,6,7,8-tétrahydro-3,5,5,8,8-pentaméthyl-2-naphtalényl)-2-propényl]phényl]-1H-tétrazole (composé CB40747) de formule:

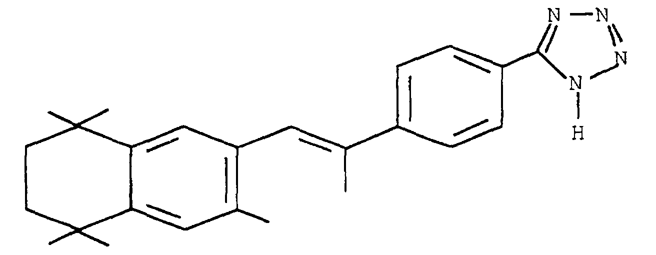

A une solution des (E) et (Z) 4-[1-(5,6,7,8-tétrahydro-3,5,5,8,8-pentaméthyl-2-naphtalényl)-2-propényl]benzonitriles préparés précédemment (0,32 g, 0,93 mmol) dans du toluène anhydre (5 ml), on additionne successivement de l'oxyde de dibutylétain (23 mg, 10% mol) et de l'azoture de triméthylsilyle (0,247 ml, 1,86 mmol, 2 éq.). Le milieu réactionnel est chauffé 16 h à reflux (110°C) sous atmosphère d'argon et agitation magnétique. On précipite avec du chloroforme rectifié pour obtenir après filtration 31 mg d'un solide blanc le (E) 5-[4-[1-(5,6,7,8-tétrahydro-3,5,5,8,8-pentaméthyl-2-naphtalényl)-2-propényl]phényl]-1*H*-tétrazole (composé CB40747) pur après séchage au dessiccateur sous vide (avec P₂O₅).
F (°C) = 256-258 (déc.).
RMN¹H 200 MHz (DMSO-D₆) : 1,24 (s, 12H, 5 et 8-Me); 1,64 (s, 4H, -CH₂-); 2,15 (d, 3H, Me *J* 0,7 Hz); 2,21 (s, 3H, Me); 7,02 (s, 1H, H vinylique); 7,16 (s, 2H, ArH); 7,81 (d, 2H, ArH méta au tétrazyl J 8,4Hz); 8,05 (d, 2H, ArH ortho au tétrazoyl *J* 8,4Hz). Pic H₂O à 3,30; pic DMSO 2,48.
MS EI 70 eV (m/z, % intensité) : 386 (M⁺, 100%); 371 (29), 359 (21), 358 (71), 343 (25), 328 (11), 327 (19), 312 (17).
HMRS EI 70 eV: Mₜᵣ = 386,2450 pour C₂₅H₃₀N₄ Mₜₕ = 386,2470.
HPLC : Colonne ODS, Ultrasphère, 5 µ, 250 x 4,6 mm, détecteur Shimadzu UV, 260 nm, éluant MeOH : H₂O = 90:10 + 0,1% TFA, débit 1 ml/min, tétrazole (CB40747) tr = 9,10 min 97,9%, impureté tr = 6,75 min 1,53%.

### Exemple 4 : Préparation du (Z) 5-[4-[1-(5,6,7,8-tétrahydro-3,5,5,8,8-pentaméthyl-2-naphtalényl)-2-propényl]phényl]-1H-tétrazole (composé CB61692) de formule:

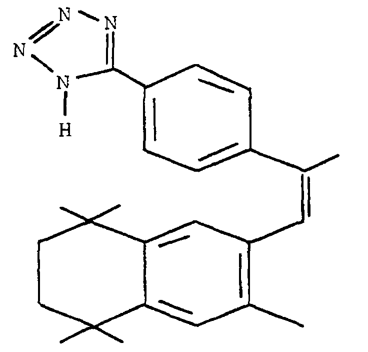

A une solution du (Z) 4-[1-(5,6,7,8-tétrahydro-3,5,5,8,8-pentaméthyl-2-naphtalényl)-2-propényl]benzonitrile précédent (0,24 g, 0,70 mmol) dans du toluène anhydre (1,4 ml), on additionne successivement de l'oxyde de dibutylétain (22,3 mg, 0,09 mmol, 10% mol) et de l'azoture de triméthylsilyle ( 0,19 ml, 1,40 mmol). Le milieu réactionnel est chauffé 23 h à reflux (110°C) sous atmosphère d'argon et agitation magnétique. Après refroidissement du milieu réactionnel, on évapore le toluène et purifie le produit brut par chromatographie éclair sur silice (éluant dichlorométhane: méthanol = 90 : 10). On obtient 0,30 g d'un produit que l'on dissout dans un minimum de chloroforme et précipite par de l'hexane. Après filtration et lavage à l'hexane puis séchage au dessicateur, on obtient 0,18 g d'un solide blanc, le (Z) 5-[4-[1-(5,6,7,8-tétrahydro-3,5,5,8,8-pentaméthyl-2-naphtalényl)-2-propényl]phényl]-1*H*-tétrazole (CB61692) (Rdt = 66%).
F (°C) = 210.
RMN¹H 200 MHz (DMSO-D₆): 0,68 (s, 6H, Me); 1,15 (s, 6H, Me); 1,30 (s, 4H, -CH₂-); 2,23 (d, 6H, Me); 6,54 (s, 1H, ArH); 6,59 (s large, 1H, H vinylique); 7,31 (s, 1H, ArH); 7,35 (d, 2H, ArH méta au tétrazole *J* 8,2Hz); 7,90 (d, 2H, ArH ortho au tétrazole J 8,2Hz).
MS EI 70 eV (m/z, % intensité) : 386 (M⁺, 100%); 371 (31), 359 (21), 358 (73), 343 (29), 328 (16), 327 (21), 312 (21).
HMRS EI 70 eV: Mₜᵣ = 386,2466 pour C₂₅H₃₀N₄ Mₜₕ = 386,2470.
HPLC : Colonne ODS, Ultrasphère, 5µ, 250 x 4,6 mm, détecteur Shimadzu UV, 260 nm, débit 1 ml/min ; éluant MeOH : H₂O = 90:10 + 0,1% TFA, tétrazole (CB61692) tr = 8,8 min 98,1%, impuretés tr = 8,0 min 0,3% et tr = 12,0 min 1,6%.

### Exemples 5 et 6 : Préparation des acides (E) et (Z) 4-[1-(3',4'-dihydro-4',4'-diméthyl-1',1'-dioxyde-2'H-1'-benzothiopyran-6'-yl)-2-propényl]benzoique (composés CB39356 et CB72484) de formules :

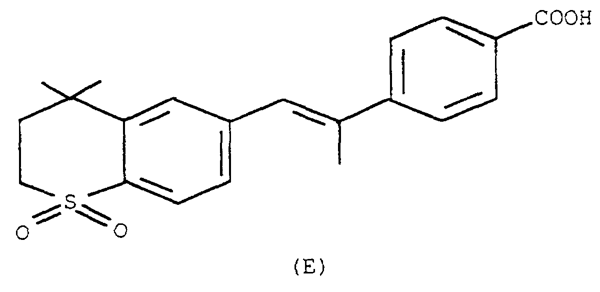

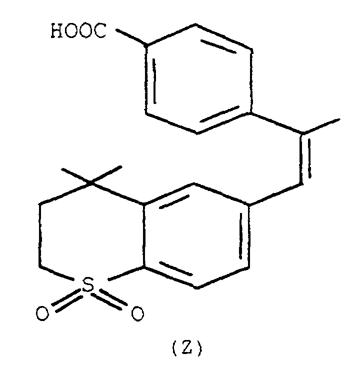

### a) Préparation du 3-méthylbut-2-ényl-4'-bromophénylsulfure

A une solution d'hydrure de sodium à 60% en dispersion dans l'huile minérale (3,2 g, 80 mmol) dans 31 ml de THF anhydre, on ajoute goutte à goutte à température ambiante une solution de 4-bromothiophénol (12,7 g, 61 mmol) dans 18,5 ml de THF anhydre. On laisse agiter à température ambiante 30 min, puis on ajoute une solution de 1-bromo-3-méthyl-but-2-ène (7,7 ml, 67 mmol) dans 15,5 ml de THF anhydre et de l'iodure de sodium (1,83 g, 12 mmol). Le milieu réactionnel est agité à température ambiante pendant 12 h. On hydrolyse à 0°C avec 30 ml d'eau. Après retour à température ambiante, on extrait à l'éther (5 x 50 ml), sèche sur MgSO₄ et évapore les solvants. On obtient 18,23 g d'une huile jaunâtre, le 3-méthylbut-2-ényl-4'-bromophénylsulfure (Rdt brut = 100%).

RMN¹H 200MHz (CDCl₃) : 1,60 (s, 3H, Me); 1,70 (s, 3H, Me); 3.50 (d, 2H, -CH2- *J* 7,7Hz); 5,20-5,30 (m, 1H, H vinylique); 7,13-7,24 (m, 2H, ArH); 7,33-7,39 (m, 2H, ArH).

### b) Préparation du 6-bromo-4,4-diméthyl-3,4-dihydro-2H-1-benzothiopyrane.

A une solution du 3-méthylbut-2-ényl-4'-bromophénylsulfure brut préparé à l'étape précédente (18,23 g, 61 mmol) dans 600 ml du dichlorométhane, on ajoute à -10°C du trichlorure d'aluminium (8, 9 g, 67 mmol). Le milieu réactionnel est agité à cette température pendant 3 h, puis versé sur 500 ml d'un mélange eau-glace volume à volume. On extrait au dichlorométhane et la phase organique est lavée par une solution aqueuse saturée en NaHCO₃, puis séchée sur MgSO₄, filtrée et evaporée. Le produit brut est purifié par chromatographie éclair sur silice (éluant éther de pétrole : éther éthylique = 100 : 5. On obtient 12,62 g d'une huile, le 6-bromo-4,4-diméthyl-3,4-dihydro-2*H*-1-benzothiopyrane (Rdt = 80%).
RMN¹H 200MHz (CDCl₃) : 1,30 (s, 6H, 2 Me); 1,80-2,00 (m, 2H, -CH₂-); 2,80-3,05 (m, 2H, -CH₂-S) ; 6,85-7,60 (m, 3H, ArH).

### c) Préparation du 6-formyl-4,4-diméthyl-3,4-dihydro-2H-1-benzothiopyrane.

A du magnésium (0,30 g, 12 mmol) dans 1 ml de THF anhydre, on ajoute à 66°C du 6-bromo-4,4-diméthyl-3,4-dihydro-2H-1-benzothiopyrane préparé à l'étape précédente (b) (2,62 g, 10 mmol) en solution dans 5 ml de THF anhydre. On agite au reflux du THF jusqu'à consommation quasi-complète du magnésium (30 min). Le milieu réactionnel est ensuite refroidi à -10°C et on ajoute à cette température de la N-formylmorpholine (1,2 ml, 12 mmol) dans 2 ml de THF. A la fin de l'addition le milieu réactionnel est porté à température ambiante pendant 1 h. Après hydrolyse par 10 ml d'une solution aqueuse saturée de NH₄Cl à 0°C et extraction à l'éther éthylique (5 x 30 ml), la phase organique est séchée sur MgSO₄, filtrée et évaporée. Le produit brut est purifié par chromatographie éclair sur silice (éluant éther de pétrole : éther éthylique : 100 : 5). On obtient 2,00 g du 6-formyl-4,4-diméthyl-3,4-dihydro-2*H*-1-benzothiopyrane (Rdt = 61%)

RMN¹H 200MHz (CDCl₃) : 1,60 (s, 6H, 2 Me); 1,90-2,00 (m, 2H, -CH₂-); 3,00-3,10 (m, 2H, -CH₂-S); 7,10-7,25 (m, 1H, ArH); 7,45-7,55 (m, 1H, ArH); 7,60 (m, 1H, Ar-H); 9,85 (s, 1H CHO).

### d) Préparation du 6-(hydroxyméthyl)-4,4-diméthyl-3,4-dihydro-2H-1-benzothiopyrane.

A une solution du 6-formyl-4,4-diméthyl-3,4-dihydro-2H-1-benzothiopyrane précédent (2,00 g, 9,7 mmol) dans 20 ml d'éthanol, on ajoute à 0°C du borohydrure de sodium (NaBH₄) (0,36 g, 9,7 mmol) . L'agitation est poursuivie à cette température 30 min. Après évaporation de l'éthanol, on reprend par 100 ml d'éther éthylique et on acidifie par une solution aqueuse de HCl 3N jusqu'à pH = 1. On extrait à l'éther, sèche sur MgSO₄, filtre et évapore les solvants. Le produit brut est purifié par chromatographie éclair sur silice (éluant éther de pétrole : éther éthylique : 100 : 10). On obtient 1,85 g du 6-(hydroxyméthyl)-4,4-diméthyl-3,4-dihydro-2*H*-1-benzothiopyrane (Rdt = 92%).

RMN¹H 200MHz (CDCl₃) : 1,30 (s, 6H, 2 Me); 1,90-1,96 (m, 2H, -CH₂-); 2,97-3,03 (m, 2H, -CH₂-S); 4,56 (s, 2H, BnzH); 6,96-7,07 (m, 2H, ArH); 7,23-7,33 (m, 1H, ArH).

### e) Préparation du 6-(bromométhyl)-4,4-diméthyl-3,4-dihydco-2H-1-benzothiopyrane.

A une solution 6-(hydroxyméthyl)-4,4-diméthyl-3, 4-dihydro-2*H*-1-benzothiopyrane précédent (1,85 g, 8,9 mmol) dans 6,2 ml de toluène anhydre, on ajoute, goutte à goutte, à la seringue entre 20 et 25 °C du tribromure de phosphore, (1 ml, 10,7 mmol). On laisse agiter à température ambiante 1 h. On hydrolyse à 0°C par de la glace puis par de l'eau, on extrait à l'éther éthylique, sèche sur MgSO₄ filtre et évapore les solvants. On obtient 2,14 g du 6-(bromométhyl)-4,4-diméthyl-3,4-dihydro-2H-1-benzothiopyrane (Rdt brut = 88%).

RMN¹H 200MHz (CDCl₃) : 1,30 (s, 6H, 2 Me); 1,89-1,95 (m, 2H, -CH₂-); 2,97-3,03 (m, 2H, -CH₂-S); 4,44 (s, 2H, BnzH); 7,03 (s, 2H, ArH); 7,33 (s, 1H, ArH).

### f) Préparation du bromure de (4,4-diméthyl-3,4-dihydro-2H-1-benzothiopyranyl)-6-méthyltriphénylphosphonium.

Le 6-(bromométhyl)-4,4-diméthyl-3,4-dihydro-2*H*-1-benzothiopyrane de l'étape précédente (2,14 g, 7,8 mmol) est mélangé à température ambiante avec de la triphénylphosphine (2,52g, 9,6 mmol) en solution dans 7 ml de dichlorométhane pendant 18 h, puis dilué à l'éther éthylique (50 ml). Le bromure de (4,4-diméthyl-3,4-dihydro-2*H*-1-benzothiopyranyl-6-méthyltriphénylphosphonium précipite, est filtré et lavé abondamment à l'éther. On obtient 4,36 g de cristaux blancs qui sont séchés au dessiccateur et utilisés tels quels (Rdt brut = 100%).

RMN¹H 200MHz (CDCl₃) : 1,00 (s, 6H, 2 Me); 1,76-1,82 (m, 2H, -CH₂-); 2,89-2,95 (m, 2H, -CH₂-S); 5,23 (d, 2H, BnzH, *J* 14Hz); 6,60-6,70 (m, 1H, ArH) ; 6,80-6,85 (m, 1H, ArH); 7,15 (m, 1H, ArH); 7,55-7,85 (m, 15H, -PPh₃).

### g) Préparation des (E) et (Z) 4 - [1- (3' ,4'-dihydro-4',4'-diméthyl-2'H-1'-benzothiopyran-6'-yl)-2-propényl]benzonitriles de formules respectives:

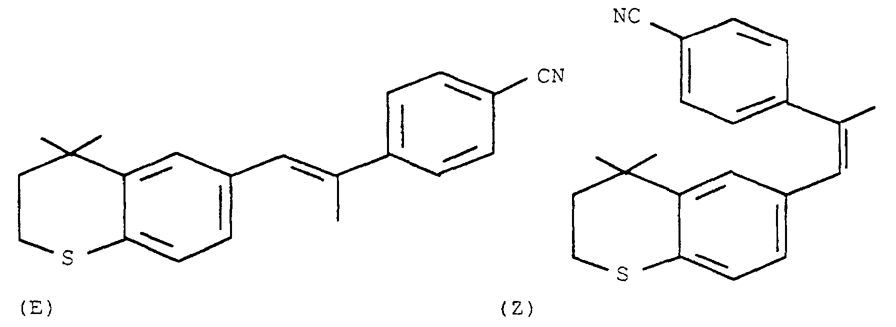

Une solution de bromure de (4,4-diméthyl-3,4-dihydro-2H-1-benzothiopyranyl)-6-méthyltriphényl phosphonium de l'étape précédente (2,41 g, 4,5 mmol), de NaH (Aldrich, 4,1 mmol) provenant de 0,16 g d'une dispersion de 60% de NaH dans l'huile dans 2,5 ml de DMSO anhydre est agitée sous argon pendant 20 minutes, puis est ajoutée une solution de 4-cyanoacétophénone (0,54 g, 3,75 mmol) dans 5 ml de DMSO. La solution est agitée pendant 24 h à l'ambiante puis versée sur de la glace (70 g). On extrait alors à l'éther (5 x 50 ml,). Le produit brut est alors purifié par chromatographie éclair sur silice (éluant éther de pétrole : éther éther éthylique = 100 : 10). On obtient 0,24 g d'un solide pur, le (E) 4-[1-(3',4'-dihydro-4',4'-diméthyl-2'H-1'-benzothiopyran-6'-yl)-2-propényl]benzonitrile (Rdt = 20%) et 0,13 g d'un solide, le (Z) 4-[1-(3',4'-dihydro-4',4'-diméthyl-2'*H*-1'-benzothiopyran-6'-yl)-2-propén-yl] benzonitrile (Rdt = 11%).

### Isomère (E) :

RMN¹H 200MHz (CDCl₃) : 1,33 (s, 6H, 2 Me); 1,93-1,99 (m, 2H, -CH₂-) ; 2,26 (s, 3H, Me vinylique); 3,03-3,06 (m, 2H, -CH₂-S) ; 6,83 (s, 1H, H vinylique); 7,07 (m, 2H, ArH); 6,78-6,87 (m, 2H, ArH); 7,33 (s, 1H, ArH); 7,50-7,70 (m, 2H, ArH).

### Isomère (Z) :

RMN¹H 200MHz (CDCl₃) : 1,10 (s, 6H, 2 Me); 1,56-1,86 (m, 2H, -CH₂-); 2,16 (s, 3H, Me vinylique); 2,90-2,96 (m, 2H, -CH₂-S) ; 6,46 (s, 1H, H vinylique); 6,60 (dd, 1H, ArH *J* 1,9Hz *J* 8,1Hz); 6,78-6,87 (m, 2H, ArH); 7,28 (dd, 2H, ArH *J* 1,8Hz, *J* 6,8Hz); 7,54 (dd, 2H, ArH *J* 1,8Hz *J* 6,8Hz).

### h) Préparation du (E) 4-[1-(3',4'-dihydro-4',4'-diméthyl-1',1'-dioxyde-2'H-1'-benzothiopyran-6'-yl)-2-propényl]benzonitrile de formule :

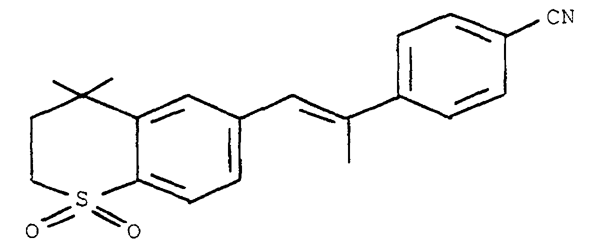

A une solution du (E) 4-[1-(3',4'-dihydro-4',4'-diméthyl-2'*H*-1'-benzothiopyran-6'-yl)-2-propényl] benzonitrile précédent (0,24 g, 0,75 mmol) dans 7 ml de chloroforme, on ajoute de l'acide métachloroperbenzoïque à 70-75% (0,37 g, 1,5 mmol) à 0°C. L'agitation est poursuivie à cette température 2 h 30. Le milieu réactionnel est dilué par 15 ml de chloroforme puis lavé avec une solution aqueuse de Na₂CO₃ à 5% (3 x 5ml). La phase organique est séchée sur MgSO₄, filtrée puis évaporée. On obtient 0,25 g d'un solide, le (E) 4-[1-(3',4'-dihydro-1',1'-dioxyde-4',4'-diméthyl-2'H-1'-benzothiopyran-6'-yl)-2-propényl]benzonitrile (Rdt brut = 96%).

RMN¹H 200MHz (CDCl₃) :1,41 (s,6H, 2 Me); 2,23 (s, 3H, Me vinylique); 2,37-2,45 (m, 2H, -CH₂-); 3,36-3,42 (m, 2H, CH₂-S); 6,85 (s, 1H, H vinylique); 7,24-7,38 (m, 2H, ArH) ; 7,56-7,67 (m, 4H, ArH) ; 7,90 (d, 1H, ArH *J* 8, 1Hz).

### i) Préparation du (Z) 4-[1-(3', 4'-dihydro-4',4'-diméthyl-1',1'-dioxyde-2'H-1'-benzothiopyran-6'-yl)-2-propényl] benzonitrile de formule :

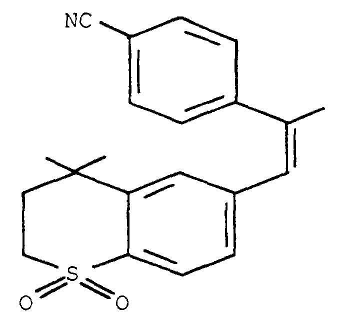

A une solution du (Z) 4-[1-(3',4'-dihydro-4',4'-diméthyl-2'*H*-1'-benzothiopyran-6'-yl)-2-propényl]-benzonitrile (0,21 g, 0,65 mmol) dans 6,1 ml de chloroforme, on ajoute de l'acide métachloroperbenzoique à 70-75% (0,32 g, 1,3 mmol) à 0°C. L'agitation est poursuivie à cette température pendant 3 h. Le milieu réactionnel est dilué par 15 ml de chloroforme puis lavé avec une solution aqueuse de Na₂CO₃ à 5% (3 x 5ml). La phase organique est séchée sur MgSO₄, filtrée puis évaporée. On obtient 0,21 g d'un solide, le (Z) 4-[1-(3',4'-dihydro-4',4'-diméthyl-1',1'-dioxyde-2'*H*-1'-benzothiopyran-6'-yl)-2-propényl]benzonitrile (Rdt brut = 95%).

RMN¹H 200MHz (CDCl₃) :0,95 (s, 6H, 2 Me); 2,15 (s, 3H, Me vinylique); 2,20-2,30 (m, 2H, -CH₂-); 3,25-3,35 (m, 2H, CH₂-S); 6,50 (s, 1H, H vinylique); 6,75 (s, 1H, ArH); 7,00 (dd,1H, ArH *J* 1,3Hz, *J* 1,3Hz); 7,20 (m, 2H, ArH); 7,55 (d, 2H, *J* 7,5Hz, ArH); 7,5 (d, 1H, *J* 7,5 Hz, ArH).

### j) Préparation de l'acide (E) 4-[1-(3',4'-dihydro4',4'-diméthyl-1',1'-dioxyde-2'H-1'-benzothiopyran-6'-yl)-2-propényl]benzoïque (composé CB39356) et de l'acide (Z) 4-[1-(3',4'-dihydro-4',4'-diméthyl-1',1'-dioxyde-2'H-1'-benzothiopyran-6'-yl)-2-propényl]benzoïque (composé CB72484) de formules respectives suivantes :

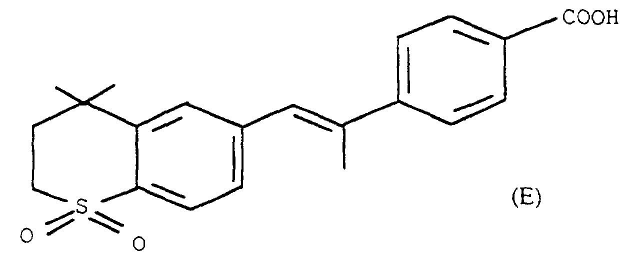

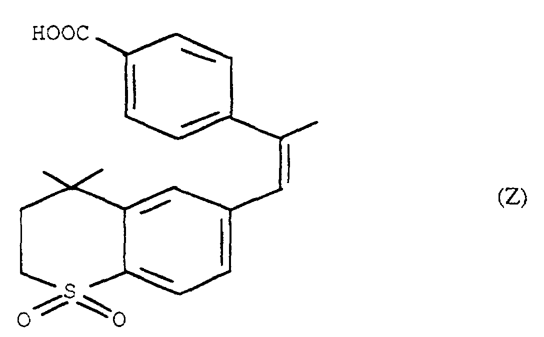

Une suspension du mélange des (E) et (Z) 4-[1-(3',4'-dihydro-4',4'-diméthyl-1',1'-dioxyde-2'*H*-1'-benzothiopyran-6'-yl)-2-propényl]benzonitriles (0,39 g, 0,75 mmol) en solution dans de la potasse (0,5 g, 9,0 mmol) hydroéthanolique (H₂O 0,25 ml et EtOH 1,6 ml) est chauffée à reflux sous agitation magnétique pendant 5 h. On évapore l'éthanol à l'évaporateur rotatif, reprend à l'eau (50 ml), acidifie par HCl 3N, extrait (3 x 25ml) à l'éther, sèche la phase éthérée sur MgSO₄, filtre et évapore. Le produit brut est purifié par HPLC préparative sur une colonne Waters HR C₁₈ (25 x 100 mm) avec comme éluant CH₃CN : H20 = 100 : 100 + 0,1% de TFA. On obtient 15,8 mg d'un solide blanc, l'acide (E) 4-[1-(3',4'-dihydro-4',4'-diméthyl-1',1'-dioxyde-2'*H*-1'-benzo-thiopyran-6'-yl)-2-propényl]benzoïque (Rdt = 6%) (composé CB39356) et 17,2 mg d'un solide blanc, l'acide (Z) 4-[1-(3',4'-dihydro-4',4'-diméthyl-1',1'-dioxyde-2'*H*-1'-benzo- thiopyran-6'-yl)-2-propényl]benzoïque (composé CB72484) (Rdt = 6 %)

Acide (E) 4-[1-(3',4'-dihydro-4',4'-diméthyl-1',1'-dioxyde-2'*H*-1'-benzothiopyran-6'-yl)-2-propényl] benzoïque (composé CB39356) :
F (°C) - 270-274.
RMN¹H 200MHz (CDCl₃) : 1,35 (s, 6H, 2 Me); 2,19 (d, 3H, Me vinylique *J* 1,0Hz); 2,31-2,37 (m, 2H, -CH₂-); 3,30-3,36 (m, 2H, CH₂-S); 6,80 (s, 1H, H vinylique); 7,30 (m, 2H, ArH); 7,49 (d, 2H, ArH J 8,3Hz); 7,82 (d, 1H, ArH *J* 8,1Hz); 7,97 (d, 2H, ArH *J* 8,3Hz).
MS EI 70 ev (m/z, % intensité) : 370 (M⁺, 100%); 338 (30); 337 (43); 44 (74); 40 (45).
MSHR EI 70 ev : Mₜᵣ = 370,1257 pour C₂₁H₂₂O₄S Mₜₕ = 370,1239.
HPLC : Colonne Waters HR C₁₈, 8 x 100 mm, 6 µ, détecteur UV Waters 486 à 300 nm, débit 2,5 ml/min, éluant CH₃CN : H₂O = 50 : 50 + 0,1% de TFA, acide (composé CB39356) tr = 4,52 min 98,8%.
Acide (Z) 4-[1-(3',4'-dihydro-4',4'-diméthyl-1',1'-dioxyde-2'*H*-1'-benzothiopyran-6'-yl)-2-propényl] benzoïque (composé CB72484) :
F(°C) = 210.
RMN¹H 200MHz (CDCl₃) : 1,02 (s, 6H, 2 Me); 2,20 (d, 3H, Me vinylique *J* 1,4Hz); 2,23-2,27 (m, 2H, -CH₂-); 3,25-3,31 (m, 2H, CH₂-S); 6,52 (s, 1H, H vinylique); 6,82 (d, 1H, ArH *J* 1,3Hz); 7,03 (dd, 1H, ArH J 8,3Hz *J* 1,4Hz); 7,24 (d, 2H, ArH *J* 7,9Hz); 7,67 (d, 1H, ArH *J* 1,3Hz); 8,03 (d, 2H, ArH *J* 8,3Hz).
MS EI 70 ev (m/z, % intensité) : 370 (M⁺, 100%); 338 (10); 337 (44).
MSHR EI 70 ev : Mₜᵣ = 370,1265 pour C₂₁H₂₂O₄S Mₜₕ = 370,1239.
HPLC : Colonne Waters HR C₁₈, 8 x 100 mm, 6 µ, détecteur UV Waters 486 à 260 nm, débit 2,5 ml/min, éluant CH₃CN : H₂O = 50 : 50 + 0,1% de TFA, acide (composé CB72484) tr = 5,51 min 97,8%.

### Exemple 7 : Préparation du (Z) 5-[4-[1-(3',4'-dihydro-4',4'-diméthyl-1',1'-dioxvde-2'H-1'-benzothiopvran-6'-yl)-2-propényl]phényl]-1H-tétrazole (composé CB39122) de formule suivante :

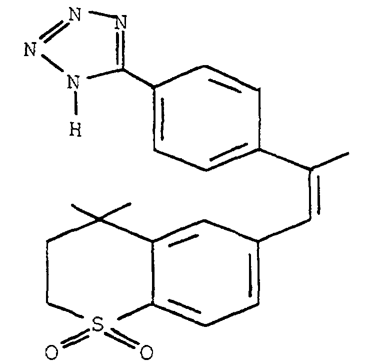

A une solution de (Z) 4-[1-(3',4'-dihydro-4',4'-diméthyl-1',1'-dioxyde-2'*H*-1'-benzothiopyran-6'-yl)-2-propényl]benzonitrile (0,31 g, 0,90 mmol ) dans du toluène anhydre on additionne successivement de l'oxyde de dibutylétain (30 mg, 0,11 mmol) et de l'azoture de triméthylsilyle (0,24 ml, 1,80 mmol). Le milieu réactionnel est chauffé 15 h à reflux (110°C) sous atmosphère d'argon et agitation magnétique. On purifie par chromatographie éclair sur silice (éluant CH₂Cl₂ : MeOH = 95 : 5) pour obtenir 0,16 g d'un solide, le (Z) 5-[4-[1-(3',4'-dihydro-4',4'-diméthyl-1',1'-dioxyde-2'H-1'-benzothiopyran-6'-yl)-2-propényl]phényl]-lH-tétrazole (composé CB39122) (Rdt = 46%).
F(°C) = 94.
RMN¹H 200MHz (CDCl₃) : 1,05 (s, 6H, 2 Me); 2,24 (s, 5H, Me vinylique et -CH₂-); 3,25 (m, 2H); 6,50 (s, 1H, H vinylique); 6,91-6,98 (m, 2H, ArH); 7,23-7,27 (m, 2H, ArH); 7,62 (d, 1H, ArH J 8Hz); 7,98 (d, 2H, ArH *J* 8Hz).
MS EI 70 ev (m/z, % intensité) : 394 (M⁺, 42,9%); 354 (59); 321 (61); 293 (69); 44 (100).
MSHR EI 70 ev Mth = 394,1464 pour C₂₁H₂₂N₄O₂S; Mₜᵣ = 394,1489.
HPLC : Colonne Waters HR C₁₈, 8 x 100 mm, 6 µ, détecteur UV Waters 486 à 260 nm, débit 2,5 ml/min, éluant MeOH : H₂O = 75 : 25 + 0,1% de TFA, sulfone (CB39122) tr = 4,6 min 97,5% ; impuretés tr = 4,3 1,9%; tr = 5,7 0,5%.

### Exemple 8 : Préparation du (E) 5-[4-[1-(3',4'-dihydro-4',4'-diméthyl-1',1'-dioxyde-2'H-1'-benzothiopyran-6'-yl)-2-propényl]phényl]-1H-tétrazole (composé CB15068) de formule suivante :

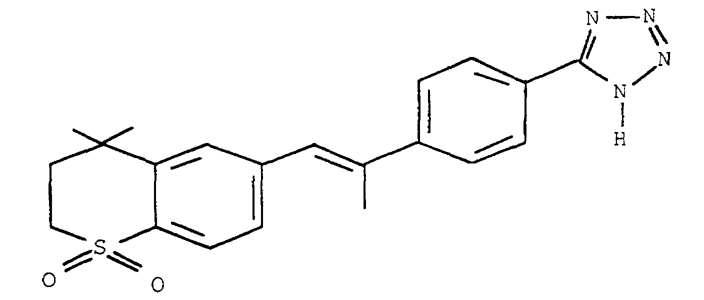

A une solution du (E) 4-[1-(3',4'-dihydro-4',4'-diméthyl-1',1'-dioxyde-2'*H*-1'-benzothiopyran-6'-yl)-2-propényl]benzonitrile (0,31 g, 0,75 mmol ) dans du toluène anhydre, on additonne successivement de l'oxyde de dibutylétain (22,4 mg, 0,09 mmol) et de l'azoture de triméthylsilyle (0,20 ml, 1,5 mmol). Le milieu réactionnel est chauffé 15 h à reflux (110°C) sous atmosphère d'argon et agitation magnétique. On purifie par HPLC préparative sur une colonne Waters HR C₁₈ (25 x 100 mm) avec comme éluant MeOH : H₂O = 75 : 25 + 0,1% de TFA pour obtenir après évaporation et séchage au dessiccateur 50 mg d'un solide blanc, le (E) 5-[4-(3',4'-dihydro-4',4'-diméthyl-1',1'-dioxyde-2'*H*-1'-benzothiopyran-6'-yl)-2-propényl]phényl]-1*H*-tétrazole (composé CB15068) (Rdt = 17%) .
F(°C) = 224.
RMN¹H 200MHz (DMSO-d₆) : 1,38 (s, 6H, 2 Me); 2,47 (s, 5H, -CH₂- et Me vinylique); 3,30-3,45 (m, 1H, -CH₂-S); 3,50-3,60 (m, 1H, -CH₂-S); 7,12 (s, 1H, H vinylique); 7,53 (d, 2H, ArH *J* 8,5HZ); 7,65 (s, 1H, ArH); 7,76-7,87 (m, 3H, ArH); 8,07 (d, 1H, ArH *J* 8,5Hz).
MS EI 70 ev (m/z, %intensité) : 394 (M⁺, 100%); 351 (47); 321 (38), 293 (39).
MSHR EI 70 ev : Mₜᵣ = 394,1492 pour C₂₁H₂₂N₄O₂S Mₜₕ = 394,1464.
HPLC : Colonne Waters HR C₁₈, 8 x 100 mm, 6 µ, détecteur UV Waters 486 à 260 nm, débit 2,5 ml/min, éluant MeOH : H₂O = 75 : 25 + 0,1% de TFA (CB15068) tr = 2,2 min 100%.

### Exemple 9 : Préparation du (E) 4-[1-(5,6,7,8-tétrahvdro-5,5,8,8-tétraméthyl-2-naphtalényl)-2-propényl]-N-(tétrazol-5-yl)-benzamide (composé CB73364) de formule suivante :

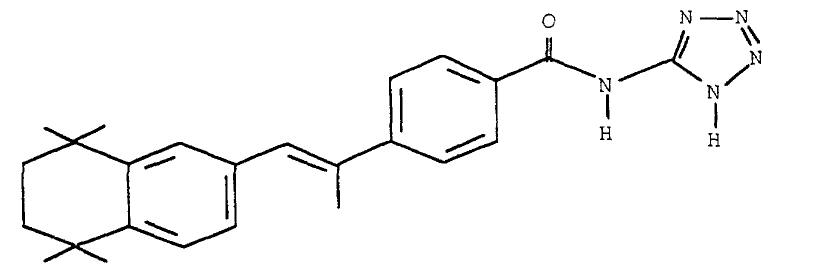

### a) Préparation du 1,1,4,4,6-pentaméthyl-1,2,3,4-tétrahydronaphtalène.

i) Dans un tricol de 250 ml muni d'un réfrigérant, d'une agitation magnétique et d'un thermomètre, on pèse du trichlorure d'aluminiun (3,0 g, 22,5 mmol). On additionne 70 ml de toluène anhydre (distillé et séché au tamis moléculaire 4Å) puis, par une ampoule à brome, on additionne une solution de 2,5-dichloro-2,5-diméthylhexane (15,00 g, 81,9 mmol) dans 30 ml de benzène anhydre. On chauffe à 100°C le milieu réactionnel pendant 2 h. Il y a un important dégagement de HCl et le milieu réactionnel devient rouge sombre. Après refroidissement du milieu réactionnel, celui-ci est versé sur un mélange eau-glace (200 ml), on extrait par de l'éther (3 x 80 ml), lave par une solution saturée de NaHCO₃ (2 x 150 ml), sèche la phase éthérée par MgSO₄, filtre et évapore. Le produit brut est purifié par distillation sous vide, on obtient 12,76 g (Rdt = 77%) de 1,1,4,4,6-pentaméthyl-1,2,3,4-tétrahydronaphtalène [Eb (°C) = 66-67 sous 1,5 mmHg].
   RMN¹H 80 MHz (CDCl₃): 1,20 (s, 12H, 1,4-Me); 1,60 (s, 4H, -CH₂-); 2,25 (s, 3H, 6-Me); 6,80-7,30 (m, 3H, ArH).
ii) A une solution de toluène distillé (20,0 g, 217,0 mmol) et de 2,2,5,5-tétraméthyltétrahydrofurane (Aldrich, 12,5 g, 97,5 mmol) est additionné à 0°C par petite fraction du AlCl₃ anhydre (Aldrich, 13,3g, 100 mmol). Le bain froid est alors écarté et le milieu réactionnel agité pendant 72 h. On additionne alors 100 ml d'une solution d'HCl 3N, sépare la phase organique et extrait la phase aqueuse à l'éther (3 x 75 ml). On rassemble les phases organiques, lave avec une solution saturée de NaHCO₃, sèche sur MgSO₄, filtre et évapore. Le produit brut obtenu est distillé sous vide, on obtient 12,2 g (Rdt = 62 %) d'une huile le 1,1,4,4,6-pentaméthyl-1,2,3,4-tétrahydronaphtalène cristallisant à froid.
   RMN¹H 80 MHz (CDCl₃): 1,20 (s, 12H, 1,4-Me); 1,60 (s, 4H, -CH₂-); 2,25 (s, 3H, 6-Me); 6,80-7,30 (m, 3H, ArH).

### b) Préparation du 6-bromométhyl-1,1,4,4,tétraméthyl-1,2,3,4-tétrahydronaphtalène.

Une solution de 1,1,4,4,6-pentaméthyl-1,1,4,4-tétrahydronaphtalène (2,14 g, 60,0 mmol), de NBS (11,21g, 63,0 mmol), de peroxyde de benzoyle (0,436 g, 1,80 mmol) dans 120 ml de CCl₄ est portée à reflux pendant 1 h. La solution est alors refroidie, diluée avec de l'éther de pétrole (120 ml), filtrée et concentrée donnant une huile jaunâtre. On distille (128-134°C, 1,2 mmHg) pour obtenir 9,24 g d'une huile incolore, le 6-bromométhyl-1,1,4,4,tétraméthyl-1,2,3,4-tétrahydro- naphtalène (Rdt = 55%).

RMN¹H 80 MHz (CDCl₃): 1,25 (s, 12H, Me); 1,65 (s, 4H, -CH₂-); 4,40 (s, 2H, BnzH); 7,2-7,0 (m, 3H, ArH).

### c) Préparation du bromure de (5,6,7,8-tétrahydro-5,5,8,8-tétraméthyl-2-naphtalényl)méthyl- triohénylphosphonium.

Le 6-bromométhyl-1,1,4,4,tétraméthyl-1,2,3,4-tétrahydronaphtalène (9,0 g, 32,0 mmol) préparé à l'étape précédente est mélangé avec de la triphénylphosphine (10,1 g, 38,4 mmol) en solution dans 50 ml de dichlorométhane pendant 24 h, puis dilué avec de l'éther éthylique (200 ml). Le bromure de (5,6,7,8-tétrahydro-5,5,8,8-tétraméthyl-2-naphtalényl)méthyltriphényl- phosphonium précipite, est filtré et lavé à l'éther. Les cristaux blancs obtenus 15,0 g (Rdt = 86%) sont séchés au dessiccateur.
F (°C) = 270-271.
RMN¹H 200 MHz (CDCl₃) : 0,54 et 0,88 (2s, 12H, 5,8-Me); 1,53 (s, 4H, 6,7-CH₂-); 5,13 (d, 1H, -CH₂P *J* 14 Hz); 6,88-6,75 (m, 2H, 1- et 3-ArH); 7,02 (d, 1H, 4-ArH *J* 8 Hz); 7,8-7,5 (m, 15H, -PPh₃).

### d) Préparation des (E) et (Z) 4-[1-(5,6,7,8-tétrahydro-5,5,8,8-tétraméthyl-2-naphtalényl)-2-propényl] benzonitriles de formules respectives :

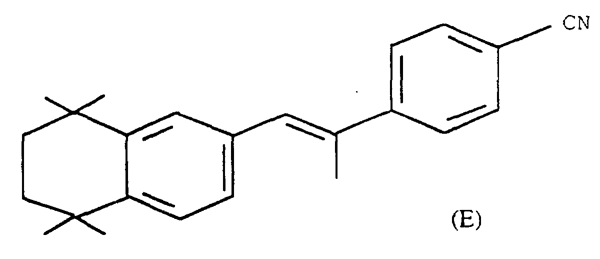

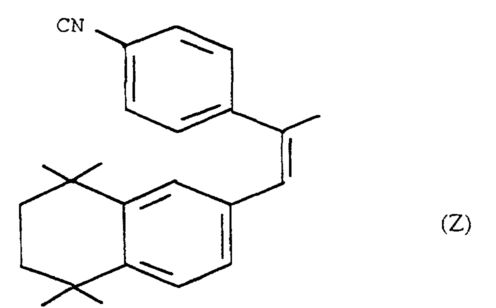

Une solution de bromure de (5,6,7,8-tétrahydro-5,5,8,8-tétraméthyl-2-naphtalényl)méthyltriphénylphosphonium (6,25 g, 11,5 mmol), de NaH (10,5 mmol), provenant de 0,42 g d'une dispersion de 60% de NaH dans l'huile, dans 35 ml de DMSO anhydre est agitée sous argon pendant 20 min, durée à laquelle est ajoutée une solution de 4-cyanoacétophénone (1,45 g, 10,0 mmol) dans 15 ml de DMSO. La solution est agitée pendant 6 h à l'ambiante puis versée sur de la glace (150 g). On extrait alors à l'éther (125 ml, 3 x 50 ml). Le produit brut est alors purifié par trois chromatographies éclair sur silice successives (1° éluant hexane : acétone = 5 : 95, 2° éluant hexane : acétone = 4 : 96, 3° éluant hexane:acétone = 2,5 : 97,5) afin de séparer les (E) et (Z) 4-[1-(5,6,7,8-tétrahydro-5,5,8,8-tétraméthyl-2-naphtalényl)-2-propényl]-benzonitriles. Pour obtenir l'isomère (Z) pur (moins polaire que l'isomère (E)), on vérifie les différentes fractions en HPLC. L'isomère (Z) peut être recristallisé dans l'hexane. L'isomère (E) cristallise dans les fractions issues de la chromatographie éclair, il est filtré, lavé à l'hexane et séché à la pompe à palette, sa pureté est déterminée par analyse HPLC.

On obtient 0,57 g de (E) 4-[1-(5,6,7,8-tétrahydro-5,5,8,8-tétraméthyl-2-naphtalényl)-2-propényl] benzonitrile sous forme d'un solide blanc (Rdt pur = 17,5%) et 0,96 g de (Z) 4-[1-(5,6,7,8-tétrahydro-5,5,8,8-tétraméthyl-2-naphtalényl)-2-propényl]benzonitrile sous forme d'un solide blanc (Rdt = 30%). On obtient également 0, 70 g d'un mélange des deux isomères (Rdt mélange = 18%).

### Isomère (E) :

F (°C) = 173.
RMN¹ H 200MHz (CDCl₃): 1, 29 (2s, 12H, 5, 8-CH3) ; 1, 69 (s, 4H, 6,7-CH₂-) ; 2,29 (d, 3H, Me vinylique *J* 1,3Hz); 6,87 (s large, 1H, H vinylique); 7,13 (dd, 1H, 3-ArH J 8Hz, *J* 1,9Hz); 7,28 (m, 1H, 1-ArH); 7,31 (d, 1H, 4-ArH J 8,4Hz); 7,60 (m, 4H, ArH ortho et méta au CN).
HPLC : Colonne Lichrosorb L5-25F, 5 µ, 250 x 4,6 mm; éluant EtOAc : hexane = 5 : 95.
débit : 1 ml/min, détection UV à 260 nm ; isomère (E) tr = 6,25 min 98,8%

### Isomère (Z) :

F (°C) = 126.
RMN¹H 200MHz (CDCl₃): 0,95 et 1,19 (2s, 12H, 5,8-CH₃); 1,57 (s, 4H, 6,7-CH₂-); 2,16 (d, 3H, Me vinylique J 1,2Hz); 6,49 (d, 1H, H vinylique *J* 1,2Hz); 6,72 (m, 1H, 3-ArH); 6,73 (s, 1H, 1-ArH); 7,07 (d, 1H, 4-ArH *J* 8,6Hz); 7,30 (d, 2H, ArH méta au CN *J* 8Hz); 7,56 (d, 2H, ArH ortho au CN *J* 8Hz).
HPLC colonne Lichrosorb L5-25F, 5 µ, 250 x 4,6 mm; éluant AcOEt : hexane = 5 : 95
débit : 1 ml/min, détection UV à 260 nm, Isomère (Z) tr = 6,8 min 98,3%, impureté isomère (E) tr = 6,29 min 1,1%.

### f) Préparation de l'acide (E) 4-[1-(5,6,7,8-tétrahydro-5,5,8,8-tétraméthyl-2-naphtalényl)-2-propényl] benzoïque de formule :

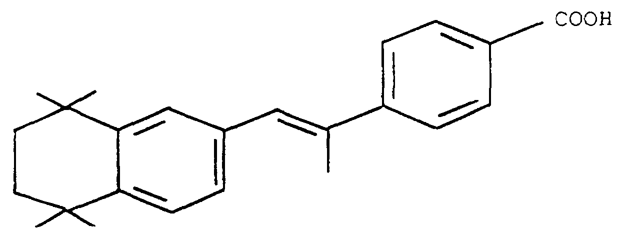

Une suspension du (E) 4-[1-(5,6,7,8-tétrahydro-5,5,8,8-tétraméthyl-2-naphtalényl)-2-propényl] benzonitrile (0,44 g, 1,20 mmol) en solution dans de la potasse (0,94 g, 16,8 mmol) hydroéthanolique (H₂O 0,55 ml et EtOH 3,3 ml) est chauffée à reflux sous agitation magnétique pendant 4 h. L'avancement de la réaction est suivi par CCM (éluant acétone : hexane = 10 : 90). On évapore l'éthanol à l'évaporateur rotatif, reprend à l'eau (50 ml), acidifie par HCl 1N, extrait 3 fois à l'éther, sèche la phase éthérée par MgSO₄, filtre et évapore pour obtenir après un lavage au pentane et séchage 0,40 g d'une poudre blanche, l'acide (E) 4-[1-(5,6,7,8-tétrahydro-5,5,8,8-tétraméthyl-2-naphtalényl)-2-propényl]benzoïque (Rdt = 96%).
F (°C) : 230-2.
RMN¹H 200MHz (CDCl₃) : 1,29 et 1,30 (2s, 12H, 5,8-CH₃); 1,69 (s, 4H, 6,7-CH₃); 2,32 (s, 3H, Me vinylique); 6,91 (s, 1H, H vinylique); 7,16 (d, 1H, 3-ArH *J* 8Hz), 7,29 (s, 1H, 1-ArH); 7,31 (d, 1H, 4-ArH *J* 7,9Hz); 7,60 (d, 2H, ArH méta au COOH *J* 8,4Hz); 7,60 (d, 2H, ArH orto au COOH *J* 8,4Hz).
HPLC : Colonne Ultrasphère ODS, 5 µ, 250 x 4,6 mm, détection UV à 260 nm, éluant MeOH : H₂O = 90:10 + 0,1% TFA, pression environ 2500 psi, acide tr = 12,7 min 99,9%, impureté tr = 9,1 min 0,1%.
IR (pur, cm⁻¹) : 3400-2200, 2928, 1672, 1602, 1424, 1280

| | | | |
|---|---|---|---|
| Microanalyse C₂₄H₂₈O₂ : | | | |
| Tr.%C | 82,06 | %H | 7,95 |
| Calc.%C | 82,72 | %H | 8,10 |

MS EI 70 ev (m/z) : 348 (M⁺, 75%); 333 (M⁺-CH₃, 100).

### g) Préparation du (E) 4-[1-(5,6,7,8-tétrahydro-5,5,8,8-tétraméthyl-2-naphtalényl)-2-propényl] -N-(tétrazol-5-yl)-benzamide (composé CB73364) de formule suivante :

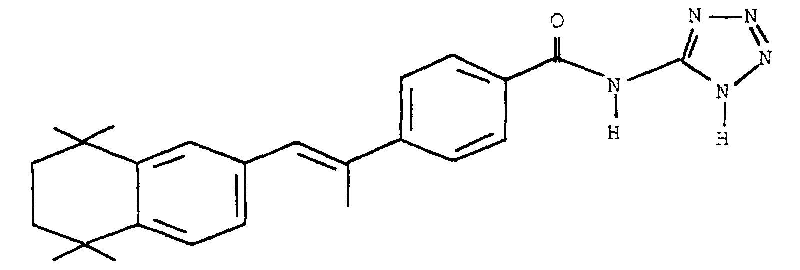

A une solution d'acide (E) 4-[1-(5,6,7,8-tétrahydro-5,5,8,8-tétraméthyl-2-naphtalényl)-2-propényl] benzoïque (0,10 g, 0,29 mmol) dans 5 ml de THF anhydre est additionné à température ambiante 1 équivalent de N,N'-carbonyldiimidazole (Aldrich, 0,46 g, 0,29 mmol). Une demi-heure plus tard on additionne 1 équivalent de 5-aminotétrazole monohydraté (Aldrich, 0,30 g, 0,29 mmol). L'agitation magnétique est poursuivie pendant 15 h 30, un précipité blanc dans le milieu réactionnel s'est formé. On évapore à sec et additionne 10 ml de HCl 1N, filtre la solution, lave à l'eau et au méthanol le filtrat, on obtient après séchage une nuit au dessiccateur (avec P₂O₅) 15 mg d'un solide blanc, le (E) 4-[1-(5,6,7,8-tétrahydro-5,5,8,8-tétraméthyl-2-naphtalényl)-2-propényl]-N-(tétrazol-5-yl)benzamide (CB73364) (Rdt = 12 %).
F (°C) = 318-321.
RMN¹H 200MHz (DMSO D₆) : 1,24 (s, 12H, 5,8-CH₃); 1,64 (s, 4H, 6,7-CH₂-); 2,27 (d, 3H, Me vinylique); 7,03 (s large, 1H, H vinylique); 7,15-7,45 (m, 3H, ArH ); 7,75 (d, 2H, ArH méta au COOH J 8,4Hz); 8,11 (d, 2H, ArH ortho au COOH J 8,4Hz). signal H₂O à 3,58, signal DMSO 2,49.
MS DIC (isobutane) 200 eV (m/z, % intensité): 416 (M⁺+1, 100%); 172 (22), 154 (48).

### Exemple 10 : Préparation du (E) 5-[4-[1-(5,6,7,8-tétrahydro-5,5,8,8-tétraméthyl-2-naphtalényl]-2-propényl]phényl]-1H-tétrazole (composé CB62458) de formule :

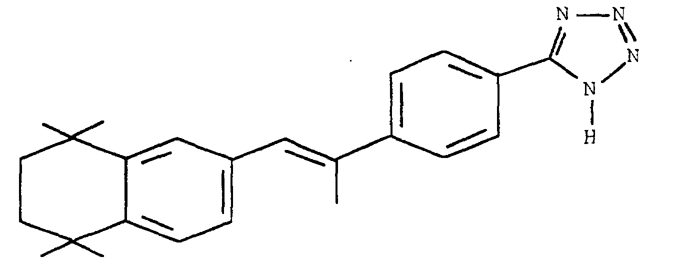

A une solution du E) 4-[1-(5,6,7,8-tétrahydro-5,5,8,8-tétraméthyl-2-naphtalényl)-2-propényl]benzonitrile (0,24 g, 0,73 mmol) dans du toluène anhydre (1,25 ml), on additionne successivement de l'oxyde de dibutylétain (15,1 mg, 10% mol) et 2 équivalents d'azoture de triméthylsilyle (0,19 ml, 1,46 mmol). Le milieu réactionnel est chauffé 16 h à reflux (110°C) sous atmosphère d'argon et agitation magnétique. On purifie par chromatographie éclair sur silice (éluant CH₂Cl₂ puis MeOH : CH₂Cl₂ = 5 : 95) pour obtenir après évaporation, 0,13 g d'une poudre blanche, le (E) 5-[4-[1-(5,6,7,8-tétrahydro-5,5,8,8-tétraméthyl-2-naphtalényl)-2-propényl]phényl]-1*H*-tétrazole (CB62458) (Rdt = 48%).
F (°C) = 224-225.
RMN¹H 200MHz (CDCl₃+DMSO D₆) : 1,02 et 1,03 (2s, 12H, 5,8-CH₃); 1,43 (s, 4H, 6,7-CH₃); 2,08 (d, 3H, Me *J* 1Hz); 6,63 (s large, 1H, H vinylique); 6,85-7,10 (m, 3H, ArH), 7,40 (d, 2H, ArH méta du tétrazoyl *J* 8,4Hz); 7,81 (d, 2H, ArH ortho du tétrazoyl *J* 8,4Hz).
MS EI 70 ev (m/z, % intensité) : 372 (M+, 100%); 357 (28); 344 (74); 329 (31); 313 (23) 298 (13).
MSHR EI 70 ev : Mₜᵣ = 372,2312 pour C₂₄H₂₈N₄ Mₜₕ = 372,2314
HPLC : Colonne ODS, Ultrasphère, 5 µ, 250 x 4,6 mm, détecteur Shimadzu UV, 260 nm, éluant MeOH : H₂O = 90:10 + 0,1% TFA, tétrazole (CB62458) tr = 11,5 min 99;4%, impureté tétrazole (CB92834) tr = 8,5 min 0,5%.

### Exemple 11 : Préparation du (Z) 5-[4-[1-(5,6,7,8-tétrahydro-5,5,8,8-tétraméthyl-2-naphtalényl)-2-propényl] phényl]-1H-tétrazole (composé CB92834) de formule :

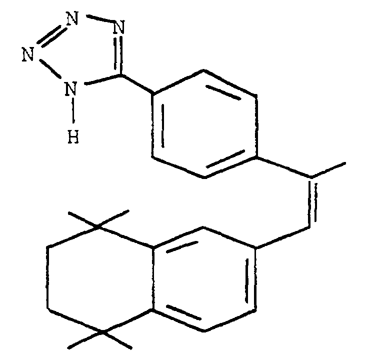

A une solution du (Z) 4-[1-(5,6,7,8-tétrahydro-5,5,8,8-tétraméthyl-2-naphtalényl)-2-propényl]benzonitrile (1,20 g, 3,64 mmol) dans du toluène anhydre (6,25 ml), on additionne successivement de l'oxyde de dibutyletain (75 mg, 10% mol) et de l'azoture de triméthylsilyle (0,97 ml, 7,28 mmol). Le milieu réactionnel est chauffé 16 h à reflux (110°C) sous atmosphère d'argon et agitation magnétique. On purifie par chromatographie éclair sur silice (éluant CH₂Cl₂ puis MeOH : CH₂Cl₂ = 5 : 95) pour obtenir après évaporation, lavage à l'hexane et séchage à la pompe à palette, 0,99 g d'une poudre blanche, le (Z) 5-[4-[1-(5,6,7,8-tétrahydro-5,5,8,8-tétraméthyl-2-naphtalényl)-2-propényl]phényl]-1*H*-tétrazole (CB92834) (Rdt = 73%).
F(°C) = 191-3.
RMN¹H 200MHz (CDCl₃): 0,91 et 1,14 (2s, 12H, 5,8-CH₃); 1,50 (s, 4H, 6,7-CH₃); 2,15 (d, 3H, Me vinylique *J* 1,2Hz); 6,46 (s large, 1H, H vinylique); 6,72 (dd, 1H, 3-ArH *J* 8,1Hz *J* 1,5Hz), 6,82 (d, 1H, 1-ArH *J* 1,6Hz); 7,01 (d, 1H, 4-ArH *J* 8,2Hz); 7,32 (d, 2H, ArH méta du tétrazoyl *J* 8,1Hz); 8,03 (d, 2H, ArH ortho du tétrazoyl *J* 8,1Hz).
RMN¹³C 50MHz (CDCl₃) : 26,7; 31,3; 31,6; 33,8; 33,9; 34,8; 122,3; 126,0; 127,4; 127,6; 127,9; 129,3; 133,7; 136,0; 143,2; 144,1; 148,2.
MS EI 70 ev (m/z, % intensité) : 372 (M⁺, 100%); 35 (M⁺-CH₃, 20); 344 (70); 329 (23); 313 (17).
MSHR EI 70 ev : Mₜᵣ = 372,2332 pour C₂₄H₂₈N₄ Mₜₕ = 372,2314.
HPLC : Colonne ODS, Ultrasphère, 5 µ, 250 x 4,6 mm, détecteur Shimadzu UV, 260 nm, éluant MeOH : H₂O = 90:10 + 0,1% TFA, tétrazole (CB92834) tr = 8,25 min 98,5%, impureté tétrazole (CB62458) tr = 11,2 min 1,5%.

### Exemple 12 : Préparation de l'acide (E) 4-[1-trifluorométhyl-2-(5,6,7,8-tétrahydro-5,5,8,8-tétraméthyl -2-naphtalényl)-1-éthényll benzoïque (composé CB36493) de formule :

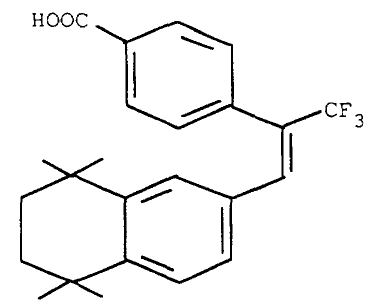

### a) Préparation du 1-(4-benzonitrile)-2,2,2-trifluoroéthanol.

Une solution de 4-cyanobenzaldéhyde (Aldrich, 1,48 g, 11,28 mmol) et de (trifluorométhyl) triméthylsilane (Fluka, 2 ml, 13,53 mmol) dans 12 ml de THF est refroidie à 0°C puis on additionne une quantité catalytique de fluorure de tétrabutylammonium TBAF 1M dans le THF (0,1 ml, 0,1 mmol). Instantanément la solution devient jaune pâle et la solution est portée à température ambiante pendant 4 h. On suit la réaction par chromatographie sur couche mince. On hydrolyse en fin de réaction par HCl 1N, additionne de l'eau puis extrait à l'éther (150 ml), sèche sur MgSO₄, filtre et évapore pour obtenir 2,15 g d'un solide, le 1-(4-benzonitrile)-2,2,2-trifluoroéthanol (Rdt brut = 95 %).
FMN¹H 200 MHz (CDCl₃) : 3,56 (s large, 1H mobile, -OH); 5,08 (q, 1H, BnzH *J* 6,4Hz); 7,55-7,70 (m, 4H, ArH).
RMN¹⁹F 100MHz (CDCl₃) : - 78,64 (d, 3F, *J* 2,9Hz).
IR (pur, cm⁻¹) : 3384, 2242, 1616, 1506, 1410, 1348, 1260, 1130

### b) 1-(4-benzonitrile)-2,2,2-trifluoroéthane-1,1-diol.

On porte à reflux pendant 24 h, sous agitation magnétique vigoureuse et atmosphère d'argon, une solution de 1-(4-benzonitrile)-2,2,2-trifluoroéthanol (2,15 g, 10,7 mmol) et de PCC (3,45 g, 16,0 mmol) dans 150 ml de dichlorométhane. En fin de réaction, on refroidit le milieu réactionnel, filtre sur florisil (Et₂O) où l'on obtient un produit brut contenant le diol attendu en mélange avec environ 15% d'alcool de départ (détection par chromatographie en phase gazeuse). On purifie ce mélange par chromatographie éclair sur silice (éluant Et₂O : éther de pétrole = 20 : 80) pour obtenir une première fraction contenant 0,70 g d'un mélange d'alcool et de diol puis une seconde fraction contenant 1,40 g d'un solide blanc le de 1-(4-benzonitrile)-2,2,2-trifluoroéthane-1,1-diol (Rdt = 60%).

IR (pur, cm⁻¹) : 3240 (large), 2246, 1504, 1406, 1248, 1154, 1056, 1018, 922.

### c) Préparation du 4-(cyano)-α, α,α-trifluoroacétophénone.

Dans un ballon de 100 ml muni d'un Dean-Stark, d'une agitation magnétique, on porte à reflux une solution de 1-(4-benzonitrile)-2,2,2-trifluoroéthane-1,1-diol (1,36 g, 6,26 mmol) dans du toluène (50 ml) pendant 3 h. On évapore à sec après refroidissement du milieu réactionnel, on obtient 1,25 g de 4-(cyano)-α,α,α-trifluoroacétophénone (Rdt brut = 100%).
F (°C) = 105
IR (pur, cm⁻¹) : 2232, 1724, 1606, 1410, 1148, 938, 856.

### d) Préparation du (E) 4-[1-trifluorométhyl-2-(5,6,7,8-tétrahydro-5,5,8,8-tétraméthyl-2-naphtalényl)-1-éthényl]benzonitrile de formule :

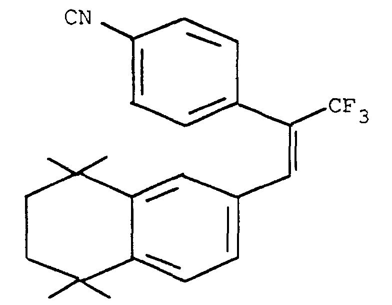

Une solution de bromure de (5,6,7,8-tétrahydro-5,5,8,8-tétraméthyl-2-naphtalényl)méthyl triphénylphosphonium (4,28 g, 7,87 mmol), de NaH (Aldrich, 7,18 mmol) provenant de 0,29 g d'une dispersion de 60% de NaH dans l'huile dans 25 ml de DMSO anhydre est agitée sous argon pendant 20 minutes, durée à laquelle est ajoutée une solution de 4-(cyano)-α,α,α-trifluoroacétophénone (1,25 g, 6,26 mmol) dans 10 ml de DMSO. La solution est agitée pendant 5 h à l'ambiante puis versée sur de la glace (100 g). On extrait alors à l'éther (3 x 50 ml). Le produit brut est alors purifié par chromatographie éclair sur silice (éluant Et₂O : éther de pétrole = 1:99). Chaque fraction est analysée par HPLC pour vérifier qu'il n'y a qu'un isomère et que la pureté est suffisante. On obtient 1,35 g d'un solide le (E) 4-[1-trifluoromèthyl-2-(5,6,7,8-tétrahydro-5,5,8,8-tétraméthyl-2-naphtalényl)-1-éthényl] benzonitrile (Rdt = 56%) que l'on recristallise dans l'hexane pour obtenir 1,10 g de solide pur (Rdt recrist. = 46%). On obtient 0,08 g de (Z) 4-[1-trifluorométhyl-2-(5,6,7,8-tétrahydro-5,5,8,8-tétraméthyl-2-naphtalényl)-1-éthényl] benzonitrile (Rdt = 4%) que l'on recristallise dans l'hexane pour obtenir 0,04 g (Rdt recrist. = 2%).

### Isomère (E) :

F (°C) = 131-132
RMN¹H 200MHz (CDCl₃) : 0,96 et 1,19 (2s, 12H, 5,8-CH₃); 1,58 (s, 4H, 6,7-CH₂); 6,49 (dd, 1H, 3-ArH J 1,8Hz *J* 8,3Hz); 6,77 (d, 1H, 1-ArH *J* 1,8Hz); 7,13 (d, 1H, 4-ArH *J* 8,3Hz); 7,23 (d, 1H, H vinylique *J* 1,8Hz) ; 7,44 (d, 2H, ArH méta au CN *J* 8,2Hz); 7,70 (d, 2H, ArH ortho au CN *J* 8,2Hz).
RMN¹⁹F 100MHz (CDCl₃) : -65,6 (s, 3F, -CF₃).
HPLC: Colonne Lichrosorb L5-25F, 5 µ, 250 x 4,6 mm; éluant AcOEt : hexane = 3 : 97, débit : 1 ml/min, détection UV à 260 nm; isomère (E) tr = 6,6 min 99,8 %.

### Isomère (Z):

RMN¹H 200MHz (CDCl₃) : 1,29 (s, 12H, 5,8-CH₃); 1,69 (s, 4H, 6,7-CH2); 7,05 (s large, 1H, H vinylique); 7,19 (dd, 1H, 3-ArH *J* 8Hz, *J* 1,9Hz); 7,32 (d, 1H, 4-ArH *J* 8Hz); 7,38 (d, 1H, 1-ArH *J* 1,9Hz); 7,56 (d, 2H, ArH méta du CN *J* 8,2Hz); 7,68 (d, 2H, ArH ortho du CN *J* 8,2Hz).
RMN¹⁹F 100MHz (CDCl₃) : -56,6 (s, 3F, -CF₃).
HPLC : Colonne Lichrosorb L5-25F, 5 µ, 250 x 4,6 mm; éluant AcOEt : hexane = 3 : 97, débit : 1 ml/min, détection UV à 260 nm; isomère (Z) tr = 12,6 min 95,2%, isomère (E) tr = 6,6 min 3,5%).

### e) Préparation de l'acide (E) 4-[1trifluorométhyl-2-(5,6,7,8-tétrahydro-5,5,8,8-tétraméthyl -2-naphtalényl)-1-éthényl]benzoïque (composé CB36493) de formule :

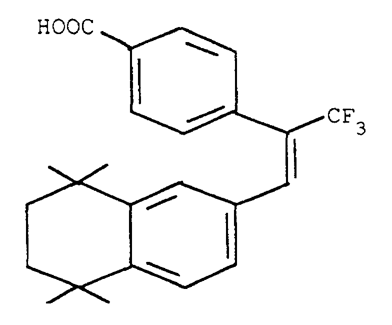

Une suspension du dérivé (E) 4-[1-trifluorométhyl-2-(5,6,7,8-tétrahydro-5,5,8,8-tétraméthyl -2-naphtalényl)-1-éthényl]benzonitrile (0,50 g, 1,30 mmol) en solution dans de la potasse (0,94 g, 16,8 mmol) hydroéthanolique (H₂O 0,55 ml et EtOH 3,3 ml) est chauffée à reflux sous agitation magnétique pendant 5 h. L'avancement de la réaction est suivi par CCM. On évapore l'éthanol à l'évaporateur rotatif, reprend à l'eau (50 ml), acidifie par HCl 1N, extrait (3 x 60 ml) à l'éther, sèche la phase étherée par MgSO₄, filtre et évapore pour obtenir un produit brut que l'on purifie par chromatographie éclair sur silice (éluant Et₂O : éther de pétrole 50 : 50, hauteur de silice = 5 cm). Après évaporation des fractions, on obtient un solide orangé clair que l'on lave au pentane (x 2). Après filtration et séchage à 50°C (24 h) au dessiccateur sous vide, on obtient 0,19 g d'un solide blanchâtre, l'acide (E) 4-[1-trifluorométhyl-2-(5,6,7,8-tétrahydro-5,5,8,8-tétraméthyl-2-naphtalényl)-1-éthényl]benzoique (composé CB36493) pur (Rdt = 36%).
F (°C) : 161-162 (pentane)
RMN¹H 200MHz (CDCl₃) : 0, 96 et 1, 19 (2s, 12H, 5,8-CH₃); 1,57 (s, 4H, 6,7-CH₂-); 6,77 (dd, 1H, 3-ArH *J* 1,8Hz *J* 8,2Hz); 6,88 (d, 1H, 1-ArH *J* 1,8Hz) ; 7,11 (d, 1H, 4-ArH *J* 8,2Hz); 7,21 (d, 1H, H vinylique *J* 1,6Hz); 7,46 (d, 2H, ArH méta du COOH J 8,2Hz); 8,16 (d, 2H, ArH ortho du COOH J 8,2Hz).
RMN¹⁹F 100MHz (CDCl₃) : -65,7 (s, 3F, -CF₃).
MS EI 70 eV (m/z, % intensité) : 402 (M⁺, 51%); 387 (M⁺-CH₃, 100); 345 (16).
HRMS EI 70 eV: Mₜᵣ 402,1815 pour C₂₄H₂₅F₃O₂ Mₜᵣ 402,1807.
HPLC : Colonne ODS, Ultrasphère, 5 µ, 250 x 4,6 mm, détecteur Shimadzu UV, 260 nm, débit 1 ml/min, éluant MeOH : H₂O = 90 : 10 + 0,1% TFA acide (CB36493) tr = 7,63 min 99,5%; impuretés tr = 6,41 min 0,15%; tr = 4,4 min 0,08%.
IR (pur, cm⁻¹) : 2958, 1692, 1608, 1416, 1284, 1160, 1105, 922, 858.

### Exemple 13 : Préparation du (E) 5-[4-[1-trifluorométhyl-2-(5,6,7,8-tétrahydro-5,5,8,8-tétraméthyl-2-naphtalényl)-1-éthényl]phényl]-1H-tétrazole (composé CB77402) de formule :

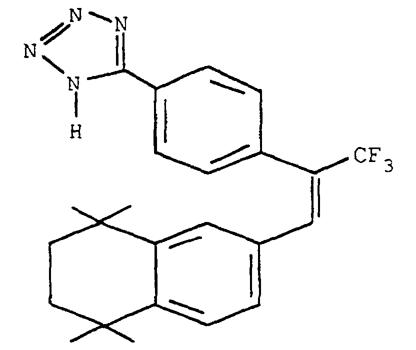

A une solution du (E) 4-[1-trifluorométhyl-2-(5,6,7,8-tétrahydro-5,5,8,8-tétraméthyl-2-naphtalényl)-1-éthényl]benzonitrile (0,24 g, 0,61 mmol) dans du toluène anhydre (1,25 ml), on additionne sucessivement de l'oxyde de dibutylétain (15,1 mg, 10% mol) et de l'azoture de triméthylsilyle (0,16 ml, 1,22 mmol). Le milieu réactionnel est chauffé 16 h à reflux (110°C) sous atmosphère d'argon et agitation magnétique. On purifie par chromatographie éclair sur silice (éluant CH₂Cl₂ puis MeOH : CH₂Cl₂ = 5 : 95) pour obtenir après évaporation puis lavage au pentane 0,15 g d'une poudre verdâtre, le (E) 5-[4-[1-trifluoraméthyl-2-(5,6,7,8-tétrahydro-5,5,8,8-tétraméthyl-2-naphtalényl)-1-éthényl]phényl]-1*H*-tétrazole (CB77402) (Rdt = 58%), que l'on sèche à 50°C (24h) au dessiccateur. On peut recristalliser ce solide avec du chloroforme.
F (°C) = 210-211.
RMN¹H 200MHz (CDCl₃) : 0,94 et 1,16 (2s, 12H, 5,8-CH₃); 1,54 (s, 4H, 6,7-CH₂-); 6,77 (dd, 1H, 3-ArH*J* 1,7Hz *J* 8,2Hz); 6,93 (d, 1H, 1-ArH *J* 1,7Hz); 7,09 (d, 1H, 4-ArH *J* 8,2Hz); 7,21 (d, 1H, H vinylique *J* 1,4Hz); 7,50 (d, 2H, ArH méta du tétrazoyl J 8,2Hz) ; 8,17 (d, 2H, ArH ortho du tétrazoyl J 8,2Hz).
RMN¹⁹F 100MHz (CDCl₃) : - 65,76 (s, 3F, -CF₃).
MS EI 70 eV (m/z, % intensité) : 426 (M⁺, 100%); 411 (M⁺-CH₃, 74); 398 (80); 383 (35); 368 (15); 352 (16); 341 (11); 326 (10).
HRMS EI 70 eV: Mₜᵣ 426,2005 pour C₂₄H₂₅F₃N₄ Mₜₕ 426,2031.
HPLC : Colonne ODS, Ultrasphère, 5 µ, 250 x 4,6 mm, détecteur Shimadzu UV, 260 nm, débit 1 ml/min, éluant MeOH : H₂O = 90 : 10 + 0,1% TFA, tétrazole (CB77402) tr = 6,11 min 98,5% impuretés: acide (CB36493) tr = 7,57 min 1,1% ; inconnue tr = 12,07 min 0,15%.

### Exemple 14 : Préparation de l'acide (E) 4-[1-trifluorométhyl-2-(5,6,7,8-tétrahydro-3,5,5,8,8-pentaméthyl] -2-naphtalényl)-1-éthényl] benzoïque (CB62899) de formule :

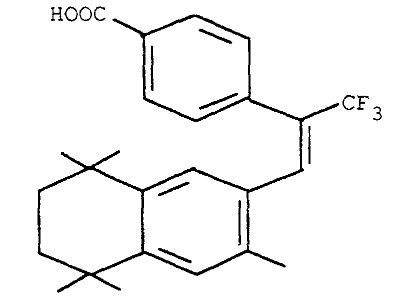

### a) Préparation du (E) 4[1-5,6,7,8-tétrahydro3,5,5,8,8-pentaméthyl-2-naphthalènyl)-2-(trifluorométhyl)-éthén-2-yl)]benzonitrile de formule :

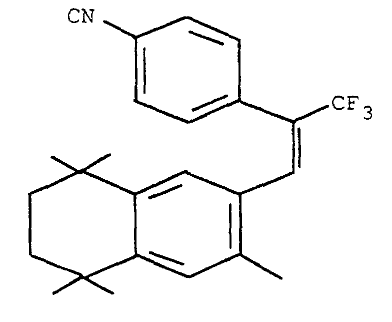

Dans un ballon de 50 ml muni d'une agitation magnétique et sous atmosphère d'argon on a introduit du bromure de (5,6,7,8-tétrahydro-3,5,5,8,8-pentaméthyl-2-naphtalényl)méthyltriphénylphosphonium (1,75 g, 3,14 mmol) en solution dans 10 ml de THF anhydre. On additione à -78°C, goutte à goutte, une solution de tBuOK 1M dans le THF (3,26 ml, 3,26 mmol) et poursuit l'agitation à -70°C pendant 2 h. On additionne à la seringue une solution de 4-(cyano)-α,α,α-trifluoroacétophénone (0,50 g, 2,51 mmol) dans 2,5 ml de THF. On poursuit l'agitation et laisse remonter à température ambiante pendant 4 h 30. On hydrolyse à 0°C par une solution de HCl 6N (7 ml) puis additionne 15 ml d'eau distillée avant d'extraire au dichlorométhane (3 x 50 ml). On sèche sur MgSO₄, filtre et évapore les solvants. Le produit brut est alors purifié par chromatographie éclair sur silice (éluant éther éthylique : éther de pétrole = 1,5 : 98,5). On obtient 0,65 g d'un solide blanc, le (E) 4-[1-trifluorométhyl-2-(5,6,7,8-tétrahydro-3,5,5,8,8-penta- méthyl-2-naphtalényl)-1-éthényl]benzonitrile de formule : 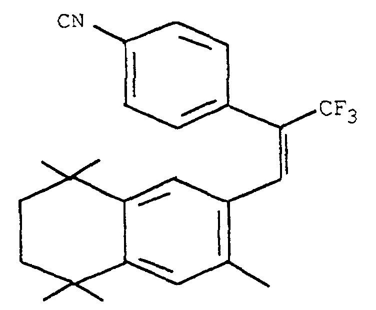 (Rdt = 65%) puis 0,01 g de (Z) 4-[1-trifluorométhyl-2-(5,6,7,8-tétrahydro-3,5,5,8,8-pentaméthyl-2-naphtalényl)-1-éthényl)benzonitrile (Rdt = 1%).

### Isomère (E) :

F (°C) = 89-90.
RMN¹H 200MHz (CDCl₃) : 0,76 et 1,21 (2s, 12H, 5,8-CH₃); 1,40-1,65 (m, 4H, 6,7-CH₂); 2,32 (s, 3H, Me); 6,54 (s, 1H, ArH); 7,06 (s, 1H, H vinylique); 7,36 (d, 2H, ArH); 7,45 (m, 1H, ArH); 7,60 (d, 2H, ArH).
FMN¹⁹F 100MHz (CDCl₃) : -65,0 (s, 3F, -CF₃).

### Isomère (Z):

RMN¹H 200MHz (CDCl₃) : 1,26 (d, 12H, Me *J* 1,6Hz) ; 1,67 (s, 4H, 6,7-CH₂); 2,27 (s, 3H, Me); 7,11 (s, 1H, ArH); 7,13 (s, 1H, ArH); 7,25 (s, 1H, H vinylique); 7,58 (d, 2H, ArH méta au CN J 8,4Hz); 7,70 (d, 2H, ArH ortho au CN J 8,4Hz).
RMN¹⁹F 100MHz (CDCl₃) : -56,8 (s, 3F, -CF₃).

### b) Préparation de l'acide (E) 4-[1-trifluorométhyl-2-(5,6,7,8-tétrahydro-3,5,5,8,8-pentaméthyl -2-naphtalényl)-1-éthényl]benzoïque (composé CB62899) de formule :

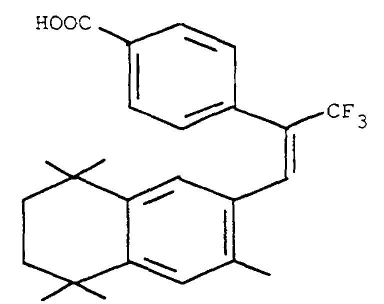

Une suspension du dérivé (E) 4-[1-trifluorométhyl-2-(5,6,7,8-tétrahydro-3,5,5,8,8-pentaméthyl-2-naphtalényl)-1-éthényl]benzonitrile (0,56 g, 1,41 mmol) en solution dans de la potasse (2,00 g, 35,71 mmol) hydroéthanolique (H₂O 6,6 ml et EtOH 1 ml) est chauffée à reflux sous agitation magnétique pendant 5 h. L'avancement de la réaction est suivi par CCM. On évapore l'éthanol à l'évaporateur rotatif, reprend à l'eau (50 ml), acidifie par HCl 1N, extrait (3 x 80 ml) à l'éther, sèche la phase étherée sur MgSO₄, filtre et évapore pour obtenir un produit brut que l'on purifie par chromatographie éclair sur silice (éluant éther : éther de pétrole = 70 : 30). Après évaporation des fractions, on obtient 0,44 g d'un solide blanc, l'acide (E) 4-[1-trifluorométhyl-2-(5,6,7,8-tétrahydro-3,5,5,8,8-penta- méthyl-2-naphtalényl)-1-éthényl]benzoïque (CB62899) pur (Rdt = 75%).
F (°C) = 151 (banc Köfler).
RMN¹H 200MHz (CDCl₃) : 0,73 et 1,19 (2s, 12H, 5,8-CH₃); 1,40-1,60 (m, 4H, 6,7-CH₂-) ; 2,32 (s, 3H, Me); 6,60 (s, 1H, ArH); 7,04 (s, 1H, ArH); 7,37 (d, 2H, ArH J 8,2Hz); 7,42 (s, 1H, H vinylique); 8,06 (d, 2H, ArH J 8,2Hz).
RMN¹⁹F 100MHz (CDCl₃) : -65,1 (s, 3F, -CF₃).
MS El 70 eV (m/z, % intensité): 416 (M⁺, 47%); 401 (M⁺-CH₃, 100); 359 (11).
HRMS EI 70 eV: Mₜᵣ 416,1962 pour C₂₅H₂₇F₃O₂ Mₜᵣ 416,1963.
HPLC : Colonne ODS, Ultrasphère, 5 µ, 250 x 4,6 mm, détecteur Shimadzu UV, 260 nm, débit 1 ml/min, éluant MeOH : H₂O = 90 : 10 + 0,1% TFA acide (CB62899) tr = 9,5 min 97%; impureté tr = 11,2 min 2,65%.

### Exemple 15 : Préparation du (E) 5-[4-[1-trifluorométhyl-2-(5,6,7,8-tétrahydro-3,5,5,8,8-pentaméthyl-2-naphtalényl)-1-éthényl]phényl]-1H-tétrazole (composé CB63237) de formule :

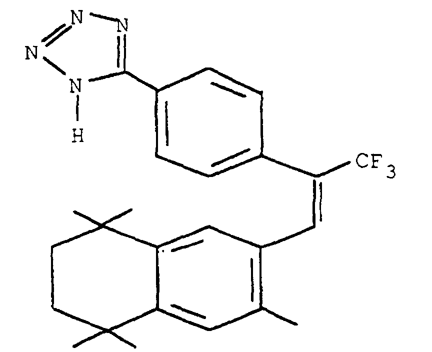

A une solution du (E) 4-[1-trifluorométhyl-2-(5,6,7,8-tétrahydro-3,5,5,8,8-pentaméthyl-2-naphtalényl)-1-éthényl]benzonitrile (0,24 g, 0,60 mmol) dans du toluène anhydre (1,25 ml), on additionne sucessivement de l'oxyde de dibutylétain (15,1 mg, 10% mol) et de l'azoture de triméthylsilyle (0,16 ml, 1,22 mmol). Le milieu réactionnel est chauffé 16 h à reflux (110°C) sous atmosphère d'argon et agitation magnétique. On purifie par chromatographie éclair sur silice (éluant CH₂Cl₂ puis MeOH : CH₂Cl₂ = 5 : 95) pour obtenir après évaporation puis lavage au pentane et séchage 0,13 g d'un solide blanc, le (E) 5-[4-[1-trifluorométhyl-2-(5,6,7,8-tétrahydro-3,5,5,8,8-pentaméthyl-2-naphtalényl)-1-éthényl]phényl]-1*H*-tétrazole (CB63237) (Rdt = 49%).
F (°C) = 203-205.
RMN¹H 200MHz (DMSO) : 0,73 et 1,19 (2s, 12H, 5,8-CH₃); 1,40-1,65 (s, 4H, 6,7-CH₂-); 2,34 (s, 3H, Me); 6,69 (s, 1H, ArH); 7,12 (s, 1H, ArH); 7,46 (d, 2H, ArH J 8,4Hz); 7,54 (s, 1H, H vinylique); 8,02 (d, 2H, ArH *J* 8,4Hz).
RMN¹⁹F 100MHz (CDCl₃) : - 67,9 (s, 3F, -CF₃).
MS EI 70 eV (m/z, % intensité) : 440 (M⁺, 90%); 425 (M⁺-CH₃, 100); 412 (31); 397 (29); 366 (10); 198 (12).
HRMS EI 70 eV: Mₜᵣ = 440,2178 pour C₂₅H₂₇F₃N₄ Mₜₕ = 440,2169.
HPLC : Colonne ODS, Ultrasphère, 5 µ, 250 x 4,6 mm, détecteur Shimadzu UV, 260 nm, débit 1 ml/min, éluant MeOH : H₂O = 90 : 10 + 0,1% TFA, tétrazole (CB63237) tr = 7,60 min 97,7% impuretés tr = 8,9 min 1,9%.

### Exemple 16 : Préparation du (E) [1-(5,6,7,8-tétrahydro-5,5,8,8-tétraméthyl-2-naphtalényl)-1-(phényl)-2-éthényl]benzène (composé CB04854) de formule suivante :

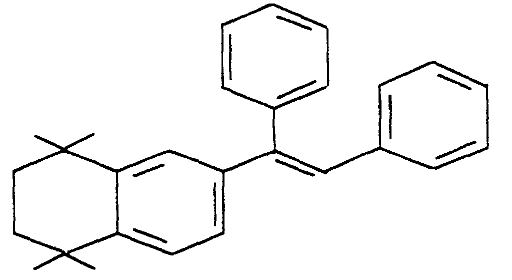

### a) Préparation du 1,1,4,4-tétraméthyl-1,2,3,4-tétrahydronaphtalène.

Dans un tricol de 100 ml muni d'un réfrigérant, d'une agitation magnétique et d'un thermomètre, on pèse du trichlorure d'aluminiun (0,60 g, 4,49 mmol). On additionne 20 ml de benzène anhydre puis, par une ampoule à brome, additionne une solution de 2,5-dichloro-2,5-diméthylhexane (3,00 g, 16,38 mmol) dans 10 ml de benzène anhydre. On chauffe à reflux le milieu réactionnel pendant 4 h. Il y a un important dégagement de HCl et le milieu réactionnel devient rouge sombre. Après refroidissement du milieu réactionnel, il est versé sur un mélange eau-glace (100 ml). On extrait par de l'éther (3 x 50 ml), lave par une solution saturée de NaHCO₃ (2 x 100 ml), sèche la phase éthérée par MgSO₄, filtre et évapore. Le produit brut est purifié par chromatographie éclair sur silice (éluant éther de pétrole). On obtient 1,95 g (Rdt = 63%) d'un liquide incolore, le 1,1,4,4-tétraméthyl-1,2,3,4-tétrahydronaphtalène.
Eb (°C) = 74 sous 0,5 mmHg.
RMN¹H 200 MHz (CDCl₃): 1,28 (s, 12H, 1,4-Me); 1,68 (s, 4H, -CH₂-); 7,05-7,40 (m, 4H, ArH).

### b) Préparation du chlorure du 4-bromophénylacétyle.

A une solution de l'acide 4-bromophénylacétique (Aldrich, 5 g, 23,2 mmol) dans 10 ml de toluène on ajoute à température ambiante le chlorure de thionyle (2,5 ml, 34,8 mmol). Le milieu réactionnel est porté au reflux pendant 2 h. Après retour à température ambiante, on évapore le toluène et on obtient 5,55 g du chlorure du 4-bromophénylacétyle brut (Rdt brut = 100 %).

### c) Préparation du 4-[-1-(5, 6, 7, 8-tétrahydro-5,5,8,8-tétraméthyl-2-naphtalényl)-1-oxo-2-éthyl]bromo benzène de formule :

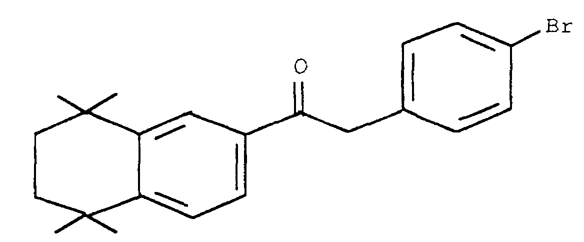

A une solution de 1,1,4,4-tétraméthyl-1,2,3,4-tétrahydronaphtalène (4,36 g, 23,2 mmol) dans 300 ml du dichlorométhane sont ajoutés successivement à - 30 °C du trichlorure d'aluminium (4,33 g, 32,5 mmol) et le chlorure de 4-bromophénylacétyle (5,55 g, 23,2 mmol). On laisse remonter à 0 °C en 30 min, verse le milieu réactionnel sur 400 ml d'un mélange eau-glace volume à volume et extrait au dichlorométhane. La phase organique est séchée sur MgSO₄, filtrée et evaporée. Le produit brut est purifié par cristallisation dans l'hexane pour obtenir 5,69 g d'un solide blanc, le 4-[-1-(5,6,7,8-tétrahydro-5,5,8,8-tétraméthyl-2-naphtalényl)-1-oxo-2-éthyl]bromobenzène (Rdt = 63 %).
F (°C) = 197.
RMN¹H 200MHz (CDCl₃) : 1,31 (s, 6H, 2 Me); 1,32 (s, 6H, 2 Me); 1,72 (s, 4H, 2 -CH₂-); 4,22 (s, 2H, BnzH); 7,16 (d, 2H, ArH *J* 8,5Hz); 7,44 (d, 2H, ArH *J* 8,5Hz); 7,41 (d, 1H, ArH, *J* 8,5Hz); 7,77 (dd, 1H, ArH *J* 1,8Hz J 8,5Hz); 8,02 (d, 1H, ArH *J* 1,8Hz).
RMN¹³C 50MHz (CDCl₃) : 31,6; 31,8; 34,4; 34,7; 34,8; 44,7; 120,8; 125,7; 127,0; 127,1; 131,3 (2C) ; 131,6 (2C); 133,8; 133,9; 145,4; 151,0; 196,6.
MS IC butane (m/z, % intensité) : 387, 385 (MH⁺, 100%, 97%).

### d) Préparation du 4-[-1-(5,6,7,8-tétrahydro-5,5,8,8-tétraméthyl-2-naphtalényl)-1-(phényl)-1-(hydroxy)-2-éthyl]bromobenzène de formule :

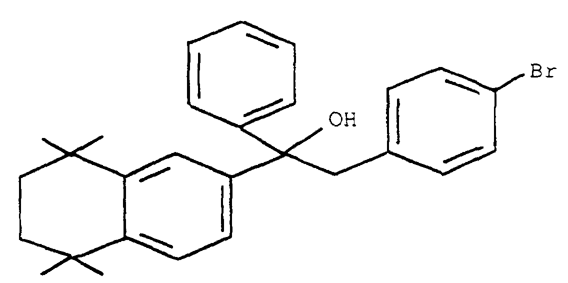

A une solution de 4-[-1-(5,6,7,8-tétrahydro-5,5,8,8-tétraméthyl-2-naphtalényl)-1-oxo-2-éthyl) bromobenzène (3,85 g, 10,0 mmol) dans 30 ml de THF, on ajoute à - 70°C une solution de phényllithium 1,4 M dans un mélange toluène : éther = 3 : 1 (8,6 ml, 12,0 mmol). On poursuit l'agitation à - 70°C pendant 45 min puis hydrolyse par 25 ml d'une solution saturée de NH₄Cl. On remonte à température ambiante et on extrait à l'éther (5 x 50 ml), sèche sur MgSO₄, filtre et évapore. Le produit brut est purifié par chromatographie éclair sur silice (éluant éther de pétrole : éther = 100 : 5). On obtient 2,48 g d'un solide blanc, le 4-[-1-(5,6,7,8-tétrahydro-5,5,8,8-tétraméthyl-2-naphtalényl)-1-(phényl)-1-(hydroxy)-2-éthyl]bromo-benzène (Rdt = 53%).
F (°C) = 130-132.
RMN¹H 200MHz (CDCl₃) : 1,20 (s, 12H, 4 Me); 1,60 (s, 4H, 2 -CH₂-); 3,52 (s, 2H, BnzH); 6,22 (d, 2H, ArH J 8Hz); 7,05 (dd, 1H, J 2Hz J 8Hz); 7,15-7,45 (m, 10H, ArH).

### e) Préparation des (E) et (Z) 4-[1-(5,6,7,8-tétrahydro-5,5,8,8-tètraméthyl-2-naphtalényl)-1-(phényl)-2-éthényl]bromobenzènes de formules respectives suivantes:

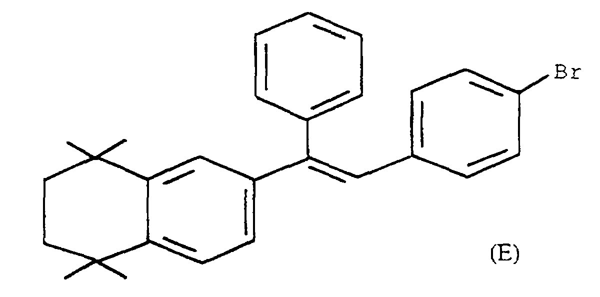

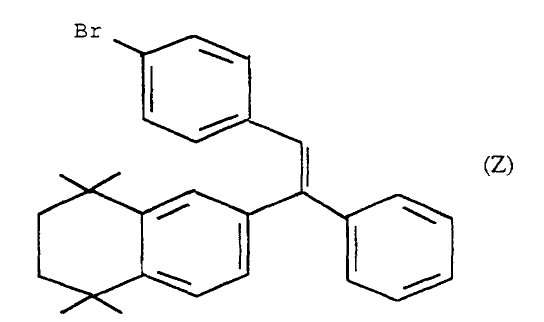

A une solution de 4-[-1-(5,6,7,8-tétrahydro-5,5,8,8-tétraméthyl-2-naphtalényl)-1-(phényl)-1-(hydroxy)-2-éthyl]bromobenzène (2,48 g, 5,34 mmol) dans 26 ml d'éthanol, on additionne à température ambiante une solution de HCl 3N (8, 5 ml, 25,5 mmol). Le milieu réactionnel est porté au reflux pendant 2 h. Après retour à l'ambiante, on ajoute 25 ml d'eau et on extrait à l'éther (5 x 50 ml), sèche sur MgSO₄, filtre et évapore. Le produit brut est recristallisé dans l'éthanol et on obtient après filtration et séchage 0,60 g d'un solide blanc le (E) 4-[1-(5,6,7,8-tétrahydro-5,5,8,8-tétraméthyl-2-naphtalényl)-1-(phényl)-2-éthényl] bromobenzène (Rdt = 25%). Le filtrat est évaporé à sec, puis le produit brut obtenu purifié par chromatographie éclair sur silice (éluant éther de pétrole). On obtient 1,13 g d'une huile, le (Z) 4-[1-(5,6,7,8-tétrahydro-5,5,8,8-tétraméthyl-2-naphtalényl)-1-(phényl)-2-éthényl]bromobenzène (Rdt = 47%) (contenant environ 10% d'isomère (E) déterminé par RMN¹H).

### Isomère (E) :

F (°C) = 147.
RMN¹H 200MHz (CDCl₃) : 1,10 (s, 6H, 2 Me); 1,15 (s, 6H, 2 Me); 1,62 (s, 4H, 2 -CH₂-); 6,80-6,90 (m, 3H, H vinylique et ArH); 6,95-7,05 (m, 1H, ArH); 7,10-7,35 (m, 9H, ArH).

### Isomère (Z) :

F (°C) = 114.
RMN¹H 200MHz (CDCl₃) : 1,05 (s, 6H, 2 Me); 1,27 (s, 6H, 2 Me); 1,65 (s, 4H, 2 -CH₂-); 6,80-6,90 (m, 3H, H vinylique et ArH); 7,07-7,12 (m, 1H, ArH); 7,15-7,40 (m, 9H, ArH).

### f) Préparation du (E) [1-(5,6,7,8-tétrahydro-5,5,8,8-tétraméthyl-2-naphtalényl)-1-(phényl)-2-éthényl]benzène (composé CB04854) de formule :

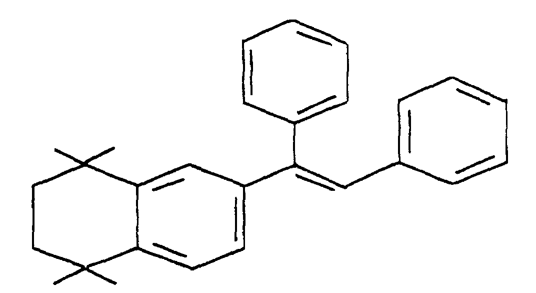

A une solution du (E) 4-[1-(5,6,7,8-tétrahydro-5,5,8,8-tétraméthyl-2-naphtalényl)-1-(phényl)-2-éthényl]bromobenzène (80 mg, 0,18 mmol) dans 2,2 ml de THF, on ajoute à - 70°C une solution de n-butyllithium 1,6 M dans l'hexane (0,12 ml, 0,19 mmol). On agite à - 70°C pendant 30 min puis laisse le milieu réactionnel remonter à - 10°C et on hydrolyse par 3 ml d'une solution de HCl 3N. On extrait à l'éther éthylique (5 x 20 ml), sèche sur MgSO₄, filtre et évapore. Le produit brut est recristallisé dans le méthanol et on obtient après filtration et séchage 50 mg d'un solide blanc, le (E) [1-(5,6,7,8-tétrahydro-5,5,8,8-tétraméthyl-2-naphtalényl)-1-(phényl)-2-éthényl]benzène (CB04854) (Rdt = 75%).
F (°C) = 157.
RMN¹H 200MHz (CDCl₃) : 1,25 (s, 6H, 2 Me); 1,30 (s, 6H, 2 Me); 1,65 (s, 4H, 2 -CH₂-); 6,80-7,10 (m, 6H, H vinylique et ArH); 7,15-7,35 (m, 8H, ArH).
MS EI 70 ev (m/z, % intensité) : 366 (M⁺, 100%); 351 (52); 179 (25).
MSHR EI 70 ev : Mₜᵣ = 366,2339 pour C₂₈H₃₀ Mₜₕ = 366,2347

### Exemple 17 : Préparation de l'acide (E) 4-[1-(5,6,7,8-tétrahydro-5,5,8,8-tétraméthyl-2-naphtalényl)-1-(phényl)-2-éthényl]benzoïque (composé CB01585) de formule :

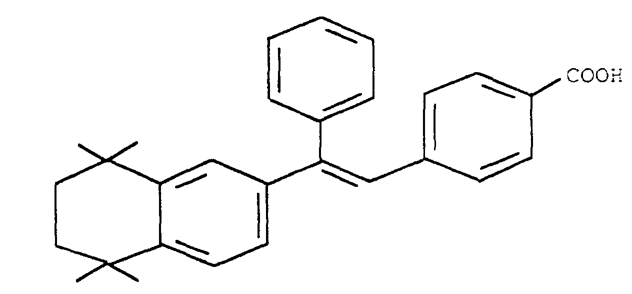

### a) Préparation du (E) 4-[1-(5,6,7,8-tetrahydro-5,5,8,8-tétraméthyl-2-naphtalényl) -1-(phényl)-2-éthényl]benzonitrile de formule :

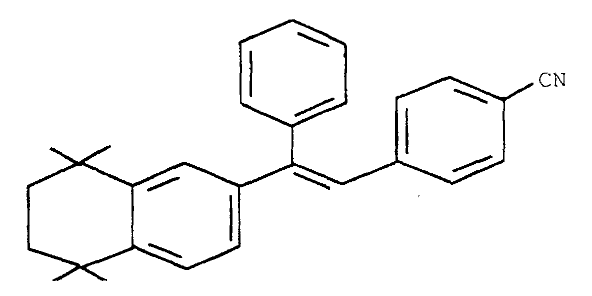

A une solution du (E) 4-[1-(5,6,7,8-tétrahydro-5,5,8,8-tétraméthyl-2-naphtalényl)-1-(phényl)-2-éthényl]bromobenzène (0,24 g, 0,53 mmol) dans 1 ml de DMF anhydre, on ajoute à température ambiante le cyanure de cuivre (55 mg, 0,61 mmol). Le milieu réactionnel est porté au reflux pendant 4 h. Après retour à température ambiante le milieu réactionnel est dilué avec de l'éther (100 ml) et filtré sur célite. On lave la phase organique avec une solution aqueuse saturée en NaHCO₃ (3 x 25 ml) puis sèche sur MgSO₄, filtre et évapore. Le produit brut est purifié par chromatographie éclair sur silice (éluant éther de pétrole : éther = 100 : 2). On obtient 30 mg d'un solide, le (E) 4-[1-(5,6,7,8-tétrahydro-5,5,8,8-tétraméthyl-2-naphtalényl)-1-(phényl)-2-éthényl) benzonitrile (Rdt = 14 %).
F (°C) = 191-193.
RMN¹H 200MHz (CDCl₃) : 1,10 (s, 6H, 2 Me); 1,15 (s, 6H, 2 Me); 6,90 (s, 1H, H vinylique) ; 6,78-7,10 (m, 3H, ArH); 7,15-7,50 (m, 9H, ArH) .
MS EI 70 ev (m/z, % intensité) : 391 (M⁺, 100 %); 376 (90); 204 (40).

### b) Préparation de l'acide (E) 4-[1-(5,6,7,8-tétrahydro-5,5,8,8-tétraméthyl-2-naphtalényl)-1-(phényl)-2-éthényl]benzoïque (composé CB01585).

Une suspension de (E) 4-[1-(5,6,7,8-tétrahydro-5,5,8,8-tétraméthyl-2-naphtalényl)-1-(phényl)-2-éthényl]benzonitrile (30 mg, 0,076 mmol) en solution dans de la potasse (42 mg, 0,75 mmol) hydroéthanolique (H₂O 0,1 ml et EtOH 1 ml) est chauffée à reflux sous agitation magnétique pendant 12 h. On évapore l'éthanol à l'évaporateur rotatif, reprend à l'eau (10 ml), acidifie par HCl 3N, extrait à l'éther éthylique (3 x 15ml), sèche la phase éthérée sur MgSO₄, filtre et évapore. Le produit est lavé par du pentane, filtré et séché. On obtient 20 mg d'un solide blanc, l'acide (E) 4-[1-(5,6,7,8-tétrahydro-5,5,8,8-tétraméthyl-2-naphtalényl)-1-(phényl)-2-éthényl]benzoïque (CB01585) (Rdt = 64%).
F (°C) = 240-241.
RMN¹H 200MHz (CDCl₃) :1,20 (s, 6H, 2 Me); 1,25 (s,6H, 2 Me); 1,65 (s, 4H, 2 -CH₂-); 4,40-5,15 (m, 1H, H mobile); 6,80-7,40 (m, 11H) ; 7,60 (d, 2H, *J* 8,1Hz).
MS EI 70ev (m/z, % intensité) : 410 (M⁺, 100 %); 395 (60,9); 223 (17,5);178 (10,2).
MSHR EI 70 ev : Mₜᵣ = 410,2238 pour C₂₉H₃₀O₂ Mₜₕ = 410,2246.
HPLC Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB01585) tr = 5,0 min 97,0%.

### Exemple 18 : Préparation de l'acide (Z) 2-[1-(5,6,7,8-tétrahydro-5,5,8,8-tétraméthyl-2-naphtalényl)-2-propényl]pyridinyl-5-carboxylique (composé CB38416) de formule :

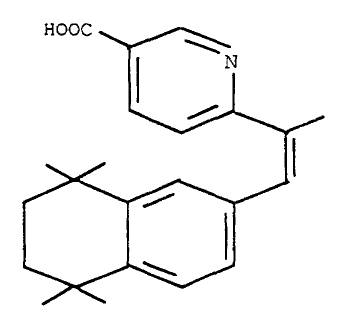

### a) Préparation des (E) et (Z) 2-[1-(5,6,7,8-tétrahydro-5,5,8,8-tétraméthyl-2-naphtalényl)-2-propényl]pyridinyl-5-carboxylates de méthyle de formules respectives suivantes :

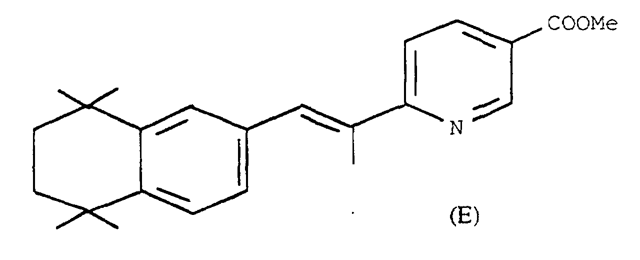

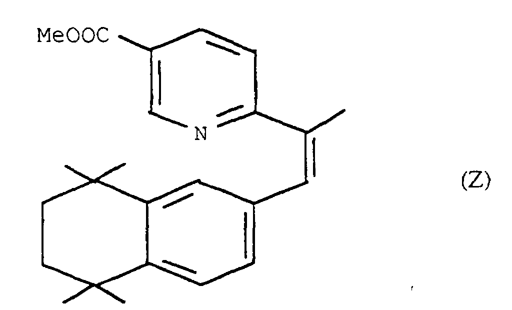

Une solution de bromure de (5,6,7,8-tétrahydro-5,5,8,8-tétraméthyl-2-naphtalényl)-méthyl- triphénylphosphonium (1,80 g, 3,3 mmol), de NaH (Aldrich, 3,3 mmol) provenant de 0,12 g d'une dispersion de 60% de NaH dans l'huile dans 10 ml de DMSO anhydre est agitée sous argon pendant 30 min, puis est ajoutée une solution de 2-acétylpyridine-5-carboxylate de méthyle (0,50 g, 2,79 mmol) dans 1 ml de DMSO. La solution est agitée pendant 70 h à l'ambiante puis versée sur de la glace (50 g). On extrait alors à l'éther (5 x 50 ml). Le produit brut est purifié par chromatographie éclair sur silice (éluant éther de pétrole : éther éther éthylique = 100 : 10). On obtient 0,64 g d'un mélange des (E) et (Z) 2-[1-(5,6,7,8-tétrahydro-5,5,8,8-tétraméthyl-2-naphtalényl)-2-propényl]pyridinyl-5-carboxylates de méthyle (Rdt = 63%) sous forme solide (rapport (E) : (Z) environ 47 : 53 déterminé par RMN¹H 200MHz).

RMN¹H 200MHz (CDCl₃) : 0,95 (s, 6 x 0,53 H, 2 Me, isomère Z); 1,18 (s, 6 x 0,53H, 2 Me, isomère Z); 1,28 (s, 12 x 0,47H, 4 Me, isomère E); 1,55 (s, 4 x 0,47H, 2 -CH₂-, isomère E); 1,68 (s, 4 x 0,53H, 2 -CH₂-, isomère Z); 2,25 (s, 3 x 0,47H, Me vinylique, isomère E); 2,37 (s, 3 x 047H, Me vinylique, isomère Z); 3,91 (s, 3 x 0,53H, -OMe, isomère Z) ; 3,93 (s, 3 x 0,47H, -OMe, isomère E) ; 6,60-6,85 (m, 2H); 7,00-7,40 (m, 2H, ArH); 7,60 (m, 1H, ArH); 8,05-8,45 (m, 1H, ArH) ; 9,15-9,25 (m, 1H).

### b) Acide (Z) 2-[1-(5,6,7,8-tétrahydro-5,5,8,8-tétraméthyl-2-naphtalényl)-2-propényl]pyridinyl-5-carboxylique (composé CB38416)

Une suspension des (E) et (Z) 2-[1-(5,6,7,8-tétrahydro-5,5,8,8-tétraméthyl-2-naphtalényl)-2-propényl]-pyridinyl-5-carboxylates de méthyle et (0,64 g, 1,76 mmol) en solution dans de la potasse (0,5 g, 9 mmol) hydrométhanolique (H₂O 2,2 ml et MeOH 19 ml) est chauffée à reflux sous agitation magnétique pendant 12 h. On évapore le méthanol à l'évaporateur rotatif, reprend à l'eau (50 ml), acidifie par HCl 3N, extrait (3 x 50ml) à l'éther, sèche la phase éthérée sur MgSO₄, filtre et évapore. Le produit brut est purifié par HPLC préparative sur une colonne Waters HR C₁₈ (25 x 100 min) avec comme éluant MeOH : H₂O = 85 : 15 + 0, 1 % de TFA. On obtient 108 mg d'un solide blanc, l'acide (Z) 2-[(1-(5,6,7,8-tétrahydro-5,5,8,8-tétraméthyl-2-naphtalényl)-2-propènyl]pyridinyl-5-carboxylique (CB38416) (Rdt = 17 %).
F (°C) = 234-236.
RMN¹H 200MHz (DMSO-d₆) : 0,91 (s, 6H, 2 Me); 1,13 (s, 6H, 2 Me); 1,52 (s, 4H, 2 -CH₂-); 2,12 (s, 3H, Me vinylique) ; 3,40 (m, 1H); 6,67 (m, 1H, H vinylique) ; 6, 70 (m, 1H, ArH); 7,10 (d, 1H, ArH *J* 8Hz); 7,24 (d, 2H, ArH *J* 8Hz); 8,10 (dd, 1H, ArH *J* 2Hz *J* 8Hz) ; 9,15 (m, 1H, ArH).
MS EI 70 ev (m/z, % intensité) : 349 (56 %) ; 348 (100); 334 (39); 278 (11); 262 (19).
MSHR EI 70 ev : Mₜᵣ = 349,1968 pour C₂₃H₂₇NO₂ Mₜₕ = 349,2042.
HPLC Colonne Waters HR C₁₈, 8 x 100 mm, 6 µ, détecteur UV Waters 486 à 260 nm, débit 3 ml/min, éluant MeOH : H₂O = 85 : 15 + 0,1% de TFA, acide (CB38416) tr = 2,8 min 98,5%.

### Exemple 19 : Préparation de l'acide (E) 5-[1-(5,6,7,8-tétrahydro-5,5,8,8-tétraméthyl-2-naphtalényl)-2-propényl]pyridinyl-2-carboxylique (composé CB12273) de formule :

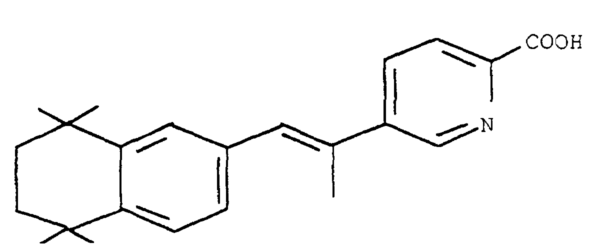

### a) Préparation des (E) et (Z) 5-[1-(5,6,7,8-Tétrahydro-5,5,8,8-tétraméthyl-2-naphtalényl)-2-propényl] pyridinyl-2-(N,N-diisopropyl)carboxamides de formules respectives suivantes :

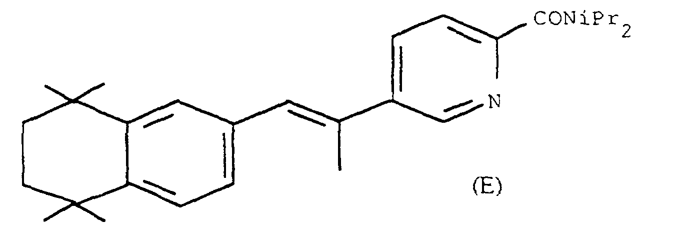

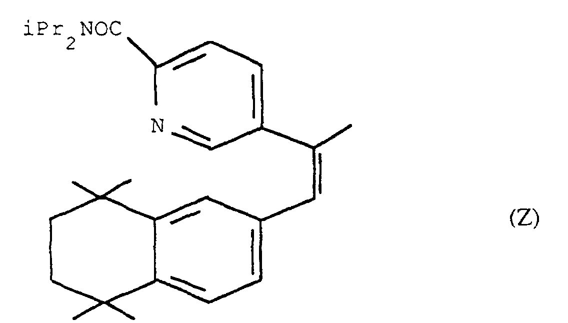

A une solution de bromure de (5,6,7,8-tétrahydro-5,5,8,8-tétraméthyl-2-naphtalényl)méthyltriphénylphosphonium (9,34 g, 17,2 mmol) dans 25 ml de THF, on ajoute à - 70 °C une solution de tBuOK (2,13 g, 18,1 mmol) dans 18,1 ml de THF. On poursuit l'agitation à - 70 °C pendant 1 h avant d'ajouter à cette température le N,N-diisopropyl-5-acétyl-pyridine-2-carboxamide.

Le milieu réactionnel est porté à température ambiante et on poursuit l'agitation pendant 90 h. On hydrolyse ensuite à 0 °C par une solution de HCl 3N (30 ml) . Après retour à température ambiante, on extrait à l'éther, sèche sur MgSO₄, filtre et évapore les solvants. Le produit brut est purifié par chromatographie éclair sur silice (éluant : éther de pétrole : éther = 100 : 25). On obtient 0,60 g d'un solide, le (E) 5-[1-(5,6,7,8-tétrahydro-5,5,8,8-tétraméthyl-2-naphtalényl)-2-propényl]pyridinyl-2-(N,N-diisopropyl)carboxamide puis 0,50 g d'un solide blanc le (Z) 5-[1-(5,6,7,8-tétrahydro-5,5,8,8-tétraméthyl-2-naphtalényl)-2-propényl]pyridinyl-2-(N,N-diisopropyl)carboxamide et 0,78 g du mélange des deux isomères (Rdt global = 60%).

### Isomère (E) :

RMN¹H 200MHz (CDCl₃) :1,05-1,25 (m, 6H, 2 Me); 1,30 (s, 12H, 4 Me); 1,40-1,60 (m, 6H, 2 Me); 1,70 (s, 4H, 2 -CH₂-); 2,30 (s, 3H, Me); 3,50 (m, 1H, -CH-N-); 3,90 (m, 1H, -CH-N-); 6,80 (m, 1H, H vinylique); 7,10-7,18 (m, 1H, ArH); 7,25-7,35 (m, 2H, ArH); 7,45 (d, 1H, ArH *J* 8Hz); 7,62 (dd, 1H, ArH *J* 2Hz et *J* 8Hz); 8,70 (m, 1H, ArH).

### Isomère (Z) :

RMN¹H 200MHz (CDCl₃) : 0,97 (s, 6H, 2 Me); 1,05-1,25 (m, 12H, 4 Me); 1,40-1,70 (m, 10H, 2 -CH₂-et 2 Me); 2,22 (s, 3H, Me); 3,47 (m, 1H, -CH-N-); 4,92 (m, 1H, -CH-N) ; 6,52 (s, 1H, H vinylique); 6,72 (dd, 1H, ArH *J* 2Hz *J* 8Hz) ; 6,85 (m, 1H, ArH); 7,05 (d, 1H, ArH *J* 8Hz); 7,30-7,40 (m, 1H, ArH); 7,57 (dd, 1H, ArH *J* 2Hz *J* 8Hz); 8,37 (m, 1H, ArH).

### b) Préparation de la (E) 5 - [1 - (5,6,7,8-tétrahydro-5,5,8,8-tétraméthyl-2-naphtalényl)-2-propényl]-2-formylpyridine de formule :

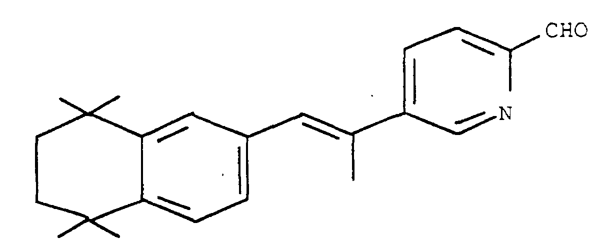

A une solution du (E) 5-[1-(5,6,7,8-tétrahydro-5,5,8,8-tétraméthyl-2-naphtalényl)-2-propényl] pyridinyl-2-(N,N-diisopropyl)carboxamide (0,51 g, 1,40 mmol) dans 7 ml de THF, on ajoute à -70 °C une solution d'hydrure de diisobutylaluminium 1,5 M dans le toluène (1 ml, 1,47 mmol) . On agite à cette température 30 min et on hydrolyse par une solution aqueuse de HCl 3N (5 ml), on remonte à température ambiante et on extrait au dichlorométhane (5 x 20 ml). La phase organique est séchée sur MgSO₄, filtrée et évaporée. Le produit brut est purifié par chromatographie éclair sur silice (éluant éther de pétrole : éther = 100 : 20). On obtient 0,14 g d'un solide blanc, la (E) 5-[1-(5,6,7,8-tétrahydro-5,5,8,8-tétraméthyl-2-naphtalényl)-2-propényl]-2-formylpyridine (Rdt = 29 %).

RMN¹H 200MHz (CDCl₃) : 1,27 (s, 12H, 4 Me); 1,67 (s, 4H, 2 -CH₂-); 2,30 (s, 3H, Me); 6,93 (s, 1H, H vinylique); 7,10-7,20 (m, 1H, ArH); 7,28-7,37 (m, 2H, ArH); 7,93 (m, 2H, ArH); 8,90 (m, 1H; ArH); 10,07 (s, 1H, -CHO).

### c) Préparation de la (Z) 5-[1-(5,6,7,8-tétrahydro-5,5,8,8-tétraméthyl-2-naphtalényl)-2-propényl]-2-formylpyridine de formule :

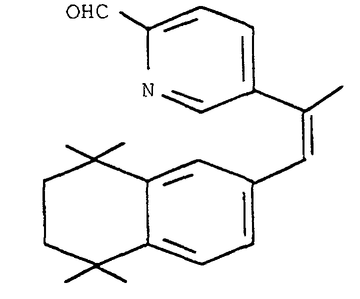

A une solution de la (Z) 5-[1-(5,6,7,8-tétrahydro-5,5,8,8-tétraméthyl-2-naphtalényl)-2-propényl] pyridinyl-2-(N,N-diisopropyl)carboxamide (0,26 g, 0,71 mmol) dans 3,5 ml de THF, on ajoute à -70 °C une solution d'hydrure de diisobutylaluminium 1,5M dans le toluène (0,5 ml, 0,75 mmol). On agite à cette température 30 min et on hydrolyse par une solution aqueuse de HCl 3N (5 ml), on remonte à température ambiante et on extrait au dichlorométhane (5 x 20 ml). La phase organique est séchée sur MgSO₄, filtrée et évaporée. Le produit brut est purifié par chromatographie éclair sur silice (éluant éther de pétrole : éther = 100 : 20). On obtient 0,15 g d'un solide, la (Z) 5-[1-(5,6,7,8-tétrahydro-5,5,8,8-tétramethyl-2-naphcalényl)-2-propényl]-2-formylpyridine (Rdt = 63 %).

RMN¹H 200MHz (CDCl₃) : 0,93 (s, 6H, 2 Me); 1,20 (s, 6H, 2 Me ) 1,55 (s, 4H, 2 -CH₂-); 2,20 (s, 3H, Me); 6,62 (s, 1H, H vinylique); 6,67-6,78 (m, 2H, ArH); 7,05 (d, 1H, ArH J 8Hz); 7,65-7,72 (m, 1H, ArH); 7,87 (d, 1H, ArH J 8Hz) ; 8,55 (m, 1H, ArH); 9,98 (s, 1H, -CHO) .

### d) Préparation des (E) et (Z) 5-[-1-(5,6,7,8-tétrahydro-5,5,8,8-tétraméthyl-2-naphtalényl)-2-propényl]pyridinyl-2-carboxylates de méthyle de formules respectives suivantes :

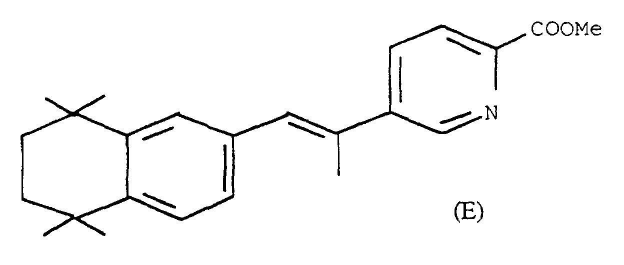

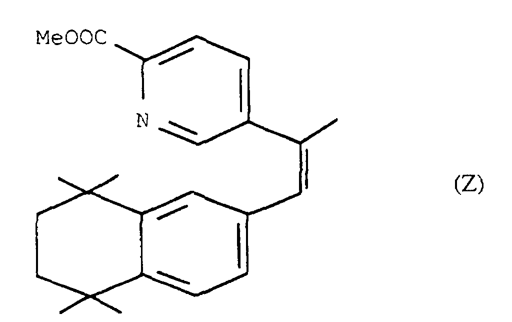

A une solution du mélange des (E) et (Z) 5-[1-(5,6,7,8-tétrahydro-5,5,8,8-tétraméthyl-2-naphtalényl)-2-propényl]-2-formylpyridines (0,72 g, 2,17 mmol) dans 60 ml de méthanol, on ajoute successivement 0,185 ml d'acide acétique, de l'oxyde de manganèse (3,98 g, 43,3 mmol) et du cyanure de sodium (0,53 g, 10,85 mmol) à température ambiante. Le milieu réactionnel est agité à cette température pendant 15 h. On filtre sur papier, évapore et reprend par 20 ml d'eau, extrait à l'éther (5 x 50 ml), sèche sur MgSO₄, filtre et évapore. Le produit brut est purifié par chromatographie éclair sur silice (éluant éther de pétrole : éther = 100 : 20 puis 100 : 25). On obtient après évaporation des fractions 0,13 g d'un solide, le (Z) 5-[1-(5,6,7,8-tétrahydro-5,5,8,8-tétraméthyl-2-naphtalényl)-2-propényl]pyridinyl-2-carboxylate de méthyle (Rdt = 16 %) puis 0,11 g d'un solide, le (E) 5-[1-(5,6,7,8-tétrahydro-5,5,8,8-tétraméthyl-2-naphtalényl)-2-propényl]pyridinyl-2-carboxylate de méthyle (Rdt = 14 %).

### Isomère (E) :

RMN¹H 200MHz (CDCl₃) :1,25 (s, 12H, 4 Me); 1,70 (s, 4H, 2 -CH₂-); 2,35 (s, 3H, Me); 4,00 (s, 3H, -OMe); 6,90 (s, 1H, H vinylique); 7,12 (dd, 1H, ArH J 2Hz J 8Hz); 7,25-7,35 (m, 2H, ArH); 7,87 (dd, 1H, ArH J 2Hz J 8Hz); 8,10 (d, 1H, ArH *J* 8Hz) ; 8, 85 (m, 1H, ArH).

### Isomère (Z) :

RMN¹H 200MHz (CDCl₃) : 0,90 (s, 6H, 2Me); 1,17 (s, 6H, 2 Me); 1,52 (s, 4H, 2 -CH₂-); 2,40 (s, 3H, Me); 4,00 (s, 3H, -OMe); 6,50-6,85 (m, 3H, H vinylique et ArH); 6, 95-7,15 (m, 1H, ArH); 7,20-7,75 (m, 2H, ArH); 8,02 (d, 1H, ArH *J* 8Hz); 8,47 (m, 1H, ArH).

### e) Préparation de l'acide (E) 5-[1-(5,6,7,8-tétrahydro-5,5,8,8-tétraméthyl-2-naphtalényl)-2-propényl]pyridinyl-2-carboxylique (CB12273)

Une suspension de (E) 5-[1-(5,6,7,8-tétrahydro-5,5,8,8-tétraméthyl-2-naphtalényl)-2-propényl] pyridinyl-2-carboxylate de méthyle, (80 mg, 0,22 mmol) en solution dans de la potasse (120 mg, 2,18 mmol) hydrométhanolique (H₂O 0,3 ml et MeOH 2,4 ml) est chauffée à reflux sous agitation magnétique pendant 3 h 30. On évapore le méthanol à l'évaporateur rotatif, reprend à l'eau (10 ml), acidifie par HCl 3N, extrait (3 x 20ml) à l'éther, sèche la phase éthérée sur MgSO₄, filtre et évapore. Le produit brut est lavé au pentane puis filtré , on obtient 70 mg d'un solide blanc, l'acide (E) 5-[1-(5,6,7,8-tétrahydro-5,5,8,8-tétraméthyl-2-naphtalényl)-2-propényl]pyridinyl-2-carboxylique (CB12273) (Rdt = 91 %).
F(°C) = 192.
RMN¹H 200MHz (CDCl₃) : 1,28 (s, 12H, 4 Me); 1,69 (s, 4H, 2 -CH₂-); 2,34 (s, 3H, Me); 6,85-7,05 (m, 1H, H vinylique); 7,10-7,20 (m, 1H, ArH); 7,30-7,40 (m, 2H, ArH); 8,0-8,10 (m, 1H, ArH); 8,15-8,30 (m, 1H, ArH); 8,75-8,95 (m, 1H, ArH).
MS EI 70 ev (m/z, % intensité) : 349 (M⁺, 100%); 334 (96); 316 (20); 190 (20).
MSHR EI 70 ev : Mₜᵣ = 349,2045 pour C₂₃H₂₇NO₂ Mₜₕ = 349,2042.
IR (cm⁻¹) : 3738; 2994; 1706; 1578; 1464.

### Exemple 20 : Préparation de l'acide (E) 2-[1-(5,6,7,8-tétrahydro-5,5,8,8-tétraméthyl-2-naphtalényl)-2-propényl)]thiényl-4-carboxylique composé (CB80660) de formule :

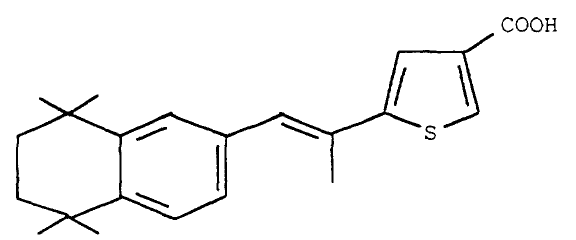

### a) Préparation du 4-bromo-2-(1'-hydroxyéthyl)-thiophène.

Dans un ballon muni d'un thermomètre, sous atmosphère d'argon et agitation magnétique, on porte à -70°C une solution de 4-bromo-2-thiophènecarboxaldéhyde (Aldrich, 1,20 g, 6,28 mmol) dans 15 ml d'éther éthylique anhydre. On additionne alors, goutte à goutte, une solution de MeLi 1,6 M dans l'éther (4,12 ml, 6,59 mmol) entre -70°C et -60°C. On poursuit l'agitation pendant 40 min à -60°C. La réaction est suivie par CCM (éluant éther : éther de pétrole = 20 : 80). On hydrolyse à froid par 5 ml d'une solution saturée de NH₄Cl, additionne 20 ml d'eau distillée et extrait à l'éther (3 x 40 ml). On sèche sur MgSO₄ la phase éthérée, filtre et évapore pour obtenir 1,28 g d'une huile légèrement jaunâtre, le 4-bromo-2-(1'-hydroxyéthyl)thiophène (Rdt brut = 98,5%).

FMN¹H 200MHz (CDCl₃) : 1,53 (d, 3H, Me J 6Hz); 2,35 (s large, 1H mobile, -OH); 5,02 (q, 1H, -CHOH J 6,0Hz); 6,84 (d, 1H, ArH *J* 1,5Hz); 7,09 (d, 1H, ArH *J* 1,5Hz).

### b) Préparation du 4-bromo-2-acétyl-thiophène.

Dans un ballon muni d'une agitation magnétique et sous atmosphère d'argon, on a introduit une solution de 4-bromo-2-(1'-hydroxyéthyl)thiophène brut (1,28 g, 6,18 mmol) et de PCC (2,66 g, 13,26 mmol) dans 20 ml de dichlorométhane. On agite vigoureusement pendant 2 h à température ambiante, le milieu réactionnel devient progressivement noir. On filtre alors sur colonne de florisil (éluant éther), évapore le solvant et purifie le produit brut obtenu par chromatographie éclair sur silice (éluant éther de pétrole pur). On obtient 1,10 g d'un solide blanc, le 4-bromo-2-acétyl-thiophène (Rdt = 87%).

RMN¹H 200MHz (CDCl₃) : 2,50 (s, 3H, Me); 7,50 (s, 1H, ArH); 7,54 (s, 1H, ArH).

### c) Préparation des (E) et (Z) 2-[1-(5,6,7,8-tétrahydro-5,5,8,8-tétraméthyl-2-naphtalényl)-2-propényl))-4-bromothiophènes de formules respectives suivantes :

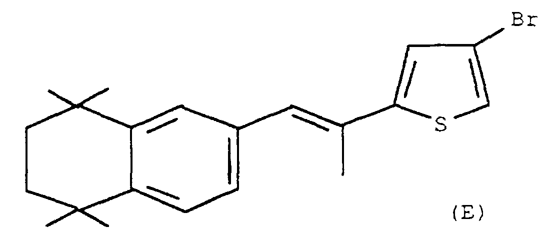

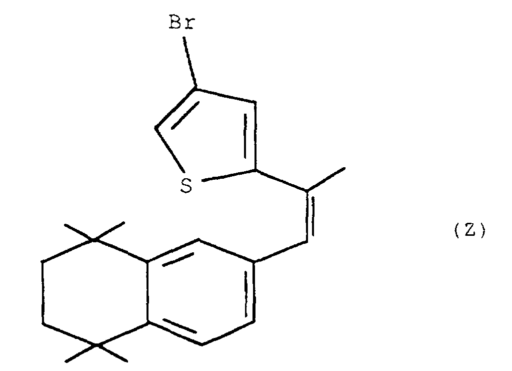

Dans un bicol de 100 ml, muni d'un thermomètre, d'une agitation magnétique et sous atmosphère d'argon, on a pesé du bromure de (5,6,7,8-tétrahydro-5,5,8,8-tétraméthyl-2-naphtalényl)méthyl- triphénylphosphonium (7,00 g, 12,56 mmol) mis en solution dans 20 ml de THF anhydre. On additionne à -70°C du tBuOK 1M dans le THF et poursuit l'agitation à -70°C pendant 1 h. On additionne alors le 4-bromo-2-acétyl-thiophène (1,29 g, 6,28 mmol) en solution dans 15 ml de THF anhydre puis laisse remonter doucement le milieu réactionnel à température ambiante et poursuit l'agitation pendant 72 h. On verse alors le milieu réactionnel dans une solution eau-glace et extrait à l'éther (5 x 50 ml). La phase organique est séchée par du MgSO₄, filtrée et évaporée pour conduire à un produit brut que l'on purifie par chromatographie éclair sur silice (éluant éther de pétrole pur). On obtient 1,97 g d'une huile incolore composeé du mélange d'isomères des (E) et (Z) 2-[1-(5,6,7,8-tétrahydro-5,5,8,8-tétraméthyl-2-naphtalényl)-2-propényl)]-4-bromothiophènes (Rdt global = 80%) (rapport (E) : (Z) = 60 : 40 déterminé par FMN¹H).

RMN¹H 200 MHz (CDCl₃) : 1,15 (s, 6 x 0,40 H, 2 Me, isomère Z); 1,25 (s, 6 x 0,40 H, 2 Me, isomère Z); 1,30 (s, 12 x 0,60 H, 4 Me, isomère E); 1,62 (s, 4 x 0,40 H, 2 -CH₂-, isomère Z); 1,70 (s, 4 x 0,60 H, 2 -CH₂, isomère E); 2,20 (d, 3 x 0,40 H, Me *J* 2,9Hz, isomère Z); 2,27 (d, 3 x 0,60 H, Me *J* 2,9Hz, isomère E); 6,51 (s large, 1 x 0,40 H, H vinylique, isomère Z); 6,75-7,35 (m, 5H, ArH isomères E et Z et 1 x 0,60 H, H vinylique isomère E).

### d) Préparation du (E) 2-[1-(5, 6, 7, 8-tétrahydro-5,5,8,8-tétraméthyl-2-naphtalényl)-2-propényl)]thiényl-4-carboxylate de méthyle de formule suivante :

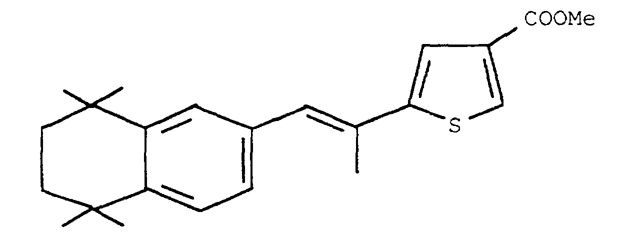

Dans un ballon de 25 ml muni d'une agitation magnétique, d'un thermomètre et sous atmosphère d'argon on a introduit une solution de (E) et (Z) 2-[1-(5,6,7,8-tétrahydro-5,5,8,8-tétraméthyl-2-naphtalényl)-2-propényl)]-4-bromothiophènes Z et E (1,22 g, 3,13 mmol) en solution dans 12,5 ml de THF anhydre. On porte le milieu réactionnel à -70°C et additionne goutte à goutte une solution de t-butyllithium 1,7 M dans le pentane (3,78 ml, 6,42 mmol). Le milieu réactionnel s'assombrit et l'on agite pendant 20 min à -70°C puis additionne du chloroformiate de méthyle (1,5 éq., 4,70 mmol) et porte le milieu réactionnel à température ambiante pendant 30 min. On additionne à 0°C 25 ml d'eau distillée et extrait à l'éther (3 x 50 ml), sèche la phase organique sur MgSO₄, filtre et évapore. On obtient un produit brut que l'on purifie par chromatographie éclair sur silice (éluant éther de pétrole puis éther : éther de pétrole = 2 : 98). On obtient 0,10 g d'un solide blanc le (E) 2-[1-(5,6,7,8-tétrahydro-5,5,8,8-tétraméthyl-2-naphtalényl)-2-propényl)]thiényl-4-carboxylate de méthyle après recristallisation dans l'hexane (Rdt = 8%).

RMN¹H 200MHz (CDCl₃) : 1,28 (s, 12H, Me); 1,68 (s, 4H, 6,7-CH₂-); 2,29 (d, 3H, Me *J* 2,9Hz); 3,87 (s, 3H, -OMe); 7,02-7,33 (m, 5H, ArH et H vinylique); 7,68 (d, 1H, ArH *J* 10Hz).

### e) Préparation de l'acide (E) 2-[1-(5,6,7,8-tétrahydro-5,5,8,8-tétraméthyl-2-naphtalényl)-2-propényl)]thiényl-4-carboxylique (composé CB80660).

Dans un ballon de 10 ml muni d'un réfrigérant et sous agitation magnétique, on a préalablement introduit une suspension de (E) 2-[1-(5,6,7,8-tétrahydro-5,5,8,8-tétraméthyl-2-naphtalényl)-2-propényl)]thiényl-4-carboxylate de méthyle (88 mg, 0,24 mmol) dans 3 ml de méthanol et 1,5 ml d'une solution aqueuse de KOH 5N. On porte à reflux pendant 6 h, refroidit le milieu réactionnel, acidifie par HCl 3N jusqu'à pH = 1 et extrait à l'éther (3 x 30 ml). On sèche la phase éthérée sur MgSO₄, filtre et évapore. Le produit brut est lavé dans un minimum d'hexane et l'on obtient 40 mg d'un solide blanc l'acide (E) 2-[1-(5,6,7,8-tétrahydro-5,5,8,8-tétraméthyl-2-naphtalényl)-2-propényl)]-thiényl-4-carboxylique (CB80660) (Rdt = 47%).
F (°C) = 232-235.
RMN¹H 200MHz (CDCl₃) : 1,28 (s, 12H, 4 Me); 1,69 (s, 4H, -CH₂-); 2,30 (s, 3H, Me); 7,00-7,35 (m, 5H, ArH et H vinylique), 7,77 (d, 1H, ArH *J* 10Hz).
MS EI 70 eV (m/z, % intensité): 354 (M⁺, 100%); 339 (M⁺-CH₃, 76) ; 297 (5); 155 (5) .
MSHR EI 70 eV: Mₜᵣ 354,1661 pour C₂₂H₂₆O₂S Mₜₕ 364,1654
IR (pastilles NaCl, cm⁻¹) : 1668 (C=O).
HPLC Colonne Ultrasphère ODS, 5 µ, 250 x 4,6 mm, détection UV, 260 nm, débit 1 ml/min éluant MeOH : H₂O = 100:0 + 0,1% TFA, acide (CB80660) tr = 4,6 min 98,9 %, impureté tr = 4,1 min 1,1 %.

### Exemples 21 et 22 : Préparation de l'acide (Z) 2-[1-(5,6,7,8-tétrahydro-5,5,8,8-tétraméthyl-2-naphtalényl)-2propényl)]thiényl-5-carboxylique (composé CB30382) de formule :

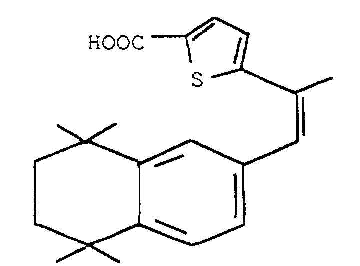

### Préparation de l'acide (E) 2-[1-(5,6,7,8-tétrahydro-5,5,8,8-tétraméthyl-2-naphtalényl)-2-propényl)]thiényl-5-carboxylique (composé CB38973) de formule :

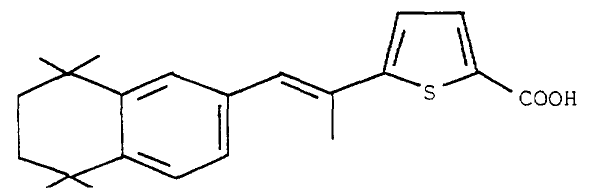

### a) (E) et (Z) 2-[1-(5,6,7,8-tétrahydro-5,5,8,8-tétraméthyl-2-naphtalényl)-2-propényl)]-5-bromothiophènes de formules respectives suivantes :

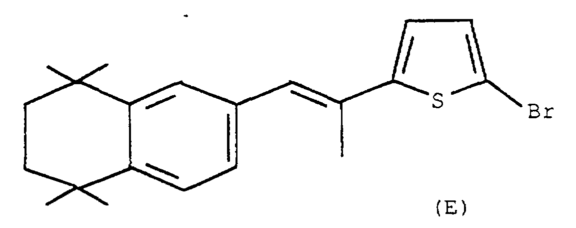

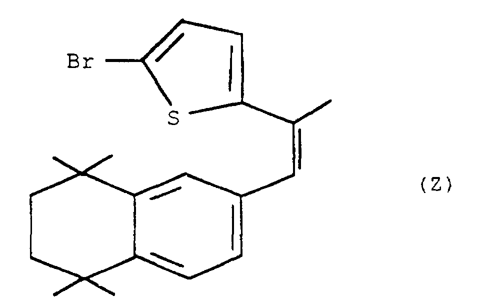

A une solution de bromure de (5,6,7,8-tétrahydro-5,5,8,8-tétraméthyl-2-naphtalényl)méthyltriphénylphosphonium (3,34 g, 6,15 mmol) dans 9 ml de THF, on ajoute à - 70 °C une solution de tertiobutylate de potassium 1M dans le THF (6,50 ml, 6,50 mmol). On poursuit l'agitation à - 70 °C pendant 1 h avant d'ajouter le 2-acétyl-5-bromothiophène (0,63 g, 3,10 mmol). Le milieu réactionnel est porté à température ambiante et on agite pendant 20 h. On hydrolyse ensuite à 0°C par une solution de HCl 3N. Après retour à température ambiante, on extrait à l'éther, sèche sur MgSO₄, filtre et évapore les solvants. Le produit brut est purifié par chromatographie éclair sur silice (éluant éther de pétrole). On obtient 0,46 g des (E) et (Z) 2-[1-(5,6,7,8-tétrahydro-5,5,8,8-tétraméthyl-2-naphtalényl)-2-propényl)]-5-bromothiophènes (Rdt = 38 %) (rapport (Z) : (E) = 70 : 30 déterminé par RMN¹H).

RMN¹H 200MHz (CDCl₃) : 1,25-1,40 (m, 12H, 4 Me); 1,65 (s, 0,3 x 4H, 2 -CH₂- isomère E); 1,70 (s, 0,7 x 4H, 2 -CH₂- isomère Z); 2,18 (s, 0,3 x 3H, Me vinylique isomère E); 2,25 (s, 0,7 x 3H, Me vinylique isomère Z); 6,45 (s, 0,30 x 1H, H vinylique isomère E); 6,67 (d, 0,7 x 1H, H vinylique isomère Z *J* 5Hz); 6,80-6,87 (m, 2H, ArH); 6,92-7,00 (m, 1H, ArH); 7,05-7,30 (m, 2H, ArH).

### b) Préparation des acides (E) et (Z) 2-[1-(5,6,7,8-tétrahydro-5,5,8,8-tétraméthyl-2-naphtalényl)-2-propényl)]thiényl-5-carboxyliques respectivement désignés composés CB38973 et CB30382 et répondant aux formules :

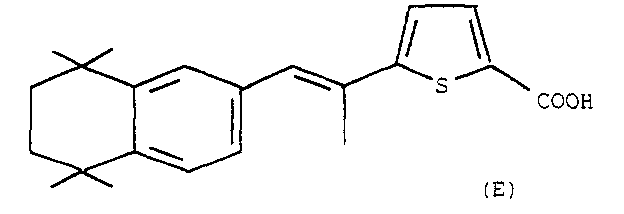

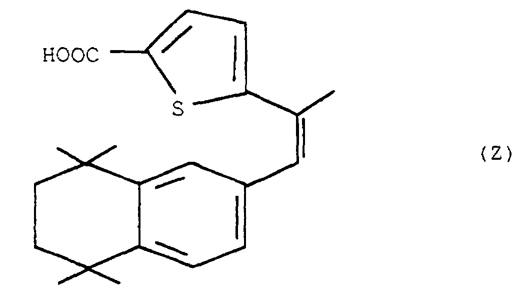

A une solution des (E) et (Z) 2-[1-(5,6,7,8-tétrahydro-5,5,8,8-tétraméthyl-2-naphtalényl)-2-propényl)]-5-bromothiophènes (0,46 g, 1,18 mmol) dans 14 ml de THF, on ajoute à -70 °C une solution de n-butyllithium 1,6 M dans l'hexane (0,8 ml, 1,18 mmol) et on agite 30 min. On fait barbotter un courant de dioxyde de carbone à travers le milieu réactionnel et on agite 30 min à - 70 °C. On laisse remonter à -10 °C et hydrolyse par une solution de HCl 3N (15 ml). On extrait à l'ether, sèche sur MgSO₄, filtre et évapore. Le produit brut est purifié par HPLC préparative sur une colonne Waters HR C₁₈ (25 x 100 mm) avec comme éluant MeOH : H₂O = 90 : 10 + 0,1% de TFA. On obtient 101,2 mg d'un solide jaunâtre, l'acide (E) 2-[1-(5,6,7,8-tétrahydro-5,5,8,8-tétraméthyl-2-naphtalényl)-2-propényl)]thiényl-5-carboxylique (CB38973) (Rdt = 25 %) et 62,9 mg d'un solide jaunâtre, l'acide (Z) 2-[1-(5,6,7,8-tétrahydro-5,1,8,8-tétraméthyl-2-naphtalényl)-2-propényl)]thiényl-5-carboxylique (CB30382) (Rdt = 16 %).

Acide (Z) 2-[1-(5,6,1,8-tétrahydro-5,5,8,8-tétraméthyl-2-naphtalényl)-2-propényl)]thiényl-5-carboxylique (CB30382) :
F (°C) = 165-167.
RMN¹H 200MHz (CDCl₃) : 1,09 (s, 6H, 2 Me); 1,23 (s, 6H, 2 Me); 1,62 (s, 4H, 2 -CH₂-); 2,22 (s, 3H, Me); 6,57 (s, 1H, H vinylique); 6,87 (m, 2H, ArH); 7,06 (m, 1H, ArH) ; 7,18 (m, 1H, ArH); 7,64 (d, 1H, ArH *J* 1,9Hz).
MS EI 70ev (m/z, % intensité) : 354 (100 %); 339 (92).
MSHR EI 70 ev : Mₜᵣ = 354,1669 pour C₂₂H₂₆O₂S Mth = 354,1654.
HPLC Colonne Waters HR C₁₈, 8 x 100 mm, 6 µ, détecteur UV Waters 486 à 260 nm, débit 2 ml/min, éluant MeOH : H₂O = 90 : 10 + 0,1% de TFA, acide (CB30382) tr = 4,4 min 97,5% ; impureté tr = 6, 0 min 1,8%.

Acide (E) 2-[1-(5,6,7,8-tétrahydro-5,5,8,8-tétraméthyl-2-naphtalényl)-2-propényl)]thiényl-5-carboxylique (CB38973) :
F (°C) = 239.
RMN¹H 200MHz (CDCl₃) : 1,28 (s, 12H, 4 Me); 1,66 (s, 4H, 2 -CH₂-) ; 2,30 (s, 3H, Me); 7,15 (m, 3H, H vinylique et 2 ArH); 7,28 (m, 2H, ArH); 7,77 (d, 1H, ArH J 2Hz).
MS EI 70 ev (m/z, % intensité) : 354 (100 %); 339 (95).
MSHR EI 70 ev : Mₜᵣ = 354,1667 pour C₂₂H₂₆O₂S Mₜₕ = 354,1654.
HPLC : Colonne Waters HR C₁₈, 8 x 100 mm, 6 µ, détecteur UV Waters 486 à 260 nm, débit 3 ml/min, éluant MeOH : H₂O = 90 : 10 + 0,1% de TFA, acide (CB38973) tr = 4,1 min 98, 1% ; impureté tr = 3,1 min 1,5%.

### Exemples 23 :

### Préparation de l'acide (Z) 5-[1-(5,6,7,8-tétrahydro-5,5,8,8-tétraméthyl-2-naphtalényl)-2-propényl)]-isoxazole-3-carboxylique (CB73069) et de l'acide (E) 5-[1-(5,6,7,8-tétrahydro-5,5,8,8-tétraméthyl-2-naphtalényl)-2-propényl)]isoxazole-3-carboxylique (CB57201)

### a) (E) et (Z) 5-[1-(5,6,7,8-Tétrahydro-5,5,8,8-tétraméthyl-2-naphtalényl)-2-propényl)]isoxazole-3-carboxylates d'éthyle.

A une solution de bromure de (5,6,7,8-tétrahydro-5,5,8,8-tétraméthyl-2-naphtalényl)-6-méthyltriphénylphosphonium 9 (4,75 g, 8,74 mmol) dans 10 ml de THF anhydre, on ajoute à -70°C une solution de t-BuOK 1M dans le THF (6,50 ml, 6,50 mmol). On poursuit l'agitation à -70 °C pendant 1 h avant d'ajouter une solution de 5-acétylisoxazole-3-carboxylate d'éthyle (0,80 g, 4,37 mmol) dans 5 ml de THF anhydre. Le milieu réactionnel est porté à température ambiante et on agite pendant 16 h. On verse alors le milieu réactionnel sur un mélange eau-glace (150 ml) puis extrait à l'éther (3 x 70 ml), sèche sur MgSO₄, filtre et évapore les solvants. Le produit brut est purifié par chromatographie éclair sur silice (éluant éther : éther de pétrole = 2 : 98 puis 5 : 95). On obtient 0,37 g d'une huile jaunâtre, le (Z) 5-[1-(5,6,7,8-tétrahydro-5,5,8,8-tétraméthyl-2-naphtalényl)-2-propényl)]isoxazole-3-carboxylate d'éthyle (Rdt = 23 %) puis 0,65 g d'un solide jaunâtre, le (E) 5-[1-(5,6,7,8-tétrahydro-5,5,8,8-tétraméthyl-2-naphtalényl)-2-propényl)]isoxazole-3-carboxylate d'éthyle (Rdt = 40,5 %).

### Isomère (Z) :

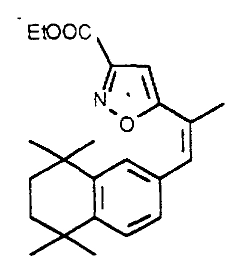

RMN¹H 200MHz (CDCl₃) : 1,16 et 1,25 (2s, 12H, 5,5,8,8-Me); 1,31 (t, 3H, Me *J* 7,1Hz); 1,64 (s, 4H, 6,7-CH₂-); 2,23 (d, 3H, Me vinylique *J* 1,5Hz); 4,37 (q, 2H, -OCH₂- *J* 7,1Hz); 6,29 (s, 1H, H isoxazole); 6,74 (s large, 1H, H vinylique); 6,91 (dd, 1H, ArH *J* 1,8Hz *J* 8,1Hz); 7,11 (d, 1H, ArH *J* 1,8Hz); 7,24 (s, 1H, ArH).
MS EI 70 eV (m/z, % intensité) : 367 (M⁺, 48 %); 352 (M⁺-CH₃, 100); 253 (17); 74 (12); 59 (21); 57 (15); 55 (15). 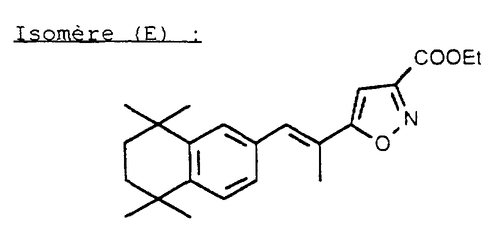
F (°C) = 89.
RMN¹H 200MHz (CDCl₃) : 1,29 et 1,31 (2s, 12H, 5,5,8,8-Me); 1,42 (t, 3H, Me *J* 7, 1Hz); 1, 68 (s, 4H, 6,7-CH₂-); 2,26 (d, 3H, Me vinylique *J* 1,4Hz) ; 4,44 (q, 2H, -OCH₂- J 7,1Hz); 6,64 (s, 1H, H isoxazole); 7,19 (dd, 1H, ArH J 1,7Hz *J* 8,2Hz); 7,27-7,40 (m, 3H, 2 ArH et H vinylique).
MS EI 70 eV (m/z, % intensité) : 367 (M⁺, 62 %); 352 (M⁺-CH₃, 100); 253 (18).

### b) Acide (Z) 5-[1-(5,6,7,8-tétrahydro-5,5,8,8-tétraméthyl-2-naphtalényl)-2-propényl)]isoxazole-3-carboxylique (CB73069)

Dans un ballon de 25 ml muni d'un réfrigérant et sous agitation magnétique, on a introduit le (Z) 5-[1-(5,6,7,8-tétrahydro-5,5,8,8-tétraméthyl-2-naphtalényl)-2-propényl)]isoxazole-3-carboxylate d'éthyle (0,35 g, 0,95 mmol) en suspension dans 6,6 ml d'éthanol et 1,1 ml d'eau distillée. On additionne de la potasse (0,53 g, 9,5 mmol) puis porte à reflux le milieu réactionnel pendant 8 h. Après refroidissement du milieu réactionnel, on acidifie par HCl 3N jusqu'à pH = 1 et extrait à l'éther (3 x 50 ml). On sèche la phase éthérée sur MgSO₄, filtre et évapore. Le produit brut est purifié par HPLC préparative sur une colonne Waters HR C₁₈ (25 x 100 mm) avec comme éluant MeOH : H₂O = 85 : 15 + 0,1% de TFA. On obtient 0,31 g d'un solide blanc, l'acide (Z) 5-[1-(5,6,7,8-tétrahydro-5,5,8,8-tétraméthyl-2-naphtalényl)-2-propényl)]isoxazole-3-carboxylique (CB73069) (Rdt = 96 %). 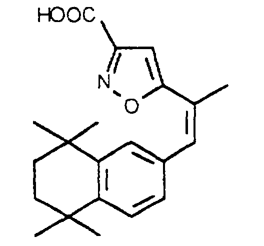
F (°C) = 114.
RMN¹H 200MHz (CDCl₃) : 1,16 et 1,25 (2s, 12H, 5,5,8,8-Me); 1,65 (s, 4H, 6,7-CH₂); 2,24 (s, 3H, Me vinylique); 6,33 (s, 1H, H isoxazole), 6,77 (s, 1H, H vinylique) ; 6,92 (d, 1H, ArH *J* 1,8Hz) 7,12 (s, 1H, ArH); 7,24 (d, 1H, ArH *J* 8.0Hz), 7,50 (s large, 1H, -COOH).
MS IC 70 isobutane (m/z, % intensité): 340 (M⁺+1, 100%); 296 (57) .
MSHR (FAB + NOBA) : MH⁺ₜᵣ 340,1916 pour C₂₁H₂₅NO₃ MH⁺ₜₕ 340,1913
IR (KBr, cm⁻¹) : 1708 (m _{c=0}).
HPLC : Colonne Waters HR C₁₈, 8 x 100 mm, 6µ, détecteur UV Waters 486 à 260 nm, débit 3 ml/min, éluant MeOH : H₂O = 85 : 15 + 0,1% de TFA, acide CB73069 tr = 3,37 min 97,6%.

### c) Acide (E) 5-[1-(5,6,7,8-tétrahydro-5,5,8,8-tétraméthyl-2-naphtalényl)-2-propényl)]isoxazole-3-carboxylique (CB57201).

Dans un ballon de 50 ml muni d'un réfrigérant et sous agitation magnétique, on a introduit le (E) 5-[1-(5,6,7,8-tétrahydro-5,5,8,8-tétraméthyl-2-naphtalényl)-2-propényl)]isoxazole-3-carboxylate d'éthyle (0,70 g, 1,90 mmol) en suspension dans 13,2 ml d'éthanol et 2,2 ml d'eau distillée. On additionne de la potasse (1,06 g, 19,00 mmol) puis porte à reflux le milieu réactionnel pendant 1 h 15. Après refroidissement du milieu réactionnel, on acidifie par HCl 3N jusqu'à pH = 1 et extrait à l'éther (3 x 50 ml). On sèche la phase éthérée sur MgSO₄, filtre et évapore. Le produit brut est repris dans un minimum d'éther éthylique et on ajoute de l'hexane jusqu'à précipitation. Après filtration et séchage, on obtient 0,36 g d'un solide blanc, l'acide (E) 5-[1-(5,6,7,8-tétrahydro-5,5,8,8-tétraméthyl-2-naphtalényl)-2-propényl)]isoxazole-3-carboxylique (CB57201) (Rdt = 96 %). 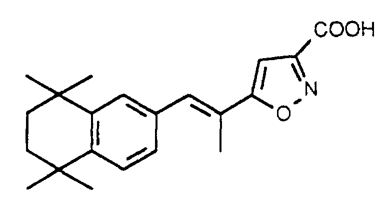
F (°C) = 205.
RMN¹H 200MHz (CDCl₃) : 1,29 et 1,30 (2s, 12H, 5,5,8,8-Me) ; 1,69 (s, 4H, 6,7-CH₂) ; 2,28 (d, 3H, Me vinylique *J* 1,1Hz); 6,71 (s, 1H, H isoxazole), 7,19 (dd, 1H, ArH *J* 0,8Hz *J* 4,0Hz); 7,25-7,45 (m, 3H, ArH et H vinylique).
MS FAB + NOBA (m/z, % intensité): 340 (MH⁺, 100%); 324 (31); 154 (49); 136 (40).
MSHR (FAB + NOBA) : MH⁻ₜᵣ 340, 1917 pour C₂₁H₂₅NO₃ MH⁻ₜᵣ 340, 1913
HPLC : Colonne Waters HR C₁₈, 8 x 100 mm, 6 µ, détecteur UV Waters 486 à 260 nm, débit 3 ml/min, éluant MeOH : H₂O = 85 : 15 + 0,1% de TFA, acide CB57201 tr = 5,21 min 98,8%.

### Exemple 24 :

### Préparation de l'acide (Z) 5-[1-(5,6,7,8-tétrahydro-3,5,5,8,8-pentamethyl-2-naphtalényl)-2-propényl)lisoxazole-3-carboxylique (CB54647) et de l'acide (E) 5-[1-(5,6,7,8-tétrahydro-3,5,5,8,8-pentaméthyl-2-naphtalényl)-2-propényl)]isoxazole-3-carboxylique (CB82718)

### a) (E) et (Z) 5-[1-(5,6,7,8-Tétrahydro-3,5,5,8,8pentaméthyl-2-naphtalényl)-2-propényl)]isoxazole-3-carboxylates d'éthyle.

A une solution de bromure de (5,6,7,8-tétrahydro-3,5,5,8,8-tétraméthyl-2-naphtalényl)-6-méthyltriphénylphosphonium (4,86 g, 8,73 mmol) dans 10 ml de THF anhydre, on ajoute à -70°C une solution de *t*-BuOK 1M dans le THF (8,73 ml, 8,73 mmol). On poursuit l'agitation à -70 °C pendant 1 h avant d'ajouter une solution de 5-acétylisoxazole-3-carboxylate d'éthyle (0,80 g, 4,37 mmol) dans 5 ml de THF anhydre. Le milieu réactionnel est porté à température ambiante et on agite pendant 3 h 30. On verse alors le milieu réactionnel sur un mélange eau-glace (150 ml) et HCl 1N (50 ml), puis extrait à l'éther (2 x 75 ml) et au dichlorométhane (2 x 75 ml), rassemble les phases organiques que l'on sèche sur MgSO₄, filtre et évapore. Le produit brut est purifié par chromatographie éclair sur silice (éluant éther : éther de pétrole = 2 : 98 jusqu'à 10 : 90). On obtient 1,80 g d'une huile incolore, le mélange des (E) et (Z) 5-[1-(5,6,7,8-tétrahydro-3,5,5,8,8-pentaméthyl-2-naphtalényl)-2-propényl)]isoxazole-3-carboxylates d'éthyle (Rdt = 54 %) en proportion déterminée par RMN¹H 200 MHz E:Z = 66:33.

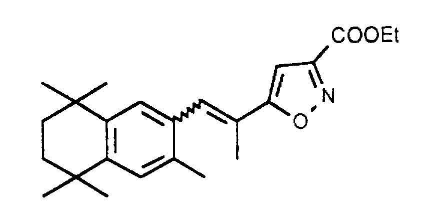

RMN¹H 200MHz (CDCl₃) : 1,12 (s, 0,33 x 6H, 2 Me isomère Z) ; 1,26 (m, 0,66 x 12H et 0,33 x 6H, 4 Me isomère E et 2 Me isomère Z); 1,32 (t, 0,33 x 3H, Me isomère Z *J* 7,1Hz); 1,42 (t, 0,66 x 3H, Me isomère Z *J* 7,1Hz); 1,63 (s, 0,33 x 4H, 6,7-CH₂- isomère Z); 1,67 (s, 0,66 x 4H, 6,7-CH₂-isomère E); 2,15 (d, 0,66 x 3H, Me vinylique isomère E *J* 1,4Hz) ; 2,16 (s, 0,33 x 3H, Me aromatique isomère Z); 2,25 (s, 0,66 x 3H, Me aromatique isomère E); 2,29 (d, 0,33 x 3H, Me vinylique isomère Z *J* 1,4Hz) ; 4,33 (q, 0,33 x 2H, -OCH₂- isomère Z *J* 7,1Hz); 4,45 (q, 0,66 x 2H, -OCH₂-isomère E *J* 7,1Hz); 5,95 (s, 0,33 x 1H, H isoxazole isomère Z); 6,64 (s, 0,66 x 1H, H isoxazole isomère E); 6,78 (s, 0,33 1H, H vinylique isomère Z); 7,01 (s, 0,33 x 1H, ArH isomère Z); 7,10-7,20 (m, 0,33 x 2H et 0,66 x 2H, ArH isomère Z et ArH isomère E); 7,42 (s, 0,66 x 1H, H vinylique isomère E).

### b) Acides (Z) et (E) 5-[1-(5,6,7,8-tétrahydro-3,5,5,8,8-pentaméthyl-2-naphtalényl)-2-propenyl)] isoxazole-3-carboxyliques CB54647 et CB82178.

Dans un ballon de 50 ml muni d'un réfrigérant et sous agitation magnétique, on a introduit un mélange de (E) et (Z) 5-[-1-(5,6,7,8-tétrahydro-3,5,5,8,8-pentaméthyl-2-naphtalényl)-2-propényl)]isoxazole-3-carboxylates d'éthyle (1,80 g, 4,72 mmol) en suspension dans 26,0 ml d'éthanol et 4,5 ml d'eau distillée. On additionne de la potasse (2,12 g, 38,0 mmol) puis porte à reflux le milieu réactionnel pendant 1 h 15. Après refroidissement du milieu réactionnel, on acidifie par HCl 3N jusqu'à pH = 1 et extrait à l'éther (3 x 70 ml). On sèche la phase éthérée sur MgSO₄, filtre et évapore pour obtenir 0,92 g d'un mélange d'acide (E) et (Z) (Rdt brut = 55%). Une partie du produit brut est purifiée par HPLC préparative sur une colonne Waters HR C₁₈ (25 x 100 mm) avec comme éluant MeOH : H₂O = 87 : 13 + 0,1% de TFA. On obtient 0,12 g d'un solide blanc, l'acide 5-[(Z)-1-(5,6,7,8-tétrahydro-3,5,5,8,8-pentaméthyl-2-naphtalényl)-2-propényl)]isoxazole-3-carboxylique (CB54647) et 0,23 g d'un solide blanc, l'acide (E) 5-[1-(5,6,7,8-tétrahydro-3,5,5,8,8-pentaméthyl-2-naphtalényl) -2-propényl)]isoxazole-3-carboxylique (CB82178).

Acide (Z) 5-[-1-(5,6,7,8-tétrahydro-3,5,5,8,8-pentaméthyl-2-naphtalényl)-2-propényl)]isoxazole-3-carboxylique (CB54647) : 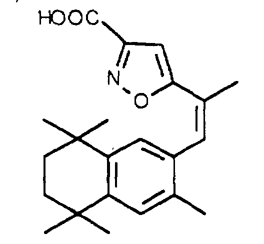
F (°C) = 162-163.
RMN¹H 200MHz (CDCl₃) : 1,11 et 1,26 (2s, 12H, 5,5,8,8-Me); 1,63 (s, 4H, 6,7-CH₂) ; 2, 17 (s, 3H, Me aromatique); 2,29 (s, 3H, Me vinylique); 5,99 (s, 1H, H isoxazole), 6,80 (s, 1H, H vinylique); 6,99 (s, 1H, ArH); 7, 12 (s, 1H, ArH).
MS FAB + NOBA (m/z, % intensité): 354 (MH⁺, 100%); 338 (35); 154 (34); 136 (29).
MSHR (FAB + NOBA) : MH⁺ₜᵣ 354,2072 pour C₂₂H₂₇NO₃ MH⁻ₜₕ 354,2069
HPLC : Colonne Waters HR C₁₈, 8 x 100 mm, 6µ, détecteur UV Waters 486 à 260 nm, débit 3 ml/min, éluant MeOH : H₂O = 87 : 13 + 0,1% de TFA, acide CB54647 tr = 3,28 min 99,1%; impureté isomère (E) (CB82178) tr = 4,45 min 0,9%.

Acide (E) 5-[1-(5,6,7,8-tétrahydro-3,5,5,8,8-pentaméthyl-2-naphtalényl)-2-propényl)]isoxazole-3-carboxylique (CB82178) : 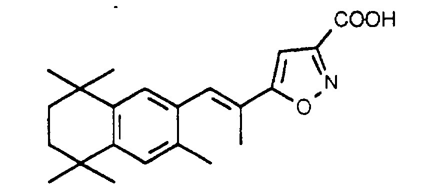
F (°C) = 204 (déc.).
RMN¹H 200MHz (CDCl₃) : 1,27 et 1,28 (2s, 12H, 5,5,8,8-Me); 1,68 (s, 4H, 6,7-CH₂) ; 2,16 (d, 3H, Me vinylique *J* 1,2Hz); 2,26 (d, 3H, Me aromatique); 5,63 (s large, 1H, -COOH); 6,70 (s, 1H, H oxazole), 7,14 (s, 1H, ArH); 7,18 (s, 1H, ArH); 7,45 (d, 1H, H vinylique *J* 1,2Hz).
MS FAB + NOBA (m/z, % intensité): 354 (MH⁺, 100%); 338 (36); 154 (25); 136 (19); 69 (12).
MSHR (FAB + NOBA) : MH⁺ₜᵣ 354,2069 pour C₂₂H₂₇NO₃ MH⁺ₜₙ 354,2069
HPLC : Colonne Waters HR C₁₈, 8 x 100 mm, 6µ, détecteur UV Waters 486 à 260 nm, débit 3 ml/min, éluant MeOH : H₂O = 85 : 15 + 0,1% de TFA, acide CB82178 tr = 4,53 min 99,6%, impureté isomère (Z) (CB54647) tr = 3,28 min 0,2%.

### Exemple 25 : Préparation de l'acide (E) 4-[1-(5,6,7,8-tétrahydro-5,5,8,8-tétraméthyl-2-naphtalényl)-3-éthoxy-2-propényl]benzoïque (CB17231)

### a) (E) 4-[1-(5,6,7,8-tétrahydro-5,5,8,8-tétraméthyl-2-naphtalényl)-3-bromo-2-propényl]benzonitrile.

Une solution de (E) 4-[1-(5,6,7,8-tétrahydro-5,5,8,8-tétraméthyl-2-naphtalényl)-2-propényl]benzonitrile (0,27 g, 0,76 mmol), de N-bromosuccinimide (0,16 g, 0,90 mmol), de péroxyde de benzoyle (6 mg) dans 3 ml de CCl₄ est portée à reflux pendant 2 h sous atmosphère d'argon et irradiation par une lampe tungstène 500-W. Après refroidissement, le milieu réactionnel est concentré, repris à l'éther et incorporé sur silice. On purifie par chromatographie éclair sur silice (éluant éther : éther de pétrole = 4 : 96) pour obtenir après évaporation un produit brut que l'on recristallise dans l'hexane. On obtient 0,12 g d'un solide blanc, le (E) 4-[1-(5,6,7,8-tétrahydro-5,5,8,8-tétraméthyl-2-naphtalényl)-3-bromo-2-propényl]benzonitrile (Rdt = 36,5%). 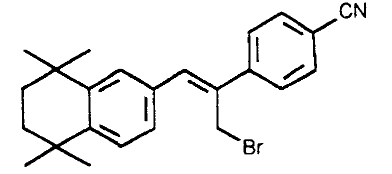
F (°C) = 151.
RMN¹H 200MHz (CDCl₃) : 1,29 et 1,32 (s, 12H, 5,5,8,8-CH₃) ; 1,70 (s, 4H, 6,7-CH₂-); 4,54 (s, 2H, BnzH); 7,02 (s, 1H, H vinyliquel; 7,25 (dd, 1H, ArH *J* 2Hz *J* 8Hz); 7,37 (d, 1H, ArH *J* 8Hz); 7,54 (d, 1H, ArH *J* 2Hz); 7,67 (s, 4H, ArH).

### b) Acide (E) 4-[1-(5,6,7,8-tétrahydro-5,5,8,8-tétraméthyl-2-naphtalényl)-3-éthoxy-2-propényl]benzoique (CB17231).

A une suspension de (E) 4-[1-(5,6,7,8-tétrahydro-5,5,8,8-tétraméthyl-2-naphtalényl)-3-bromo-2-propényl]-benzonitrile (0,12 g, 0,28 mmol) en solution hydroéthanolique (H₂O 0,55 ml et EtOH 3,3 ml) est ajoutée de la potasse (0,50 g, 8,42 mmol). On chauffe à reflux sous agitation magnétique pendant 20 h. Après refroidissement, on acidifie par 10 ml d'une solution HCl 1N, extrait à l'éther (3 x 50 ml), sèche par MgSO₄, filtre et évapore. Le produit brut est purifié par HPLC préparative sur une colonne Waters HR C₁₈ (25 x 100 mm) avec comme éluant MeOH : H₂O = 85 : 15 + 0,1% de TFA. On obtient 0,03 g d'un solide blanc, l'acide (E) 4-[1-(5,6,7,8-tétrahydro-5,5,8,8-tétraméthyl-2-naphtalényl)-3-éthoxy-2-propényl]benzoïque (CB17231) (Rdt = 27%). 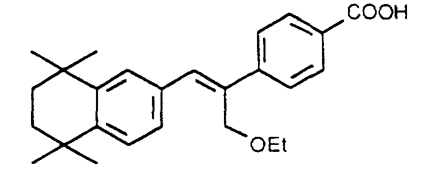
F (°C) = 135.
RMN¹H 200MHz (CDCl₃) : 1,27 (t, 3H, Me *J* 7Hz); 1,29 et 1,31 (s, 12H, 5,5,8,8-CH₃); 1,70 (s, 4H, 6,7-CH₂-); 3,59 (q, 2H, -OCH₂- *J* 7Hz); 7,15 (s, 1H, H vinylique); 7,15-7,45 (m, 3H, ArH); 7,89 (dm, 2H, ArH méta au -COOH *J* 8Hz); 8,09 (dm, 2H, ArH ortho au -COOH J 8Hz).
MS El 70 eV (m/z, % intensité) : 392 (M⁺, 95 %); 377 (66), 323 (14); 281 (37); 215 (22); 149 (100); 91 (21); 71 (16); 69 (44); 57 (47); 55 (46).
MSHR EI 70 eV : Mₜᵣ = 398,2359 pour C₂₆H₃₂O₃ Mₜₕ = 392,2351.
HPLC : Colonne Waters HR C₁₈, 8 x 100 mm, 6µ, détecteur UV Waters 486 à 260 nm, débit 3 ml/min, éluant MeOH : H₂O = 85 : 15 + 0,1% de TFA, acide (CB17231) tr = 7,12 min 98,3% ; impureté tr = 4,73 min 1,40%.

### Exemple 26 : Préparation du (E) N-Carbéthoxy-4-[1-(5,6,7,8-tétrahydro-5,5,8,8-tétraméthyl-2-naphtalényl)-2-propényl]aniline (CB21282).

A une suspension d'acide (E) 4-[1-(5,6,7,8-tétrahydro-5,5,8,8-tétraméthyl-2-naphtalényl)-2-propényl]benzoique (CB24159) (0,56 g, 1,61 mmol) dans 5 ml de toluène, on ajoute successivement à la seringue de l'azoture de diphénylphosphoryle (0,37 ml, 1,70 mmol) et de la triéthylamine (0,24 ml, 1,69 mmol). On porte à 80°C sous atmosphère d'argon et agitation magnétique pendant 1 h. Après refroidissement, on ajoute 0,95 ml d'éthanol puis porte à 80°C pendant 3 h. On évapore à sec après refroidissement, reprend à l'éther et incorpore sur silice. On purifie le mélange brut par chromatographie éclair sur silice (éluant éther : éther de pétrole = 3 : 97 jusqu'à 5 : 95). On obtient après évaporation 0,50 g d'un solide blanc, la (E) N-carbéthoxy-4-[1-(5,6,7,8-tétrahydro-5,5,8,8-tétraméthyl-2-naphtalényl)-2-propényl]aniline (CB21282) (Rdt = 79,5%). 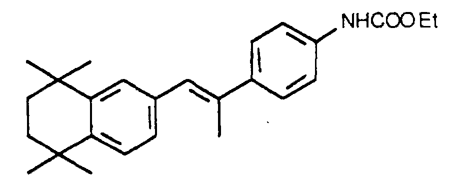
F (°C) = 124-5.
RMN¹H 200MHz (CDCl₃) : 1,31 et 1,32 (2s, 12H, 5,5,8,8-CH₃); 1,30 (t, 3H, Me *J* 7,1Hz); 1,71 (s, 4H, 6,7-CH₂-); 2,28 (d, 3H, Me vinylique *J* 1,2Hz); 4,24 (q, 2H, -OCH₂- *J* 7,1Hz); 6,76 (s large, 1H, -NH-); 6,79 (s, 1H, H vinylique); 7,15 (dd, 1H, ArH J 1,8Hz J 8,5Hz); 7,25-7,35 (m, 2H, ArH); 7,38 (d, 2H méta au -NH- *J* 8,7Hz); 7,48 (d, 2H ortho au -NH- *J* 8,7Hz).
MS EI 70 eV (m/z, % intensité) : 391 (M⁺, 100%); 376 (69); 330 (17); 69 (25); 57 (17).
MSHR EI 70 eV : Mₜᵣ = 391,2502 pour C₂₆H₃₃NO₂ Mₜₕ = 391,2512
HPLC : Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, *N*-carbéthoxyaniline (CB21282) tr = 4,04 min pureté 97,6% ; impureté tr = 3,20 min 2,1%.

### Exemple 27 : Préparation du (Z) N-(Carbéthoxy)-4-[1-(5,6,7,8-tétrahydro-5,5,8,8-tétraméthyl-2-naphtalényl)-2-pronényl]aniline (CB96682)

A une suspension d'acide (Z) 4-[1-(5,6,7,8-tétrahydro-5,5,8,8-tétraméthyl-2-naphtalényl)-2-propényl]-benzoïque (CB28628) (0,44 g, 1,26 mmol) dans 4 ml de toluène, on ajoute successivement à la seringue de l'azoture de diphénylphosphoryle (0,29 ml, 1,34 mmol) et de la triéthylamine (0,19 ml, 1,33 mmol). On porte à 80°C sous atmosphère d'argon et agitation magnétique pendant 1 h. Après refroidissement, on ajoute 1,0 ml d'éthanol puis porte à 80°C pendant 3 h. On évapore à sec après refroidissement, reprend à l'éther et incorpore sur silice. On purifie le mélange brut par chromatographie éclair sur silice (éluant éther : éther de pétrole = 3 : 97 jusqu'à 5 : 95). On obtient après évaporation 0,40 g d'une huile incolore, la (Z) *N*-(carbéthoxy)-4-[1-(5,6,7,8-tétrahydro-5,5,8,8-tétraméthyl-2-naphtalényl)-2-propényl]aniline (CB96682) (Rdt = 81%). 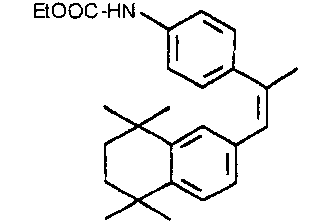
F (°C) = 47.
RMN¹H 200MHz (CDCl₃) : 1,00 et 1,19 (2s, 12H, 5,5,8,8-CH₃) ; 1,30 (t, 3H, Me *J* 7,1Hz); 1,57 (s, 4H, 6,7-CH₂-); 2,14 (d, 3H, Me vinylique *J* 1,3Hz); 4,21 (q, 2H, -OCH₂- *J* 7,0Hz); 6,37 (d, 1H, H vinylique *J* 1,3Hz); 6,56 (s large, 1H, -NH-); 6,74 (dd, 1H, ArH *J* 1,9Hz *J* 8,2Hz); 6,86 (d, 1H, ArH *J* 1,9Hz); 7,04 (d, 1H, ArH *J* 8,2Hz); 7,14 (dm, 2H, ArH méta au -NH- J 8,6Hz); 7,48 (dm, 2H ortho au -NH- J 8,6Hz).
MS EI 70 eV (m/z, % intensité) : 391 (M, 100 %); 376 (49); 330 (14); 215 (31); 57 (14).
MSHR EI 70 eV : Mₜᵣ = 391,2512 pour C₂₆H₃₃NO₂ Mₜₕ = 391,2512
HPLC : Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, *N*-carbéthoxyaniline (CB96682) tr = 3,17 min pureté 98,8% ; impureté tr = 1,45 min 0,6%.

### Exemple 28 : Préparation du (E) 1-[4-[1-(5,6,7,8-Tétrahydro-5,5,8,8-tétraméthyl-2-naphtalényl)-2-propényl]phényl]-5-trifluorométhyl-1H-tétrazole (CB59741)

### a) (E) 4-[1-(5,6,7,8-Tétrahydro-5,5,8,8-tétraméthyl-2-naphtalényl)-2-propényl]aniline.

Une suspension de (E) *N*-(carbéthoxy)-4-[1-(5,6,7,8-tétrahydro-5,5,8,8-tétraméthyl-2-naphtalényl)-2-propényl]aniline (CB21282) (0,50 g, 1,28 mmol) dans de la soude 10N (3,84 ml, 38,4 mmol) et 1,5 ml d'éthanol est portée à 80°C pendant 3 h sous atmosphère d'argon et agitation magnétique. Après refroidissement, on extrait au dichlorométhane (4 x 25 ml), sèche sur MgSO₄, filtre et évapore pour obtenir 0,31 g d'une huile brute jaunâtre, la (E) 4-[1-(5,6,7,8-tétrahydro-5,5,8,8-tétraméthyl-2-naphtalényl)-2-propényl]aniline (Rdt = 76%).

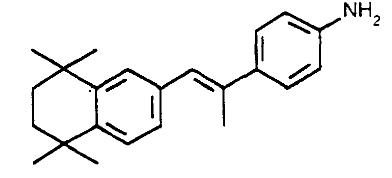

RMN¹H 200MHz (CDCl₃) : 1,37 et 1,38 (2s, 12H, 5,5,8,8-CH₃); 1,77 (s, 4H, 6,7-CH₂-); 2,33 (d, 3H, Me vinylique *J* 1,2Hz); 3,70 (s large, 2H, -NH₂-); 6,72 (dm, 2H, ArH *J* 8,6Hz); 6,81 (d, 1H, H vinylique *J* 1,3Hz) ; 7,21 (dd, 1H, ArH *J* 1,7Hz *J* 8,2Hz); 7,30-7,50 (m, 4H, ArH).

### b) (E) N-(Trifluoroacétyl)-4-[1-(5,6,7,8-tétrahydro-5,5,8,8-tétraméthyl-2-naphtalényl)-2 propényl]aniline.

On mélange pendant 30 min à température ambiante, sous atmosphère d'argon, une solution de (E) 4-[1-(5,6,7,8-tétrahydro-5,5,8,8-tétraméthyl-2-naphtalényl)-2-propényl]-aniline (0,31 g, 0,97 mmol) et de 2-(trifluoroacétoxy)pyridine (0,15 ml, 1,07 mmol) dans 3 ml d'éther anhydre. La phase éthérée est alors lavée à l'eau distillée, sèchée sur MgSO₄, filtrée et évaporée. On obtient 0,40 g d'un solide jaunâtre, la (E) *N*-(trifluoroacétyl)-4-[1-(5,6,7,8-tétrahydro-5,5,8,8-tétraméthyl-2-naphtalényl)-2-propényl]aniline (Rdt brut 99%). 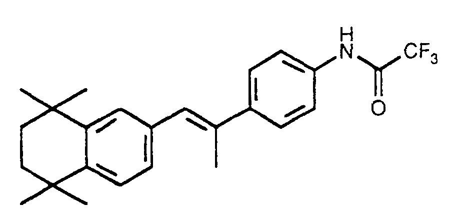
F (°C) = 124.
RMN¹H 200MHz (CDCl₃) : 1,28 et 1,29 (2s, 12H, 5,5,8,8-CH₃); 1,69 (s, 4H, 6,7-CH₂-); 2,27 (d, 3H, Me vinylique *J* 1,0Hz); 6,80 (s, 1H, H vinylique); 7,13 (dd, 1H, ArH *J* 1,8Hz *J* 8,0Hz); 7,27 (s, 1H, ArH); 7,30 (d, 1H, ArH *J* 8,0Hz) ; 7,54 (s, 4H, ArH); 7,88 (s large, 1H, -NH-).
FMN¹⁹F 50MHz (CDCl₃) : -76,23 (s, 0,77x3F, CF₃ amide trans); -76,62 (s, 0,23x3F, CF₃ amide cis).

### c) Chlorure de (E) N-[4-[1-(5,6,7,8-tétrahydro-5,5,8,8-tétraméthyl-2-naphtalényl)-2-propényl]phényl]-2,2,2-trifluoroacétimidoyle

Une solution de (E) *N*-(trifluoroacétyl)-4-[1-(5,6,7,8-tétrahydro-5,5,8,8-tétraméthyl-2-naphtalényl)-2-propényl]aniline (0,90 g, 0,96 mmol), de triphénylphosphine (0,60 g, 2,30 mmol) dans 3,5 ml de tétrachlorure de carbone anhydre est portée à reflux pendant 6 h sous atmosphère d'argon et agitation magnétique. Après refroidissement, on évapore le solvant, reprend au dichlorométhane et incorpore sur silice. Le produit brut est purifié par chromatographie éclair sur silice (éluant éther : éther de pétrole = 4 : 96). On obtient après évaporation 0,32 g d'un solide blanc, le chlorure de (E) *N*-[4-[1-(5,6,7,8-tétrahydro-5,5,8,8-tétraméthyl-2-naphtalényl)-2-propényl]phényl]-2,2,2-trifluoroacétimidoyle (Rdt = 77%). 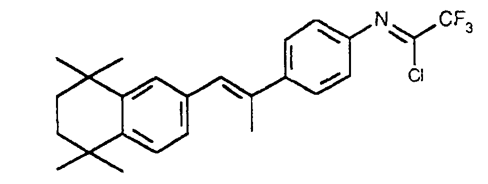
F (°C) = 60.
RMN¹H 200MHz (CDCl₃) : 1,30 et 1,31 (2s, 12H, 5,5,8,8-CH₃); 1,50 (s, 4H, 6,7-CH₂-); 2,31 (s, 3H, Me vinylique); 6,87 (s, 1H, H vinylique); 7,10-7,20 (m, 3H, ArH); 7,25-7,35 (m, 2H, ArH); 7,59 (d, 2H, ArH *J* 8,5Hz).
RMN¹⁹F 50MHz (CDCl₃) : -71,94 (s, 3F, CF₃).

### d) (E) 1-[4-[1-(5,6,7,8-Tétrahydro-5,5,8,8-tétraméthyl-2-naphtalényl)-2-propényl]phényl]-5-trifluorométhyl-1H-tétrazole (CB59741)

On porte à 70°C pendant 2 h, sous atmosphère d'argon et agitation magnétique, une solution de (E) N-[4-[1(5,6,7,8-tétrahydro-5,5,8,8-tétraméthyl-2-naphtalényl)-2-propényl]phényl]-2,2,2-trifluoroacétimidoyle (0,32 g, 0,74 mmol), d'azoture de sodium (0,10 g, 1,48 mmol) dans 1,5 ml d'acide acétique glacial. Après refroidissement du milieu réactionnel, on évapore le solvant, reprend au dichlorométhane et incorpore sur silice. On purifie par chromatographie éclair sur silice (éluant éther : éther de pétrole = 7 : 93). Après évaporation des solvants, on obtient 0,30 g d'une huile incolore, le (E) 1-[4-[1-(5,6,7,8-tétrahydro-5,5,8,8-tétraméthyl-2-naphtalényl)-2-propényl]phényl]-5-trifluorométhyl-1*H*-tétrazole (CB59741) (Rdt = 92%). 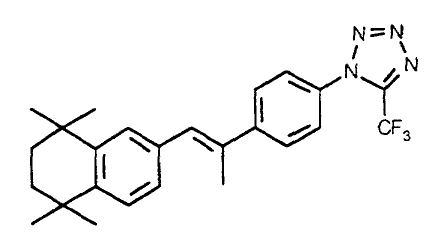
RMN¹H 200MHz (CDCl₃) : 1,30 et 1,31 (2s, 12H, 5,5,8,8-CH₃); 1,70 (s, 4H, 6,7-CH₂-); 2,34 (s, 3H, Me vinylique); 6,91 (s, 1H, H vinylique); 7,16 (dd, 1H, ArH *J* 1,9Hz *J* 8,1Hz); 7,27-7,37 (m, 2H, ArH); 7,47 (d, 2H, ArH *J* 8,6Hz); 7,72 (d, 2H, ArH *J* 8,6Hz).
RMN¹⁹F 50MHz (CDCl₃) : -60,39 (s, 3F, CF₃).
MS EI 70 eV (m/z, % intensité) : 440 (M⁺, 100 %); 397 (100); 343 (15); 248 (36); 215 (46); 111 (24); 69 (57).
MSHR EI 70 eV : Mₜᵣ = 440,2180 pour C₂₅H₂₇N₄F₃ Mₜₕ = 440,2188
HPLC : Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, tétrazole (CB59741) tr = 3,88 min pureté 96,6% ; impureté tr = 3,24 min 2,7%.

### Exemple 29 : Préparation du (Z) 1-[4-[1-(5,6,7,8-Tétrahydro-5,5,8,8-tétraméthyl-2-naphtalényl)-2-propényl]phényl]-5-trifluorométhyl-1H-tétrazole (CB29830)

### a) (Z) 4-[1-(5,6,7,8-Tétrahydro-5,5,8,8-tétraméthyl-2-naphtalényl)-2-propényl]aniline

Une suspension de (Z) *N*-(carbéthoxy)-4-[1-(5,6,7,8-tétrahydro-5,5,8,8-tétraméthyl-2-naphtalényl)-2-propényl]aniline (CB96682) (0,28 g, 1,28 mmol) dans de la soude 10N (3,84 ml, 38,4 mmol) et 1,5 ml d'éthanol est portée à 80°C pendant 3 h sous atmosphère d'argon et agitation magnétique. Après refroidissement, on extrait au dichlorométhane (4 x 25 ml), sèche sur MgSO₄, filtre et évapore pour obtenir 0,22 g d'une huile brute orangée, la (Z) 4-[1-(5,6,7,8-tétrahydro-5,5,8,8-tétraméthyl-2-naphtalényl)-2-propényl]aniline (Rdt brut = 96.5%).

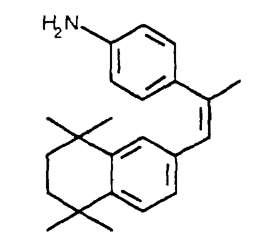

RMN¹H 200MHz (CDCl₃) : 1,04 et 1,20 (2s, 12H, 5,5,8,8-CH₃); 1,59 (s, 4H, 6,7-CH₂-) ; 2,14 (d, 3H, Me vinylique *J* 1,4Hz); 3,62 (s large, 2H, -NH₂-); 6,32 (s, 1H, H vinylique); 6,72 (dm, 2H, ArH J 8,4Hz); 6,78 (dd, 1H, ArH *J* 1,8Hz *J* 8,2Hz) ; 6,93 (d, 1H, ArH *J* 1,8Hz); 7,01 (dm, 2H, ArH *J* 8,4Hz); 7,05 (d, 1H, ArH *J* 8,2Hz).

### b) (Z) N-(Trifluoroacétyl)-4-[1-(5,6,7,8-tétrahydro-5,5,8,8-tétraméthyl-2-naphtalényl)-2 propényl]aniline.

On mélange pendant 30 min à température ambiante, sous atmosphère d'argon, une solution de (Z) 4-[1-(5,6,7,8-tétrahydro-5,5,8,8-tétraméthyl-2-naphtalényl)-2-propényl]-aniline (0,22 g, 0,69 mmol) et de 2-(trifluoroacétoxy)pyridine (0,11 ml, 0,76 mmol) dans 2,5 ml d'éther anhydre. La phase éthérée est alors lavée à l'eau distillée, séchée sur MgSO₄, filtrée et évaporée. On reprend à l'éther et incorpore sur silice pour purifier par chromatographie éclair sur silice (éluant èther : éther de pétrole 7 : 93). On obtient après évaporation des solvants 0,20 g d'une huile incolore, la (Z) *N*-(trifluoroacétyl)-4-[1-(5,6,7,8-tétrahydro-5,5,8,8-tétraméthyl-2-naphtalényl)-2-propényl]aniline (Rdt = 70%). 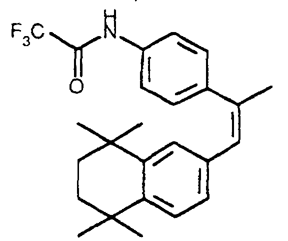
RMN¹H 200MHz (CDCl₃) : 1,00 et 1,20 (2s, 12H, 5,5,8,8-CH₃); 1,58 (s, 4H, 6,7-CH₂-); 2,16 (d, 3H, Me vinylique *J* 1,4Hz); 6,43 (s, 1H, H vinylique); 6,73 (dd, 1H, ArH *J* 1,8Hz J 8,2Hz); 6,85 (d, 1H ArH *J* 1,8Hz); 7,05 (d, 1H, ArH *J* 8,2Hz); 7,23 (dm, 2H, ArH *J* 8,6Hz); 7,49 (dm, 2H, ArH *J* 8,6Hz); 7,92 (s large, 1H, -NH-).
RMN¹⁹F 50MHz (CDCl₃) : -76,15 (s, 3F, CF₃).

### c) Chlorure de (Z) N-[4-[1-(5,6,7,8-tétrahydro-5,5,8,8-tétraméthyl-2-naphtalényl)-2-propényl]phényl]-2,2,2-trifluoroacétimidoyle

Une solution de (Z) *N*-(trifluoroacétyl)-4-[1-(5,6,7,8-tétrahydro-5,5,8,8-tétraméthyl-2-naphtalényl)-2-propényl]aniline (0,20 g, 0,48 mmol), de triphénylphosphine (0,30 g, 1,15 mmol) dans 3 ml de tétrachlorure de carbone anhydre est portée à reflux pendant 18 h sous atmosphère d'argon et agitation magnétique. Après refroidissement, on évapore le solvant, reprend au dichlorométhane et incorpore sur silice. Le produit brut est purifié par chromatographie éclair sur silice (éluant éther de pétrole pur). On obtient après évaporation 0,16 g d'un solide blanc, le chlorure de *N*-[4-[(Z)-1-(5,6,7,8-tétrahydro-5,5,8,8-técraméthyl-2-naphtalényl)-2-propényl]phényl]-2,2,2-trifluoroacétimidoyle (Rdt = 77%). 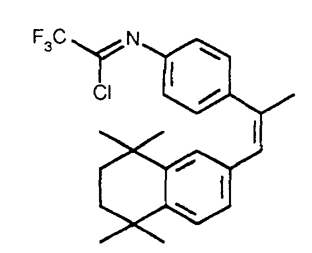
F (°C) = 76.
RMN¹H 200MHz (CDCl₃) : 0,96 et 1,18 (2s, 12H, 5,5,8,8-CH₃) ; 1,55 (s, 4H, 6,7-CH₂-) ; 2,17 (d, 3H, Me vinylique *J* 1,4Hz) ; 6,43 (s, 1H, H vinylique); 6,76 (dd, 1H, ArH *J* 1,9Hz *J* 8,1Hz); 6,84 (d, 1H ArH *J* 1,9Hz); 7,00-7,15 (m, 3H, ArH); 7,27 (dm, 2H, ArH *J* 8,6Hz).
RMN¹⁹F 50MHz (CDCl₃) : -72,01 (s, 3F, CF3) .

### d) (Z) 1-[4-[1- (5,6,7,8-Tétrahydro-5,5,8,8-tétraméthyl-2-naphtalényl)-2-propényl]phényl]-5-trifluorométhyl-1H-tétrazole (CB29830)

On porte à 70°C pendant 20 h, sous atmosphère d'argon et agitation magnétique, une solution de (Z) *N*-[4-[1-(5,6,7,8-tétrahydro-5,5,8,8-tétraméthyl-2-naphtalényl)-2-propényl]phényl]-2,2,2-trifluoroacétimidoyle (0,16 g, 0,37 mmol), d'azoture de sodium (0,05 g, 1,74 mmol) dans 0,75 ml d'acide acétique glacial. Après refroidissement du milieu réactionnel, on évapore le solvant, reprend au dichlorométhane et incorpore sur silice. On purifie par chromatographie éclair sur silice (éluant éther : éther de pétrole = 4 : 96). Après évaporation des solvants, on obtient 0,07 g d'un solide blanc, le (Z) 1-[4-[1-(5,6,7,8-tétrahydro-5,5,8,8-tétraméthyl-2-naphtalényl)-2-propényl]-phényl]-5-trifluorométhyl-1*H*-tétrazole (CB29830) (Rdt = 43%). 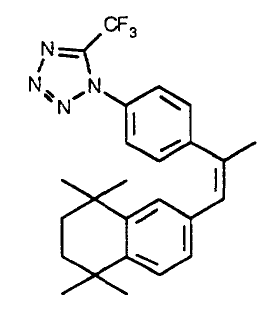
F (°C) = 111.
RMN¹H 200MHz (CDCl₃) : 0,97 et 1,20 (2s, 12H, 5,5,8,8-CH₃); 1,57 (s, 4H, 6,7-CH₂-); 2,22 (d, 3H, Me vinylique *J* 1,4Hz); 6,54 (s, 1H, H vinylique); 6,76 (dd, 1H, ArH *J* 1,7Hz *J* 8,1Hz); 6,83 (d, 1H, ArH *J* 1,7Hz); 7,10 (d, 1H, ArH *J* 8,1Hz); 7,42 (m, 4H, ArH).
RMN¹⁹F 50MHz (CDCl₃) : -60,32 (s, 3F, CF₃).
MS EI 70 eV (m/z, % intensité) : 440 (M⁻, 100 %); 397 (63); 248 (9); 69 (33).
MSHR EI 70 eV : Mₜᵣ = 440,2173 pour C₂₅H₂₇N₄F₃ Mₜₕ = 440, 2188
HPLC : Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, tétrazole (CB29830) tr = 3,37 min pureté 98,7% ; impureté tr = 4,05 min 0,8%.

### Exemple 30 : Préparation de l'acide (Z) 2-[1-(5,6,7,8-tètrahydro-5,5,8,8-tétraméthyl-2-naphtalényl)-2-propényl]thiényl-5-phos-phonique (CB46802)

### a) (E) et (Z) 2-[1-(5,6,7,8-Tétrahydro-5,5,8,8-tétraméthyl-2-naphtalényl)-2-propényl]thiényl-5-phosphonates de diéthyle.

On porte à reflux pendant 15 h, sous atmosphère d'argon et agitation magnétique, une suspension de (Z) 2-[1-(5,6,7,8-tétrahydro-5,5,8,8-tétraméthyl-2-naphtalényl)-2-propényl]-5-bromothiophéne (2,52 g, 6,47 mmol), de diéthylphosphite (1,67 ml, 12,94 mmol), de triéthylamine (1,80 ml, 12,94 mmol), de tétrakis (triphénylphosphine) palladium (0,87 g, 0,75 mmol) dans 3 ml de THF anhydre. Après refroidissement du milieu réactionnel, on reprend à l'acétate d'éthyle (100 ml) et lave par une solution de HCl 1N puis par une solution de NaCl saturée. Après évaporation du solvant, le produit brut est purifié par chromatographie éclair sur silice (éluant éther : éther de pétrole = 50 : 50 puis 70 : 30). On obtient après évaporation 0,83 g d'une huile jaunâtre (Rdt = 29%) composée d'un mélange des (E) et (Z) 2-[1-(5,6,7,8-tétrahydro-5,5,8,8-tétraméthyl-2-naphtalényl)-2-propényl]thiényl-5-phosphonates de diéthyle (proportion (E) : (Z) = 75 : 25 par RMN¹H 80MHz).

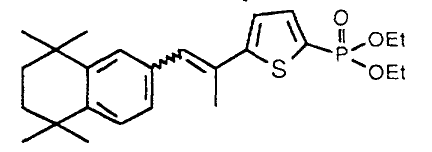

RMN¹H 80MHz (CDCl₃): 1,05-1,50 (m, 18H, 6Me); 1,55-1,70 (m, 4H, 6,7-CH₂); 2,20 (s, 0,25x3H, Me vinylique isomère Z); 2,30 (s, 0,75x3H, Me vinylique isomère E); 3,90-4,35 (m, 4H, -OCH₂-); 6,50-7,65 (m, 6H, ArH et H vinyliques).

### b) Acide (E) 2-[1-(5,6,7,8-tétrahydro-5,5,8,8-tétraméthyl-2-naphtalényl)-2-propényl]thiényl-5-phosphonique (CB46802)

On additionne à la seringue du bromotriméthylsilane (1,58 ml, 11,97 mmol) sur une suspension du mélange des (E) et (Z) 2-[1-(5,6,7,8-tétrahydro-5,5,8,8-tétraméthyl-2-naphtalényl)-2-propényl]-thiényl-5-phosphonates de diéthyle (0,83 g, 1,86 mmol) dans 12 ml d'acétonitrile. On porte à reflux pendant 2 h, sous agitation magnétique et atmosphère d'argon, le milieu réactionnel. Après refroidissement, on évapore à sec et reprend à l'éthanol. On évapore de nouveau et précipite un solide blanc par un minimum d'éther éthylique. On filtre, sèche pour obtenir 0,11 g d'un solide blanc, l'acide (E) 2-[1-(5,6,7,8-tétrahydro-5,5,8,8-tétraméthyl-2-naphtalényl)-2-propényl]thiényl-5-phosphonique (CB46802) (Rdt = 15%). 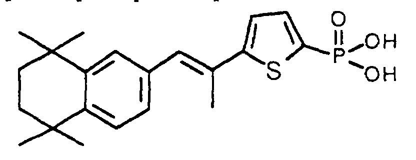
F (°C) = 175-176.
RMN¹H 200MHz (CDCl₃) : 1,26 et 1,27 (2s, 12H, 5,5,8,8-Me); 1,69 (s, 4H, 6,7-CH₂) ; 2,26 (d, 3H, Me vinylique J 0,9Hz); 7,02 (s, 1H, H vinylique); 7,09 (dd, 1H, ArH *J* 1,7Hz *J* 8,6Hz); 7,15-7,35 (m, 4H, ArH); 7,45 (dd, 1H, ArH J = 3,7Hz J = 8,6Hz).
MS EI 70 eV (m/z, % intensité) : 390 (M⁺, 0,1%); 375 (0,4%); 310 (87), 295 (100); 262 (35); 183 (27).
HPLC : Colonne Waters HR C₁₈, 8 x 100 mm, 6µ, détecteur UV Waters 486 à 280 nm, débit 3 ml/min, éluant MeOH : H₂O = 85 : 15 + 0,1% de TFA, acide (CB46802) tr = 2,93 min 98,8%; impureté tr = 1,36 min 0,7 %.

### Exemple 31 : Préparation de l'acide (Z) 4-[1-(5,6,7,8-tétrahydro-5,5,8,8-tétraméthyl-2-naphtalényl)-1-(phényl)-2-éthényl] benzoïque (CB59892).

### 1) (Z) 4 - [1- (5,6,7, 8-Tétrahydro-5,5,8,8-tétraméthyl-2-naphtalényl)-1-(phényl)-2-éthényl]benzonitrile.

A une solution du (Z) 4-[1-(5,6,7,8-tétrahydro-5,5,8,8-tétraméthyl-2-naphtalényl)-1-(phényl)-2-éthényl]-bromobenzène (2,21 g, 4,96 mmol) dans 18,5 ml de DMF anhydre, on ajoute à température ambiante le cyanure de cuivre (0,52 g, 5,81 mmol). Le milieu réactionnel est porté à reflux pendant 15 h. Après retour à température ambiante, le milieu réactionnel est dilué avec de l'éther (200 ml) et filtré sur célite. On lave la phase organique avec une solution aqueuse saturée en NaHCO₃ (3 x 50 ml) puis sèche sur MgSO₄, filtre et évapore. Le produit brut est purifié par chromatographie éclair sur silice (éluant éther de pétrole : éther = 100 : 2). On obtient 1,29 g d'une huile jaunâtre, le (Z) 4-[(5,6,7,8-tétrahydro-5,5,8,8-tétraméthyl-2-naphtalényl)-1-(phényl)-2-éthényl]benzonitrile (Rdt = 66 %).

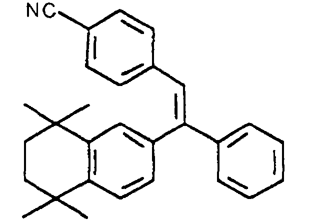

RMN¹H 200MHz (CDCl₃) : 1,05 et 1,35 (2s, 12H, 5,5,8,8 Me) ; 1,65 (s, 4H, 6,7-CH₂-) ; 6,80-6,90 (m, 2H, H vinylique et ArH); 7,00-7,10 (m, 3H, ArH); 7,20-7,50 (m, 8H, ArH).

### 2) Acide (Z) 4-[1-(5,6,7,8-tétrahydro-5,5,8,8-tétraméthyl-2-naphtalényl)-1-(phényl)-2-éthényl]benzoique (CB59892).

Une suspension de (Z) 4-[1-(5,6,7,8-tétrahydro-5,5,8,8-tétraméthyl-2-naphtalényl)-1-(phényl)-2-éthényl]benzonitrile (0,50 g, 1,27 mmol) en solution dans de la potasse (1,4 g, 25 mmol) hydroéthanolique (H₂O 1,8 ml et EtOH 17,5 ml) est chauffée à reflux sous agitation magnétique pendant 48 h. On évapore l'éthanol à l'évaporateur rotatif, reprend à l'eau (10 ml), acidifie par HCl 3N, extrait à l'éther éthylique (3 x 50ml), sèche la phase éthérée sur MgSO₄, filtre et évapore. Le produit est lavé par du pentane, filtré et séché. On obtient 521 mg d'un solide blanc, l'acide (Z) 4-[1-(5,6,7,8-tétrahydro-5,5,8,8-tétraméthyl-2-naphtalényl)-1-(phényl)-2-éthényl]benzoïque (CB59892) (Rdt = 63%). 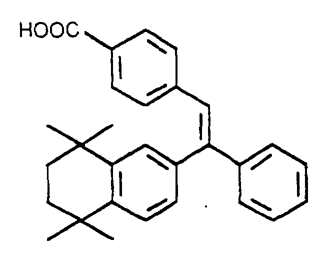
F (°C) = 222.
IR (cm⁻¹) : 2954; 1676; 1604; 1416; 1284.
RMN¹H 200MHz (CDCl₃) :1,03 et 1,20 (2s, 12H, 5,5,8,8-Me); 1,65 (s, 4H, 6,7-CH₂-); 6,85 (dd, 1H, *J* 2Hz et J 8Hz); 6,92 (s, 1H H vinylique); 7,04-7,08 (m, 3H, ArH); 7,21-7,39 (m, 6H, ArH) ; 7,82 (d, 2H, *J* 8Hz).
MS EI 70eV (m/z, % intensité) : 410 (M⁻, 100 %); 395 (48) .
MSHR EI 70 eV : Mₜᵣ = 410,2246 pour C₂₉H₃₀O₂ Mth = 410,2246.
HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, acide (CB59892) tr = 5,34 min 99,8%.

### Exemple 32 : Préparation du (Z) 5-[4-[1-(5,6,7,8-tétrahydro-5,5,8,8-tétraméthyl-2-naphtalényl)-1-(phényl)-2-éthényl]phényl]-1H-tétrazole (CB96561).

A une solution du (Z) 4-[1-(5,6,7,8-tétrahydro-5,5,8,8-tétraméthyl-2-naphtalényl)-1-(phényl)-2-éthényl]-benzonitrile (0,35 g, 0,89 mmol) dans du toluène anhydre (1,8 ml), on additionne sucessivement de l'oxyde de dibutylétain (22,8 mg, 0,09 mmol) et de l'azoture de triméthylsilyle (0,235 ml, 1,78 mmol). Le milieu réactionnel est chauffé 18 h à reflux (110°C) sous atmosphère d'argon et agitation magnétique. On purifie par chromatographie éclair sur silice (éluant MeOH : CH₂Cl₂ = 5 : 95) pour obtenir après évaporation 260 mg d'une poudre blanche, le (Z) 5-[4-[1-(5,6,7,8-tétrahydro-5,5,8,8-tétraméthyl-2-naphtalényl)-1-(phényl)-2-éthényl]phényl]-1*H*-tétrazole (CB96561) (Rdt = 67 %). 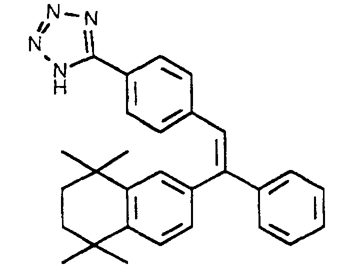
F (°C) = 232.
IR (cm⁻¹) : 2958; 1604; 1496; 1446; 1060;.880.
RMN¹H 200MHz (CDCl₃) : 1,03 et 1,26 (2s, 12H, 5,5,8,8-Me) ; 1,63 (s, 4H, 6,7-CH₂-) ; 6,88-6,90 (m, 2H) ; 7,08-7,35 (m, 9H, ArH) ; 7,79 (d, 2H, ArH, *J* 8Hz).
MS EI 70eV (m/z, % intensité) : 434 (M⁺, 62 %); 406 (100); 204 (65); 178 (13).
MSHR EI 70 eV : Mₜᵣ = 434,2484 pour C₂₉H₃₀N₄ Mₜₕ = 434,2470.
HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, tétrazole (CB96561) tr = 3,69 min 99,7%.

### Exemple 33 : Préparation du (E) 5-[4-[1-(5,6,7,8-tétrahydro-5,5,8,8-tétraméthyl-2-naphtalényl)-1-(phényl)-2-éthényl]phényl]-1H-tétrazole (CB56898).

A une solution du (E) 4-[1-(5,6,7,8-tétrahydro-5,5,8,8-tétraméthyl-2-naphtalényl)-1-phényl-2-éthényl]-benzonitrile (0,29 g, 0,74 mmol) dans du toluène anhydre (1,5 ml), on additionne successivement de l'oxyde de dibutylétain (23,3 mg, 0,089 mmol) et de l'azoture de triméthylsilyle (0,200 ml, 1,48 mmol) . Le milieu réactionnel est chauffé 15 h à reflux (110°C) sous atmosphère d'argon et agitation magnétique. On purifie par chromatographie éclair sur silice (éluant MeOH : CH₂Cl₂ = 5 : 95) pour obtenir après évaporation 40 mg d'une poudre blanche, le 5-[4-[(E)-1-(5,6,7,8-tétrahydro-5,5,8,8-tétraméthyl-2-naphtalényl)-1-(phényl)-2-éthényl]phényl]-1*H*-tétrazole (CB56898) (Rdt = 12 %) . 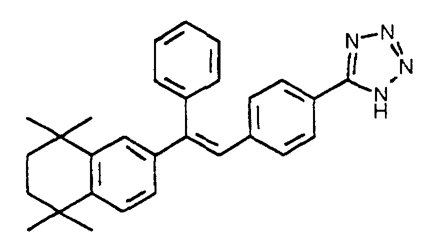
F (°C) = 254.
IR (cm⁻¹) : 2958; 1666; 1610; 1496; 1456; 1404; 1362; 1272; 1056;.878
RMN¹H 200MHz (CDCl₃) :1,22 et 1,26 (2s, 12H, 5,5,8,8-Me); 1,67 (s, 4H, 6,7-CH₂-) ; 6,95 (s, 1H, H vinylique); 7,14 (dd, 1H, ArH, *J* 2Hz et *J* 8Hz); 7,21 (m, 9H, ArH); 7,78 (d, 2H, ArH, *J* 8Hz).
MS EI 70ev (m/z, % intensité) : 434 (M⁺, 66 %); 406 (100); 219 (39); 204 (57).
MSHR EI 70 ev : Mₜᵣ = 434,2473 pour C₂₉H₃₀N₂ Mₜₕ = 434,2470.
HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, tétrazole (CB56898)
tr = 4,17 min 99,4%

### Exemple 34 : Préparation des (E) et (Z) 5-[2-[1-(5,6,7,8-tétrahydro-5,5,8,8-tétraméthyl-2-naphtalenyl)-2-propényl)]-5-thiényl]-1H-tétrazoles (CB07734 et CB92855).

### 1) (E) et (Z) 2-[1-(5,6,7,8-tétrahydro-5,5,8,8-tétraméthyl-2-naphtalényl)-2-propényl)]-5-cyanothiophénes.

A une solution de bromure de (5,6,7,8-tétrahydro-5,5,8,8-tétraméthyl-2-naphtalényl)-6-méthyltriphénylphosphonium (7,28 g, 13,22 mmol) dans 20 ml de THF, on ajoute à - 70 °C une solution de tBuOK 1M dans le THF (14 ml, 14 mmol). On poursuit l'agitation à - 70 °C pendant 1 h avant d'ajouter le 2-acétyl-5-cyanothiophène (1 g, 6,61 mmol) en solution dans 10 ml de THF. Le milieu réactionnel est porté à température ambiante et on agite pendant 14 h. On hydrolyse ensuite à 0 °C par une solution de HCl 3N. Après retour à température ambiante, on extrait à l'éther, sèche sur MgSO₄, filtre et évapore les solvants. Le produit brut est purifié par chromatographie éclair sur silice (éluant éther de pétrole = éther éthylique 100 : 1). On obtient 0,41 g d'une huile jaune, le (Z) 2-[1-(5,6,7,8-tétrahydro-5,5,8,8-tétraméthyl-2-naphtalényl)-2-propényl)]-5-cyanothiophène et 0,39 g d'une poudre blanche, le (E) 2-[1-(5,6,7,8-tétrahydro-5,5,8,8-tétraméthyl-2-naphtalényl)-2-propényl)]-5-cyanothiophène et 0,70 g du mélange des deux isomères (Z) et (E) (Rdt global = 68 %) .

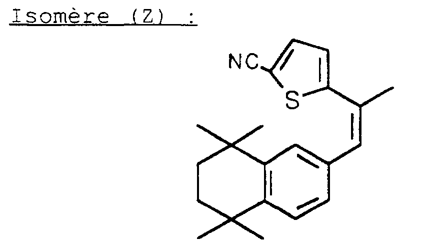

RMN¹H 200MHz (CDCl₃) : 1,10 et 1,25 (2s, 12H, 5,5,8,8-Me); 1,67 (s, 4H, 6,7-CH₂) ; 2,20 (s, 3H, Me vinylique); 6,65 (s, 1H, H vinylique); 6,90 (d, 2H, ArH, *J* 4Hz); 7,05 (s, 1H, ArH); 7,22 (d, 2H, ArH, *J* 8Hz); 7,40 (d, 1H, ArH, *J* 4Hz). 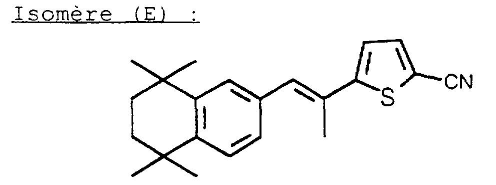
F(°C) = 108.
RMN¹H 200MHz (CDCl₃) : 1,30 (s, 12H, 5,5,8,8-Me); 1,70 (s, 4H, 6,7-CH₂) ; 2,30 (s, 3H, Me vinylique); 7,02 (s, 1H, H vinylique); 7,10-7,20 (m, 2H, ArH); 7,25-7,35 (m, 2H, ArH) ; 7,55 (d, 1H, ArH, *J* 4Hz).

### 2) (E) 5-[2-[1-(5,6,7,8-tétrahydro-5,5,8,8-tétraméthyl-2-naphtalényl)-2-propényl)]-5-thiényl]-1H-tétrazole (CB07734).

A une solution du (E) 2-[1-(5,6,7,8-tétrahydro-5,5,8,8-tétraméthyl-2-naphtalényl)-2-propényl)]-5-cyanothiophène (0,39 g, 1,16 mmol) dans du toluène anhydre (2,3 ml), on additionne successivement de l'oxyde de dibutylétain (38,3 mg, 0,14 mmol) et de l'azoture de triméthylsilyle (0,31 ml, 2,32 mmol). Le milieu réactionnel est chauffé 14 h à reflux (110°C) sous atmosphère d'argon et agitation magnétique. On purifie par chromatographie éclair sur silice (éluant MeOH : CH₂Cl₂ = 10 : 90) pour obtenir après évaporation 118,8 mg d'une poudre blanche, le (E) 5-[2-[1-(5,6,7,8-tétrahydro-5,5,8,8-tétraméthyl-2-naphtalényl)-2-propényl)]-5-thiényl]-*1H*-tétrazole (CB07734) (Rdt = 27%). 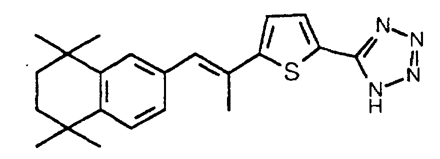
F (°C) = 186-189.
IR (cm⁻¹) : 3644; 3272; 1594; 1458; 1406.
RMN¹H 200MHz (CDCl₃) :1,27 et 1,28 (2s, 12H, 5,5,8,8-Me); 1,67 (s, 4H, 6,7-CH₂-); 2,28 (s, 3H, Me vinylique); 7,02 (s, 1H, H vinylique); 7,08-7,15 (m, 2H, ArH); 7,24-7,30 (m, 2H, ArH); 7,74 (d, 1H, ArH, *J* 4Hz).
MS EI 70eV (m/z, % intensité) : 378 (M⁺, 100 %); 358 (83) ; 69 (67).
MSHR EI 70 eV : Mₜᵣ = 378,1880 pour C₂₂H₂₆N₄S Mₜₕ = 378,1878.
HPLC Colonne Waters HR C₁₈, 8 x 100 mm, 6m, détecteur UV Waters 486 à 260 nm, débit 3 ml/min, éluant MeOH : H₂O = 90 : 10 + 0,1% de TFA, tétrazole (CB07734) tr = 3,49 min 98,9%.

### 3) (Z) 5-[2-[1-(5,6,7,8-tétrahydro-5,5,8,8-tétraméthyl-2-naphtalényl)-2-propényl)]-5-thiényl]-1H-tétrazole (CB92855).

A une solution du (Z) 2- [(5,6,7,8-tétrahydro-5,5,8,8-tétraméthyl-2-naphtalényl)-2-propényl]-5-cyanothiophène (0,41 g, 1,22 mmol) dans du toluène anhydre (2,4 ml), on additionne successivement de l'oxyde de dibutylétain (39,2 mg, 0,15 mmol) et de l'azoture de triméthylsilyle (0,325 ml, 2,44 mmol). Le milieu réactionnel est chauffé 15 h à reflux (110°C) sous atmosphère d'argon et agitation magnétique. On purifie par chromatographie éclair sur silice (éluant MeOH : CH₂Cl₂ = 5 : 95) suivie d'une HPLC préparative (éluant MeOH : H₂O = 92 : 8 + 0,1 % de TFA) pour obtenir après évaporation 192,6 mg d'une poudre blanche, le (Z) 5-[2-[1-(5,6,7,8-tétrahydro-5,5,8,8-tétraméthyl-2-naphtalényl)-2-propényl)]-5-thiényl]-*1H*-tétrazole (CB92855) (Rdt = 42%). 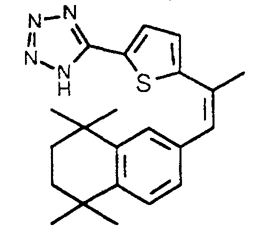
F (°C) = 126.
IR (cm⁻¹) : 2950; 1552; 1460; 1408; 1362.
RMN¹H 200MHz (CDCl₃) :1,07 et 1,20 (2s, 12H, 5,5,8,8-Me); 1,58 (s, 4H, 6,7-CH₂-); 2,22 (s, 3H, Me vinylique); 6,56 (s, 1H, H vinylique); 6,88-7,09 (m, 2H, ArH); 7,10-7,16 (m, 2H, ArH); 7,66 (d, 1H, ArH, *J* 4Hz).
MS EI 70eV (m/z, % intensité) : 378 (M⁺, 86 %); 350 (74 %); 319 (11 %); 165 (15 %); 69 (100 %).
MSHR El 70 eV : Mₜᵣ = 378,1889 pour C₂₂H₂₆N₄S Mₜₕ = 378,1878.
HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, tétrazole (CB07734) tr = 2,87 min 98,9%.

### Exemple 35 : Préparation de l'acide (Z) 5-[1-(5,6,7,8-tétrahydro-5,5,8,8-tétraméthyl-2-naphtalényl)-2-propényl]pyridinyl-2-carboxylique (CB16279).

Une suspension de (Z) 5-[1-(5,6,7,8-tétrahydro-5,5,8,8-tétraméthyl-2-naphtalényl)-2-propényl]pyridinyl-2-carboxylate de méthyle, (0,3 g, 0,82 mmol) en solution dans de la potasse (0,46g, 2,18 mmol) hydrométhanolique (H₂O 1,1 ml et MeOH 9 ml) est chauffée à reflux sous agitation magnétique pendant 3 h. On évapore le méthanol à l'évaporateur rotatif, reprend à l'eau (10 ml), acidifie par HCl 3N, extrait (5 x 30ml) au dichlorométhane, sèche la phase éthérée sur MgSO₄, filtre et évapore. Le produit brut est lavé au pentane puis filtré, on obtient 0,28 g d'un solide blanc, l'acide (Z) 5-[1-(5,6,7,8-tétrahydro-5,5,8,8-tétraméthyl-2-naphtalényl)-2-propényl]pyridinyl-2-carboxylique (CB16279) (Rdt = 98 %). 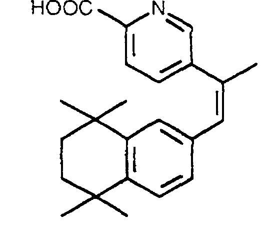
F(°C) = 62-64.
IR (cm⁻¹) : 3250; 2954; 1898; 1706; 1590.
RMN¹H 200MHz (CDCl₃) : 0,94 et 1,19 (2s, 12H, 5,5,8,8-Me); 1,56 (s, 4H, 6,7-CH₂-); 2,23 (d, 3H, Me vinylique, *J* 1,3 Hz); 6,67 (m, 1H, H vinylique); 6,73 (s, 2H, ArH); 7,09 (d, 1H, ArH, *J* 8Hz); 7,82 (d, 1H, ArH, *J* 8Hz); 8,15 (d, 1H, ArH, *J* 8Hz); 8,41 (s, 1H, ArH).
MS EI 70 eV (m/z, % intensité) : 349 (M⁺, 76%); 334 (100); 316 (5).
MSHR EI 70 eV : Mₜᵣ = 349,2059 pour C₂₃H₂₇NO₂ Mₜₕ = 349,2042.
HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, acide (CB16279) tr = 5,86 min 99,7%.

### Exemple 36 : Préparation des acides (E) et (Z) 2-[1-(5,6,7,8-tétrahydro-3,5,5,8,8-pentaméthyl-2-naphtalényl)-2-propényl]pyri-dinyl-5-carboxyliques (CB90525) et (CB93634).

### 1) (E) et (Z) 2-[1-(5,6,7,8-Tétrahydro-3,5,5,8,8-pentaméthyl-2-naphtalényl)-2-propényl]pyridinyl-5-carboxylates de méthyle.

A une solution de bromure de (5,6,7,8-tétrahydro-3,5,5,8,8-pentaméthyl-2-naphtalényl)-6-méthyltriphénylphosphonium (5,28 g, 9,48 mmol) dans 15 ml de THF, on ajoute à - 70 °C une solution de tBuOK 1M dans le THF (10 ml, 10 mmol). On poursuit l'agitation à - 70 °C pendant 45 min avant d'ajouter une solution du 2-acétylpyridine-5-carboxylate de méthyle (0,85 g, 4,74 mmol) dans 7 ml de THF à cette température. Le milieu réactionnel est porté à température ambiante et on poursuit l'agitation pendant 13 h. On hydrolyse ensuite à 0 °C par une solution de HCl 3N (30 ml). Après retour à température ambiante, on extrait au dichlorométhane, sèche sur MgSO₄, filtre et évapore les solvants. Le produit brut est purifié par chromatographie éclair sur silice (éluant éther de pétrole : éther = 100 : 10 puis 100 : 15). On obtient 0,59 g des esters (E) et (Z) 2-[1-(5,6,7,8-tétrahydro-3,5,5,8,8-pentaméthyl-2-naphtalényl)-2-propényl]pyridinyl-5-carboxylates de méthyle (Rdt = 34 %) ((E) : (Z) = 64 : 34).

### Isomère (Z) :

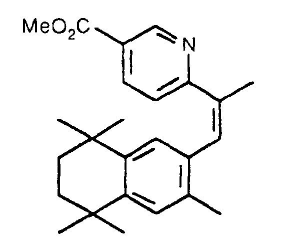

RMN¹H 200MHz (CDCl₃) : 0,60 et 1,15 (2 s, 12H, 5,5,8,8-Me); 1,40-1,60 (m, 4H, 6,7-CH₂-) ; 2,25 (s, 3H); 2,32 (s, 3H); 3,95 (s, 3H, MeO-); 6,60 (s, 1H); 6,75 (s, 1H); 7,05 (s, 1H, ArH); 7,62 (m, 1H, ArH); 7,92 (dd, 1H, ArH, *J* 2Hz et *J* 8Hz ); 9,15-9,25 (m, 1H, ArH).

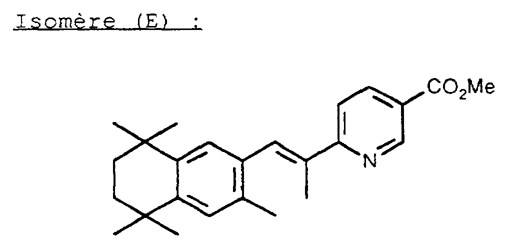

RMN¹H 200MHz (CDCl₃) : 1,30 et 1,32 (2 s, 12H, 5,5,8,8-Me); 1,40 (s, 4H, 6,7-CH₂-) ; 2,27 (s, 3H); 2,32 (s, 3H) ; 3,97 (s, 3H, MeO-); 6,82 (s, 1H); 6,90 (s, 1H); 7,08 (s, 1H, ArH); 7,20 (m, 1H, ArH); 8,02 (dd, 1H, ArH, *J* 2Hz et *J* 8Hz) ; 9,15-9,25 (m, 1H, ArH).

### 2) Acides (E) et (Z) 2-[1-(5,6,7,8-tétrahydro-3,5,5,8,8-pentaméthyl-2-naphtalényl)-2-propényl]pyri-dinyl-5-carboxylates de méthyle (CB90525 et CB93634).

Une suspension des (E) et (Z) du 2-[1-(5,6,7,8-tétrahydro-3,5,5,8,8-pentaméthyl-2-naphtalényl)-2-propényl]-pyridinyl-5-carboxylates de méthyle ((Z) : (E) = 64 : 36)), (0,59 g, 1,56 mmol) en solution dans de la potasse (0,87g, 15,6 mmol) hydrométhanolique (H₂O 2 ml et MeOH 17,6 ml) est chauffée à reflux sous agitation magnétique pendant 3 h. On évapore le méthanol à l'évaporateur rotatif, reprend à l'eau (10 ml), acidifie par HCl 3N, extrait (5 x 30ml) au dichlorométhane, sèche la phase éthérée sur MgSO₄, filtre et évapore. Le produit brut est purifié par HPLC préparative (éluant: MeOH : H₂O : 86 : 14) , on obtient 138 mg d'un solide blanc, l'acide (Z) 2-[1-(5,6,7,8-tétrahydro-3,5,5,8,8-pentaméthyl-2-naphtalényl)-2-propényl]pyridinyl-5-carboxylique (CB90525) (Rdt = 25 %) et 126 mg. d'un solide blanc, l'acide (E) 2-[1-(5,6,7,8-tétrahydro-3,5,5,8,8-pentaméthyl-2-naphtalényl)-2-propényl]pyridinyl-5-carboxylique (CB93634) (Rdt = 22 %) .

### Acide (Z) 2-[1-(5,6,7,8-tétrahydro-3,5,5,8,8-pentaméthyl-2-naphtalényl]-2-propényl]pyridinyl-5-carboxylique (CB90525).

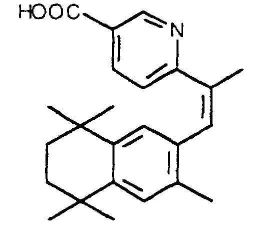

F(°C) = 85.
IR (cm⁻¹) : 2956; 2866; 1722; 1898; 1602; 1450; 1390.
RMN¹H 200MHz (CDCl₃) : 0,79 et 1,19 (2 s, 12H, 5,5,8,8-Me); 1,23-1,51 (m, 4H, 6,7-CH₂-) ; 2,27 (s, 3H) ; 2,37 (s, 3H); 6,51 (s, 1H); 6,95 (s, 1H); 7,02 (s, 1H, ArH) ; 7,16 (s, 1H); 8,30 (d, 1H, ArH, *J* 5Hz) ; 9,40 (s, 1H, ArH) ; 12,7 (s, 1H, H mobile).
MS IC négative 200 eV (m/z, % intensité) : 363 (M⁺, 67%); 362 (100).
HPLC Colonne Waters HR C₁₈, 8 x 100 mm, 6µ, détecteur UV Waters 486 à 260 nm, débit 3 ml/min, éluant MeOH : H₂O = 85 : 15 + 0,1% de TFA, acide (CB90525) tr = 3,32 min 98,4%.

### Acide (E) 2-[1-(5,6,7,8-tétrahydro-3,5,5,8,8-pentaméthyl-2-naphtalényl)-2-propényl]pyridinyl-5-carboxylique (CB93634).

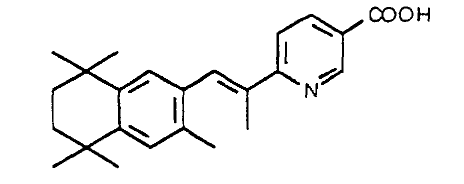

F(°C) = 157.
IR (cm⁻¹) : 3106; 2956; 2595; 1734; 1644; 1600; 1440; 1392; 1242; 1194; 1142.
RMN¹H 200MHz (CDCl₃) : 1,27 et 1,29 (2 s, 12H, 5,5,8,8-Me); 1,69 (s, 4H, 6,7-CH₂-) ; 2,29 (s, 3H); 2,35 (s, 3H) ; 7,16 (s, 1H); 7,21 (s, 1H); 7,55 (m, 1H, H mobile); 7,65 (s, 1H); 7,92 (d, 1H, J 8Hz); 8,73 (d, 1H, J 8 Hz); 9,47 (s, 1H,).
MS IC négative 200 ev (m/z, % intensité) : 363 (M⁺, 84%); 362 (100).
HPLC Colonne Waters HR C₁₈, 8 x 100 mm, 6µ, détecteur UV Waters 486 à 260 nm, débit 3 ml/min, éluant MeOH : H₂O = 90 : 10 + 0,1% de TFA, acide (CB93634) tr = 4,72 min 98,1%.

### Exemple 37 : Préparation des acides (E) et (Z) 5-[1-(5,6,7,8-tétrahydro-3,5,5,8,8-pentaméthyl-2-naphtalényl)-2-propényl]pyridinyl-2-carboxyliques (CB56004) et (CB71329).

### 1) (E) et (Z) 5-[1-(5,6,7,8-tétrahydro-3,5,5,8,8-pentaméthyl-2-naphtalényl)-2-propényl]pyridinyl-2-(N,N-diisopropyl)amides.

A une solution de bromure de (5,6,7,8-tétrahydro-3,5,5,8,8-pentaméthyl-2-naphtalényl)-6-méthyltriphénylphosphonium (11,1 g, 19,9 mmol) dans 31 ml de THF, on ajoute à - 70 °C une solution de tBuOK 1M dans le THF (21 ml, 21 mmol). On poursuit l'agitation à - 70 °C pendant 1 h avant d'ajouter le 5-acétyl-2-(*N*,*N*-diisopropyl)amide (2,47 g, 9,94 mmol) à cette température. Le milieu réactionnel est porté à température ambiante et on poursuit l'agitation pendant 18 h. On hydrolyse ensuite à 0 °C par une solution de HCl 3N (25 ml). Après retour à température ambiante, on extrait au dichlorométhane, sèche sur MgSO₄, filtre et évapore les solvants. Le produit brut est purifié par chromatographie éclair sur silice (éluant éther de pétrole : éther = 100 : 10 et 100 : 20). On obtient 3,59 g d'une gomme jaunâtre, les (E) et (Z) 5-[1-(5,6,7,8-tétrahydro-3,5,5,8,8-pentaméthyl-2-naphtalényl)-2-propényl]pyridinyl-2-(*N*,*N*-diisopropyl)amides (Rdt global = 83%) ((E) : (Z) = 1 : 2).

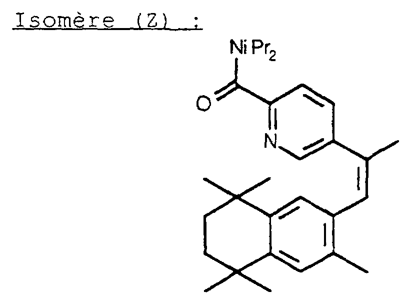

RMN¹H 200MHz (CDCl₃) : 0,85 (s, 6H); 1,05-1,20 (m, 12H); 1,25 (s, 6H); 1,40-1,60 (m, 4H, 6,7-CH₂); 2,20 (s, 3H) ; 2,25 (s, 3H); 3,35-3,65 m, 2H); 6,60 (s, 1H); 6,65 (s, 1H); 7,00 (s, 1H); 7,30 (m, 1H); 7,45 (m, 1H) ; 8,25 (m, 1H).

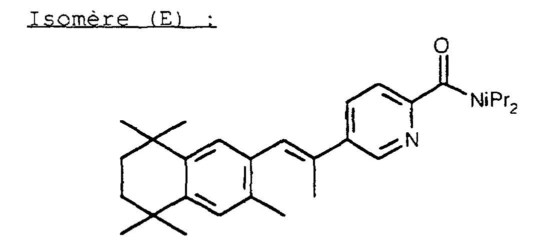

RMN¹H 200MHz (CDCl₃) : 0,9 (s, 12H, 5,5,8,8-Me); 1,05-1,20 (m, 12H); 1,30 (s, 4H, 6,7-CH₂) ; 2,17 (s, 3H); 2,22 (s, 3H) ; 3,70-3,95 (m, 2H); 6,92 (m, 1H) ; 7,10 (m, 1H); 7,20 (m, 1H); 7,50 (m, 1H); 7,80-7,90 (m, 1H); 8,45 (m, 1H).

### 2) (E) et (Z) 5-[1-(5,6,7,8-tétrahydro-3,5,5,8,8-pentaméthyl-2-naphtalényl)-2-propényl)-2-formyl pyridines.

A une solution des (E) et (Z) 5-[1-(5,6,7,8-tétrahydro-3,5,5,8,8-pentaméthyl-2-naphtalényl)-2-propényl]-pyridinyl-2-(*N*,*N*-diisopropyl)amides ((E) : (Z) = 1 : 2) (2,56 g, 5,86 mmol) dans 28 ml de THF, on ajoute à -70 °C une solution d'hydrure de diisobutylaluminium 1,5 M dans le toluène (15,9 ml, 8,8 mmol). On laisse remonter à température ambiante et agite à cette température pendant 2 h. On refroidit à - 70 °C et on hydrolyse par une solution aqueuse de HCl 3N (25 ml), on remonte à température ambiante et on extrait au dichlorométhane (5 x 50 ml). La phase organique est séchée sur MgSO₄, filtrée et évaporée. Le produit brut est purifié par chromatographie éclair sur silice (éluant éther de pétrole : éther = 100 : 10). On obtient 0,57 g d'une huile jaune, les (E) et (Z) 5-[1-(5,6,7,8-tétrahydro-3,5,5,8,8-pentaméthyl-2-naphtalényl)-2-propényl]-2-formylpyridines (Rdt = 28 %) (E : Z = 1 : 1).

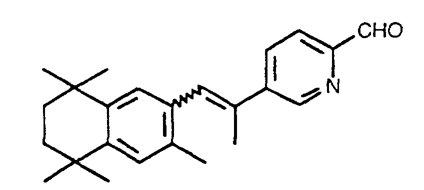

RMN¹H 200MHz (CDCl₃) : 0,76 (s, 0,5x6H); 1,20 (s, 0,5x6H); 1,28 (s, 0,5x12H); 1,40-1,60 (m, 0,5x4H) ; 1,68 (s, 0,5x4H); 2,15-2,30 (m, 6H); 6,51 (s, 0,5x1H); 6,72 (s, 0,5x1H); 6,95-7,05 (m, 1H) ; 7,14 (s, 0,5x1H); 7,18 (s, 0,5x1H); 7,55-7,70 (m, 0,5x1H); 7,80 (d, 0,5x1H, J 8Hz); 7,96 (m, 1H); 8,45 (m, 0,5x1H) ; 8,95 (m, 0,5x1H); 9,96 (s, 0,5x1H); 10,08 (s, 0,5x1H).

### 3) (E) et (Z) 5-[1-(5.6,7,8-tétrahydro-3,5,5,8,8-pentaméthyl-2-naphtalényl)-2-propényl]pyridinyl-2-carboxylates de méthyle,

A une solution du mélange des (E) et (Z) 5-[1-(5,6,7,8-tétrahydro-3,5,5,8,8-pentaméthyl-2-naphtalényl)-2-propényl]-2-formylpyridines (0,57 g, 1,64 mmol) dans 41 ml de méthanol, on ajoute successivement 0,145 ml d'acide acétique, de l'oxyde de manganèse (1,43 g, 16,5 mmol) et du cyanure de sodium (0,40 g, 8,20 mmol) à température ambiante. Le milieu réactionnel est agité à cette température pendant 4 h. On filtre sur papier, évapore et reprend par 20 ml d'eau, extrait à l'éther (5 x 50 ml), sèche sur MgSO₄, filtre et évapore. Le produit brut est purifié par chromatographie éclair sur silice (éluant éther de pétrole : éther = 70 : 30). On obtient après évaporation 0,11 g d'une pate jaune, le (Z) 5-[1-(5,6,7,8-tétrahydro-3,5,5,8,8-pentaméthyl-2-naphtalényl)-2-propényl]pyridinyl-2-carboxylate de méthyle (Rdt = 18 %) et 0,09 g d'une poudre blanche, le (E) 5-[1-(5,6,7,8-tétrahydro-3,5,5,8,8-pentaméthyl-2-naphtalényl)-2-propényl]pyridinyl-2-carboxylate de méthyle (Rdt = 14 %).

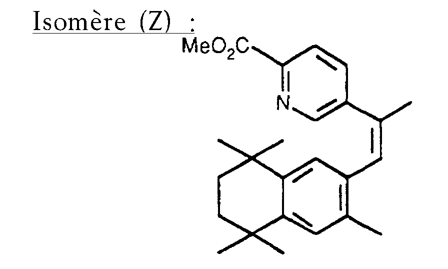

RMN¹H 200MHz (CDCl₃) : 0,78 et 1,18 (s, 6H, 5,5,8,8-Me); 1,40-1,65 (m, 4H, 6,7-CH₂-); 2,21 (s, 3H, ArMe); 2,24 (s, 3H, Me vinylique); 3,94 (s, 3H, -OMe); 6,52 (s, 1H); 6,67 (s, 1H); 6,98 (s, 1H, ArH); 7,55-7,59 (m, 1H, ArH); 7,94 (d, 1H, ArH *J* 7Hz); 8,38 (m, 1H, ArH). 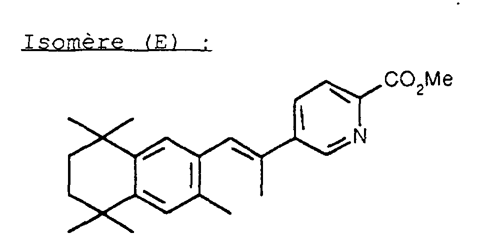
F (°C) = 132.
RMN¹H 200MHz (CDCl₃) : 1,27 (s, 12H, 5,5,8,8-Me); 1,68 (s, 4H, 6,7-CH₂-); 2,20 (s, 3H, Me vinylique); 2,23 (s, 3H, ArMe); 4,00 (s, 3H, -DMe); 6,94 (s, 1H); 7,12 (s, 1H); (m, 2H, ArH); 7,18 (s, 1H); 7,89-7,94 (m, 1H, ArH); 8,11 (d, 1H, ArH, *J* 8Hz); 8,89 (m, 1H, ArH).

### 4) Acide (Z) 5-[1-(5,6,7,8-tétrahydro-3,5,5,8,8-pentaméthyl-2-naphtalényl)-2-propényl]pyridinyl-2-carboxylique (CB56004).

Une suspension de (Z) 5-[1-(5,6,7,8-tétrahydro-3,5,5,8,8-pentaméthyl-2-naphtalényl)-2-propényl]pyridinyl-2-carboxylate de méthyle, (110 mg, 0,29 mmol) en solution dans de la potasse (160 mg, 2,90 mmol) hydrométhanolique (H₂O 0,3 ml et MeOH 3,1 ml) est chauffée à reflux sous agitation magnétique pendant 6 h. On évapore le méthanol à l'évaporateur rotatif, reprend à l'eau (10 ml), acidifie par HCl 3N, extrait (3 x 20ml) au dichlorométhane, sèche la phase éthérée sur MgSO₄, filtre et évapore. Le produit brut est lavé au pentane puis filtré, on obtient 80 mg d'un solide, l'acide (Z) 5-[1-(5,6,7,8-tétrahydro-3,5,5,8,8-pentaméthyl-2-naphtalényl)-2-propényl)pyridinyl-2-carboxylique (CB56004) (Rdt = 76 %). 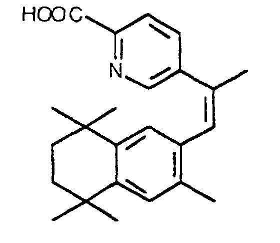
F(°C) = 66.
IR (cm⁻¹) : 3250; 2924; 1706; 1590; 1446; 1282; 1240; 1150; 1030
RMN¹H 200MHz (CDCl₃) : 0,75 et 1,20 (2s, 12H, 5,5,8,8-Me); 1,23 (s, 3H, Me vinylique); 1,50-1,56 (m, 4H, 6,7-CH₂-); 2,26 (s, 3H, ArMe); 6,48 (s, 1H); 6,73 (s, 1H); 7,03 (s, 1H); 7,96 (dd, 1H, ArH, *J* 1,7 Hz et *J* 8 Hz); 8,05 (d, 1H, ArH *J* 8 Hz); 8,24 (s, 1H, ArH).
MS EI 70 eV (m/z, % intensité) : 363 (M⁺, 70%); 348 (100); 330 (35).
MSHR EI 70 eV : Mₜᵣ = 363,2201 pour C₂₄H₂₉NO₂ Mₜₕ = 363,2198.
HPLC Colonne Waters HR C₁₈, 8 x 100 mm, 6µ, détecteur UV Waters 486 à 260 nm, débit 3 ml/min, éluant MeOH : H₂O = 85 : 15 + 0,1% de TFA, acide (CB56004) tr = 3,22 min 97,3%

### 5) Acide (E) 5-[1-(5,6,7,8-tétrahydro-3,5,5,8,8-pentaméthyl-2-naphtalényl)-2-propényl]pyridinyl-2-carboxylique (CB71329).

Une suspension de (E) 5-[1-(5,6,7,8-tétrahydro-3,5,5,8,8-pentaméthyl-2-naphtalényl)-2-propényl]pyridinyl-2-carboxylate de méthyle, (90 mg, 0,23 mmol) en solution dans de la potasse (130 mg, 2,30 mmol) hydrométhanolique (H₂O 0,25 ml et MeOH 2,5 ml) est chauffée à reflux sous agitation magnétique pendant 2 h. On évapore le méthanol à l'évaporateur rotatif, reprend à l'eau (10 ml), acidifie par HC1 3N, extrait (3 x 20ml) au dichlorométhane, sèche la phase éthérée sur MgSO₄, filtre et évapore. Le produit brut est lavé au pentane puis filtré , on obtient 80 mg d'un solide blanc, l'acide (E) 5-[1-(5,6,7,8-tétrahydro-3,5,5,8,8-pentaméthyl-2-naphtalényl)-2-propényl]pyridinyl-2-carboxylique (CB71329) (Rdt = 95 %). 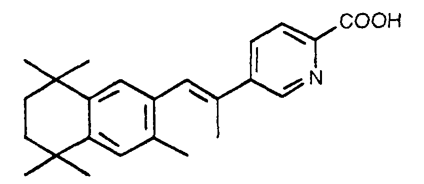
F(°C) = 172.
IR (cm⁻¹) : 3406; 2924; 2458; 1894; 1710; 1584; 1452; 1320; 1128; 1132.
RMN¹H 200MHz (CDCl₃) : 1,27 (s, 12H, 5,5,8,8-Me); 1,68 (s,4H, 6,7-CH₂-) ; 2,21 (s, 3H); 2,24 (s, 3H); 6,98 (s, 1H, H vinylique); 7,13 (s, 1H, ArH); 7,17 (s, 1H); 8,03 (d, 1H, ArH, *J* 8 Hz); 8,21 (d, 1H, ArH *J* 8 Hz); 8,77 (m, 1H, ArH).
MS EI 70 eV (m/z, % intensité) : 363 (M⁺, 75%); 348 (100); 330 (44); 304 (33).
MSHR EI 70 eV : Mₜᵣ = 363,2195 pour C₂₄H₂₉NO₂ Mₜₙ = 363,2198.
HPLC Colonne Waters HR C₁₈, 8 x 100 mm, 6µ, détecteur UV Waters 486 à 260 nm, débit 3 ml/min, éluant MeOH : H₂O = 85 : 15 + 0,1% de TFA, acide (CB71329) tr = 4,67 min 97,8%.

## Revendications

1. Dérivés polycycliques aromatiques de type rétinoïde de formule générale (I) : 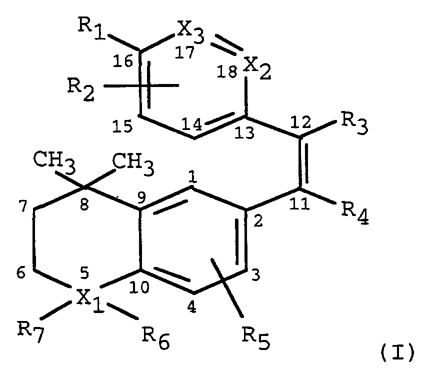
dans laquelle les groupements R₃ et R₄ portés par la double liaison entre les carbones 11 et 12 sont en cis et R₁, R₂, R₃, R₄, R₅, R₆, R₇, X₁, X₂ et X₃ ont les significations suivantes :
- R₁ représente un groupe tétrazoyle ou un groupe -COOH.
- R₂ représente un atome d'hydrogène, un atome d'halogène et plus particulièrement un atome de fluor, un radical alkyle en C₁ à C₆, un groupe de formule -COOH, -DR₁₁, -SR₁₁, -(CF₂)ₙCF₃ où n est un nombre entier compris entre 0 et 10, ou un groupe -DCDR₁₁, et quand cela est possible leurs sels avec des acides tolérés physiologiquement, ou un groupe aminé de formule : 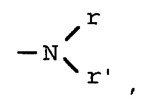
dans laquelle r et r' identiques ou différents, représentent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle et R₁₁ représente un atome d'hydrogène, un radical alkyle en C₁ à C₆, un radical fluoroalkyle ayant de 1 à 6 atomes de carbone et de 3 à 7 atomes de fluor, un radical aryle ou un radical aralkyle.
- R₃ représente un atome d'hydrogène, un radical trifluorométhyle, un radical aryle, un radical aralkyle ou un radical alkyle en C₁ à C₆, éventuellement substitué par un hydroxyle ou par un ou plusieurs atomes, de fluor, par un alcoxy en C₁ à C₆ ou par un groupe de formule -(C=O)R₁₂, dans laquelle R₁₂ représente un atome d'hydrogène, un radical alkyle en C₁ à C₆, un radical hydroxyle, un radical alcoxy en C₁ à C₆ ou un groupe aminé de formule : 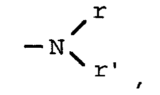
dans laquelle r et r' ont la même signification que précédemment.
- R4 représente un atome d'hydrogène ou un radical aryle.
- R₅ représente un atome d'hydrogène, un radical alkyle en C₁ à C₆, un atome d'halogène, un radical fluoroalkyle ayant de 1 à 6 atomes de carbone et de 3 à 7 atomes de fluor, ou un groupe de formule -OR₁₃ où R₁₃ représente un atome d'hydrogène, un radical alkyle en C₁ à C₆, un radical aryle ou un radical aralkyle ou un groupement trifluorométhyle.
- X₁ représente un atome de carbone ou un atome de soufre, et alors,
- R₆ et R₇ sont :
. des radicaux méthyles, dans le cas où X₁ est un atome de carbone,
. identiques ou différents et représentent soit rien (cas d'un sulfure, -S-), soit un oxygène (cas d'un sulfoxyde -SO- ou d'une sulfone -SO₂-), dans le cas où X₁ est un atome de soufre.
- X₂ et X₃, identiques ou différents, représentent un atome de carbone, un atome d'oxygène ou un atome d'azote, ou encore X₂-X₃ sont un seul atome de soufre, d'oxygène, ou d'azote, ainsi le noyau porteur de X₂ et X₃ peut être benzénique, pyridinique, thiophénique, furanique, pyrrolique, ou, dans le cas où X₂ est un atome d'oxygène et X₃ un atome de carbone, C₁₃ et C₁₄ représente un seul et même atome de carbone, ainsi le noyau porteur de X₂ et X₃ peut être isoxazolique.

2. Dérivés selon la revendication 1, **caractérisés en ce que** dans la formule (I), R₄ représente un atome d'hydrogène.

3. Dérivés selon les revendications 1 ou 2, **caractérisés en ce que** dans la formule (I), R₃ représente un alkyle en C₁ à C₆ ou un radical trifluorométhyle ou un groupe -(CH₂)ₙCF₃ où n est un nombre entier compris entre 0 et 10.

4. Dérivés selon l'une quelconque des revendications 1 à 3, **caractérisés en ce que** dans la formule (I), R2 représente un atome d'hydrogène.

5. Les dérivés selon l'une quelconque des revendications 1 à 4 :
- L'acide (Z) 2-[1-(5,6,7,8-tétrahydro-5,5,8,8-tétraméthyl-2-naphthalényl)-2-propényl]pyridinyl-5-carboxylique.
- L'acide (E) 4-[1-trifluorométhyl-2-(5,6,7,8-tétrahydro-5,5,8,8-tétraméthyl-2-naphtalényl)-1-éthényl]benzoïque.
- L'acide (Z) 4-[1-(5,6,7,8-tétrahydro-3,5,5, 8,8-pentaméthyl-2-naphtalényl)-2-propényl]benzoïque.
- L'acide (E) 4-[1-trifluorométhyl-2-(5,6,7,8-tétrahydro-3,5,5,8,8-pentaméthyl-2-naphtalényl)-1-éthényl]benzoïque.
- L'acide (Z) 4-[1-(3',4'-dihydro-4',4'-diméthyl-1',1'-dioxyde-2'H-1'-benzothiopyran-6'-yl)-2-propényl]benzoïque.
- Le (Z)-5-[4-[1-(5,6,7,8-tétrahydro-5,5,8,8-tétraméthyl-2-naphtalényl)-2-propényl]phényl]-1H-tétrazole.
- L'acide (E) 5-[4-[1-trifluorométhyl-2-(5,6,7,8-tétrahydro-5,5,8,8-tétraméthyl-2-naphtalényl)-1-éthényl]phényl]-1H-tétrazole.
- Le (Z) 5-[4-[1-(5,6,7,8-tétrahydro-3,5,5,8,8-pentaméthyl-2-naphtalényl)-2-propényl]phényl]-1H-tétrazole.
- L'acide (E) 5-[4-[1-trifluorométhyl-2-(5,6,7,8-tétrahydro-3,5,5,8,8-pentaméthyl-2-naphtalényl)-1-éthényl]phényl]-1H-tétrazole.
- Le (Z) 5 [4-[1-(3',4'-dihydro-4',4'-diméthyl-1',1'-dioxyde-2'H-1'-benzothiopyran-6'-yl)-2-propényl]phényl]-1H-tétrazole.
- L'acide (Z) 2-[1-(5,6,7,8-tétrahydro-5,5,8,8-tétraméthyl-2-naphtalényl)-2-propényl]thiényl-5-carboxylique.
- Le (2) 5-[2-[1-(5,6,7,8-Tétrahydro-5,5,8,8-tétraméthyl-2-naphtalényl)-2-propényl)]5-thiényl]-1*H* tétrazole.
- L'acide (Z) 5-[1-(5,6,7,8-tétrahydro-5,5,8,8-tétraméthyl-2-naphtalényl) -2-propényl]pyridinyl-2-carboxylique.
- L'acide (Z) 2-[1-(5,6,7,8-tétrahydro-3,5,5,8,8-pentaméthyl-2-naphtalényl)-2-propényl]pyridinyl-5-carboxylique.
- L'acide (Z) 5-[1-(5,6,7,8-tétrahydro-3,5,5,8,8-pentaméthyl-2-naphtalényl)-2-propényl]pyridinyl-2-carboxylique.
- L'acide (Z) 5-[1-(5,6,7,8-tétrahydro-5,5,8,8-tétraméthyl-2-naphtalényl)-2-propényl)]isoxazole-3-carboxylique.
- L'acide (Z) 5-[1-(5,6,7,8-tétrahydro-3,5,5,8,8-pentaméthyl-2-naphtalényl)-2-propényl)]isoxazole-3-carboxylique.
- Le (Z) 1-[4-[1-(5,6,7,8-Tétrahydro-5,5,8,8-tétraméthyl-2-naphtalényl)-2-propényl]phényl]-5-trifluorométhyl-1*H*-tétrazole.

6. Composition thérapeutique, dermatologique ou cosmétique renfermant au moins un dérivé de formule I défini dans l'une quelconque des revendications 1 à 5, sous forme libre ou sous forme d'un sel pharmaceutiquement acceptable, en association avec un véhicule ou diluant traditionnel.

7. Utilisation d'un dérivé de formule (I) défini dans l'une quelconque des revendications 1 à 5, pour la fabrication d'une composition dermatologique ou cosmétique utile dans le traitement ou la prévention des maladies de la peau.

8. Utilisation d'un dérivé de formule (I) suivante 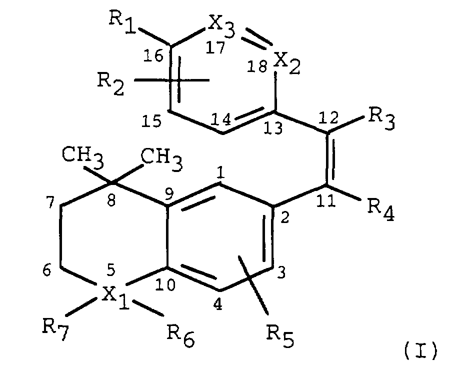
dans laquelle R₁ représente un atome d'hydrogène, un radical alkyle en C₁ à C₆, ou un groupe de formule -CH₂OH, -OH, -CHO, -COOH, -COR₈, -CH₂OCOR₉, -SH, -S-alkyl, p-hydroxyphénylaminocarbonyl, tétrazol-5-ylaminocarbonyl, tétrazol-5-yl, et quand cela est possible leurs sels avec des acides tolérés physiologiquement, où R₈ et R₉ sont :
. un atome d'hydrogène, un groupe -OH, un radical alkyle en C₁ à C₆ ou un groupe de formule -OR₁₀, où R₁₀ représente un radical alkyle ramifié ou non ayant de 1 à 20 atomes de carbone, un radical alkényle ramifié ou non ayant de 2 à 20 atomes de carbone, un radical aryle ou aralkyle, ou
. un groupe aminé de formule : 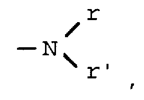
dans laquelle r et r', identiques ou différents, représentent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle.
et R₂, R₃, R₄, R₅, R₆, R₇, X₁, X2 et X3 ont les mêmes significations que dans les revendications 1 à 3, pour la fabrication d'une composition thérapeutique utile dans le traitement ou la prévention des cancers.

9. Utilisation d'un dérivé de formule (I) défini dans l'une quelconque des revendications 1 à 5, pour la fabrication d'une composition thérapeutique utile dans le traitement ou la prévention des cancers.

10. Utilisation d'un dérivé de formule (I) suivante 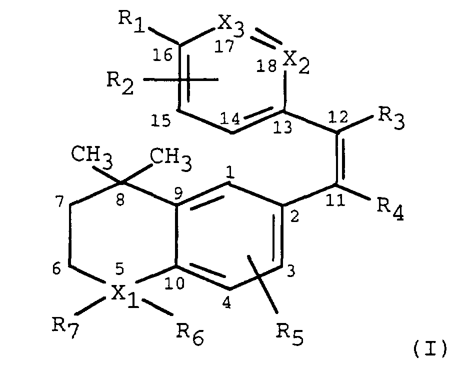
dans laquelle R₁ représente un atome d'hydrogène, un radical alkyle en C₁ à C₆, ou un groupe de formule -CH₂OH, -OH, -CHO, -COOH, -COR₈, -CH₂OCOR₉, -SH, -S-alkyl, p-hydroxyphénylaminocarbonyl, tétrazol-5-ylaminocarbonyl, tétrazol-5-yl, et quand cela est possible leurs sels avec des acides tolérés physiologiquement, où R₈ et R₉ sont :
. un atome d'hydrogène, un groupe -OH, un radical alkyle en C₁ à C₆ ou un groupe de formule -OR₁₀, où R₁₀ représente un radical alkyle ramifié ou non ayant de 1 à 20 atomes de carbone, un radical alkényle ramifié ou non ayant de 2 à 20 atomes de carbone, un radical aryle ou aralkyle, ou
. un groupe aminé de formule : 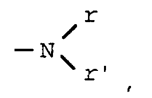
dans laquelle r et r', identiques ou différents, représentent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle.
et R₂, R₃, R₄, R₅, R₆, R₇, X₁, X₂ et X₃ ont les mêmes significations que dans les revendications 1 à 3, pour la fabrication d'une composition thérapeutique utile dans le traitement ou la prévention du diabète non insulino-dépendant.

11. Utilisation d'un dérivé de formule (I) défini dans l'une quelconque des revendications 1 à 5, pour la fabrication d'une composition thérapeutique utile dans le traitement ou la prévention du diabète non insulino-dépendant.

12. Utilisation d'un dérivé de formule I suivante : 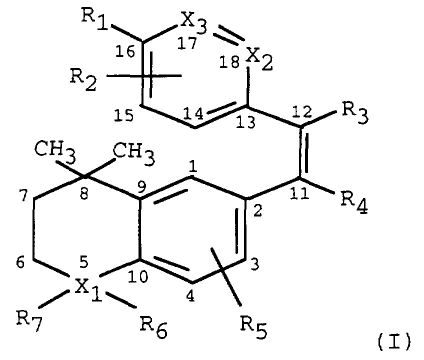
dans laquelle R₁ représente un atome d'hydrogène, un radical alkyle en C₁ à C₆, ou un groupe de formule -CH₂OH, -OH, -CHO, -COOH, -COR₈, -CH₂OCOR₉, -SH, -S-alkyl, p-hydroxyphénylaminocarbonyl, tétrazol-5-ylaminocarbonyl, tétrazol-5-yl, et quand cela est possible leurs sels avec des acides tolérés physiologiquement, où R₈ et R₉ sont :
un atome d'hydrogène, un groupe -OH, un radical alkyle en C₁ à C₆ ou un groupe de formule -OR₁₀, où R₁₀ représente un radical alkyle ramifié ou non ayant de 1 à 20 atomes de carbone, un radical alkényle ramifié ou non ayant de 2 à 20 atomes de carbone, un radical aryle ou aralkyle, ou
. un groupe aminé de formule : 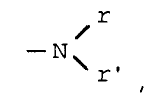
dans laquelle r et r', identiques ou différents, représentent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle.
et R₂, R₃, R₄, R₅, R₆, R₇, X₁, X₂ et X₃ ont les mêmes significations que dans les revendications 1 à 3, pour la fabrication d'une composition thérapeutique utile dans le traitement ou la prévention des maladies inflammatoires.

13. Utilisation d'un dérivé de formule (I) défini dans l'une quelconque des revendications 1 à 5, pour la fabrication d'une composition thérapeutique utile dans le traitement ou la prévention des maladies inflammatoires.

14. Utilisation d'un dérivé de formule (I) suivante 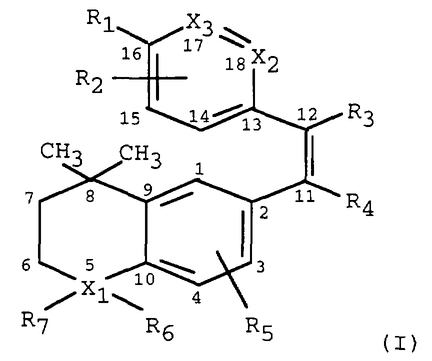
dans laquelle R₁ représente un atome d'hydrogène, un radical alkyle en C₁ à C₆, ou un groupe de formule -CH₂OH, -OH, -CHO, -COOH, -COR₈, -CH₂OCOR₉, -SH, -S-alkyl, p-hydroxyphénylaminocarbonyl, tétrazol-5-ylaminocarbonyl, tétrazol-5-yl, et quand cela est possible leurs sels avec des acides tolérés physiologiquement, où R₈ et R₉ sont :
. un atome d'hydrogène, un groupe -OH, un radical alkyle en C₁ à C₆ ou un groupe de formule -OR₁₀, où R₁₀ représente un radical alkyle ramifié ou non ayant de 1 à 20 atomes de carbone, un radical alkényle ramifié ou non ayant de 2 à 20 atomes de carbone, un radical aryle ou aralkyle, ou
. un groupe aminé de formule : 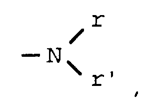
dans laquelle r et r', identiques ou différents, représentent un atome d'hydrogène, un radical alkyle en C₁ à C₆, un radical aryle ou aralkyle, un reste d'α-aminoacide, un reste de sucre ou un hétérocycle dans lequel r et r' pris ensemble forment un hétérocycle.
et R₂, R₃, R₄, R₅, R₆, R₇, X₁, X₂ et X₃ ont les mêmes significations que dans les revendications 1 à 3, pour la fabrication d'une composition thérapeutique utile dans le traitement ou la prévention des maladies immunitaires.

15. Utilisation d'un dérivé de formule (I) défini dans l'une quelconque des revendications 1 à 5, pour la fabrication d'une composition thérapeutique utile dans. le traitement ou la prévention des maladies immunitaires.

## Patentansprüche

1. Polyzyklisches aromatisches Derivat vom Retinoid-Typ der allgemeinen Formel (I): 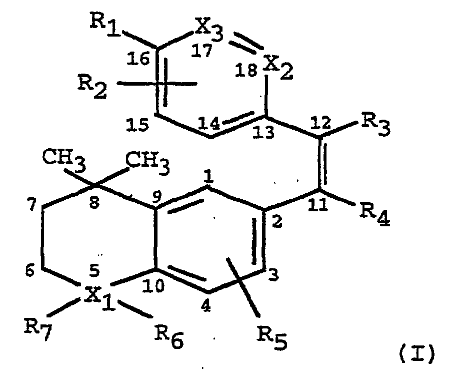
in der die Gruppen R₃ und R₄ an der Doppelbindung zwischen den Kohlenstoffatomen 11 und 12 in cis-Stellung sind und R₁, R₂, R₃, R₄, R₅, R₆, R₇, X₁, X₂ und X₃ die folgenden Bedeutungen haben:
- R₁ stellt eine Tetrazoylgruppe oder eine -COOH-Gruppe dar.
- R₂ steht für ein Wasserstoffatom, ein Halogenatom, besonders ein Fluoratom, einen C₁ bis C₆-Alkylrest, eine Gruppe der Formel COOH, OR₁₁, SR₁₁, -(CF₂)ₙCF₃, wobei n eine ganze Zahl zwischen 0 und 10 ist, oder eine Gruppe OCOR₁₁, und, soweit möglich, ihre Salze mit physiologisch akzeptablen Säuren, oder eine Aminogruppe der Formel: 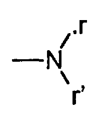
in der r und r', identisch oder verschieden, ein Wasserstoffatom, einen C₁ bis C₆-Alkylrest, einen Aryl- oder Aralkylrest, einen α-Aminosäurerest, einen Zuckerrest oder einen Heterozyklus, bei dem r und r' gemeinsam den Heterozyklus bilden darstellen und R₁₁ ein Wasserstoffatom, einen C₁ bis C₆-Alkylrest, einen Fluoralkylrest mit 1 bis 6 Kohlenstoffatomen und 3 bis 7 Fluoratomen, einen Arylrest oder einen Aralkylrest darstellt.
- R3 steht für ein Wasserstoffatom, einen Trifluoromethylrest, einen Arylrest, einen Aralkylrest oder einen C₁ bis C₆-Alkylrest, eventuell substituiert mit einem Hydroxylrest, oder mit einem oder mehreren Fluoratomen, mit einer C₁ bis C₆-Alkoxygruppe oder einer Gruppe der Formel -(C=O)R12, in der R12 ein Wasserstoffatom, einen C₁ bis C₆-Alkylrest, eine Hydroxylgruppe, eine C₁ bis C₆ Alkoxygruppe, oder eine Aminogruppe der Formel 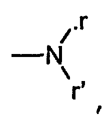 darstellt, in der r und r' die gleiche Bedeutung wie vorstehend haben.
- R4 stellt ein Wasserstoffatom oder einen Arylrest dar.
- R5 steht für ein Wasserstoffatom, einen C₁ bis C₆-Alkylrest, ein Halogenatom, einen Fluoralkylrest mit 1 bis 6 Kohlenstoffatomen und 3 bis 7 Fluoratomen, oder eine Gruppe der Formel OR₁₃, bei der R₁₃ ein Wasserstoffatom, einen C₁ bis C₆-Alkylrest, einen Arylrest oder einen Aralkylrest oder eine Trifluoromethylgruppe darstellt.
- X₁ ein Kohlenstoff- oder Schwefelatom darstellt, und dann,
- R₆ und R₇
. Methylreste sind, wenn X1 ein Kohlenstoffatom ist,
. identisch oder verschieden sind, und entweder nichts darstellen (Fall eines Sulfids, -S-, oder einen Sauerstoff (Fall eines Sulfoxyds -SO- oder eines Sulfons -SO₂-), wenn X1 ein Schwefelatom ist
- X₂ und X₃, identisch oder verschieden, stehen für ein Kohlenstoffatom, ein Sauerstoffatom oder ein Stickstoffatom, oder X₂ und X₃ zusammen sind ein einziges Schwefel- Sauerstoff- oder Stickstoffatom, so dass der Ring mit X₂ und X₃ ein Benzen-, Pyridin-, Thiophen-, Furan-, Pyrrolring sein kann, oder, im Fall dass X₂ ein Sauerstoffatom ist und X₃ ein Kohlenstoffatom ist, stellen C₁₃ und C₁₄ ein einziges Kohlenstoffatom dar, so dass der Ring mit X₂ und X₃ ein Isoxazolring sein kann.

2. Derivate nach Anspruch 1, die **dadurch gekennzeichnet** werden, dass in der Formel (I) R₄ ein Wasserstoffatom darstellt.

3. Derivate nach Anspuch 1 oder 2, die **dadurch gekennzeichnet** werden, dass in der Formel (I) R₃ einen C₁ bis C₆-Alkylrest oder einen Trifluoromethylrest oder eine -(CF₂)ₙCF₃-Gruppe darstellt, wobei n eine ganze Zahl zwischen 0 und 10 ist.

4. Derivate nach irgendeinem der Ansprüche 1 bis 3, die **dadurch gekennzeichnet** werden, dass in der Formel (I) R₂ ein Wasserstoffatom darstellt.

5. Die Derivate nach irgendeinem der Ansprüche 1 bis 4:
- Die (Z) 2-[1- (5,6,7,8-Tetrahydro- 5,5,8,8-tetramethyl- 2-naphtalenyl)- 2-propenyl ] pyridinyl -5-carbonsäure.
- Die (E) 4-[ 1-Trifluoromethyl- 2-(5,6,7,8-tetrahydro- 5,5,8,8-tetramethyl- 2-naphtalenyl)- 1-ethenyl] benzoesäure
- Die (Z) 4-[1- (5,6,7,8-Tetrahydro- 3,5,5,8,8-pentamethyl- 2-naphtalenyl))- 2-propenyl ] benzoesäure
- Die (E) 4-[1-Trifluoromethyl- 2-(5,6,7,8-tetrahydro- 3,5,5,8,8-pentamethyl- 2-naphtalenyl))- 1-ethenyl] benzoesäure
- Die (Z) 4-[1- (3',4'-Dihydro- 4',4'-dimethyl-1',1'-dioxo- 2'H- 1'-benzothiopyran-6'-yl)- 2-propenyl] benzoesäure
- Das (Z) 5-[4-[1- (5,6,7,8-Tetrahydro- 5,5,8,8-tetramethyl- 2-naphtalenyl)- 2-propenyl ] phenyl] 1H-tetrazol.
- Die (E) 5-[4-[1-Trifluoromethyl- 2-(5,6,7,8-tetrahydro- 5,5,8,8-tetramethyl- 2-naphtalenyl)-1-ethylenyl ] phenyl] 1H-tetrazolsäure.
- Das (Z) 5-[4-[1- (5,6,7,8-Tetrahydro- 3,5,5,8,8-pentamethyl- 2-naphtalenyl)- 2-propenyl ] phenyl] 1H-tetrazol.
- Die (E) 5-[4-[1-Trifluoromethyl- 2-(5,6,7,8-tetrahydro- 3,5,5,8,8-pentamethyl- 2-naphtalenyl)- 1-ethylenyl ] phenyl] 1H-tetrazolsäure.
- Das (Z) 5[4-[1-(3',4'-Dihydro-4',4'-dimethyl-1',1'-dioxo-2'H- 1'-benzothiopyran-6'-yl)- 2-propenyl] phenyl] 1H-tetrazol.
- Die (Z) 2-[1- (5, 6,7,8-Tetrahydro- 5,5,8,8-tetramethyl- 2-naphtalenyl)- 2-propenyl ] thienyl -5-carbonsäure.
- Das (Z) 5-[2-[1- (5,6,7,8-Tetrahydro- 5,5,8,8-tetramethyl- 2-naphtalenyl)- 2-propenyl ] 5-thienyl]- 1H-tetrazol.
- Die (Z) 5-[1- (5,6,7,8-Tetrahydro-5,5,8,8-tetramethyl- 2-naphtalenyl)- 2-propenyl ] pyridinyl -2-carbonsäure.
- Die (Z) 2-[1- (5,6,7,8-Tetrahydro- 3,5,5,8,8-pentamethyl- 2-naphtalenyl)- 2-propenyl ] pyridinyl -5-carbonsäure.
- Die (Z) 5-[1- (5, 6,7,8-Tetrahydro- 3,5,5,8,8-pentamethyl- 2-naphtalenyl)- 2-propenyl ] pyridinyl -2-carbonsäure.
- Die (Z) 5-[1-(5, 6,7,8-Tetrahydro-5,5,8,8-tetramethyl- 2-naphtalenyl)- 2-propenyl ] isoxazol -3-carbonsäure.
- Die (Z) 5-[1- (5,6,7,8-Tetrahydro- 3,5,5,8,8-pentamethyl- 2-naphtalenyl)- 2-propenyl ] isoxazol -3-carbonsäure.
- Das (Z) 1-[4-[1-(5,6,7,8-Tetrahydro- 5,5,8,8-tetramethyl- 2-naphtalenyl)- 2-propenyl ] phenyl] -5-trifluoromethyl -1H-tetrazol.

6. Therapeutisches, dermatologisches oder kosmetisches Präparat, das mindestens ein Derivat der Formel (I) wie in irgendeinem der Ansprüche 1 bis 5 definiert enthält, in freier Form oder in der Form eines pharmazeutisch akzeptablen Salzes, in Verbindung mit einem traditionellen Vehikel oder Verdünnungsmittel.

7. Verwendung eines Derivats der Formel (I) wie in irgendeinem der Ansprüche 1 bis 5 definiert zur Herstellung eines dermatologischen oder kosmetischen Präparats, das der Behandlung oder Vorbeugung von Hautkrankheiten dient.

8. Verwendung eines Derivats der folgenden Formel (I) : 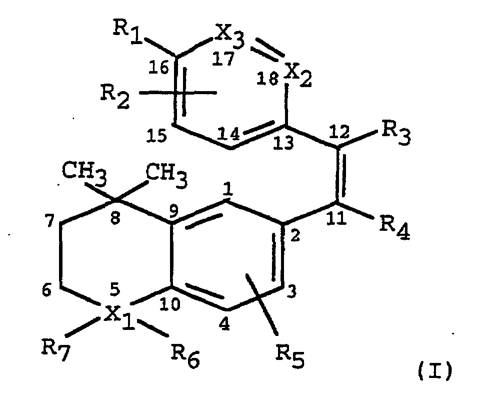
in der R1 steht für ein Wasserstoffatom, einen C₁ bis C₆-Alkylrest oder eine Gruppe der Formel -CH₂OH, -OH, -CHO, COOH, COR₈, CH₂OCOR₉, SH, S-Alkyl, p-Hydroxyphenylaminocarbonyl, Tetrazol-5-yl-aminocarbonyl, Tetrazol-5-yl, und, soweit möglich, ihrer Salze mit physiologisch akzeptablen Säuren, und R₈ und R₉ sind:
. ein Wasserstoffatom, eine OH-Gruppe, ein C₁ bis C₆-Alkylrest oder eine Gruppe der Formel -OR₁₀, wobei R₁₀ einen verzweigten oder unverzweigten Alkylrest mit 2 bis 20 Kohlenstoffatomen, einen verzweigten oder unverzweigten Alkenrest mit 2 bis 20 Kohlenstoffatomen, einen Arylrest oder einen Aralkylrest darstellt, oder
. eine Aminogruppe der Formel 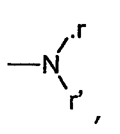
in der r und r', identisch oder verschieden, ein Wasserstoffatom, einen C₁ bis C₆-Alkylrest, einen Aryl- oder Aralkylrest, einen α-Aminosäurerest, einen Zuckerrest oder einen Heterozyklus, bei dem r und r' gemeinsam den Heterozyklus bilden darstellen.
und R₂, R₃, R₄, R₅, R₆, R₇, X₁, X₂ und X₃ die gleichen Bedeutungen haben wie in den Ansprüchen 1 bis 3, für die Herstellung eines therapeutischen Präparats, das der Behandlung oder Vorbeugung von Krebs dient.

9. Verwendung eines Derivats der Formel (I) wie in irgendeinem der Ansprüche 1 bis 5 definiert, für die Herstellung eines pharmazeutischen Präparats, das der Behandlung oder Vorbeugung von Krebs dient.

10. Verwendung eines Derivats der folgenden Formel (I) : 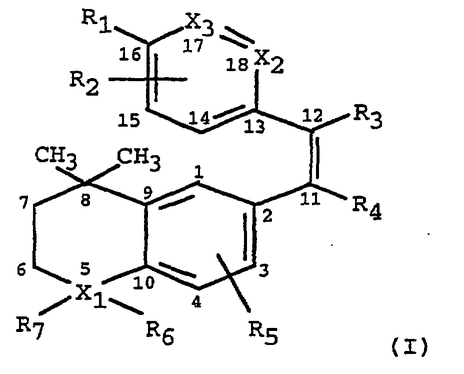
in der R1 steht für ein Wasserstoffatom, einen C₁ bis C₆-Alkylrest oder eine Gruppe der Formel -CH₂OH, -OH, -CHO, COOH, COR₈, CH₂OCOR₉, SH, S-Alkyl, p-Hydroxyphenylaminocarbonyl, Tetrazol-5-ylaminocarbonyl, Tetrazol-5-yl, und, soweit möglich ihrer Salze mit physiologisch akzeptablen Säuren, und R₈ und R₉ sind:
. ein Wasserstoffatom, eine OH-Gruppe, ein C₁ bis C₆-Alkylrest oder eine Gruppe der Formel -OR₁₀, wobei R₁₀ einen verzweigten oder unverzweigten Alkylrest mit 2 bis 20 Kohlenstoffatomen, einen verzweigten oder unverzweigten Alkenrest mit 2 bis 20 Kohlenstoffatomen, einen Arylrest oder einen Aralkylrest darstellt, oder
. eine Aminogruppe der Formel 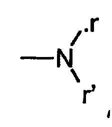
in der r und r', identisch oder verschieden, ein Wasserstoffatom, einen C₁ bis C₆-Alkylrest, einen Aryl- oder Aralkylrest, einen α-Aminosäurerest, einen Zuckerrest oder einen Heterozyklus, bei dem r und r' gemeinsam den Heterozyklus bilden darstellen.
und R₂, R₃, R₄, R₅, R₆, R₇, X₁, X₂ und X₃ die gleichen Bedeutungen haben wie in den Ansprüchen 1 bis 3, für die Herstellung eines therapeutischen Präparats, das der Behandlung oder Vorbeugung von nicht insulinabhängiger Diabetes dient.

11. Verwendung eines Derivats der Formel (I) wie in irgendeinem der Ansprüche 1 bis 5 definiert, für die Herstellung eines pharmazeutischen Präparats, das der Behandlung oder Vorbeugung von nicht insulinabhängiger Diabetes dient.

12. Verwendung eines Derivats der folgenden Formel (I): 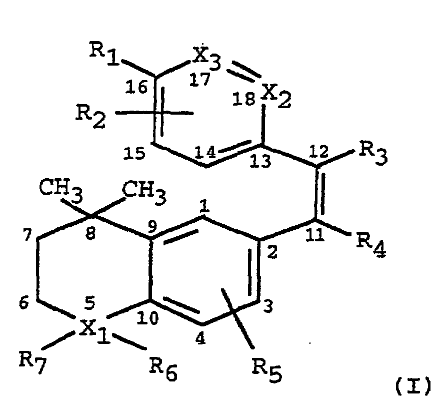
in der R1 steht für ein Wasserstoffatom, einen C₁ bis C₆-Alkylrest oder eine Gruppe der Formel -CH₂OH, -OH, -CHO, COOH, COR₈, CH₂OCOR₉, SH, S-Alkyl, p-Hydroxyphenylaminocarbonyl, Tetrazol-5-ylaminocarbonyl, Tetrazol-5-yl, und, soweit möglich ihrer Salze mit physiologisch akzeptablen Säuren, und R₈ und R₉ sind:
. ein Wasserstoffatom, eine OH-Gruppe, ein C₁ bis C₆-Alkylrest oder eine Gruppe der Formel -OR₁₀, wobei R₁₀ einen verzweigten oder unverzweigten Alkylrest mit 2 bis 20 Kohlenstoffatomen, einen verzweigten oder unverzweigten Alkenrest mit 2 bis 20 Kohlenstoffatomen, einen Arylrest oder einen Aralkylrest darstellt, oder
. eine Aminogruppe der Formel 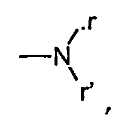
in der r und r', identisch oder verschieden, ein Wasserstoffatom, einen C₁ bis C₆-Alkylrest, einen Aryl- oder Aralkylrest, einen α-Aminosäurerest, einen Zuckerrest oder einen Heterozyklus, bei dem r und r' gemeinsam den Heterozyklus bilden darstellen.
und R₂, R₃, R₄, R₅, R₆, R₇, X₁, X₂ und X₃ die gleichen Bedeutungen haben wie in den Ansprüchen 1 bis 3, für die Herstellung eines therapeutischen Präparats, das der Behandlung oder Vorbeugung von Entzündungskrankheiten dient.

13. Verwendung eines Derivats der Formel (I) wie in irgendeinem der Ansprüche 1 bis 5 definiert, für die Herstellung eines pharmazeutischen Präparats, das der Behandlung oder Vorbeugung von Entzündungskrankheiten dient.

14. Verwendung eines Derivats der folgenden Formel (I): 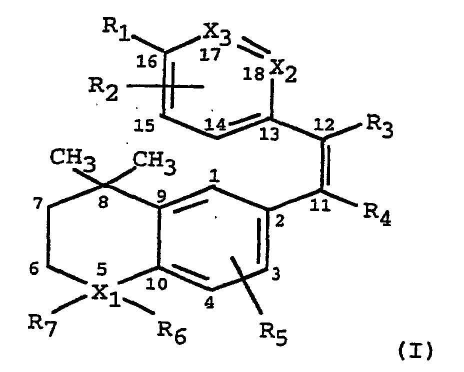
in der R1 steht für ein Wasserstoffatom, einen C₁ bis C₆-Alkylrest oder eine Gruppe der Formel -CH₂OH, -OH, -CHO, COOH, COR₈, CH₂OCOR₉, SH, S-Alkyl, p-Hydroxyphenylaminocarbonyl, Tetrazol-5-ylaminocarbonyl, Tetrazol-5-yl, und, soweit möglich ihrer Salze mit physiologisch akzeptablen Säuren, und R₈ und R₉ sind:
. ein Wasserstoffatom, eine OH-Gruppe, ein C₁ bis C₆-Alkylrest oder eine Gruppe der Formel -OR₁₀, wobei R₁₀ einen verzweigten oder unverzweigten Alkylrest mit 2 bis 20 Kohlenstoffatomen, einen verzweigten oder unverzweigten Alkenrest mit 2 bis 20 Kohlenstoffatomen, einen Arylrest oder einen Aralkylrest darstellt, oder
. eine Aminogruppe der Formel 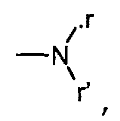
in der r und r', identisch oder verschieden, ein Wasserstoffatom, einen C₁ bis C₆-Alkylrest, einen Aryl- oder Aralkylrest, einen α-Aminosäurerest, einen Zuckerrest oder einen Heterozyklus, bei dem r und r' gemeinsam den Heterozyklus bilden darstellen.
und R₂, R₃, R₄, R₅, R₆, R₇, X₁, X₂ und X₃ die gleichen Bedeutungen haben wie in den Ansprüchen 1 bis 3, für die Herstellung eines therapeutischen Präparats, das der Behandlung oder Vorbeugung von Immunkrankheiten dient.

15. Verwendung eines Derivats der Formel (I) wie in irgendeinem der Ansprüche 1 bis 5 definiert, für die Herstellung eines pharmazeutischen Präparats, das der Behandlung oder Vorbeugung von Immunkrankheiten dient.

## Claims

1. Aromatic polycyclic derivatives of the retinoic type of general formula (I): 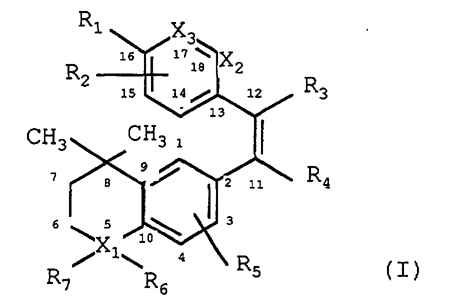
in which the groupings R₃ and R₄ carried by the double bond between carbons 11 and 12 are in cis position and R₁, R₂, R₃, R₄, R₅, R₆, R₇, X₁, X₂ and X₃, have the following meanings:
- R₁ represents a tetrazoyl group or a -COOH group.
- R₂ represents a hydrogen atom, a halogen atom and more particularly a fluorine atom, an alkyl radical in C₁ to C₆, and group of formula -COOH, -OR₁₁, -SR₁₁, -(CF₂)nCF₃ where n is a whole number comprised between 0 and 10, or an -OCOR₁₁ group, and when possible their salts with physiologically tolerated acids, or an amine group of formula: 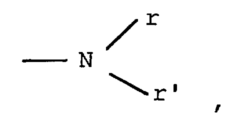
in which r and r', identical or different, represent a hydrogen atom, an alkyl radical in C₁ to C₆, an aryl or aralkyl radical, a remainder of α-aminoacid, a remainder of sugar or a heterocycle in which r and r' taken together form a heterocycle and R₁₁ represents a hydrogen atom, an alkyl radical in C₁ to C₆, a fluoroalkyl radical with from 1 to 6 carbon atoms and from 3 to 7 fluorine atoms, an aryl radical or an aralkyl radical.
- R₃ represents a hydrogen atom, a trifluoromethyl radical, an aryl radical, an aralkyl radical or an alkyl radical in C₁ to C₆, possibly substituted by a hydroxyl or by one or several atoms, of fluorine, by an alkoxy in C₁ to C₆ or by a group of formula -(C=O)R₁₂, in which R₁₂ represents an atom of hydrogen, an alkyl radical in C₁ to C₆, a hydroxyl radical, an alkoxy radical in C₁ to C₆ or an amined group of formula: 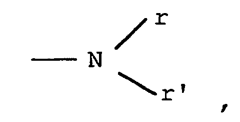
in which r and r' have the same meaning as above.
- R₄ represents a hydrogen atom or an aryl radical.
- R₅ represents a hydrogen atom, an alkyl radical in C₁ to C₆, a halogen atom, a fluoralkyl radical with from 1 to 6 carbon atoms and from 3 to 7 fluorine atoms, or a group of formula -OR₁₃ where R₁₃ represents a hydrogen atom, an alkyl radical in C₁ to C₆, an aryl radical or an aralkyl radical or a trifluoromethyl grouping.
- X₁ represents a carbon atom or a sulphur atom, and then,
- R₆ and R₇ are:
. methyl radicals, in the case where X₁ is an atom of carbon,
. identical or different and representing either nothing (the case of a sulphide, -S-), or an oxygen (the case of a sulphoxide -SO- or of a sulphone -SO₂-), in the case where X₁ is an atom of sulphur.
- X₂ and X₃, identical or different, represent a carbon atom, an oxygen atom or a nitrogen atom, or further X₂-X₃ are a single atom of sulphur, of oxygen, or of nitrogen, in which case the carrier nucleus of X₂ and X₃ can be benzenic, pyridinic, thiophenic, furanic, pyrrolic or, in the case where X₂ is an atom of oxygen and X₃ an atom of carbon, C₁₃ and C₁₄ represent one and the same atom of carbon, then the carrier nucleus of X₂ and X₃ can be isoxazolic.

2. Derivatives according to claim 1, **characterised in that** in the formula (I), R₄ represents a hydrogen atom.

3. Derivatives according to either one of claims 1 or 2, **characterised in that** in the formula (I), R₃ represents an alkyl in C₁ to C₆ or a trifluoromethyl radical or a group -(CH₂)ₙCF₃ where n is a whole number comprised between 0 and 10.

4. Derivatives according to any one of claims 1 to 3, **characterised in that** in the formula (I), R₂ represents a hydrogen atom.

5. Derivatives according to any one of claims 1 to 4:
- (Z) 2-[1-(5,6,7,8-tetrahydro-5,5,8,8-tetramethyl-2-naphthalenyl)-2-propenyl]pyridinyl-5-carboxylic acid.
- (E) 4-[1-trifluoromethyl-2-(5,6,7,8-tetrahydro-5,5,8,8-tetramethyl-2-naphthalenyl)-1-ethenyl] benzoic acid.
- (Z) 4-[1-(5,6,7,8-tetrahydro-3,5,5,8,8-pentamethyl-2-naphthalenyl)-2-propenyl] benzoic acid.
- (E) 4-[1-trifluoromethyl-2-(5,6,7,8-tetrahydro-3,5,5,8,8-pentamethyl-2-naphthalenyl) -1-ethenyl] benzoic acid.
- (Z) 4-[1-(3',4'-dihydro-4',4'-dimethyl-1',1'-dioxide-2'H-1'-benzothiopyran-6'-yl) -2-propenyl] benzoic acid.
- (Z)-5-[4-[1-(5,6,7,8-tetrahydro-5,5,8,8-tetra-methyl-2-naphthalenyl)-2-propenyl]phenyl]-1H-tetrazole.
- (E) 5-[4-[1-trifluoromethyl-2-(5,6,7,8-tetrahydro-5,5,8,8-tetramethyl-2-naphthalenyl)-1-ethenyl]phenyl]-1H-tetrazole acid.
- (Z) 5-[4-[1-(5,6,7,8-tetrahydro-3,5,5,8,8-pentamethyl-2-naphthalenyl)-2-propenyl]phenyl]-1H-tetrazole.
- (E) 5-[4-[1-trifluoromethyl-2-(5,6,7,8-tetrahydro-3,5,5,8,8-pentamethyl-2-naphthalenyl)-1-ethenyl]phenyl]-1H-tetrazole acid.
- (Z) 5 [4-[1-(3',4'-dihydro-4',4'-dimethyl-1',1'-dioxide-2'H-1'-benzothiopyran-6'-yl)-2-propenyl]phenyl],1H-tetrazole.
- (Z) 2-[1-(5,6,7,8-tetrahydro-5,5,8,8-tetramethyl-2-naphthalenyl)-2-propenyl]thienyl-5-carboxylic acid.
- (Z) 5-[2-[1-(5,6,7,8-tetrahydro-5,5,8,8-tetramethyl-2-naphthalenyl)-2-propenyl)]5-thienyl]-1H tetrazole.
- (Z) 5-[1-(5,6,7,8-tetrahydro-5,5,8,8-tetramethyl-2-naphthalenyl)-2-propenyl]pyridinyl-2-carboxylic acid.
- (Z) 2-[1-(5,6,7,8-tetrahydro-3,5,5,8,8-pentamethyl-2-naphthalenyl)-2-propenyl]pyridinyl-5-carboxylic acid.
- (Z) 5-[1-(5,6,7,8-tetrahydro-3,5,5,8,8-pentamethyl-2-naphthalenyl)-2-propenyl]pyridinyl-2-carboxylic acid.
- (Z) 5-[1-(5,6,7,8-tetrahydro-5,5,8,8-tetramethyl-2-naphthalenyl)-2-propenyl)]isoxazol-3-carboxylic acid.
- (Z) 5-[1-(5,6,7,8-tetrahydro-3,5,5,8,8-pentamethyl-2-naphthalenyl)-2-propenyl)]isoxazol-3-carboxylic acid.
- (Z) 1-[4-[1-(5,6,7,8-tetrahydro-5,5,8,8-tetramethyl-2-naphthalenyl)-2-propenyl]phenyl]-5-trifluoromethyl-lH-tetrazole.

6. Therapeutic, dermatological or cosmetic composition including at least one derivative of formula (I) defined in any one of claims 1 to 5, under free form or under the form of a pharmaceutically acceptable salt in association with a traditional carrier or diluent.

7. Utilisation of a derivative of formula (I) defined in any one of claims 1 to 5, for manufacturing a dermatological or cosmetic composition useful for treating or preventing skin diseases.

8. Utilisation of a derivative of formula (I) below: 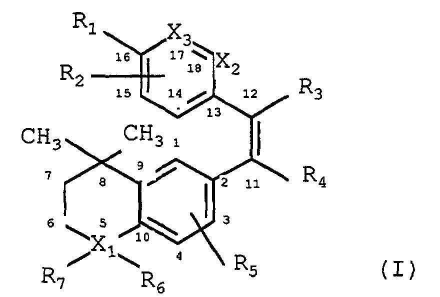
in which R₁ represents a hydrogen atom, an alkyl radical in C₁ to C₆, or a group of formula -CH₂OH, -OH,-CHO, -COOH, -COR₈, -CH₂OCOR₉, -SH, -S-alkyl, p-hydroxyphenylaminocarbonyl, tetrazole-5-ylaminocarbonyl, tetrazole-5-yl, and when possible their salts with physiologically tolerated acids, where R₈ and R₉ are:
. a hydrogen atom, an -OH group, an alkyl radical in C₁ to C₆ or a group of formula -OR₁₀, where R₁₀ represents an alkyl radical, branched or not, having from 1 to 20 atoms of carbon, an alkenyl radical, branched or not, having from 2 to 20 atoms of carbon, an aryl or aralkyl radical or
. an amined group of formula: 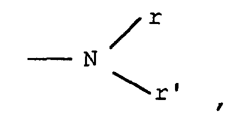
in which r and r', identical or different, represent a hydrogen atom, an alkyl radical in C₁ to C₆, an aryl or aralkyl radical, a remainder of α-aminoacid, a remainder of sugar or a heterocycle in which r and r' taken together form a heterocycle.
And R₂, R₃, R₄, R₅, R₆, R₇, X₁, X₂ and X₃ have the same meanings as in claims 1 to 3, for manufacturing a therapeutic composition useful in the treatment or prevention of cancers.

9. Utilisation of a derivative of formula (I) defined in any one of claims 1 to 5, for the manufacture of a therapeutic composition useful in the treatment or prevention of cancers.

10. Utilisation of a derivative of formula (I) below: 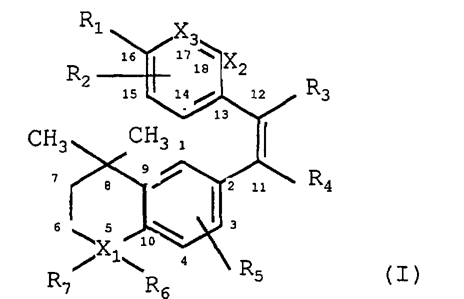
in which R₁ represents an atom of hydrogen, an alkyl radical in C₁ to C₆, or a group of formula -CH₂OH, -OH,-CHO, -COOH, -COR₈, -CH₂OCOR₉, -SH, -S-alkyl, p-hydroxy-phenylaminocarbonyl, tetrazole-5-ylaminocarbonyl, tetrazole-5-yl, and when possible their salts with physiologically tolerated acids, where R₈ and R₉ are:
. a hydrogen atom, an -OH group, an alkyl radical in C₁ to C₆ or a group of formula -OR₁₀, where R₁₀ represents an alkyl radical, branched or not, having from 1 to 20 atoms of carbon, an alkenyl radical, branched or not, having from 2 to 20 atoms of carbon, an aryl or aralkyl radical, or
. an amined group of formula: 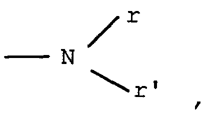
in which r and r', identical or different, represent a hydrogen atom, an alkyl radical in C₁ to C₆, an aryl or aralkyl radical, a remainder of α-aminoacid, a remainder of sugar or a heterocycle in which r and r' taken together form a heterocycle
and R₂, R₃, R₄, R₅, R₆, R₇, X₁, X₂ and X₃ have the same meanings as in claims 1 to 3, for manufacturing a therapeutic composition useful in the treatment or prevention of non insulin-dependent diabetes.

11. Utilisation of a derivative of formula (I) as defined in any one of claims 1 to 5, for the manufacture of a therapeutic composition useful in the treatment or prevention of non insulin-dependent diabetes.

12. Utilisation of a derivative of formula (I) below: 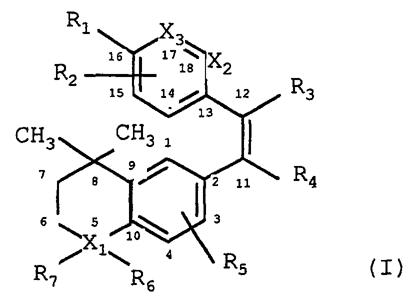
in which R₁ represents a hydrogen atom, an alkyl radical in C₁ to C₆, or a group of formula -CH₂OH, -OH,-CHO, -COOH, -COR₈, -CH₂OCOR₉, -SH, -S-alkyl, p-hydroxyphenylaminocarbonyl, tetrazole-5-ylaminocarbonyl, tetrazole-5-yl, and when possible their salts with physiologically tolerated acids, where R₈ and R₉ are:
. a hydrogen atom, an -OH group, an alkyl radical in C₁ to C₆ or a group of formula -OR₁₀, where R₁₀ represents an alkyl radical, branched or not, having from 1 to 20 atoms of carbon, an alkenyl radical, branched or not, having from 2 to 20 atoms of carbon, an aryl or aralkyl radical, or
. an amined group of formula: 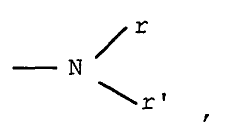
in which r and r', identical or different, represent a hydrogen atom, an alkyl radical in C₁ to C₆, an aryl or aralkyl radical, a remainder of α-aminoacid, a remainder of sugar or a heterocycle in which r and r' taken together form a heterocycle
and R₂, R₃, R₄, R₅, R₆, R₇, X₁, X₂ and X₃ have the same meanings as in claims 1 to 3, for manufacturing a therapeutic composition useful in the treatment or prevention of inflammatory illnesses.

13. Utilisation of a derivative of formula (I) as defined in any one of claims 1 to 5, for manufacturing a therapeutic composition useful in the treatment or the prevention of inflammatory illnesses.

14. Utilisation of a derivative of formula (I) below: 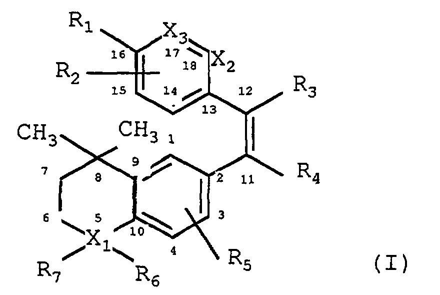
in which R₁ represents a hydrogen atom, an alkyl radical in C₁ to C₆, or a group of formula -CH₂OH, -OH,-CHO, -COOH, -COR₈, -CH₂OCOR₉, -SH, -S-alkyl, p-hydroxyphenylaminocarbonyl, tetrazole-5-ylaminocarbonyl, tetrazole-5-yl, and when possible their salts with physiologically tolerated acids, where R₈ and R₉ are:
. a hydrogen atom, an -OH group, an alkyl radical in C₁ to C₆ or a group of formula -OR₁₀, where R₁₀ represents an alkyl radical, branched or not, having from 1 to 20 atoms of carbon, an alkenyl radical, branched or not, having from 2 to 20 atoms of carbon, an aryl or aralkyl radical, or
. an amined group of formula: 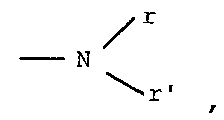
in which r and r', identical or different, represent a hydrogen atom, an alkyl radical in C₁ to C₆, an aryl or aralkyl radical, a remainder of α-aminoacid, a remainder of sugar or a heterocycle in which r and r' taken together form a heterocycle
and R₂, R₃, R₄, R₅, R₆, R₇, X₁, X₂ and X₃ have the same meanings as in claims 1 to 3, for manufacturing a therapeutic composition useful in the treatment or prevention of immunitary illnesses.

15. Utilisation of a derivative of formula (I) as defined in any one of claims 1 to 5, for manufacturing a therapeutic composition useful in the treatment or the prevention of immunitary illnesses.
